(19) Europäisches Patentamt
European Patent Office
Office européen des brevets

(11) EP 4 234 553 A2

(12) EUROPEAN PATENT APPLICATION

(43) Date of publication:
**30.08.2023 Bulletin 2023/35**

(21) Application number: **23160423.2**

(22) Date of filing: **09.05.2019**

(51) International Patent Classification (IPC):
***C07D 403/14*** $^{(2006.01)}$ ***A61P 21/00*** $^{(2006.01)}$
***A61K 31/4178*** $^{(2006.01)}$

(52) Cooperative Patent Classification (CPC):
**C07D 403/14; A61P 21/00; C12Q 1/6883;**
C12Q 2600/156

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.05.2018 US 201862669155 P**

(62) Document number(s) of the earlier application(s) in accordance with Art. 76 EPC:
**19729405.1 / 3 790 872**

(71) Applicant: **Design Therapeutics, Inc.**
**Solana Beach, California 92075 (US)**

(72) Inventors:
- **ANSARI, Aseem**
  **Solana Beach, California, 92075 (US)**
- **SHAH, Pratik**
  **Solana Beach, California, 92075 (US)**

(74) Representative: **Carpmaels & Ransford LLP**
**One Southampton Row**
**London WC1B 5HA (GB)**

Remarks:
This application was filed on 07-03-2023 as a divisional application to the application mentioned under INID code 62.

# (54) METHODS AND COMPOUNDS FOR THE TREATMENT OF GENETIC DISEASE

(57) The present disclosure relates to compounds and methods for modulating the expression of *c9orf72* (brain expressed, associated with NEDD4) and treating diseases and conditions in which *c9orf72* plays an active role. The compound can be a transcription modulator molecule having a first terminus, a second terminus, and oligomeric backbone, wherein: a) the first terminus comprises a DNA-binding moiety capable of noncovalently binding to a nucleotide repeat sequence GGGGCC; b) the second terminus comprises a protein-binding moiety binding to a regulatory molecule that modulates an expression of a gene comprising the nucleotide repeat sequence GGGGCC; and c) the oligomeric backbone comprising a linker between the first terminus and the second terminus.

EP 4 234 553 A2

## Description

### CROSS REFERENCE

This application claims the benefit of U.S. Application No. 62/669,155 filed May 9, 2018, which is hereby incorporated by reference in its entirety.

### FIELD OF INVENTION

Disclosed herein are new chimeric heterocyclic polyamide compounds and compositions and their application as pharmaceuticals for the treatment of disease. Methods to modulate the expression of *c9orf72* (chromosome 9 open reading frame 72) in a human or animal subject are also provided for the treatment diseases such as ALS.

### BACKGROUND

Amyotrophic lateral sclerosis ("ALS") is a degenerative disease characterised by muscle atrophy, loss of muscle mass, and decreased ability to control motion (ataxia). ALS is caused by the degeneration of motor neurons, leading both to involuntary twitching (fasciculations) and paralysis. Most patients with ALS die within 5 years of diagnosis, generally from respiratory failure.

Although the majority of ALS cases are sporadic, with no identifiable risk factor or genetic basis, a fraction of ALS cases are inherited. The presence of a GGGGCC repeat sequence in the non-coding region of the *c9orf72* gene is associated with 25% to 40% of inherited ALS cases, as well as a smaller fraction of sporadic cases. This genetic defect is also observed in a disease termed frontal-temporal lobe dementia ("FTD", also referred to as frontotemporal lobar degeneration with TDP-43 pathology, or "FTLD-TDP"), which is characterized by atrophy of the frontal-temporal lobe. Certain individuals present with symptoms of both diseases, a condition which is termed ALS-FTD. Healthy individuals typically display 2-23 of these hexanucleotide repeats; in FTD/ALS patients, this number is estimated at 700-1600 repeat units. RNA foci comprising the hexanucleotide repeat have been identified in individuals afflicted with FTD, suggesting that the presence of the defective RNA may be responsible for the observed phenotype.

The FDA has approved two drugs: riluzole (RILUTEK®) and edaravone (RADICAVA®) for the treatment of ALS; however, neither agent is considered curative. Much of the treatment of ALS is for alleviation of symptoms and for palliative care.

### SUMMARY

This disclosure utilizes regulatory molecules present in cell nuclei that control gene expression. Eukaryotic cells provide several mechanisms for controlling gene replication, transcription, and/or translation. Regulatory molecules that are produced by various biochemical mechanisms within the cell can modulate the various processes involved in the conversion of genetic information to cellular components. Several regulatory molecules are known to modulate the production of mRNA and, if directed to *c9orf72*, would modulate the productionof *c9orf72* mRNA that causes ALS, and thus reverse the progress of the disease.

The disclosure provides compounds and methods for recruiting a regulatory molecule into close proximity to *c9orf72.* The compounds disclosed herein contain: (a) a recruiting moiety that will bind to a regulatory molecule, linked to (b) a DNA binding moiety that will selectively bind to *c9orf72.* The compounds will counteract the expression of defective *c9orf72* in the following manner:

The DNA binding moiety will bind selectively the characteristic GGGGCC hexanucleotide repeat sequence of *c9orf72;*

The recruiting moiety, linked to the DNA binding moiety, will thus be held in proximity to *c9orf72*;

The recruiting moiety, now in proximity to *c9orf72,* will recruit the regulatory molecule into proximity with the gene; and

The regulatory molecule will modulate expression, and therefore counteract the production of defective *c9orf72* by direct interaction with the gene.

The mechanism set forth above will provide an effective treatment for ALS, which is caused by the expression of defective *c9orf72.* Correction of the expression of the defective *c9orf72* gene thus represents a promising method for the treatment of ALS.

The disclosure provides recruiting moieties that will bind to regulatory molecules. Small molecule inhibitors of regulatory molecules serve as templates for the design of recruiting moieties, since these inhibitors generally act via noncovalent binding to the regulatory molecules.

The disclosure further provides for DNA binding moieties that will selectively bind to one or more copies of the

GGGGCC hexanucleotide repeat that is characteristic of the defective *c9orf72* gene. Selective binding of the DNA binding moiety to *c9orf72*, made possible due to the high GGGGCC count associated with the defective *c9orf72* gene, will direct the recruiting moiety into proximity of the gene, and recruit the regulatory molecule into position to modulate gene transcription.

The DNA binding moiety will comprise a polyamide segment that will bind selectively to the target GGGGCC sequence. Polyamides have been designed by Dervan and others that can selectively bind to selected DNA sequences. These polyamides sit in the minor groove of double helical DNA and form hydrogen bonding interactions with the Watson-Crick base pairs. Polyamides that selectively bind to particular DNA sequences can be designed by linking monoamide building blocks according to established chemical rules. One building block is provided for each DNA base pair, with each building block binding noncovalently and selectively to one of the DNA base pairs: A/T, T/A, G/C, and C/G. Following this guideline, hexanucleotides will bind to molecules with six amide units, i.e. hexaamides. In general, these polyamides will orient in either direction of a DNA sequence, so that the 5'-GGGGCC-3' hexanucleotide repeat sequence of *c9orf72* can be targeted by polyamides selective either for GGGGCC or for CCGGGG. Furthermore, polyamides that bind to the complementary sequence, in this case, CCCCGG or GGCCCC, will also bind to the hexanucleotide repeat sequence of *c9orf72* and can be employed as well.

In principle, longer DNA sequences can be targeted with higher specificity and/or higher affinity by combining a larger number of monoamide building blocks into longer polyamide chains. Ideally, the binding affinity for a polyamide would simply be equal to the sum of each individual monoamide / DNA base pair interaction. In practice, however, due to the geometric mismatch between the fairly rigid polyamide and DNA structures, longer polyamide sequences do not bind to longer DNA sequences as tightly as would be expected from a simple additive contribution. The geometric mismatch between longer polyamide sequences and longer DNA sequences induces an unfavorable geometric strain that subtracts from the binding affinity that would be otherwise expected.

The disclosure therefore provides DNA moieties that comprise hexaamide or pentaamide subunits that are connected by flexible spacers. The spacers alleviate the geometric strain that would otherwise decrease binding affinity of a larger polyamide sequence.

Disclosed herein are polyamide compounds that can bind to one or more copies of the hexanucleotide repeat sequence GGGGCC, and can modulate the expression of the defective *c9orf72* gene. Treatment of a subject with these compounds will counteract the expression of the defective *c9orf72* gene, and this can reduce the occurrence, severity, and/or frequency of symptoms associated with ALS. Certain compounds disclosed herein will provide higher binding affinity and/or selectivity than has been observed previously for this class of compounds.

**INCORPORATION BY REFERENCE**

All publications, patents, and patent applications mentioned in this specification are herein incorporated by reference to the same extent as if each individual publication, patent, or patent application was specifically and individually indicated to be incorporated by reference.

**DETAILED DESCRIPTION**

The transcription modulator molecule described herein represents an interface of chemistry, biology and precision medicine in that the molecule can be programmed to regulate the expression of a target gene containing nucleotide repeat GGGGCC. The transcription modulator molecule contains DNA binding moieties that will selectively bind to one or more copies of the GGGGCC hexanucleotide repeat that is characteristic of the defective *c9orf72* gene. The transcription modulator molecule also contains moieties that bind to regulatory proteins. The selective binding of the target gene will bring the regulatory protein into proximity to the target gene and thus downregulates transcription of the target gene. The molecules and compounds disclosed herein provide higher binding affinity and selectivity than has been observed previously for this class of compounds and can be more effective in treating diseases associated with the defective c9orf72 gene.

Treatment of a subject with these compounds will modulate the expression of the defective *c9orf72* gene, and this can reduce the occurrence, severity, or frequency of symptoms associated with ALS. The transcription modulator molecules described herein recruits the regulatory molecule to modulate the expression of the defective *c9orf72* gene and effectively treats and alleviates the symptoms associated with diseases such as ALS.

<u>Transcription Modulator Molecule</u>

The transcription modulator molecules disclosed herein possess useful activity for modulating the transcription of a target gene having one or more GGGGCC repeats (e.g., *c9orf72*), and may be used in the treatment or prophylaxis of a disease or condition in which the target gene (e.g., *c9orf72*) plays an active role. Thus, in broad aspect, certain

embodiments also provide pharmaceutical compositions comprising one or more compounds disclosed herein together with a pharmaceutically acceptable carrier, as well as methods of making and using the compounds and compositions. Certain embodiments provide methods for modulating the expression of *c9orf72.* Other embodiments provide methods for treating a c9orj72-mediated disorder in a patient in need of such treatment, comprising administering to said patient a therapeutically effective amount of a compound or composition according to the present disclosure. Also provided is the use of certain compounds disclosed herein for use in the manufacture of a medicament for the treatment of a disease or condition ameliorated by the modulation of the expression of *c9orf72.*

Some embodiments relate to a transcription modulator molecule or compound having a first terminus, a second terminus, and oligomeric backbone, wherein: a) the first terminus comprises a DNA-binding moiety capable of noncovalently binding to a nucleotide repeat sequence GGGGCC; b) the second terminus comprises a protein-binding moiety binding to a regulatory molecule that modulates an expression of a gene comprising the nucleotide repeat sequence GGGGCC; and c) the oligomeric backbone comprising a linker between the first terminus and the second terminus. In some embodiments, the second terminus is not a Brd4 binding moiety.

In certain embodiments, the compounds have structural Formula I:

$$X\text{-}L\text{-}Y \qquad (I)$$

or a salt thereof, wherein:

X comprises a is a recruiting moiety that is capable of noncovalent binding to a regulatory moiety within the nucleus;
Y comprises a DNA recognition moiety that is capable of noncovalent binding to one or more copies of the hexanucleotide repeat sequence GGGGCC; and
L is a linker;

Certain compounds disclosed herein may possess useful activity for modulating the transcription of *c9orf72*, and may be used in the treatment and/or prophylaxis of a disease or condition in which *c9orf72* plays an active role. Thus, in broad aspect, certain embodiments also provide pharmaceutical compositions comprising one or more compounds disclosed herein together with a pharmaceutically acceptable carrier, as well as methods of making and using the compounds and compositions. Certain embodiments provide methods for modulating the expression of *c9orf72.* Other embodiments provide methods for treating a *c9orf72* -mediated disorder in a patient in need of such treatment, comprising administering to said patient a therapeutically effective amount of a compound or composition according to the present disclosure. Also provided is the use of certain compounds disclosed herein for use in the manufacture of a medicament for the treatment of a disease or condition ameliorated by the modulation of the expression of *c9orf72.*

In certain embodiments, the regulatory molecule is chosen from a bromodomain-containing protein, a nucleosome remodeling factor (NURF), a bromodomain PHD finger transcription factor (BPTF), a ten-eleven translocation enzyme (TET), methylcytosine dioxygenase (TET1), a DNA demethylase, a helicase, an acetyltransferase, and a histone deacetylase ("HDAC").

In some embodiments, the first terminus is Y, and the second terminus is X, and the oligomeric backbone is L.

In In certain embodiments, the compounds have structural Formula II:

$$X\text{-}L\text{-}(Y_1\text{-}Y_2\text{-}Y_3\text{-}Y_4\text{-}Y_5\text{-}Y_6)_n\text{-}Y_0 \qquad (II)$$

or a salt thereof, wherein:

X comprises a recruiting moiety that is capable of noncovalent binding to a regulatory molecule within the nucleus;
L is a linker;
$Y_1$, $Y_2$, $Y_3$, $Y_4$, $Y_5$, and $Y_6$ are internal subunits, each of which comprises a moiety chosen from a heterocyclic ring or a $C_{1-6}$straight chain aliphatic segment, and each of which is chemically linked to its two neighbors;
$Y_0$ is an end subunit which comprises a moiety chosen from a heterocyclic ring or a straight chain aliphatic segment, which is chemically linked to its single neighbor;

each subunit can noncovalently bind to an individual nucleotide in the GGGGCC repeat sequence;

n is an integer between 1 and 15, inclusive; and
$(Y_1\text{-}Y_2\text{-}Y_3\text{-}Y_4\text{-}Y_5\text{-}Y_6)_n\text{-}Y_0$ combine to form a DNA recognition moiety that is capable of noncovalent binding to one or more copies of the the hexanucleotide repeat sequence GGGGCC.

In certain embodiments, the compounds of structural Formula II comprise a subunit for each individual nucleotide

in the GGGGCC repeat sequence.

In certain embodiment, each internal subunit has an amino (-NH-) group and a carboxy (-CO-) group.

In certain embodiments, the compounds of structural Formula II comprise amide (-NHCO-) bonds between each pair of internal subunits.

In certain embodiments, the compounds of structural Formula II comprise an amide (-NHCO-) bond between L and the leftmost internal subunit.

In certain embodiments, the compounds of structural Formula II comprise an amide bond between the rightmost internal subunit and the end subunit.

In certain embodiments, each subunit comprises a moiety that is independently chosen from a heterocycle and an aliphatic chain.

In certain embodiments, the heterocycle is a monocyclic heterocycle. In certain embodiments, the heterocycle is a monocyclic 5-membered heterocycle. In certain embodiments, each heterocycle contains a heteroatom independently chosen from N, O, or S. In certain embodiments, each heterocycle is independently chosen from pyrrole, imidazole, thiazole, oxazole, thiophene, and furan.

In certain embodiments, the aliphatic chain is a $C_{1-6}$straight chain aliphatic chain. In certain embodiments, the aliphatic chain has structural formula $-(CH_2)_m-$, for m chosen from 1, 2, 3, 4, and 5. In certain embodiments, the aliphatic chain is $-CH_2CH_2-$.

In certain embodiments, each subunit comprises a moiety independently chosen from

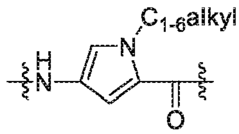 (Py), 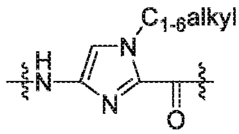 (Im), 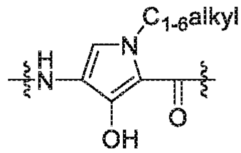 (Hp), 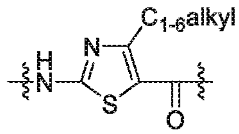 (Th), 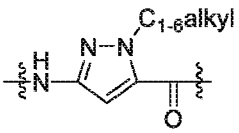 (Pz), 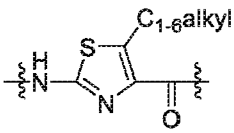 (Nt), 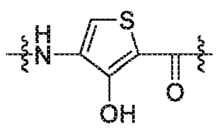 (Ht), 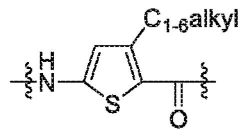 (Tn), 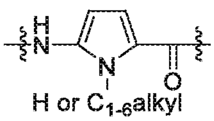 (Nh), 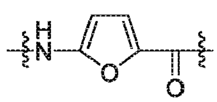 (Fr), 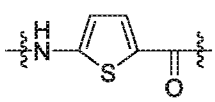 (Tp), 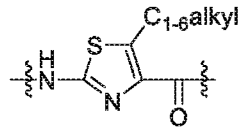 (iNt), 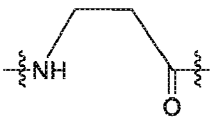 (β), 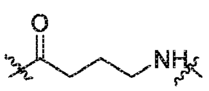 (gAB), 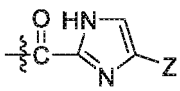 (PyT), 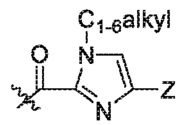 (ImT), 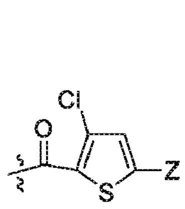 (CTh), 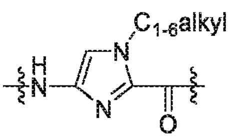 (iIm), 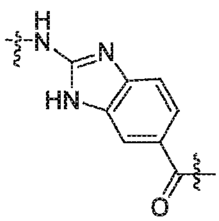 (Bi), 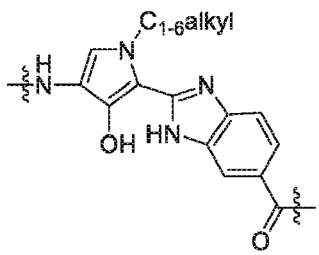

(HpBi), 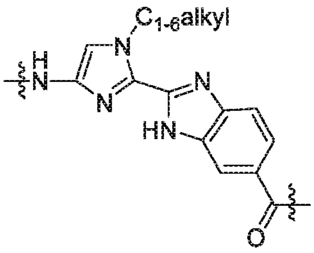 (ImBi), 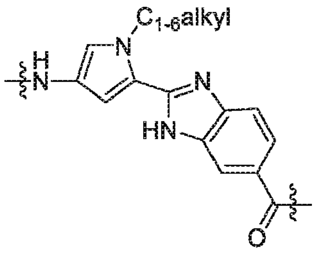 (PyBi), 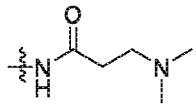 ("Dp"),

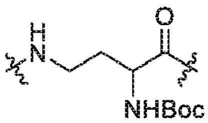 , 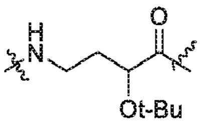 , 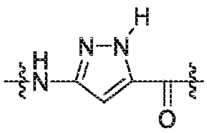 , 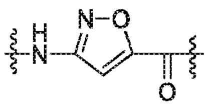 , 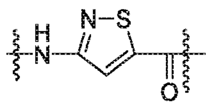 ,

NH-benzopyrazinylene-CO-, -NH-phenylene-CO-, -NH-pyridinylene-CO-, -NH-piperidinylene-CO-, -NH-pyrimidinylene-CO-, -NH-anthracenylene-CO-, -NH-quinolinylene-CO-, and

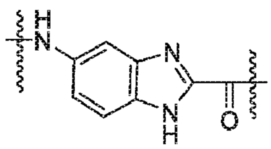

,

wherein Z is H, $NH_2$, $C_{1-6}$ alkyl, $C_{1-6}$ haloalkyl or $C_{1-6}$ alkyl-$NH_2$.

In some emebodiments, Py is

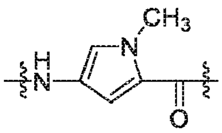

,

Im is

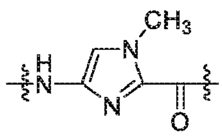

,

Hp is

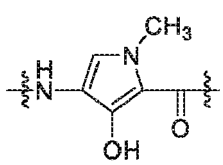

,

Th is

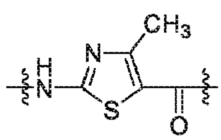

,

Pz is

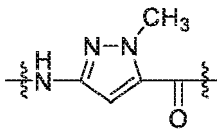

,

Nt is

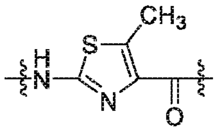

,

Tn is

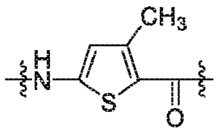

,

Nh is

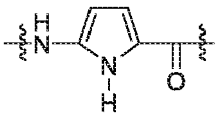

,

iNt is

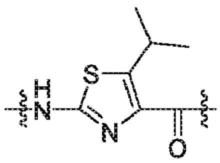

,

iIm is

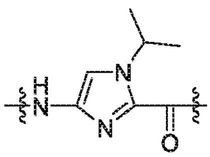

,

HpBi is

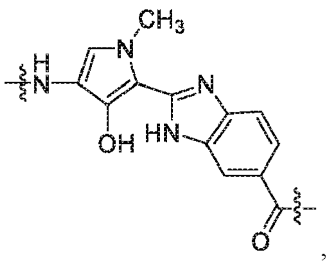

ImBi is

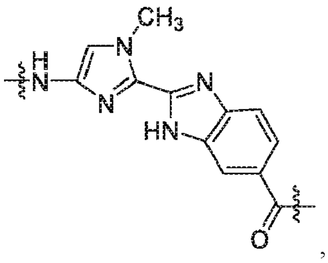

PyBi is

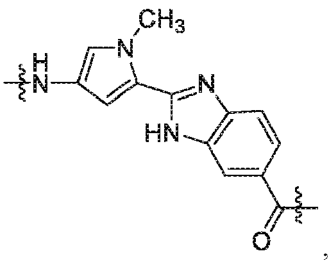

Dp is

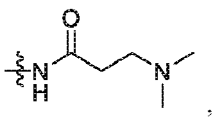

-NH-benzopyrazinylene-CO- is

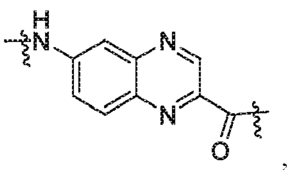

-NH-phenylene-CO- is

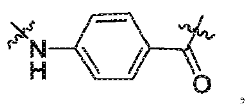

-NH-pyridinylene-CO- is

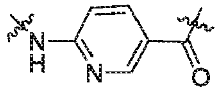,

-NH-piperidinylene-CO- is

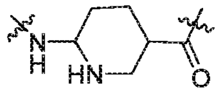,

-NH-pyrazinylene-CO- is

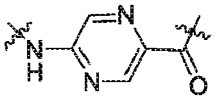,

-NH-anthracenylene-CO- is

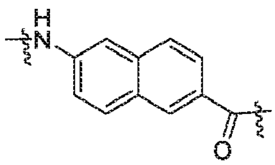,

and -NH-quinolinylene-CO- is

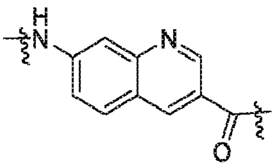.

In some embodiments, Py

is 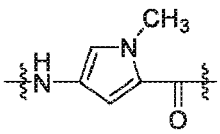,

Im is

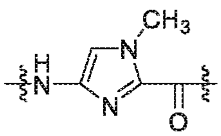,

Hp is

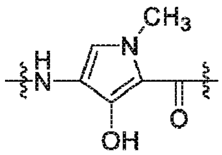,

Th is

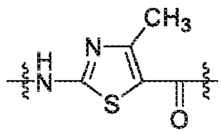,

Pz is

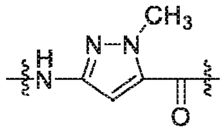,

Nt is

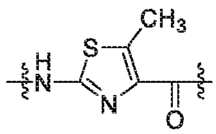,

Tn is

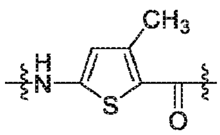,

Nh is

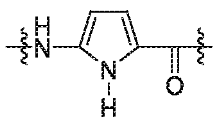

, iNt is

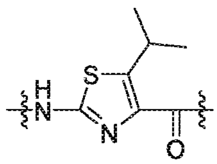,

and ihn is

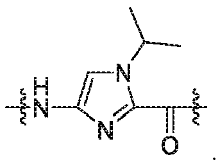

In certain embodiments, n is an integer between 1 and 5, inclusive.
In certain embodiments, n is an integer between 1 and 3, inclusive.
In certain embodiments, n is an integer between 1 and 2, inclusive.
In certain embodiments, n is 1.
In certain embodiments, L comprises a $C_{1-6}$straight chain aliphatic segment.
In certain embodiments, L comprises $(CH_2OCH_2)_m$; and m is an integer between 1 to 20, inclusive. In certain further embodiments, m is an integer between 1 to 10, inclusive. In certain further embodiments, m is an integer between 1 to 5, inclusive.
In certain embodiments, the compounds have structural Formula III:

$$X\text{-}L\text{-}(Y_1\text{-}Y_2\text{-}Y_3\text{-}Y_4\text{-}Y_5\text{-}Y_6)\text{-}(W\text{-}Y_1\text{-}Y_2\text{-}Y_3\text{-}Y_4\text{-}Y_5\text{-}Y_6)_n\text{-}Y_0 \qquad \text{(III)}$$

or a salt thereof, wherein:

X comprises a recruiting moiety that is capable of noncovalent binding to a regulatory molecule within the nucleus;
L is a linker;
$Y_1$, $Y_2$, $Y_3$, $Y_4$, $Y_5$, and $Y_6$ are internal subunits, each of which comprises a moiety chosen from a heterocyclic ring or a $C_{1-6}$straight chain aliphatic segment, and each of which is chemically linked to its two neighbors;
$Y_0$ is an end subunit which comprises a moiety chosen from a heterocyclic ring or a straight chain aliphatic segment, which is chemically linked to its single neighbor;
each subunit can noncovalently bind to an individual nucleotide in the GGGGCC repeat sequence;
W is a spacer;
n is an integer between 1 and 10, inclusive; and
$(Y_1\text{-}Y_2\text{-}Y_3\text{-}Y_4\text{-}Y_5\text{-}Y_6)\text{-}(W\text{-}Y_1\text{-}Y_2\text{-}Y_3\text{-}Y_4\text{-}Y_5\text{-}Y_6)_n\text{-}Y_0$ combine to form a DNA recognition moiety that is capable of noncovalent binding to one or more copies of the the hexanucleotide repeat sequence GGGGCC.

In certain embodiments, $Y_1\text{-}Y_2\text{-}Y_3\text{-}Y_4\text{-}Y_5\text{-}Y_6$ is:

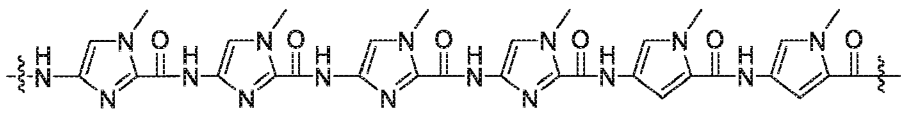

("Im-Im-Im-Im-Py-Py").
In certain embodiments, $Y_1\text{-}Y_2\text{-}Y_3\text{-}Y_4\text{-}Y_5\text{-}Y_6$ is:

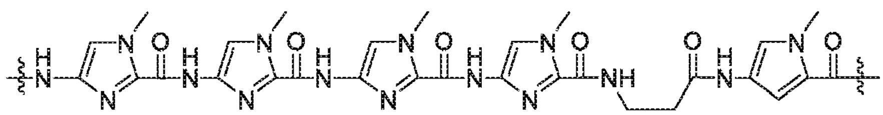

("Im-Im-Im-Im-β-Py").
In certain embodiments, $Y_1\text{-}Y_2\text{-}Y_3\text{-}Y_4\text{-}Y_5\text{-}Y_6$ is Py-Py-Im-Im-Im-Im.
In certain embodiments, $Y_1\text{-}Y_2\text{-}Y_3\text{-}Y_4\text{-}Y_5\text{-}Y_6$ is Py-β-Im-Im-Im-Im.
In certain embodiments, $Y_1\text{-}Y_2\text{-}Y_3\text{-}Y_4\text{-}Y_5\text{-}Y_6$ is Py-Py-Py-Py-Im-Im.
In certain embodiments, $Y_1\text{-}Y_2\text{-}Y_3\text{-}Y_4\text{-}Y_5\text{-}Y_6$ is Py-β-Py-Py-Im-Im.
In certain embodiments, $Y_1\text{-}Y_2\text{-}Y_3\text{-}Y_4\text{-}Y_5\text{-}Y_6$ is Im-Im-Py-Py-Py-Py.
In certain embodiments, $Y_1\text{-}Y_2\text{-}Y_3\text{-}Y_4\text{-}Y_5\text{-}Y_6$ is Im-Im-Py-Py-β-Py.
In certain embodiments, the compounds have structural Formula IV:

$$X\text{-}L\text{-}(Y_1\text{-}Y_2\text{-}Y_3\text{-}Y_4\text{-}Y_5\text{-}Y_6)\text{-}V\text{-}(Y_7\text{-}Y_8\text{-}Y_9\text{-}Y_{10}\text{-}Y_{11}\text{-}Y_{12})\text{-}Y_0 \qquad \text{(IV)}$$

or a salt thereof, wherein:

X comprises a recruiting moiety that is capable of noncovalent binding to a regulatory molecule within the nucleus;
$Y_1$, $Y_2$, $Y_3$, $Y_4$, $Y_5$, $Y_6$, $Y_7$, $Y_8$, $Y_9$, $Y_{10}$, $Y_{11}$, and $Y_{12}$ are internal subunits, each of which comprises a moiety chosen from a heterocyclic ring or a $C_{1-6}$straight chain aliphatic segment, and each of which is chemically linked to its two neighbors;
$Y_0$ is an end subunit which comprises a moiety chosen from a heterocyclic ring or a straight chain aliphatic segment, which is chemically linked to its single neighbor;
each subunit can noncovalently bind to an individual nucleotide in the GGGGCC repeat sequence;
L is a linker;
V is a turn component for forming a hairpin turn; and
$(Y_1\text{-}Y_2\text{-}Y_3\text{-}Y_4\text{-}Y_5\text{-}Y_6)\text{-}V\text{-}(Y_7\text{-}Y_8\text{-}Y_9\text{-}Y_{10}\text{-}Y_{11}\text{-}Y_{12})\text{-}Y_0$ combine to form a DNA recognition moiety that is capable of noncovalent binding to one or more copies of the the hexanucleotide repeat sequence GGGGCC.

In certain embodiments, V is $-HN\text{-}CH_2CH_2CH_2\text{-}CO-$.

In certain embodiments, the compounds have structural Formula Va:

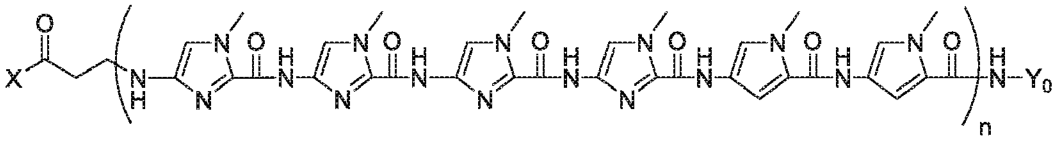

(Va) or a salt thereof, wherein:

X comprises a recruiting moiety that is capable of noncovalent binding to a regulatory molecule within the nucleus;
$Y_0$ is an end subunit which comprises a moiety chosen from a heterocyclic ring or a straight chain aliphatic segment, which is chemically linked to its single neighbor; and n is an integer between 1 and 5, inclusive.

In certain embodiments, the compounds have structural Formula Vb:

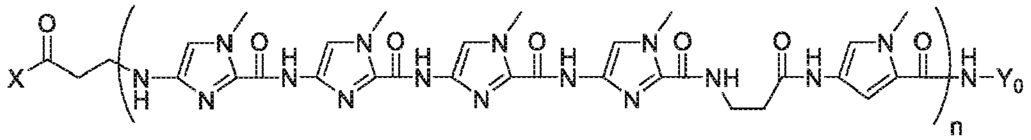

(Vb)

or a salt thereof, wherein:

X comprises a recruiting moiety that is capable of noncovalent binding to a regulatory molecule within the nucleus;
$Y_0$ is an end subunit which comprises a moiety chosen from a heterocyclic ring or a straight chain aliphatic segment, which is chemically linked to its single neighbor; and
n is an integer between 1 and 5, inclusive.

In certain embodiments, the compounds have structural Formula VIa:

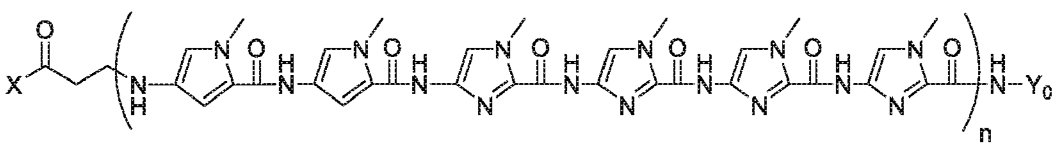

(VIa)

or a salt thereof, wherein:

X comprises a recruiting moiety that is capable of noncovalent binding to a regulatory molecule within the nucleus;
$Y_0$ is an end subunit which comprises a moiety chosen from a heterocyclic ring or a straight chain aliphatic segment, which is chemically linked to its single neighbor; and
n is an integer between 1 and 5, inclusive.

In certain embodiments, the compounds have structural Formula VIb:

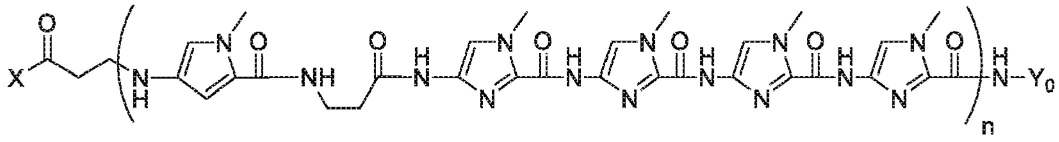

(VIb)

or a salt thereof, wherein:

X comprises a recruiting moiety that is capable of noncovalent binding to a regulatory molecule within the nucleus;
$Y_0$ is an end subunit which comprises a moiety chosen from a heterocyclic ring or a straight chain aliphatic segment, which is chemically linked to its single neighbor; and
n is an integer between 1 and 5, inclusive.

In certain embodiments, the compounds have structural Formula VII:

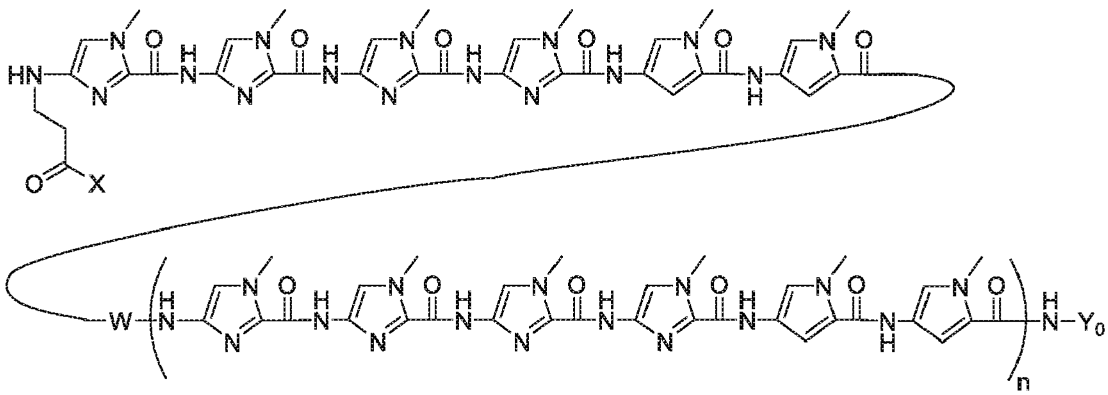

(VII)

or a salt thereof, wherein:

X comprises a recruiting moiety that is capable of noncovalent binding to a regulatory molecule within the nucleus; and
W is a spacer;
$Y_0$ is an end subunit which comprises a moiety chosen from a heterocyclic ring or a straight chain aliphatic segment, which is chemically linked to its single neighbor; and
n is an integer between 1 and 5, inclusive.

In certain embodiments of the compounds of structural Formula VII,

W is $-NHCH_2-(CH_2OCH_2)_p-CH_2CO-$; and
p is an integer between 1 and 4, inclusive.

In certain embodiments, the compounds have structural Formula VIII:

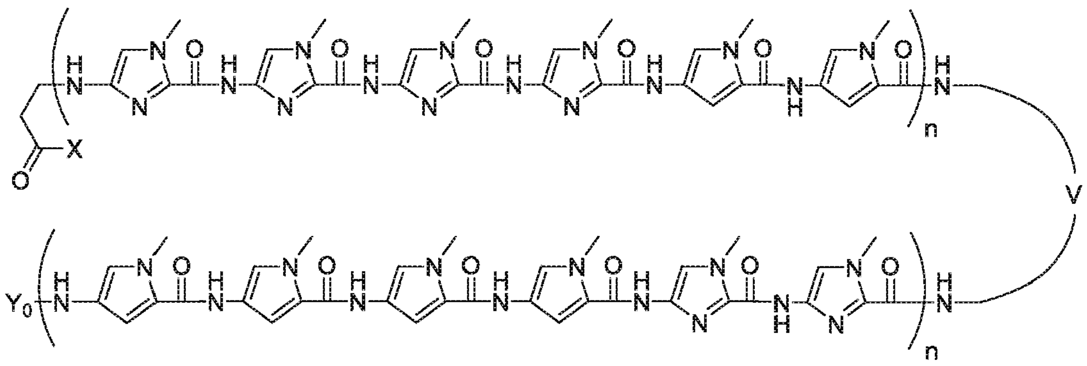

(VIII)

or a salt thereof, wherein:

X comprises a recruiting moiety that is capable of noncovalent binding to a regulatory molecule within the nucleus;
V is a turn component for forming a hairpin turn;
$Y_0$ is an end subunit which comprises a moiety chosen from a heterocyclic ring or a straight chain aliphatic segment, which is chemically linked to its single neighbor; and
n is an integer between 1 and 5, inclusive.

In certain embodiments of the compounds of structural Formula VIII, V is $-(CH_2)q-NH-(CH_2)_q-$; and q is an integer between 2 and 4, inclusive.

In some embodiments, V is $-(CH_2)_a-NR^1-(CH_2)_b-$, $-(CH_2)_a-$, $-(CH_2)_a-O-(CH_2)_b-$, $-(CH_2)_a-CH(NHR^1)-$, $-(CH_2)_a-CH(NHR^1)-$, $-(CR^2R^3)_a-$, or $-(CH_2)_a-CH(NR^1{}_3)^+-(CH_2)_b-$, wherein each a is independently an integer between 2 and 4; $R^1$ is H, an optionally substituted $C_{1-6}$ alkyl, an optionally substituted $C_{3-10}$ cycloalkyl, an optionally substituted $C_{6-10}$ aryl, an optionally substituted 4-10 membered heterocyclyl, or an optionally substituted 5-10 membered heteroaryl; each $R^2$ and $R^3$ are independently H, halogen, OH, NHAc, or $C_{1-4}$ alky. In some embodiments, $R^1$ is H. In some embodiments, $R^1$ is $C_{1-6}$ alkyl optionally substituted by 1-3 substituents selected from -C(O)-phenyl. In some embodiments, V is $-(CR^2R^3)-(CH_2)a-$ or $-(CH_2)a-(CR^2R^3)-(CH_2)_b-$, wherein each a is independently 1-3, b is 0-3, and each $R^2$ and $R^3$ are independently H, halogen, OH, NHAc, or $C_{1-4}$ alky. In some embodiments, V is $-(CH_2)-CH(NH_3)^+-(CH_2)-$ or $-(CH_2)-CH_2CH(NH_3)^+-$.

In one aspect, the compounds of the present disclosure bind to the GGGGCC of *c9orf72* and recruit a regulatory moiety to the vicinity of *c9orf72.* The regulatory moiety, due to its proximity to the gene, will be more likely to modulate the expression of *c9orf72.*

Also provided are embodiments wherein any compound disclosed above, including compounds of Formulas I - VIII, are singly, partially, or fully deuterated. Methods for accomplishing deuterium exchange for hydrogen are known in the art.

Also provided are embodiments wherein any embodiment above may be combined with any one or more of these embodiments, provided the combination is not mutually exclusive.

As used herein, two embodiments are "mutually exclusive" when one is defined to be something which is different than the other. For example, an embodiment wherein two groups combine to form a cycloalkyl is mutually exclusive with an embodiment in which one group is ethyl the other group is hydrogen. Similarly, an embodiment wherein one group is $CH_2$ is mutually exclusive with an embodiment wherein the same group is NH.

In one aspect, the compounds of the present disclosure bind to the GGGGCC of *c9orf72* and recruit a regulatory moiety to the vicinity of *c9orf72.* The regulatory moiety, due to its proximity to the gene, will be more likely to modulate the expression of *c9orf72.*

In one aspect, the compounds of the present disclosure provide a polyamide sequence for interaction of a single polyamide subunit to each base pair in the GGGGCC repeat sequence. In one aspect, the the compounds of the present disclosure provide a turn component V, in order to enable hairpin binding of the compound to the GGGGCC, in which each nucleotide pair interacts with two subunits of the polyamide.

In one aspect, the compounds of the present disclosure are more likely to bind to the repeated GGGGCC of *c9orf72* than to GGGGCC elsewhere in the subject's DNA, due to the high number of GGGGCC repeats associated with *c9orf72.*

In one aspect, the compounds of the present disclosure provide more than one copy of the polyamide sequence for noncovalent binding to the GGGGCC. In one aspect, the compounds of the present disclosure bind to *c9orf72* with

an affinity that is greater than a corresponding compound that contains a single polyamide sequence.

In one aspect, the compounds of the present disclosure provide more than one copy of the polyamide sequence for noncovalent binding to the GGGGCC, and the individual polyamide sequences in this compound are linked by a spacer W, as defined above. The spacer W allows this compound to adjust its geometry as needed to alleviate the geometric strain that otherwise affects the noncovalent binding of longer polyamide sequences.

***First terminus* - *DNA binding moiety***

The first terminus interacts and binds with the gene, particularly with the minor grooves of the GGGGCC sequence. In one aspect, the compounds of the present disclosure provide a polyamide sequence for interaction of a single polyamide subunit to each base pair in the GGGGCC repeat sequence. In one aspect, the compounds of the present disclosure provide a turn component (e.g, aliphatic amino acid moiety), in order to enable hairpin binding of the compound to the GGGGCC, in which each nucleotide pair interacts with two subunits of the polyamide.

In one aspect, the compounds of the present disclosure are more likely to bind to the repeated GGGGCC of *c9orf72* than to GGGGCC elsewhere in the subject's DNA, due to the high number of GGGGCC repeats associated with *c9orf72*.

In one aspect, the compounds of the present disclosure provide more than one copy of the polyamide sequence for noncovalent binding to GGGGCC. In one aspect, the compounds of the present disclosure bind to *c9orf72* with an affinity that is greater than a corresponding compound that contains a single polyamide sequence.

In one aspect, the compounds of the present disclosure provide more than one copy of the polyamide sequence for noncovalent binding to the GGGGCC, and the individual polyamide sequences in this compound are linked by a spacer W, as defined above. The spacer W allows this compound to adjust its geometry as needed to alleviate the geometric strain that otherwise affects the noncovalent binding of longer polyamide sequences.

In certain embodiments, the DNA recognition or binding moiety binds in the minor groove of DNA.

In certain embodiments, the DNA recognition or binding moiety comprises a polymeric sequence of monomers, wherein each monomer in the polymer selectively binds to a certain DNA base pair.

In certain embodiments, the DNA recognition or binding moiety comprises a polyamide moiety.

In certain embodiments, the DNA recognition or binding moiety comprises a polyamide moiety comprising heteroaromatic monomers, wherein each heteroaromatic monomer binds noncovalently to a specific nucleotide, and each heteroaromatic monomer is attached to its neighbor or neighbors via amide bonds.

In certain embodiments, the DNA recognition moiety binds to a sequence comprising at least 1000 pentanucleotide repeats. In certain embodiments, the DNA recognition moiety binds to a sequence comprising at least 500 pentanucleotide repeats. In certain embodiments, the DNA recognition moiety binds to a sequence comprising at least 200 pentanucleotide repeats. In certain embodiments, the DNA recognition moiety binds to a sequence comprising at least 100 pentanucleotide repeats. In certain embodiments, the DNA recognition moiety binds to a sequence comprising at least 50 pentanucleotide repeats. In certain embodiments, the DNA recognition moiety binds to a sequence comprising at least 20 pentanucleotide repeats.

In certain embodiments, the compounds comprise a cell-penetrating ligand moiety.

In certain embodiments, the cell-penetrating ligand moiety is a polypeptide.

In certain embodiments, the cell-penetrating ligand moiety is a polypeptide containing fewer than 30 amino acid residues.

In certain embodiments, the polypeptide is chosen from any one of SEQ ID NO. 1 to SEQ ID NO. 37, inclusive.

In certain embodiments, the compounds have structural Formula II:

$$X\text{-}L\text{-}(Y_1\text{-}Y_2\text{-}Y_3\text{-}Y_4\text{-}Y_5)_n\text{-}Y_0 \qquad \text{(II)}$$

or a salt thereof, wherein:

X comprises a recruiting moiety that is capable of noncovalent binding to a regulatory molecule within the nucleus;
L is a linker;
$Y_1$, $Y_2$, $Y_3$, $Y_4$, and $Y_5$ are internal subunits, each of which comprises a moiety chosen from a heterocyclic ring or a $C_{1-6}$straight chain aliphatic segment, and each of which is chemically linked to its two neighbors;
$Y_0$ is an end subunit which comprises a moiety chosen from a heterocyclic ring or a straight chain aliphatic segment, which is chemically linked to its single neighbor;
each subunit can noncovalently bind to an individual nucleotide in the GGGGCC repeat sequence;
n is an integer between 1 and 15, inclusive; and
$(Y_1\text{-}Y_2\text{-}Y_3\text{-}Y_4\text{-}Y_5)_n\text{-}Y_0$ combine to form a DNA recognition moiety that is capable of noncovalent binding to one or more copies of the the pentanucleotide repeat sequence GGGGCC.

In certain embodiments, the compounds of structural Formula II comprise a subunit for each individual nucleotide in the GGGGCC repeat sequence.

In certain embodiment, each internal subunit has an amino (-NH-) group and a carboxy (-CO-) group.

In certain embodiments, the compounds of structural Formula II comprise amide (-NHCO-) bonds between each pair of internal subunits.

In certain embodiments, the compounds of structural Formula II comprise an amide (-NHCO-) bond between L and the leftmost internal subunit.

In certain embodiments, the compounds of structural Formula II comprise an amide bond between the rightmost internal subunit and the end subunit.

In certain embodiments, each subunit comprises a moiety that is independently chosen from a heterocycle and an aliphatic chain.

In certain embodiments, the heterocycle is a monocyclic heterocycle. In certain embodiments, the heterocycle is a monocyclic 5-membered heterocycle. In certain embodiments, each heterocycle contains a heteroatom independently chosen from N, O, or S. In certain embodiments, each heterocycle is independently chosen from pyrrole, imidazole, thiazole, oxazole, thiophene, and furan.

In certain embodiments, the aliphatic chain is a $C_{1-6}$straight chain aliphatic chain. In certain embodiments, the aliphatic chain has structural formula $-(CH_2)_m-$, for m chosen from 1, 2, 3, 4, and 5. In certain embodiments, the aliphatic chain is $-CH_2CH_2-$.

The form of the polyamide selected can vary based on the target gene. The first terminus can include a polyamide selected from the group consisting of a linear polyamide, a hairpin polyamide, a H-pin polyamide, an overlapped polyamide, a slipped polyamide, a cyclic polyamide, a tandem polyamide, and an extended polyamide. In some embodiments, the first terminus comprises a linear polyamide. In some embodiments, the first terminus comprises a hairpin polyamide.

The binding affinity between the polyamide and the target gene can be adjusted based on the composition of the polyamide. In some embodiments, the polyamide is capable of binding the DNA with an affinity of less than about 600 nM, about 500 nM, about 400 nM, about 300 nM, about 250 nM, about 200 nM, about 150 nM, about 100 nM, or about 50nM. In some embodiments, the polyamide is capable of binding the DNA with an affinity of less than about 300 nM. In some embodiments, the polyamide is capable of binding the DNA with an affinity of less than about 200 nM. In some embodiments, the polyamide is capable of binding the DNA with an affinity of greater than about 200 nM, about 150 nM, about 100 nM, about 50 nM, about 10 nM, or about 1 nM. In some embodiments, the polyamide is capable of binding the DNA with an affinity in the range of about 1-600 nM, 10-500 nM, 20-500 nM, 50-400 nM, or 100-300 nM.

The binding affinity between the polyamide and the target DNA can be determined using a quantitative footprint titration experiment. The experiment involve measuring the dissociation constant Kd of the polyamide for target sequence at either 24° C. or 37° C., and using either standard polyamide assay solution conditions or approximate intracellular solution conditions.

The binding affinity between the regulatory protein and the ligand on the second terminus can be determined using an assay suitable for the specific protein. The experiment involve measuring the dissociation constant Kd of the ligand for protein and using either standard protein assay solution conditions or approximate intracellular solution conditions.

In some embodiments, the first terminus comprises -NH-Q-C(O)-, wherein Q is an optionally substituted $C_{6-10}$ arylene group, optionally substituted 4-10 membered heterocyclene, optionally substituted 5-10 membered heteroarylene group, or an optionally substituted alkylene group. In some embodiments, Q is an optionally substituted $C_{6-10}$ arylene group or optionally substituted 5-10 membered heteroarylene group. In some embodiments, Q is an optionally substituted 5-10 membered heteroarylene group. In some embodiments, the 5-10 membered heteroarylene group is optionally substituted with 1-4 substituents selected from H, OH, halogen, $C_{1-10}$ alkyl, $NO_2$, CN, NR'R", $C_{1-6}$haloalkyl, $C_{1-6}$alkoxyl, $C_{1-6}$ haloalkoxy, ($C_{1-6}$alkoxy)$C_{1-6}$alkyl, $C_{2-10}$ alkenyl, $C_{2-10}$alkynyl, $C_{3-7}$carbocyclyl, 4-10 membered heterocyclyl, $C_{6-10}$aryl, 5-10 membered heteroaryl, ($C_{3-7}$carbocyclyl)$C_{1-6}$ alkyl, (4-10 membered heterocyclyl)$C_{1-6}$ alkyl, ($C_{6-10}$ aryl)$C_{1-6}$ alkyl, ($C_{6-10}$ aryl)$C_{1-6}$ alkoxy, (5-10 membered heteroayl)$C_{1-6}$ alkyl, ($C_{3-7}$carbocyclyl)-amine, (4-10 membered heterocyclyl)amine, ($C_{6-10}$aryl)amine, (5-10 membered heteroaryl)amine, acyl, C-carboxy, O-carboxy, C-amido, N-amido, S-sulfonamido, N-sulfonamido, -SR', COOH, or CONR'R"; wherein each R' and R" are independently H, $C_{1-10}$ alkyl, $C_{1-10}$ haloalkyl, $C_{1-10}$ alkoxyl.

In some embodiments, the first terminus comprises at least three aromatic carboxamide moieties selected to correspond to the nucleotide repeat sequence GGGGCC and at least one aliphatic amino acid residue chosen from the group consisting of glycine, β-alanine, γ-aminobutyric acid, 2,4-diaminobutyric acid, and 5-aminovaleric acid. In some embodiments, the first terminus comprises at least one β-alanine subunit.

In some embodiments, the monomer element is independently selected from the group consisting of optionally substituted pyrrole carboxamide monomer, optionally substituted imidazole carboxamide monomer, optionally substituted C-C linked heteromonocyclic/heterobicyclic moiety, and β-alanine.

The transcription modulator molecule of claim 1, wherein the first terminus comprises a structure of Formula

(A-1):

$-L_{1a}-[A-M]_p-E_1$ (A-1)

wherein:

each [A-M] appears p times and p is an integer in the range of 1 to 10,
$L_{1a}$ is a bond, a $C_{1-6}$ alkylene, $-NR^a-C_{1-6}$alkylene-C(O)-, $NR^aC(O)$-, -NW-$C_{1-6}$ alkylene, -0-, or -O-$C_{1-6}$ alkylene;
each A is selected from the group consisting of a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, -$C_{1-10}$ alkylene-$NR^a$-, -CO-, -$NR^a$-, - $CONR^a$-,-$CONR^aC_{1-4}$alkylene-, -$NR^aO$-$C_{1-4}$alkylene-, -C(O)O-, -O-, -S-, - C(=S)NH-, -C(O)-NH-NH-, -C(O)-N=N-, -C(O)-CH=CH-, $(CH_2)_{0-4}CH=CH-(CH_2)_{0-4}$, -$N(CH_3)$-$C_{1-6}$ alkylene, and

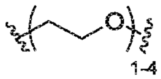;

NH- $C_{1-6}$ alkylene-NH-, -0- $C_{1-6}$ alkylene-O-, -NH-N=N-, -NH-C(O)-NH-, and any combinations thereof, and at least one A is -CONH-;
each M is an optionally substituted $C_{6-10}$ arylene group, optionally substituted 4-10 membered heterocyclene, optionally substituted 5-10 membered heteroarylene group, or an optionally substituted alkylene;
$E_1$ is H or -$A^E$-G;
$A^E$ is absent or NHCO-;
G is selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)$C_{1-5}$alkylene-$NR^aR^b$, $C_{0-4}$ alkylene-NHC(=NH)$R^a$, -CO-halogen, and optionally substituted amine; and
each $R^a$ and $R^b$ are independently selected from the group consisting of H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, and optionally substituted 5-10 membered heteroaryl.

In some embodiments, the first terminus can comprise a structure of Formula (A-2):

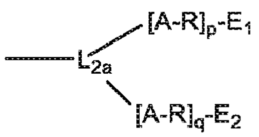

(A-2)

wherein:

$L_{2a}$ is a linker selected from -$C_{1-12}$ alkylene-$CR^a$, -CH, N, -$C_{1-6}$ alkylene-N, -C(O)N, -$NR^a$-$C_{1-6}$ alkylene-CH, -O-$C_{0-6}$ alkylene-CH,

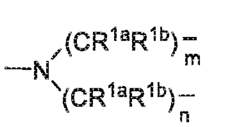, 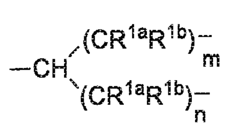,

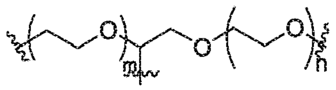, or 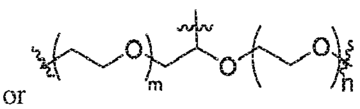;

each p and q are independently an integer in the range of 1 to 10;

each m and n are independently an integer in the range of 0 to 10;

each A is independently selected from a bond, $C_{1-10}$ alkylene, -$C_{1-10}$ alkylene-C(O)-, -$C_{1-10}$ alkylene-$NR^a$-, -CO-, -$NR^a$-, -$CONR^a$-,-$CONR^aC_{1-4}$alkylene-, -$Nr^aCO$-$C_{1-4}$ alkylene-, -C(O)O-, -0-, -S-, -C(=S)-NH-, -C(O)-NH-NH-, -C(O)-N=N-, or -C(O)-CH=CH-, and at least one A is CONH-;

each M is independently an optionally substituted $C_{6-10}$ arylene group, optionally substituted 4-10 membered heterocyclene, optionally substituted 5-10 membered heteroarylene group, or an optionally substituted alkylene;

each $E_1$ and $E_2$ are independently H or -$A^E$-G;

each $A^E$ is independently absent or NHCO;

each G is independently selected from the group consisting of $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$lkylene-CH(=$N^+H_2$)($NR^aR^b$)$C_{1-5}$alkylene-$NR^aR^b$, $C_{0-4}$ alkylene-NHC(=NH) $R^a$, -CO-halogen, and optionally substituted amine; and

each $R^a$ and $R^b$ are independently selected from the group consisting of H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, and an optionally substituted 5-10 membered heteroaryl; and

each $R^{1a}$ and $R^{1b}$ is independently H, or $C_{1-6}$ alkyl.

In certain embodiments, the integers p and q are 2≦p+q≦20. In some embodiments, p is in the range of about 2 to 10. In some embodiments, p is in the range of about 4 to 8. In some embodiments, q is in the range of about 2 to 10. In some embodiments, q is in the range of about 4 to 8.

In certain embodiments, $L^{2a}$ is-$C_{2-8}$alkylene-CH,

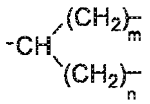 or 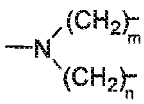 ,

and wherein each m and n is independently an integer in the range of 0 to 10. In certain embodiments, $L^{2a}$ is

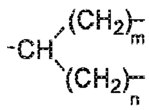 .

In some embodiments, $L^{2a}$ is -$C_{2-8}$ alkylene-CH. In some embodiments, $L^{2a}$ is

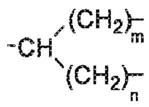 ,

wherein (m+n) is in the range of about 1 to 4. In some embodiments, $L^{2a}$ is

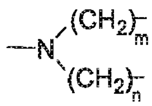 ,

and (m+n) is in the range of about 2 to

5. In some embodiments, $L^{2a}$ is

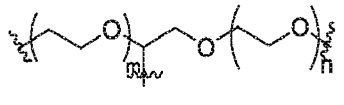 , 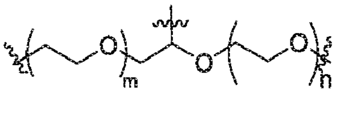 ;

wherein (m+n) is in the range of about 1 to 6.

The transcription modulator molecule of claim 1, wherein the first terminus comprises a structure of Formula (A-3):

$$-L_{1a}-[A-M]_{p1}-L_{3a}-[M-A]_{q1}-E_1 \quad \text{(A-3)}$$

wherein:

$L_{1a}$ is a bond, a $C_{1-6}$ alkylene, -NH-$C_{0-6}$ alkylene-C(O)-, -N($CH_3$)-$C_{0-6}$ alkylene, or -O-$C_{0-6}$ alkylene;
$L_{3a}$ is a bond, $C_{1-6}$ alkylene, -NH-$C_{0-6}$ alkylene-C(O)-, -N($CH_3$)-$C_{0-6}$ alkylene, -O-$C_{0-6}$ alkylene, -$(CH_2)_a$-$NR^a$-$(CH_2)_b$-, -$(CH_2)_a$-, -$(CH_2)_a$-O-$(CH_2)_b$-, -$(CH_2)_a$-CH($NHR^a$)-, -$(CH_2)_a$-CH($NHR^a$)-, -$(CR^{1a}R^{1b})_a$-, or -$(CH_2)_a$-CH($NR^aR^b$)-$(CH_2)_b$-;
each a and b are independently an integer between 2 and 4;
each $R^a$ and $R^b$ are independently selected from H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, and an optionally substituted 5-10 membered heteroaryl;
each $R^{1a}$ and $R^{1b}$ is independently H, halogen, OH, NHAc, or $C_{1-4}$ alkyl;
each [A-M] appears $p^1$ times and $p^1$ is an integer in the range of 1 to 10;
each [M-A] appears $q^1$ times and $q^1$ is an integer in the range of 1 to 10;
each A is selected from a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, -$C_{1-10}$ alkylene-$NR^a$-, -CO-, -$NR^a$-, -$CONR^a$-,-$CONR^aC_{1-4}$akylene-, -$NR^aCO$-$C_{1-4}$alkylene-, -C(O)O-, -O-, -S-, -C(=S)-NH-, -C(O)-NH-NH-, -C(O)-N=N-, -C(O)-CH=CH-, $(CH_2)_{0-4}$-CH=CH-$(CH_2)_{0-4}$, -N($CH_3$)-$C_{1-6}$ alkylene, and

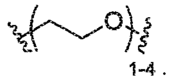
;

-NH- $C_{1-6}$ alkyleneNH-, -0- $C_{1-6}$ alkylene-O-, -NH-N=N-, -NH-C(O)-NH-, and any combinations thereof, and at least one A is NHCO;
each M in each [A-M] and [M-A] unit is independently an optionally substituted $C_{6-10}$ arylene group, optionally substituted 4-10 membered heterocyclene, optionally substituted 5-10 membered heteroarylene group, or an optionally substituted alkylene; and
$E_1$ is selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR_2$), -$C_{0-4}$alkylene-CH(=$N^+H_2$)($NR^aR^b$)$C_{1-5}$alkylene- $NR^aR^b$, $C_{0-4}$ alkylene-NHC(=NH) $R^a$, -CO-halogen.

In certain embodiments, the integers $p^1$ and $q^1$ are $2 \leqq p^1+q^1 \leqq 20$.

In some embodiments, for Formula (A-1) to (A-4), each A is indepedently a bond, $C_{1-6}$ alkylene, optionally substituted phenylene, optionally substituted thiophenylene, optionally substituted furanylene, - $C_{1-10}$ alkylene-C(O)-, -$C_{1-10}$ alkylene-NH-, —CO—, -$NR^a$-, -$CONR^a$-, -$CONR^aC_{1-4}$alkylene-, - $NR^aCO$-$C_{1-4}$alkylene-, -C(O)O-, -O-, -S-, -C(=S)-NH-, -C(O)-NH-NH-, -C(O)-N=N-, —C(O)-CH=CH—, -CH=CH-, -NH-N=N-, -NH-C(O)-NH-, -N($CH_3$)-$C_{1-6}$ alkylene, and

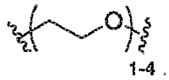
;

-NH-$C_{1-6}$ alkylene-NH-, -O-$C_{1-6}$ alkylene-O-, and any combinations optionally substituted 5-10 membered heteroarylene group. In some embodiments, in Formula (A-1) and (A-3), $L_{1a}$ is a bond. In some embodiments, in Formula (A-1) and (A-3), $L_{1a}$ is a $C_{1-6}$ alkylene. In some embodiments, in Formula (A-1) and (A-3), $L_{1a}$ is -NH-$C_{1-6}$ alkylene-C(O)-. In some embodiments, in Formula (A-1) and (A-3), $L_{1a}$ is - N($CH_3$)-$C_{1-6}$alkylene-. In some embodiments, in Formula (A-1) and (A-3), $L_{1a}$ is -O-$C_{0-6}$alkylene-.

In some embodiments, $L_{3a}$ is a bond. In some embodiments, $L_{3a}$ is $C_{1-6}$ alkylene. In some embodiments, $L_{3a}$ is -NH-$C_{1-6}$ alkylene-C(O)-. In some embodiments, $L_{3a}$ is -N($CH_3$)-$C_{1-6}$ alkylene C(O)-. In some embodiments, $L_{3a}$ is -O-$C_{0-6}$ alkylene. In some embodiments, $L_{3a}$ is -$(CH_2)_a$-$NR^a$-$(CH_2)_b$-. In some embodiments, $L_{3a}$ is -$(CH_2)_a$-O-$(CH_2)_b$-.

In some embodiments, $L_{3a}$ is $-(CH_2)_a-CH(NHR^a)-$. In some embodiments, $L_{3a}$ is $-(CH_2)_a-CH(NHR^a)-$. In some embodiments, $L_{3a}$ is $-(CR^{1a}R^{1b})_a-$. In some embodiments, $L_{3a}$ is $-(CH_2)_a-CH(NR^aR^b)-(CH_2)_b-$.

In some embodiments, for Formula (A-1) to (A-4), at least one A is NH and at least one A is C(O). In some embodiments, for Formula (A-1) to (A-4), at least two A is NH and at least two A is C(O). In some embodiments, when M is a bicyclic ring, A is a bond. In some embodiments, at least one A is a phenylene optionally substituted with one or more alkyl. In some embodiments, at least one A is thiophenylene optionally substituted with one or more alkyl. In some embodiments, at least one A is a furanylene optionally substituted with one or more alkyl. In some embodiments, at least one A is $(CH_2)_{0-4}-CH=CH-(CH_2)_{0-4}$, preferably -CH=CH-. In some embodiments, at least one A is -NH-N=N-. In some embodiments, at least one A is -NH-C(O)-NH-. In some embodiments, at least one A is $-N(CH_3)-C_{1-6}$ alkylene. In some embodiments, at least one A is

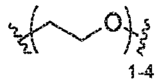

.

In some embodiments, at least one A is -NH- $C_{1-6}$ alkylene-NH-. In some embodiments, at least one A is $-O-C_{1-6}$ alkylene-O-.

In some embodiments, each M in [A-M] of Formula (A-1) to (A-4) is $C_{6-10}$ arylene group, 4-10 membered heterocyclene, optionally substituted 5-10 membered heteroarylene group, or $C_{1-6}$ alkylene; each optionally substituted by 1-3 substituents selected from H, OH, halogen, $C_{1-10}$ alkyl, $NO_2$, CN, $NR^aR^b$, $C_{1-6}$ haloalkyl, $-C_{1-6}$ alkoxyl, $C_{1-6}$ haloalkoxy, ($C_{1-6}$ alkoxy)$C_{1-6}$ alkyl, $C_{2-10}$alkenyl, $C_{2-10}$alkynyl, $C_{3-7}$ carbocyclyl, 44-10 membered heterocyclyl, $C_{6-10}$aryl, 5-10 membered heteroaryl, -($C_{3-7}$carbocyclyl)$C_{1-6}$alkyl, (4-10 membered heterocyclyl)$C_{1-6}$alkyl, ($C_{6-10}$aryl)$C_{1-6}$alkyl, ($C_{6-10}$aryl)$C_{1-6}$alkoxy, (5-10 membered heteroaryl)$C_{1-6}$alkyl, -($C_{3-7}$carbocyclyl)-amine, (4-10 membered heterocyclyl)amine, ($C_{6-10}$aryl)amine, (5-10 membered heteroaryl)amine, acyl, C-carboxy, O-carboxy, C-amido, N-amido, S-sulfonamido, N-sulfonamido, -SR', COOH, or $CONR^aR^b$; wherein each $R^a$ and $R^b$ are independently H, $C_{1-10}$ alkyl, $C_{1-10}$ haloalkyl, $-C_{1-10}$ alkoxyl. In some embodiments, each M in [A-M] of Formula (A-1) to (A-3) is a 5-10 membered heteroarylene containing at least one heteroatoms selected from O, S, and N or a $C_{1-6}$ alkylene, and the heteroarylene or the a $C_{1-6}$ alkylene is optionally substituted with 1-3 substituents selected from OH, halogen, $C_{1-10}$ alkyl, $NO_2$, CN, $NR^aR^b$, $C_{1-6}$ haloalkyl, $-C_{1-6}$ alkoxyl, $C_{1-6}$ haloalkoxy, $C_{3-7}$ carbocyclyl, 4-10 membered heterocyclyl, $C_{6-10}$aryl, 5-10 membered heteroaryl, -SR', COOH, or $CONR^aR^b$; wherein each $R^a$ and $R^b$ are independently H, $C_{1-10}$ alkyl, $C_{1-10}$haloalkyl, $-C_{1-10}$alkoxyl. In some embodiments, each R in [A-R] of Formula (A-1) to (A-3) is a 5-10 membered heteroarylene containing at least one heteroatoms selected from O, S, and N, and the heteroarylene is optionally substituted with 1-3 substituents selected from OH, $C_{1-6}$alkyl, halogen, and $C_{1-6}$ alkoxyl.

In some embodiments, for Formula (A-1) to (A-4), at least one M is a 5 membered heteroarylene having at least one heteroatom selected from O, N, S and optionally substituted with one or more $C_{1-10}$ alkyl. In some embodiments, at least one M is a pyrrole optionally subsituted with one or more $C_{1-10}$ alkyl. In some embodiments, at least one M is a immidazole optionally subsituted with one or more $C_{1-10}$ alkyl. In some embodiments, for Formula (A-1) to (A-4), at least one M is a $C_{2-6}$ alkylene optionally substituted with one or more $C_{1-10}$ alkyl. In some embodiments, at least one M is a pyrrole optionally subsituted with one or more $C_{1-10}$ alkyl. In some embodiments, for Formula (A-1) to (A-4), at least one M is a bicyclic heteroarylene or arylene. In some embodiments, at least one M is a phenylene optionally subsituted with one or more $C_{1-10}$ alkyl. In some embodiments, at least one M is a benzimmidazole optionally subsituted with one or more $C_{1-10}$ alkyl.

In some embodiments, the first terminus comprises a structure of Formula (A-4):

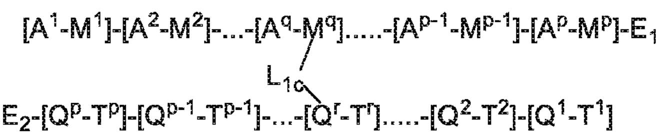

(A-4)

wherein:

$L_{1c}$ is a bivalent or trivalent group selected from

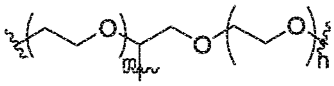,

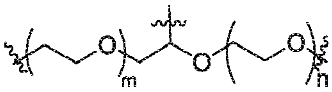, 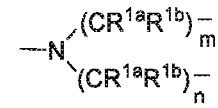, 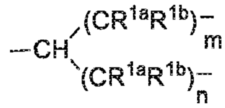,

a $C_{1-10}$ alkylene, -NH-$C_{0-6}$ alkylene-C(O)-, -N($CH_3$)-$C_{0-6}$ alkylene, and

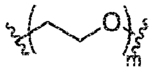;

p is an integer in the range of 3 to 10;

$$2 \leq q \leq (p-1);$$

$$2 \leq r \leq (p-1);$$

m and n are each independently an integer in the range of 0 to 10;

each $A^2$ through $A^p$ is independently selected from the group consisting of a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-,-$C_{1-10}$ alkyleneNR'-, -CO-, -$NR^a$-,-$CONR^a$-,-$CONR^aC_{1-4}$alkylene-, -$NR^aCO$-$C_{1-4}$alkylene-, -C(O)O-, -O-, -S-, -C(=S)-NH-, -C(O)-NH-NH-, -C(O)-N=N-,-C(O)-CH=CH-, $(CH_2)_{0-4}$-CH=CH-$(CH_2)_{0-4}$, -N($CH_3$)-$C_{1-6}$ alkylene,

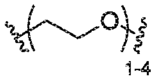,

-NH- $C_{1-6}$ alkylene-NH-, -O- $C_{1-6}$ alkylene-O-, -NH-N=N-, -NH-C(O)-NH-, and any combinations thereof, and at least one $A^2$ through $A^p$ is NHCO;

each $M^1$ through $M^p$ is an optionally substituted $C_{6-10}$ arylene group, optionally substituted 4-10 membered heterocyclene, optionally substituted 5-10 membered heteroarylene group, or an optionally substituted alkylene;

each $T^2$ through $T^p$ is independently selected from the group consisting of a bond, $C_{1-10}$ alkylene, optionally substituted $C_{6-10}$ arylene group, optionally substituted 4-10 membered heterocyclene, optionally substituted 5-10 membred heteroarylene group, -$C_{1-10}$ alkylene-C(O)-,-$C_{1-10}$ alkylene-$NR^a$-, -CO-, -$NR^a$-, -$CONR^a$-,-$CONR^aC_{1-4}$alkylene-, -$NR^aCO$-$C_{1-4}$alkylene-, -C(O)O-, -O-, -S-, -C(=S)-NH-, -C(O)-NH-NH-, -C(O)-N=N-,-C(O)-CH=CH-, $(CH_2)_{0-4}$-CH=CH-$(CH_2)_{0-4}$, -N($CH_3$)-$C_{1-6}$ alkylene,

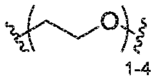,

-NH- $C_{1-6}$ alkylene-NH-, -O- $C_{1-6}$ alkylene-O-, -NH-N=N-, and -NH-C(O)NH-, and any combinations thereof,

each $Q^1$ to $Q^p$ is an optionally substituted $C_{6-10}$ arylene group, optionally substituted 4-10 membered heterocyclene, optionally substituted 5-10 membered heteroarylene group, or an optionally substituted alkylene;

each $A^1$, $A^2$, $E_1$, and $E_2$ are indpendently H or -$A^E$-G;

each $A^E$ is independently absent or NHCO;

each G is independently selected from the group consisting of optionally substituted H, $C_{6-10}$ aryl, optionally sub-

stituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;

when $L_{1c}$ is a trivalent group, the oligomeric backbone is attached to the first terminus through $L_{1c}$, and each G is an end group independently selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;

when $L_{1c}$ is a divalent group, the oligomeric backbone is attached to the first terminus through one of $A^1$, $T^1$, $E_1$, and $E_2$, and each G is independently selected from the group consisting of a bond, a -$C_{1-6}$ alkylene-, -NH-$C_{0-6}$ alkylene-C(O)-, -N($CH_3$)-$C_{0-6}$ alkylene, -C(O)-, -C(O)-$C_{1-10}$alkylene, and -O-$C_{0-6}$ alkylene, optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-1}$alkylene-CH(=NH)($NR^aR^b$), -$C_{0-4}$alkylene-CH(=$N^+H_2$)($NR^aR^b$)C1-5alkylene-$NR^aR^b$, $C_{0-4}$alkylene-NHC(=NH)$R^a$, -CO-halogen, and optionally substituted amine; or

when $L_{1c}$ is a bivalent group, the oligomeric backbone is attached to the first terminus through a nitrogen or carbon atom on one of $M^1$, $M^2$, ...$M^{p-1}$, $M^p$, $T^1$, $T^2$, ...$T^{p-1}$, and $T^p$, and each G is an end group independently selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene-$NR^aR^b$, $C_{0-4}$ alkylene-NHC(=NH)$R^a$, -CO-halogen, and optionally substituted, and

each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl; each $R^{11}$ and $R^{1b}$ are independently H or an optionally substituted $C_{1-6}$ alkyl.

In some embodiments, the first terminus comprises a structure of Formula (A-4a) or (A-4b):

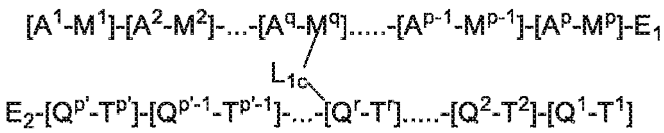

Formula (A-4a)

Or

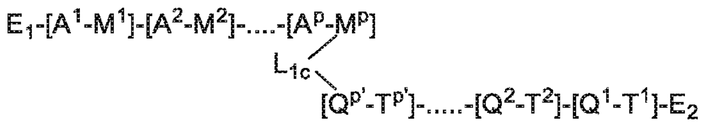

Formula (A-4b)

wherein:

$L_{1c}$ is a bivalent or trivalent group selected from

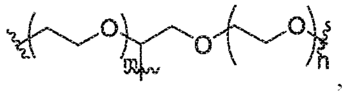

,

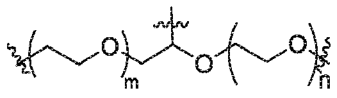,
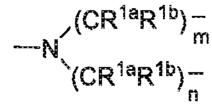,
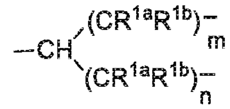,

a $C_{1-10}$ alkylene, -NH-$C_{0-6}$ alkylene-C(O)-, -N($CH_3$)-$C_{0-6}$ alkylene, and

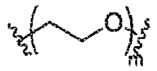;

p is an integer in the range of 2 to 10;
p' is an integer in the range of 2 to 10;

$$2 \leqq q \leqq (p-1);$$

$$2 \leqq r \leqq (p-1);$$

m and n are each independently an integer in the range of 0 to 10;
each $A^2$ through $A^p$ is independently selected from the group consisting of a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, - $C_{1-10}$alkylene-$NR^a$-, -CO-, -$NR^a$-,-CON$R^a$-,-CON$R^a$$C_{1-4}$alkylene-, -$NR^a$CO-$C_{1-4}$alkylene-, -C(O)O-, -O-, -S-, -C(=S)-NH-, -C(O)-NH-NH-, -C(O)-N=N-,-C(O)-CH=CH-, $(CH_2)_{0-4}$-CH=CH-$(CH_2)_{0-4}$, -N($CH_3$)-$C_{1-6}$ alkylene, and

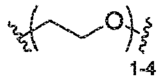;

-NH-$C_{1-6}$ alkylene-NH-, -O- $C_{1-6}$ alkylene-O-, -NH-N=N-, -NH-C(O)-NH-, and any combinations thereof, and at least one of $A^2$ through $A^p$ is -CONH-;
each $M^1$ through $M^p$ is an optionally substituted $C_{6-10}$ arylene group, optionally substituted 4-10 membered heterocyclene, optionally substituted 5-10 membered heteroarylene group, or an optionally substituted alkylene;
each $T^2$ through $T^{p'}$ in formula (A-4a) is independently selected from the group consisting of a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, -$C_{1-10}$ alkylene-$NR^a$-, -CO-, -$NR^a$-, -CON$R^a$-,-CON$R^a$$C_{1-4}$alkylene-, - $NR^a$CO-$C_{1-4}$alkylene-, -C(O)O-, -O-, -S-, -C(=S)-NH-, -C(O)-NH-NH-, -C(O)-N=N-, -C(O)-CH=CH-, $(CH_2)_{0-4}$-CH=CH-$(CH_2)_{0-4}$, -N($CH_3$)-$C_{1-6}$ alkylene, and

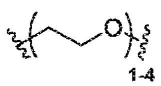;

NH- $C_{1-6}$ alkylene-NH-, -O- $C_{1-6}$ alkylene-O-, -NH-N=N-, -NH-C(O)-NH-, and any combinations thereof, and at least one of $T^2$ through $T^p$ is -CONH-;
each $Q^1$ to $Q^{p'}$ is an optionally substituted $C_{6-10}$ arylene group, optionally substituted 4-10 membered heterocyclene, optionally substituted 5-10 membered heteroarylene group, or an optionally substituted alkylene;
each $A^1$, $T^1$, $E_1$, and $E_2$ are independently H or -$A^E$-G,
each $A^E$ is independently absent or NHCO,
each G is independently selected from the group consisting of optionally substituted H, $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted

amine;

when $L_{1c}$ is a trivalent group, the oligomeric backbone is attached to the first terminus through $L_{1c}$, when $L_{1c}$ is a bivalent group, the oligomeric backbone is attached to the first terminus through one of $A^1$, $T^1$, $E_1$, and $E_2$, or the oligomeric backbone is attached to the first terminus through a nitrogen or carbon atom on one of $M^1$, $M^{2,}$ ...$M^{p-1}$, $M^p$, $T^1$, $T^{2,}$ ...$T^{p'-1}$, and $T^{p'}$, and

each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl;

each $R^{1a}$ and $R^{1b}$ are independently H or an optionally substituted $C_{1-6}$ alkyl

In certain embodiments, $L_{1a}$ is

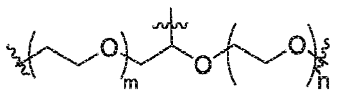

or

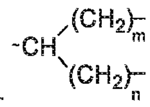

,

$C_{1-10}$ alkylene, or

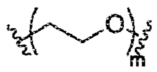

.

In certain embodiments, $L_{1c}$ is $C_{3-8}$ alkylene. In certain embodiments, $L_{1c}$ is

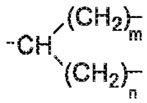

,

and wherein 2≦m+n≦10. In some embodiments, $L_{1c}$ is $C_{2-8}$ alkylene. In some embodiments, $L_{1c}$ is $C_{3-8}$ alkylene. In some embodiments, $L_{1c}$ is $C_{4-8}$ alkylene. In some embodiments, $L_{1c}$ is $C_3$ alkylene, $C_4$ alkylene, $C_5$ alkylene, $C_6$ alkylene, $C_7$ alkylene, $C_8$ alkylene, or $C_9$ alkylene.

In certain embodiments, 3≦m+n≦7. In certain embodiments, (m+n) is 3, 4, 5, 6, 7, 8, or 9. In certain embodiments, m is in the range of 3 to 8. In certain embodiments, m is 3, 4, 5, 6, 7, 8, or 9.

In certain embodiments, $M^q$ is a five to 10 membered heteroaryl ring comprising at least one nitrogen; $Q^q$ is a five to 10 membered heteroaryl ring comprising at least one nitrogen; and $M^q$ is linked to $Q^q$ through $L_{1c}$.In certain embodiments, $M^q$ is a five membered heteroaryl ring comprising at least one nitrogen; $Q^q$ is a five membered heteroaryl ring comprising at least one nitrogen; $M^q$ is linked to $Q^q$ through $L_{1c}$, and $L_{3c}$ is attached to the nitrogen atom on $M^q$ and $L_{1c}$ is attached to the nitrogen atom on $Q^q$.

In certain embodiments, each $M^1$ through $M^p$ is independently selected from an optionally substituted pyrrolylene, an optionally substituted imidazolylene, an optionally substituted pyrazolylene, an optionally substituted thioazolylene, an optionally substituted diazolylene, an optionally substituted benzopyridazinylene, an optionally substituted benzopyrazinylene, an optionally substituted phenylene, an optionally substituted pyridinylene, an optionally substituted thiophenylene, an optionally substituted furanylene, an optionally substituted piperidinylene, an optionally substituted pyrimidinylene, an optionally substituted anthracenylene, an optionally substituted quinolinylene, and an optionally substituted $C_{1-6}$ alkylene.

In certain embodiments, at least one M of $M^1$ through $M^p$ is a 5 membered heteroarylene having at least one heteroatom selected from O, N, S and optionally substituted with one or more $C_{1-10}$ alkyl. In certain embodiments, at least two M of $M^1$ through $M^p$ is a 5 membered heteroarylene having at least one heteroatom selected from O, N, S and optionally substituted with one or more $C_{1-10}$ alkyl. In certain embodiments, at least three, four, five, or six M of $M^1$ through $M^p$ is a 5 membered heteroarylene having at least one heteroatom selected from O, N, S and optionally substituted with one or more $C_{1-10}$ alkyl. In some embodiments, at least one of $M^1$ through $M^p$ is a pyrrole optionally subsituted with one or more $C_{1-10}$ alkyl. In some embodiments, at least one of $M^1$ through $M^p$ is a immidazole optionally subsituted with one or more $C_{1-10}$ alkyl. In some embodiments, at least one of $M^1$ through $M^p$ is a $C_{2-6}$ alkylene optionally substituted with one or more $C_{1-10}$ alkyl. In some embodiments, at least one of $M^1$ through $M^p$ is a phenyl optionally subsituted with one or more $C_{1-10}$ alkyl. In some embodiments, at least one of $M^1$ through $M^p$ is a bicyclic heteroarylene or arylene. In some embodiments, at least one of $M^1$ through $M^p$ is a phenylene optionally subsituted with one or more $C_{1-10}$ alkyl. In

some embodiments, at least one of $M^1$ through $M^p$ is a benzimmidazole optionally subsituted with one or more $C_{1-10}$ alkyl.

In certain embodiments, each $Q^1$ to $Q^p$ is independently selected from an optionally substituted pyrrolylene, an optionally substituted imidazolylene, an optionally substituted pyrazolylene, an optionally substituted thioazolylene, an optionally substituted diazolylene, an optionally substituted benzopyridazinylene, an optionally substituted benzopyrazinylene, an optionally substituted phenylene, an optionally substituted pyridinylene, an optionally substituted thiophenylene, an optionally substituted furanylene, an optionally substituted piperidinylene, an optionally substituted pyrimidinylene, an optionally substituted anthracenylene, an optionally substituted quinolinylene, and an optionally substituted $C_{1-6}$ alkylene.

In certain embodiments, at least one Q of Q' through $Q^p$ is a 5 membered heteroarylene having at least one heteroatom selected from O, N, S and optionally substituted with one or more $C_{1-10}$ alkyl. In certain embodiments, at least two Q of Q' through $Q^p$ is a 5 membered heteroarylene having at least one heteroatom selected from O, N, S and optionally substituted with one or more $C_{1-10}$ alkyl. In certain embodiments, at least three, four, five, or six Q of Q' through $Q^p$ is a 5 membered heteroarylene having at least one heteroatom selected from O, N, S and optionally substituted with one or more $C_{1-10}$ alkyl. In some embodiments, at least one of $Q^1$ through $Q^p$ is a pyrrole optionally subsituted with one or more $C_{1-10}$ alkyl. In some embodiments, at least one of $Q^1$ through $Q^p$ is a immidazole optionally subsituted with one or more $C_{1-10}$ alkyl. In some embodiments, at least one of Q' through $Q^p$ is a $C_{2-6}$ alkylene optionally substituted with one or more $C_{1-10}$ alkyl. In some embodiments, at least one of $Q^1$ through $Q^p$ is a phenyl optionally subsituted with one or more $C_{1-10}$ alkyl. In some embodiments, at least one of Q' through $Q^p$ is a bicyclic heteroarylene or arylene. In some embodiments, at least one of Q' through $Q^p$ is a phenylene optionally subsituted with one or more $C_{1-10}$ alkyl. In some embodiments, at least one of Q' through $Q^p$ is a benzimmidazole optionally subsituted with one or more $C_{1-10}$ alkyl.

In some embodiments, at least one of $A^2$ through $A^p$ is NH and at least one of $A^2$ through $A^p$ is C(O). In some embodiments, at least two of $A^2$ through $A^p$ is NH and at least two of $A^2$ through $A^p$ is C(O). In some embodiments, when one of $M^2$ through $M^p$ is a bicyclic ring, the adjacent A is a bond. In some embodiments, one of $A^2$ through $A^p$ is a phenylene optionally substituted with one or more alkyl. In some embodiments, one of $A^2$ through $A^p$ is thiophenylene optionally substituted with one or more alkyl. In some embodiments, one of $A^2$ through $A^p$ is a furanylene optionally substituted with one or more alkyl. In some embodiments, one of $A^2$ through $A^p$ is $(CH_2)_{0-4}$-CH=CH-$(CH_2)_{0-4}$, preferably -CH=CH-. In some embodiments, one of $A^2$ through $A^p$ is -NH-N=N-. In some embodiments, one of $A^2$ through $A^p$ is NH-C(O)-NH-. In some embodiments, one of $A^2$ through $A^p$ is -N($CH_3$)-$C_{1-6}$ alkylene. In some embodiments, one of $A^2$ through $A^p$ is

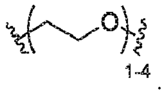

In some embodiments, one of $A^2$ through $A^p$ is NH- $C_{1-6}$ alkylene-NH-. In some embodiments, one of $A^2$ through $A^p$ is -O-$C_{1-6}$ alkylene-O-.

In certain embodiments, each $A^2$ through $A^p$ is independently selected from a bond, $C_{1-10}$ alkylene, optionally substituted phenylene, optionally substituted thiophenylene, optionally substituted furanylene, - $C_{1-10}$ alkylene-C(O)-, -$C_{1-10}$ alkylene-NH-, -CO-, -$NR^a$-, -$CONR^a$-,-$CONR^aC_{1-4}$alkylene-, - $NR^aCO$-$C_{1-4}$alkylene-, -C(O)O-, -O-, -S-, -C(=S)-NH-, -C(O)-NH-NH-, -C(O)-N=N-, —C(O)-CH=CH—, -CH=CH-, -NH-N=N-, -NH-C(O)-NH-, -N($CH_3$)-$C_{1-6}$ alkylene,

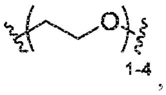

-NH- $C_{1-6}$ alkylene-NH-, and -O-$C_{1-6}$ alkylene-O-, and any combinations thereof.

In some embodiments, at least one T of $T^2$ through $T^p$ is NH and at least one of T of $T^2$ through $T^p$ is C(O). In some embodiments, at least two T of $T^2$ through $T^p$ is NH and at least two T of $T^2$ through $T^p$ is C(O). In some embodiments, when one Q of $Q^2$ through $Q^p$ is a bicyclic ring, the adjacent T is a bond. In some embodiments, one T of $T^2$ through $T^P$ is a phenylene optionally substituted with one or more alkyl. In some embodiments, one T of $T^2$ through $T^p$ is thiophenylene optionally substituted with one or more alkyl. In some embodiments, one T of $T^2$ through $T^p$ is a furanylene optionally substituted with one or more alkyl. In some embodiments, one T of $T^2$ through $T^p$ is $(CH2)_{0-4}$-CH=CH-$(CH_2)_{0-4}$, preferably -CH=CH-. In some embodiments, one T of $T^2$ through $T^p$ is -NH-N=N-. In some embodiments, one T of $T^2$ through $T^p$ is -NH-C(O)-NH-. In some embodiments, one T of $T^2$ through $T^p$ is -N($CH_3$)-$C_{1-6}$ alkylene. In some embodiments, one T of $T^2$ through $T^p$ is

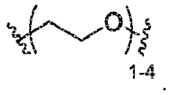

In some embodiments, one T of $T^2$ through $T^p$ is -NH- $C_{1-6}$ alkylene-NH-. In some embodiments, one T of $T^2$ through $T^p$ is -O-$C_{1-6}$ alkylene-O-.

In certain embodiments, each $T^2$ through $T^p$ is independently selected from a bond, $C_{1-10}$ alkylene, optionally substituted phenylene, optionally substituted thiophenylene, optionally substituted furanylene, - $C_{1-10}$ alkylene-C(O)-, -$C_{1-10}$ alkylene-NH-, -CO-, -$NR^a$-, -$CONR^a$-,-$CONR^aC_{1-4}$alkylene-, - $NR^aCO$-$C_{1-4}$alkylene-, -C(O)O-, -O-, -S-, -C(=S)-NH-, -C(O)-NH-NH-, -C(O)-N=N-, —C(O)-CH=CH—, -CH=CH-, -NH-N=N-, -NH-C(O)-NH-, -N($CH_3$)-$C_{1-6}$ alkylene, and

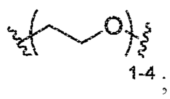

-NH-$C_{1-6}$ alkylene-NH-, -O-$C_{1-6}$ alkylene-O-, and any combinations thereof.

In certain embodiments, each $A^1$, $T^1$, $E_1$, and $E_2$ are independently -$A^E$-G, and each $A^E$ is independently absent or NHCO. In certain embodiments, each $A^1$, $T^1$, $E_1$, and $E_2$ are independently -$A^E$-G and each $A^E$ is independently NHCO.

In certain embodiments, for Formula (A-1) to (A-4), each end group G independently comprises a moiety selected from the group consisting of optionally substituted $C_{6-10}$ aryl, optionally substituted 4-10 membered heterocyclyl, a 5-10 membered heteroaryl optionally substituted with 1-3 substituents selected from $C_{1-6}$ alkyl, -NHCOH, halogen, -$NR^aR^b$, an optionally substituted $C_{1-6}$ alkyl, $C_{0-4}$ alkylene-NHC(=NH)NH, $C_{0-4}$ alkylene-NHC(=NH)-$R_E$, -$C_{1-4}$ alkylene-$R_E$, -CN, -$C_{0-4}$alkylene-CH(=NH)($NR^aR^b$), -$C_{0-4}$alkylene-CH(=$N^+H_2$)($NR^aR^b$)$C_{1-5}$ alkylene-$NR^aR^b$, $C_{0-4}$ alkylene-NHC(=NH) $R^a$, -CO-halogen, and optionally substituted amine, wherein each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl. In certain embodiments, for Formula (A-1) to (A-4), each end group G independently comprises a NH or CO group. In certain embodiments, each $R^a$ and $R^b$ are independently H or $C_{1-6}$ alkyl. In certain embodiments, for formula (A-1) to (A-4), at least one of the end groups is H. In certain embodiments, for Formula (A-1) to (A-4), at least two of the end groups are H. In certain embodiments, for formula (A-1) to (A-4), at least one of the end groups is H. In certain embodiments, for Formula (A-1) to (A-4), at least one of the end groups is NH-5-10 membered heteroaryl ring optionally substituted with one or more alkyl or -CO-5-10 membered heteroaryl ring optionally substituted with one or more alkyl.

In certain embodiments, for Formula (A-1) to (A-4), each end group G is independently selected from $C_{1-4}$alkylNHC(NH)$NH_2$,

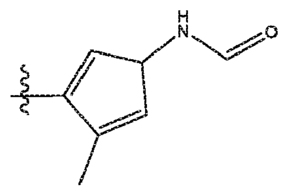

,

-CH(=NH)($NH_2$),

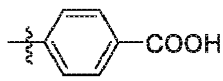

,

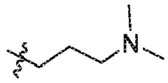 ,-CN, 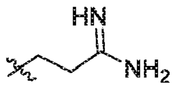 , 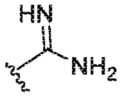 , 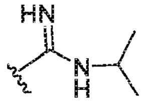 , 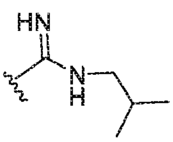 , 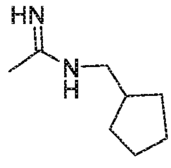 , 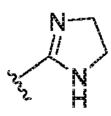

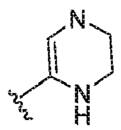, 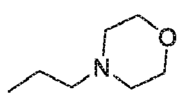, 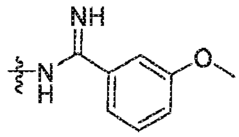, 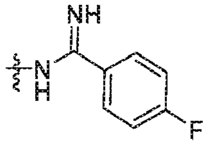, 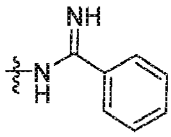,

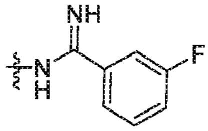, 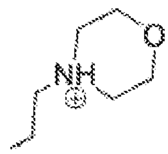, 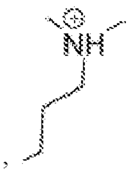, 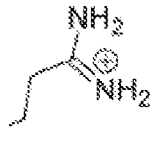, , 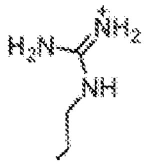, 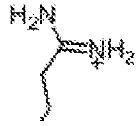,

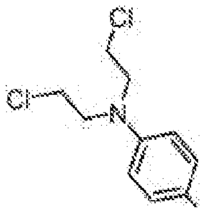, 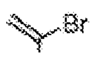, 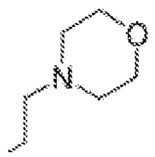, 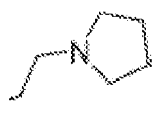, 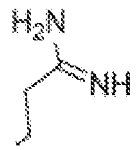, 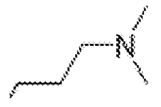,

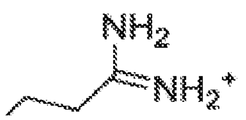, 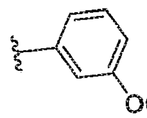, 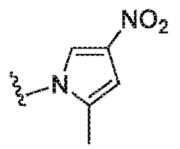, 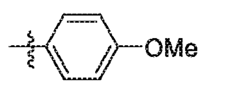, 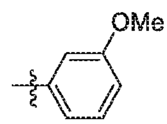,

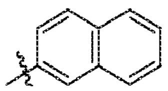, 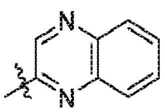, 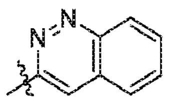, 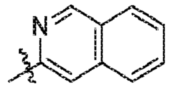, 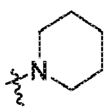, 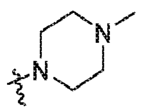, 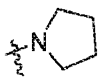,

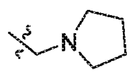, and 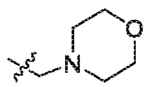.

In certain embodiments, for Formula (A-1) to (A-4), each $E_1$ independently comprises an optionally substituted thiophene-containing moiety, optionally substituted pyrrole containing moiety, optionally substituted imidazole containing moiety, or optionally substituted amine.

In certain embodiments, for Formula (A-1) to (A-4), each $E_2$ independently comprises an optionally substituted thiophene-containing moiety, optionally substituted pyrrole containing moiety, optionally substituted imidazole containing moiety, or optionally substituted amine

In certain embodiments, for Formula (A-1) to (A-4), each $E_1$ and $E_2$ independently comprises a moiety selected from the group consisting of optionally substituted N-methylpyrrole, optionally substituted N-methylimidazole, optionally substituted benzimidazole moiety, and optionally substituted 3-(dimethylamino)propanamidyl. In certain embodiments, each $E_1$ and $E_2$ independently comprises thiophene, benzothiophene, C-C linked benzimidazole/thiophene-containing moiety, or C-C linked hydroxybenzimidazole/thiophene-containing moiety. In certain embodiments, for Formula (A-1) to (A-4), each $E_1$ and $E_2$ independently also comprises NH or CO group.

In certain embodiments, for Formula (A-1) to (A-4), each $E_1$ or $E_2$ independently comprises a moiety selected from the group consisting of isophthalic acid; phthalic acid; terephthalic acid; morpholine; N,N-dimethylbenzamide; N,N-bis(trifluoromethyl)benzamide; fluorobenzene; (trifluoromethyl)benzene; nitrobenzene; phenyl acetate; phenyl 2,2,2-trifluoroacetate; phenyl dihydrogen phosphate; 2H-pyran; 2H-thiopyran; benzoic acid; isonicotinic acid; and nicotinic acid; wherein one, two, or three ring members in any of the end-group candidates can be independently substituted with C, N, S or O; and where any one, two, three, four or five of the hydrogens bound to the ring can be substituted with $R^{3a}$, wherein $R_5$ may be independently selected from H, OH, halogen, $C_{1-10}$ alkyl, $NO_2$, $NH_2$, $C_{1-10}$ haloalkyl, $-OC_{1-10}$ haloalkyl, COOH, and $CONR^{1c}R^{1d}$; wherein each $R^{1c}$ and $R^{1d}$ are independently H, $C_{1-10}$ alkyl, $C_{1-10}$ haloalkyl, or $-C_{1-10}$ alkoxyl.

In some embodiments, the first terminus comprises the structure of Formula (A-5a) or Formula (A-5b):

$A^{1a}$-NH-$Q^1$-C(O)-NH-$Q^2$-C(O)-NH-$Q^3$-C(O)... -NH-$Q^{p-1}$C(O)-NH-C(O)NH-G (A-Sa)

or

$T^{1a}$-C(O)-$Q^1$-NH-C(O)-$Q^2$NH-C(O)-$Q^3$-NH-... -C(O)-$Q^{p-1}$NH-C(O)-$Q^p$-NHC(O)-G (A-Sb)

wherein:

each $Q^1$, $Q^2$, $Q^3$... through $Q^p$ are independently an optionally substituted $C_{6-10}$ arylene group, optionally substituted 4-10 membered heterocyclene, optionally substituted 5-10 membered heteroarylene group, or an optionally substituted alkylene;

each $A^{1a}$ and $T^{1a}$ are independently a bond, H, a -$C_{1-6}$ alkylene-, -NH-$C_{0-6}$ alkylene-C(O)-, N($CH_3$)-$C_{0-6}$ alkylene, -C(O)-, -C(O)-$C_{1-10}$akylene, and -O-$C_{0-6}$ alkylene, optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene-$NR^aR^b$, $C_{0-4}$ alkylene-NHC(=NH) $R^a$, -CO-halogen, and optionally substituted amine;

p is an integer between 2 and 10; and

G is selected from the group consisting of optionally substituted $C_{6-10}$ aryl, optionally substituted 4-10 membered heterocyclyl, optionally substituted 5-10 membered heteroaryl, or an optionally substituted alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;

each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl; and

wherein the first terminus is connected to the oligomeric backbone through either A' or $T^1$, or a nitrogen or carbon atom on one of $Q^1$ through $Q^p$.

In certain embodiments, the first terminus comprises the structure of Formula (A-5c):

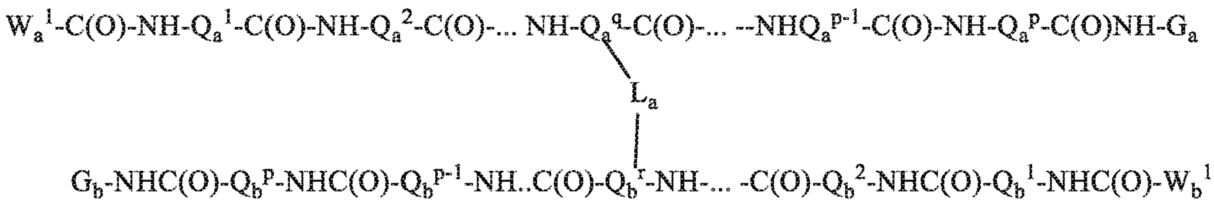

(A-5c)

wherein:

each $Q_a^1$, $Q_a^2$...$O_a^q$...through $Q_a^p$ are independently an optionally substituted $C_{6-10}$ arylene group, optionally substituted 4-10 membered heterocyclene, optionally substituted 5-10 membered heteroarylene group, or an optionally substituted alkylene;

each $Q_b^1$,$Q_b^2$...$Q_b^r$....through $Q_b^p$ are independently an optionally substituted $C_{6-10}$ arylene group, optionally substituted 4-10 membered heterocyclene, optionally substituted 5-10 membered heteroarylene group, or an optionally substituted alkylene;

p is an integer between 3 and 10;

$$2 \leqq q \leqq (p-1);$$

$$2 \leqq r \leqq (p-1);$$

$L_a$ is selected from a divalent or trivalent group selected from the group consisting of

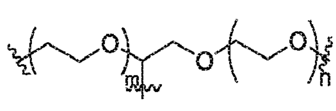, 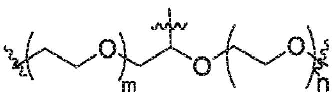, 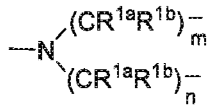,

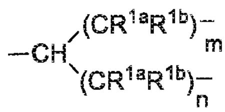,

a $C_{1-10}$ alkylene, -NH-$C_{0-6}$ alkylene-C(O)-, -N($CH_3$)-$C_{0-6}$ alkylene, and

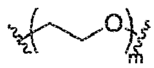;

each m and n are independently an integer in the range of 1 to 10;
n is an integer in the range of 1 to 10;
each $R^{1a}$ and $R^{1b}$ are independently H, or $C_{1-6}$ alkyl;
when $L_a$ is a trivalent group, the oligomeric backbone is attached to the first terminus through $L_a$, and each $W_a{}^1$, $G_a$, $G_b$, and $W_b{}^1$ are end groups independently selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;
when $L_a$ is a divalent group, the oligomeric backbone is attached to the first terminus through one of $W_a{}^1$, $G_a$, $G_b$, and $W_b{}^1$, and each $W_a{}^1$, $G_a$, $G_b$, and $W_b{}^1$ are independently selected from the group consisting of a bond, a -$C_{1-6}$ alkylene-, -NH-$C_{0-6}$ alkylene-C(O)-, -N($CH_3$)-$C_{0-6}$ alkylene, - C(O)-, -C(O)-$C_{1-10}$alkylene, and -O-$C_{0-6}$ alkylene, optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine; or
when $L_a$ is a bivalent group, the oligomeric backbone is attached to the first terminus through a nitrogen or carbon atom on one of $Q_a{}^1$, $Q_a{}^2$, ... $Q_a{}^{p-1}$, $Q_a{}^p$, $Q_b{}^1$, $Q_a{}^2$, ... $Q_b{}^{p-1}$, and $Q_b{}^p$, and each $W_a{}^1$, $G_a$, $G_b$, and $W_b{}^1$ are end groups independently selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene-$NR^aR^b$, $C_{0-4}$ alkylene-NHC(=NH) $R^a$, -CO-halogen, and optionally substituted amine and
each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl.

In some embodiments, the first terminus comprises the structure of Formula (A-5c) or (A-5d):

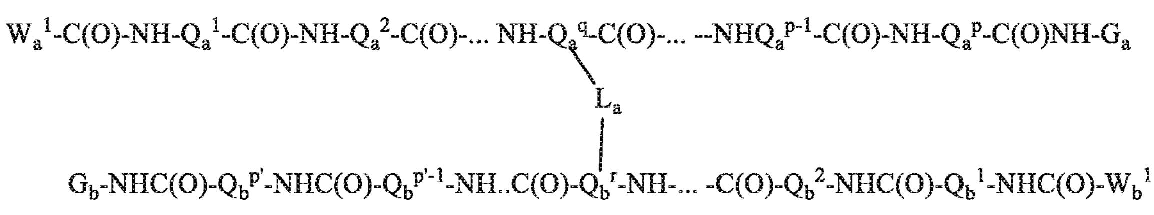 (A-5c)

or

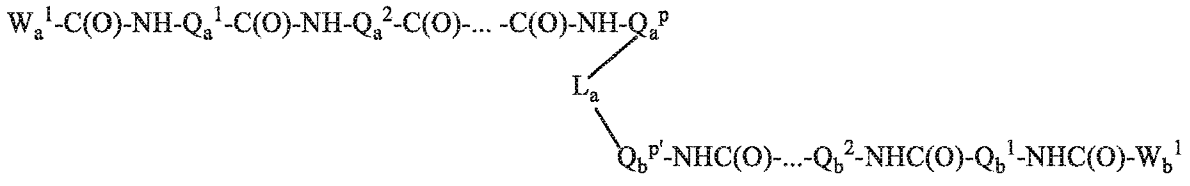

(A-5d)

wherein:

each $Q_a^1$, $Q_a^2$...$Q_a^q$...through $Q_a^p$ are independently an optionally substituted $C_{6-10}$ arylene group, optionally substituted 4-10 membered heterocyclene, optionally substituted 5-10 membered heteroarylene group, or an optionally substituted alkylene;

each $Q_b^1$,$Q_b^2$...$Q_b^r$...trough $Q_b^{p'}$ are independently an optionally substituted $C_{6-10}$ arylene group, optionally substituted 4-10 membered heterocyclene, optionally substituted 5-10 membered heteroarylene group, or an optionally substituted alkylene;

p and p' are independently an integer between 3 and 10;

$$2 \leqq q \leqq (p-1);$$

$$2 \leqq r \leqq (p-1);$$

$L_a$ is selected from a divalent or trivalent group selected from the group consisting of

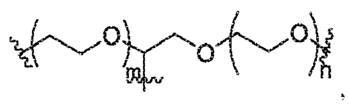,
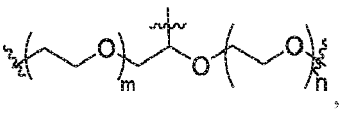,
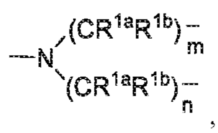,

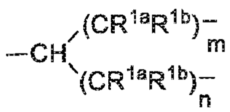,

a $C_{1-10}$ alkylene, -NH-$C_{0-6}$ alkylene-C(O)-, -N($CH_3$)-$C_{0-6}$ alkylene, and

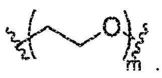;

each m and n are independently an integer in the range of 1 to 10;

n is an integer in the range of 1 to 10;

each $R^{1a}$ and $R^{1b}$ are independently H, or $C_{1-6}$ alkyl;

each $W_a^1$, $G_a$, $G_b$, and $W_b^1$ are end groups independently selected from the group consisting of optionally substituted H, $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;

when $L_a$ is a trivalent group, the oligomeric backbone is attached to the first terminus through L,; and when $L_a$ is a divalent group, the oligomeric backbone is attached to the first terminus through one of $W_a^1$, $E_a$, $E_b$, and $W_b^1$, or the oligomeric backbone is attached to the first terminus through a nitrogen or carbon atom on one of $Q_a^1$, $Q_a^2$, ... $Q_a^{p-1}$, $Q_a^p$, $Q_b^1$, $Q_a^2$, ... $Q_b^{p'-1}$, and $Q_b^{p'}$; and

each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted

5-10 membered heteroaryl.

In certain embodiements of Formula (A-5c)-(A-5d), $L_a$ is a $C_{2-8}$ alkylene. In certain embodiments, $L_a$ is $C_{3-8}$ alkylene. In certain embodiments, $L_a$ is

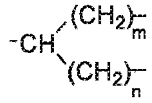

,

and wherein $2 \leqq m+n \leqq 10$. In some embodiments, $L_a$ is $C_{4-8}$ alkylene. In some embodiments, $L_a$ is $C_{3-7}$ alkylene. In some embodiments, $L_a$ is $C_3$ alkylene, $C_4$ alkylene, $C_5$ alkylene, $C_6$ alkylene, $C_7$ alkylene, $C_8$ alkylene, or $C_9$ alkylene.

In certain embodiments, for Formula (A-5c)-(A-5d), $3 \leqq m+n \leqq 7$. In certain embodiments, (m+n) is 3, 4, 5, 6, 7, 8, or 9. In certain embodiments, m is in the range of 3 to 8. In certain embodiments, m is 3, 4, 5, 6, 7, 8, or 9. In certain embodiments, for Formula (A-5c), p is 2-10. In certain embodiments, for formula (A-5c), p is 3-8. In certain embodiments, for formula (A-5c), p is 2, 3, 4, 5, 6, 7, or 8. In certain embodiments, for Formula (A-5c), q is 2-5. In certain embodiments, for formula (A-5c), p is 2-4. In certain embodiments, for Formula (A-5c), p is 2, 3, 4, 5, or 6.

In certain embodiments, $Q_a^q$ is a five to 10 membered heteroaryl ring comprising at least one nitrogen; $Q_b^{q'}$ is a five to 10 membered heteroaryl ring comprising at least one nitrogen; and $Q_a^q$ is linked to $Q_b^r$ through $L_a$.In certain embodiments, $Q_a^q$ is a five membered heteroaryl ring comprising at least one nitrogen; $Q_b^r$ is a five membered heteroaryl ring comprising at least one nitrogen; $Q_a^q$ is linked to $Q_b^r$ through $L_a$, and $L_a$ is attached to the nitrogen atom on $Q_a^q$ and $L_{1c}$ is attached to the nitrogen atom on $Q_b^r$.

In certain embodiments, each $Q_a^1$ through $Q_a^p$ is independently selected from an optionally substituted pyrrolylene, an optionally substituted imidazolylene, an optionally substituted pyrazolylene, an optionally substituted thioazolylene, an optionally substituted diazolylene, an optionally substituted benzopyridazinylene, an optionally substituted benzopyrazinylene, an optionally substituted phenylene, an optionally substituted pyridinylene, an optionally substituted thiophenylene, an optionally substituted furanylene, an optionally substituted piperidinylene, an optionally substituted pyrimidinylene, an optionally substituted anthracenylene, an optionally substituted quinolinylene, and an optionally substituted $C_{1-6}$ alkylene.

In certain embodiments, at least one Q of $Q_a^1$ through $Q_a^p$ is a 5 membered heteroarylene having at least one heteroatom selected from O, N, S and optionally substituted with one or more $C_{1-10}$ alkyl. In certain embodiments, at least two Q of $Q_a^1$ through $Q_a^p$ is a 5 membered heteroarylene having at least one heteroatom selected from O, N, S and optionally substituted with one or more $C_{1-10}$ alkyl. In certain embodiments, at least three, four, five, or six Q of $Q_a^1$ through $Q_a^p$ is a 5 membered heteroarylene having at least one heteroatom selected from O, N, S and optionally substituted with one or more $C_{1-10}$ alkyl. In some embodiments, at least one Q of $Q_a^1$ through $Q_a^p$ is a pyrrole optionally subsituted with one or more $C_{1-10}$ alkyl. In some embodiments, at least one of Q of $Q_a^1$ through $Q_a^p$ is a immidazole optionally subsituted with one or more $C_{1-10}$ alkyl. In some embodiments, at least one Q of $Q_a^1$ through $Q_a^p$ is a $C_{2-6}$ alkylene optionally substituted with one or more $C_{1-10}$ alkyl. In some embodiments, at least one Q of $Q_a^1$ through $Q_a^p$ is a phenyl optionally subsituted with one or more $C_{1-10}$ alkyl. In some embodiments, at least one Q of $Q_a^1$ through $Q_a^p$ is a bicyclic heteroarylene or arylene. In some embodiments, at least one Q of $Q_a^1$ through $Q_a^p$ is a phenylene optionally subsituted with one or more $C_{1-10}$ alkyl. In some embodiments, at least one Q of $Q_a^1$ through $Q_a^p$ is a benzimmidazole optionally subsituted with one or more $C_{1-10}$ alkyl.

In certain embodiments, each $Q_b^1$ through $Q_b^p$ is independently selected from an optionally substituted pyrrolylene, an optionally substituted imidazolylene, an optionally substituted pyrazolylene, an optionally substituted thioazolylene, an optionally substituted diazolylene, an optionally substituted benzopyridazinylene, an optionally substituted benzopyrazinylene, an optionally substituted phenylene, an optionally substituted pyridinylene, an optionally substituted thiophenylene, an optionally substituted furanylene, an optionally substituted piperidinylene, an optionally substituted pyrimidinylene, an optionally substituted anthracenylene, an optionally substituted quinolinylene, and an optionally substituted $C_{1-6}$ alkylene.

In certain embodiments, at least one Q of $Q_b^1$ through $Q_b^{p'}$ is a 5 membered heteroarylene having at least one heteroatom selected from O, N, S and optionally substituted with one or more $C_{1-10}$ alkyl. In certain embodiments, at least two Q of $Q_b^1$ through $Q_b^{p'}$ is a 5 membered heteroarylene having at least one heteroatom selected from O, N, S and optionally substituted with one or more $C_{1-10}$ alkyl. In certain embodiments, at least three, four, five, or six Q of $Q_b^1$ through $Q_b^{p'}$ is a 5 membered heteroarylene having at least one heteroatom selected from O, N, S and optionally substituted with one or more $C_{1-10}$ alkyl. In some embodiments, at least one of $Q_b^1$ through $Q_b^{p'}$ is a pyrrole optionally subsituted with one or more $C_{1-10}$ alkyl. In some embodiments, at least one of $Q_b^1$ through $Q_b^{p'}$ is a immidazole optionally subsituted with one or more $C_{1-10}$ alkyl. In some embodiments, at least one of $Q_b^1$ through $Q_b^{p'}$ is a $C_{2-6}$ alkylene optionally substituted with one or more $C_{1-10}$ alkyl. In some embodiments, at least one of $Q_b^1$ through $Q_b^{p'}$ is a phenyl

optionally subsituted with one or more $C_{1-10}$ alkyl. In some embodiments, at least one of $Q_b^1$ through $Q_b^{p'}$ is a bicyclic heteroarylene or arylene. In some embodiments, at least one of $Q_b^1$ through $Q_b^{p'}$ is a phenylene optionally subsituted with one or more $C_{1-10}$ alkyl. In some embodiments, at least one of $Q_b^1$ through $Q_b^{p'}$ is a benzimmidazole optionally subsituted with one or more $C_{1-10}$ alkyl.

In certain embodiments, for Formula (A-5c), each end group $G_a$, $G_b$, $W_a^1$, and $W_b^1$ is independently selected from the group consisting of optionally substituted $C_{6-10}$ aryl, optionally substituted 4-10 membered heterocyclyl, a 5-10 membered heteroaryl optionally substituted with 1-3 substituents selected from $C_{1-6}$ alkyl, -NHCOH, halogen, $-NR^aR^b$, an optionally substituted $C_{1-6}$ alkyl, $C_{0-4}$ alkylene-NHC(=NH)NH, $C_{0-4}$ alkylene-NHC(=NH)-$R^a$, -$C_{1-4}$ alkylene-$R^a$, -CN, -$C_{0-4}$alkylene-CH(=NH)($NR^aR^b$), -$C_{0-4}$alkylene-CH(=$N^+H_2$)($NR^aR^b$)$C_{1-5}$ alkylene-$NR^aR^b$, $C_{0-4}$ alkylene-NHC(=NH) $R^a$, -CO-halogen, and optionally substituted amine, wherein each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl. In certain embodiments, each $R^a$ and $R^b$ are independently H or $C_{1-6}$ alkyl. In certain embodiments, at least one of the end groups is 5-10 membered heteroaryl optionally subsittuted with $C_{1-6}$ alkyl, COOH, or OH. In certain embodiments, at least two of the end groups are 5-10 membered heteroaryl optionally subsittuted with $C_{1-6}$ alkyl, COOH, or OH. In certain embodiments, for Formula (A-1) to (A-5d), at least one of the end groups is 5-10 membered heteroaryl optionally subsittuted with $C_{1-6}$ alkyl, COOH, or OH. In certain embodiments, at least one of the end groups is 5-10 membered heteroaryl ring optionally substituted with one or more alkyl.

In some embodiments, $A^E$ is absent. In some embodiments, $A^E$ is NHCO-.

In some embodiments, the first terminus comprises at least one $C_{3-5}$ achiral aliphatic or heteroaliphatic amino acid.

In some embodiments, the first terminus comprises one or more subunits selected from the group consisting of optionally substituted pyrrole, optionally substituted imidazole, optionally substituted thiophene, optionally substituted furan, optionally substituted beta-alanine, γ-aminobutyric acid, (2-aminoethoxy)-propanoic acid, 3((2-aminoethyl)(2-oxo-2-phenyl-1$\lambda^2$-ethyl)amino)-propanoic acid, or dimethylaminopropylamide monomer.

In some embodiments, the first terminus comprises a polyamide having the structure of Formula (A-6)

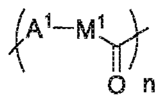

(A-6)

wherein:

each $A^1$ is NH- or -NH-$(CH_2)_m$-$CH_2$-C(O)-NH-;
each M is an optionally substituted $C_{6-10}$ arylene group, optionally substituted 4-10 membered heterocyclene, optionally substituted 5-10 membered heteroarylene group, or optionally substituted alkylene;
m is an integer between 1 to 10; and
n is an integer between 1 and 6.

In some embodiments, each $M^1$ in [$A^1$-$M^1$] of Formula (A-6) is a $C_{6-10}$ arylene group, 4-10 membered heterocyclene, optionally substituted 5-10 membered heteroarylene group, or $C_{1-6}$ alkylene; each optionally substituted by 1-3 substituents selected from H, OH, halogen, $C_{1-10}$ alkyl, $NO_2$, CN, NR'R", $C_{1-6}$ haloalkyl, -$C_{1-6}$ alkoxyl, $C_{1-6}$ haloalkoxy, ($C_{1-6}$ alkoxy)$C_{1-6}$ alkyl, $C_{2-10}$alkenyl, $C_{2-10}$alkynyl, $C_{3-7}$ carbocyclyl, 4-10 membered heterocyclyl4-10 membered heterocyclyl, $C_{6-10}$aryl, 5-10 membered heteroaryl, -($C_{3-7}$carbocyclyl)$C_{1-6}$alkyl, (4-10 membered heterocyclyl4-10 membered heterocyclyl)$C_{1-6}$alkyl, ($C_{6-10}$aryl)$C_{1-6}$alkyl, ($C_{6-10}$aryl)$C_{1-6}$alkoxy, (5-10 membered heteroaryl)$C_{1-6}$alkyl, -($C_{3-7}$carbocyclyl)-amine, (4-10 membered heterocyclyl)amine, ($C_{6-10}$aryl)amine, (5-10 membered heteroaryl)amine, acyl, C-carboxy, O-carboxy, C-amido, N-amido, S-sulfonamido, N-sulfonamido, -SR', COOH, or CONR'R"; wherein each R' and R" are independently H, $C_{1-10}$ alkyl, $C_{1-10}$ haloalkyl, -$C_{1-10}$ alkoxyl. In some embodiments, each $R^1$ in [$A^1$-$R^1$] of Formula (A-6) is a 5-10 membered heteroarylene containing at least one heteroatoms selected from O, S, and N or a $C_{1-6}$ alkylene, and the heteroarylene or the a $C_{1-6}$ alkylene is optionally substituted with 1-3 substituents selected from OH, halogen, $C_{1-10}$ alkyl, $NO_2$, CN, NR'R", $C_{1-6}$ haloalkyl, -$C_{1-6}$ alkoxyl, $C_{1-6}$ haloalkoxy, $C_{3-7}$ carbocyclyl, 4-10 membered heterocyclyl, $C_{6-10}$aryl, 5-10 membered heteroaryl, -SR', COOH, or CONR'R"; wherein each R' and R" are independently H, $C_{1-10}$ alkyl, $C_{1-10}$ haloalkyl, -$C_{1-10}$ alkoxyl. In some embodiments, each $R^1$ in [$A^1$-$R^1$] of Formula (A-6) is a 5-10 membered heteroarylene containing at least one heteroatoms selected from O, S, and N, and the heteroarylene is optionally substituted with 1-3 substituents selected from OH, $C_{1-6}$ alkyl, halogen, and $C_{1-6}$ alkoxyl.

In some embodiments, the first terminus has a structure of Formula (A-7):

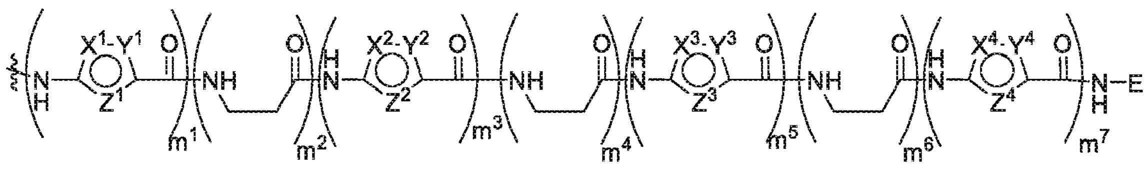

(A-7)

or a salt thereof, wherein:

E is an end subunit which comprises a moiety chosen from a heterocyclic group or a straight chain aliphatic group, which is chemically linked to its single neighbor;
$X^1$, $Y^1$, and $Z^1$ in each $m^1$ unit are independently selected from $CR^4$, N, $NR^5$, O, or S;
$X^2$, $Y^2$, and $Z^2$ in each $m^3$ unit are independently selected from $CR^4$, N, $NR^5$, O, or S;
$X^3$, $Y^3$, and $Z^3$ in each $m^5$ unit are independently selected from $CR^4$, N, $NR^5$, O, or S;
$X^4$, $Y^4$, and $Z^4$ in each $m^7$ unit are independently selected from $CR^4$, N, $NR^5$, O, or S;
each $R^4$ is independently H, -OH, halogen, $C_{1-6}$ alkyl, $C_{1-6}$ alkoxyl;
each $R^5$ is independently H, $C_{1-6}$ alkyl or $C_{1-6}$alkylamine;
each $m^1$, $m^3$, $m^5$ and $m^7$ are independently an integer between 0 and 5;
each $m^2$, $m^4$ and $m^6$ are independently an integer between 0 and 3; and
$m^1 + m^2 + m^3 + m^4 + m^5 + m^6 + m^7$ is between 3 and 15.

In some embodiments, $m^1$ is 3, and $X^1$, $Y^1$, and $Z^1$ in the first unit is respectively CH, $N(CH_3)$, and CH; $X^1$, $Y^1$, and $Z^1$ in the second unit is respectively CH, $N(CH_3)$, and N; and $X^1$, $Y^1$, and $Z^1$ in the third unit is respectively CH, $N(CH_3)$, and N. In some embodiments, $m^3$ is 1, and $X^2$, $Y^2$, and $Z^2$ in the first unit is respectively CH, $N(CH_3)$, and CH. In some embodiments, $m^5$ is 2, and $X^3$, $Y^3$, and $Z^3$ in the first unit is respectively CH, $N(CH_3)$, and N; $X^3$, $Y^3$, and $Z^3$ in the second unit is respectively CH, $N(CH_3)$, and N. In some embodiments, $m^7$ is 2, and $X^4$, $Y^4$, and $Z^4$ in the first unit is respectively CH, $N(CH_3)$, and CH; $X^4$, $Y^4$, and $Z^4$ in the second unit is respectively CH, $N(CH_3)$, and CH. In some embodiments, each $m^2$, $m^4$ and $m^6$ are independently 0 or 1. In some embodiments, each of the $X^1$, $Y^1$, and $Z^1$ in each $m^1$ unit are independently selected from CH, N, or $N(CH_3)$. In some embodiments, each of the $X^2$, $Y^2$, and $Z^2$ in each $m^3$ unit are independently selected from CH, N, or $N(CH_3)$. In some embodiments, each of the $X^3$, $Y^3$, and $Z^3$ in each $m^5$ unit are independently selected from CH, N, or $N(CH_3)$. In some embodiments, each of the $X^4$, and $Z^4$ in each $m^7$ unit are independently selected from CH, N, or $N(CH_3)$. In some embodiments, each $Z^1$ in each $m^1$ unit is independently selected from $CR^4$ or $NR^5$. In some embodiments, each $Z^2$ in each $m^3$ unit is independently selected from $CR^4$ or $NR^5$. In some embodiments, each $Z^3$ in each $m^5$ unit is independently selected from $CR^4$ or $NR^5$. In some embodiments, each $Z^4$ in each $m^7$ unit is independently selected from $CR^4$ or $NR^5$. In some embodiments, $R^4$ is H, $CH_3$, or OH. In some embodiments, $R^5$ is H or $CH_3$.

In some embodiments, for Formula (A-7), the sum of $m^2$, $m^4$ and $m^6$ is between 1 and 6. In some embodiments, for formula (A-7), the sum of $m^2$, $m^4$ and $m^6$ is between 2 and 6. In some embodiments, for Formula (A-7), the sum of $m^1$, $m^3$, $m^5$ and $m^7$ is between 2 and 10. In some embodiments, the sum of $m^1$, $m^3$, $m^5$ and $m^7$ is between 3 and 8. In some embodiments, for Formula (A-7), ($m^1 + m^2 + m^3 + m^4 + m^5 + m^6 + m^7$) is between 3 and 12. In some embodiments, ($m^1 + m^2 + m^3 + m^4 + m^5 + m^6 + m^7$) is between 4 and 10.

In some embodiments, for Formula (A-1) to (A-7), the first terminus comprises at least one beta-alanine moiety. In some embodiments, for Formula (A-1) to (A-7), the first terminus comprises at least two beta-alanine moieties. In some embodiments, for Formula (A-1) to (A-7), the first terminus comprises at least three or four beta-alanine moieties.

In some embodiments, the first terminus has the structure of Formula (A-8):

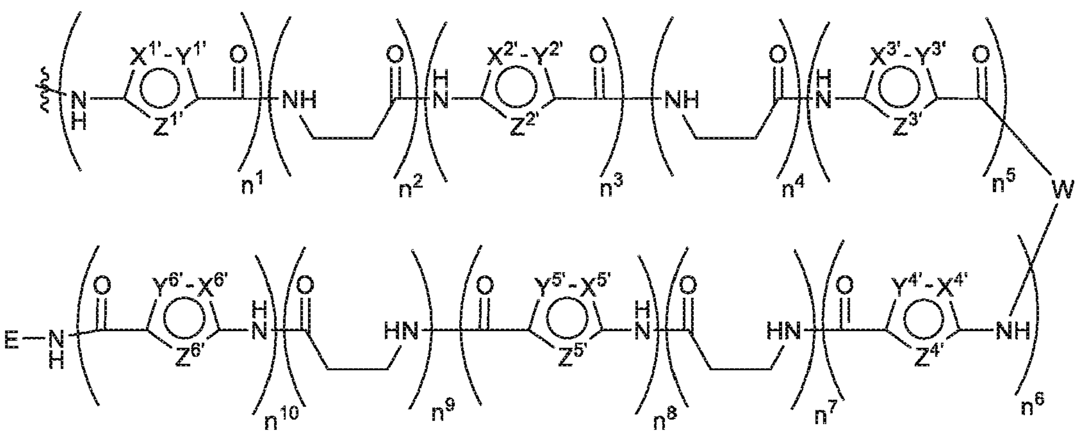

(A-8)

or a salt thereof, wherein:

E is an end subunit which comprises a moiety chosen from a heterocyclic group or a straight chain aliphatic group, which is chemically linked to its single neighbor;
W is $C_{1-6}$ alkylene,

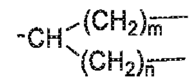 , or 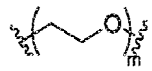 ;

$X^{1'}$, $Y^{1'}$, and $Z^{1'}$ in each $n^1$ unit are independently selected from $CR^4$, N, $NR^5$, O, or S;
$X^{2'}$, $Y^{2'}$, and $Z^{2'}$ in each $n^3$ unit are independently selected from $CR^4$, N, $NR^5$, O, or S;
$X^{3'}$, $Y^{3'}$, and $Z^{3'}$ in each $n^5$ unit are independently selected from $CR^4$, N, $NR^5$, O, or S;
$X^{4'}$, and $Z^{4'}$ in each $n^6$ unit are independently selected from $CR^4$, N, $NR^5$, O, or S;
$X^{5'}$, $Y^{5'}$, and $Z^{5'}$ in each $n^8$ unit are independently selected from $CR^4$, N, $NR^5$, O, or S;
$X^{6'}$, $Y^{6'}$, and $Z^{6'}$ in each $n^{10}$ unit are independently selected from $CR^4$, N, $NR^5$, O, or S;
each $R^4$ is independently H, -OH, halogen, $C_{1-6}$ alkyl, $C_{1-6}$ alkoxyl;
each $R^5$ is independently H, $C_{1-6}$ alkyl or $C_{1-6}$alkylaminen is an integer between 1 and 5;
each $n^1$, $n^3$, $n^5$, $n^6$, $n^8$ and $n^{10}$ are independently an integer between 0 and 5;
each $n^2$, $n^4$, $n^7$ and $n^9$ are independently an integer between 0 and 3, and
$n^1 + n^2 + n^3 + n^4 + n^5 + n^6 + n^7 + n^8 + n^9 + n^{10}$ is between 3 and 15.

In some embodiments, for Formula (A-8), the sum of $n^2$, $n^4$, $n^7$ and $n^9$ is between 1 and 6. In some embodiments, for Formula (A-8), the sum of $n^2$, $n^4$, $n^7$ and $n^9$ is between 2 and 6. In some embodiments, for Formula (A-8), the sum of $n^1$, $n^3$, $n^5$, $n^6$, $n^8$ and $n^{10}$ is between 3 and 13. In some embodiments, the sum of $n^1$, $n^3$, $n^5$, $n^6$, $n^8$ and $n^{10}$ is between 4 and 10. In some embodiments, for Formula (A-8), ($n^1 + n^2 + n^3 + n^4 + n^5 + n^6 + n^7 + n^8 + n^9 + n^{10}$) is between 3 and 12. In some embodiments, ($n^1 + n^2 + n^3 + n^4 + n^5 + n^6 + n^7 + n^8 + n^9 + n^{10}$) is between 4 and 10.

In some embodiments, $n^1$ is 3, and $X^{1'}$, $Y^{1'}$, and $Z^{1'}$ in the first unit is respectively CH, $N(CH_3)$, and CH; $X^{1'}$, $Y^{1'}$, and $Z^{1'}$ in the second unit is respectively CH, $N(CH_3)$, and N; and $X^{1'}$, $Y^{1'}$, and $Z^{1'}$ in the third unit is respectively CH, $N(CH_3)$, and N. In some embodiments, $n^3$ is 1, and $X^{2'}$, $Y^{2'}$, and $Z^{2'}$ in the first unit is respectively CH, $N(CH_3)$, and CH. In some embodiments, $n^5$ is 2, and $X^{3'}$, $Y^{3'}$, and $Z^{3'}$ in the first unit is respectively CH, $N(CH_3)$, and N; $X^{3'}$, $Y^{3'}$, and $Z^{3'}$ in the second unit is respectively CH, $N(CH_3)$, and N. In some embodiments, $n^6$ is 2, and $X^{4'}$, and $Z^{4'}$ in the first unit is respectively CH, $N(CH_3)$, and N; $X^{4'}$, $Y^{4'}$, and $Z^{4'}$ in the second unit is respectively CH, $N(CH_3)$, and N. In some embodiments, the $X^{1'}$, $Y^{1'}$, and $Z^{1'}$ in each $n^1$ unit are independently selected from CH, N, or $N(CH_3)$. In some embodiments, the $X^{2'}$, $Y^{2'}$, and $Z^{2'}$ in each $n^3$ unit are independently selected from CH, N, or $N(CH_3)$. In some embodiments, the $X^{3'}$, $Y^{3'}$, and $Z^{3'}$ in each $n^5$ unit are independently selected from CH, N, or $N(CH_3)$. In some embodiments, the $X^{4'}$, $Y^{4'}$, and $Z^{4'}$ in each $n^6$ unit are independently selected from CH, N, or $N(CH_3)$. In some embodiments, the $X^{5'}$, $Y^{5'}$, and $Z^{5'}$ in each $n^8$ unit are independently selected from CH, N, or $N(CH_3)$. In some embodiments, the $X^{6'}$, $Y^{6'}$, and $Z^{6'}$ in each $n^{10}$ unit are independently selected from CH, N, or $N(CH_3)$. In some embodiments, each $Z^{1'}$ in each $n^1$ unit is independently selected from $CR^1$ or $NR^2$. In some embodiments, each $Z^{2'}$ in each $n^3$ unit is independently selected from $CR^4$ or $NR^5$. In some embodiments, each $Z^{3'}$ in each $n^5$ unit is independently selected from $CR^4$ or $NR^5$. In some

embodiments, each $Z^{4'}$ in each $n^6$ unit is independently selected from $CR^1$ or $NR^2$. In some embodiments, each $Z^{5'}$ in each $n^8$ unit is independently selected from $CR^1$ or $NR^2$. In some embodiments, each $Z^{6'}$ in each $n^{10}$ unit is independently selected from $CR^4$ or $NR^5$. In some embodiments, $R^4$ is H, $CH_3$, or OH. In some embodiments, $R^5$ is H or $CH_3$.

In some embodiments, the first terminus has the structure of Formula (A-9):

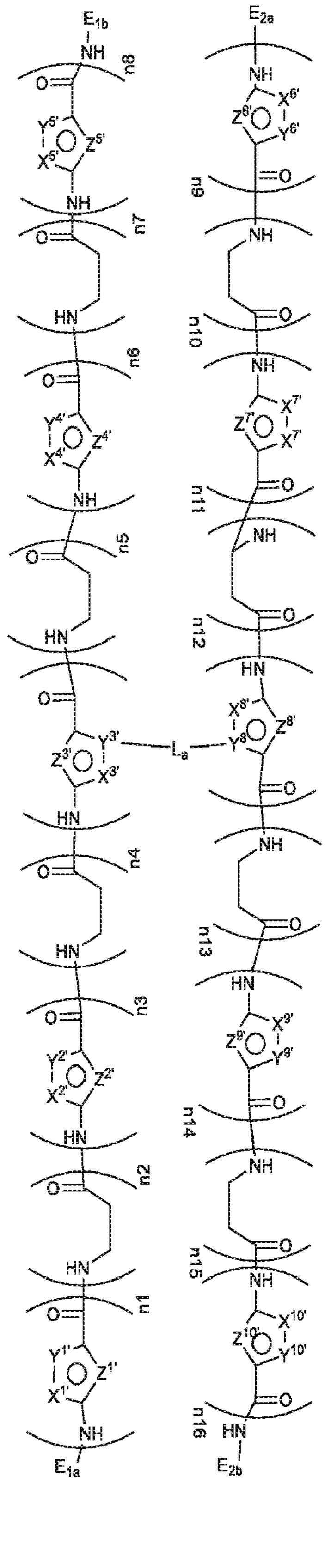

(A-9)

or a salt thereof, wherein:

$X^{1'}$, $Y^{1'}$, and $Z^{1'}$ in each $n^1$ unit are independently selected from $CR^4$, N, $NR^5$, O, or S;
$X^{2'}$, $Y^{2'}$, and $Z^{2'}$ in each $n^3$ unit are independently selected from $CR^4$, N, $NR^5$, O, or S;
$X^{3'}$, $Y^{3'}$, and $Z^{3'}$ are independently selected from $CR^4$, N, $NR^5$, O, or S;
$X^{4'}$, $Y^{4'}$, and $Z^{4'}$ in each $n^6$ unit are independently selected from $CR^4$, N, $NR^5$, O, or S;
$X^{5'}$, $Y^{5'}$, and $Z^{5'}$ in each $n^8$ unit are independently selected from $CR^4$, N, $NR^5$, O, or S;
$X^{6'}$, $Y^{6'}$, and $Z^{6'}$ in each $n^9$ unit are independently selected from $CR^4$, N, $NR^5$, O, or S;
$X^{7'}$, $Y^{7'}$, and $Z^{7'}$ in each $n^{11}$ unit are independently selected from $CR^4$, N, $NR^5$, O, or S;
$X^{8'}$, $Y^{8'}$, and $Z^{8'}$ are independently selected from $CR^4$, N, $NR^5$, O, or S;
$X^{9'}$, $Y^{9'}$, and $Z^{9'}$ in each $n^{14}$ unit are independently selected from $CR^4$, N, $NR^5$, O, or S;
$X^{10'}$, $Y^{10'}$, and $Z^{10'}$ in each $n^{16}$ unit are independently selected from $CR^4$, N, $NR^5$, O, or S;
each $R^4$ is independently H, -OH, halogen, $C_{1-6}$ alkyl, $C_{1-6}$ alkoxyl;
each $R^5$ is independently H, $C_{1-6}$ alkyl or $C_{1-6}$alkylamine;
each $n^1$, $n^3$, $n^6$, $n^8$, $n^9$, $n^{11}$, $n^{14}$, and $n^{16}$ are independently an integer between 0 and 5;
each $n^2$, $n^4$, $n^5$, $n^7$, $n^{10}$, $n^{13}$, and $n^{15}$ are independently an integer between 0 and 3,
$n^1 + n^2 + n^3 + n^4 + n^5 + n^6 + n^7 + n^8 + n^9 + n^{10} + n^{11} + n^{12} + n^{13} + n^{14} + n^{15} + n^{16}$ is between 3 and 18
or a salt thereof, wherein:

$L_a$ is selected from a divalent or trivalent group selected from the group consisting of

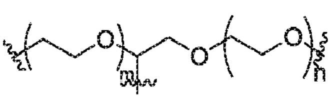,
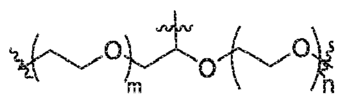,
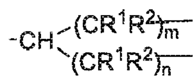,
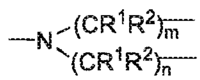,

a $C_{1-10}$ alkylene, -NH-$C_{0-6}$ alkylene-C(O)-, -N($CH_3$)-$C_{0-6}$ alkylene, and

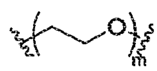;

each $R^{1a}$ and $R^{1b}$ are independently H, or an $C_{1-6}$ alkyl;
each m and n are independently an integer between 1 and 10;
when $L_a$ is a trivalent group, the oligomeric backbone is attached to the first terminus through $L_a$, and each $E_{1a}$, $E_{2a}$, $E_{1b}$, and $E_{2b}$ are end groups independently selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, and optionally substituted amine;
when $L_a$ is a divalent group, the oligomeric backbone is attached to the first terminus through one of $B_{1a}$, $B_{2a}$, $E_{1b}$, and $E_{2b}$, and each $E_{1a}$, $E_{2a}$, $E_{1b}$, and $E_{2b}$ are independently selected from the group consisting of a bond, a -$C_{1-6}$ alkylene-, -NH-$C_{0-6}$ alkylene-C(O)-, -N($CH_3$)-$C_{0-6}$ alkylene, -C(O)-, -C(O)-$C_{1-10}$alkylene, and - O-$C_{0-6}$ alkylene, optionally substituted $C_{6-10}$ aryl, optionally substituted 4-10 membered heterocyclyl, optionally substituted 5-10 membered heteroaryl, an optionally substituted $C_{1-6}$ alkyl, and optionally substituted amine; or
when $L_a$ is a bivalent group, the oligomeric backbone is attached to the first terminus through a nitrogen or carbon atom on one of five-membered heteroaryl rings, and each $E_{1a}$, $E_{2a}$, $E_{1b}$, and $E_{2b}$ are end groups independently selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, and optionally substituted amine.

In some embodiments, the first terminus comprises a polyamide having the structure of Formula (A-10):

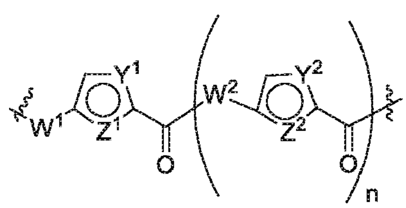
(A-10)

wherein:

each $Y^1$, $Y^2$, $Z^1$, and $Z^2$ are independently $CR^4$, N, $NR^5$, O, or S;
each $R^4$ is independently H, -OH, halogen, $C_{1-6}$ alkyl, or $C_{1-6}$ alkoxyl;
each $R^5$ is independently H, $C_{1-6}$ alkyl, or $C_{1-6}$ alkylamine ;
each $W^1$ and $W^2$ are independently a bond, NH, a $C_{1-6}$ alkylene, -NH-$C_{1-6}$ alkylene, -NH-5-10 membered heteroarylene, -NH-5-10 membered heterocyclene, -N($CH_3$)-$C_{0-6}$ alkylene, -C(O)-, - C(O)-$C_{1-10}$alkylene, or -O-$C_{0-6}$ alkylene; and
n is an integer between 2 and 11.

In some embodiments, each $R^4$ is independently H, -OH, halogen, $C_{1-6}$ alkyl, $C_{1-6}$ alkoxyl; and each $R^2$ is independently H, $C_{1-6}$ alkyl or $C_{1-6}$alkylamine. In some embodiments, each $R^4$ is selected from the group consisting of H, COH, Cl, NO, N-acetyl, benzyl, $C_{1-6}$ alkyl, $C_{1-6}$ alkoxyl, $C_{1-6}$ alkenyl, $C_{1-6}$ alkynyl, $C_{1-6}$ alkylamine, -C(O)NH-$(CH_2)_{1-4}$-C(O)NH -$(CH_2)_{1-4}$-$NR^aR^b$; and each $R^a$ and $R^b$ are independently hydrogen or $C_{1-6}$ alkyl.

In dome embodiments, $R^5$ is independently selected from the group consisting of H, $C_{1-6}$ alkyl, and $C_{1-6}$ alkyl$NH_2$, preferably H, methyl, or isopropyl.

In some embodiments, $R^4$ in Formula (A-7) to (A-8) is independently selected from H, OH, $C_{1-6}$ alkyl, halogen, and $C_{1-6}$ alkoxyl. In some embodiments, $R^4$ in Formula (A-7) to (A-8) is selected from H, OH, halogen, $C_{1-10}$ alkyl, $NO_2$, CN, NR'R", $C_{1-6}$ haloalkyl, -$C_{1-6}$ alkoxyl, $C_{1-6}$ haloalkoxy, ($C_{1-6}$ alkoxy)$C_{1-6}$ alkyl, $C_{2-10}$alkenyl, $C_{2-10}$alkynyl, $C_{3-7}$ carbocyclyl, 4-10 membered heterocyclyl, $C_{1-10}$aryl, 5-10 membered heteroaryl, -($C_{3-7}$carbocyclyl)$C_{1-6}$alkyl, (4-10 membered heterocyclyl)$C_{1-6}$alkyl, ($C_{6-10}$aryl)$C_{1-6}$alkyl, ($C_{6-10}$aryl)$C_{1-6}$alkoxy, (5-10 membered heteroaryl)$C_{1-6}$alkyl, -($C_{3-7}$carbocyclyl)-amine, (4-10 membered heterocyclyl)amine, ($C_{6-10}$aryl)amine, (5-10 membered heteroaryl)amine, acyl, C-carboxy, O-carboxy, C-amido, N-amido, S-sulfonamido, N-sulfonamido, -SR, COOH, or CONR'R"; wherein each R' and R" are independently H, $C_{1-10}$ alkyl, $C_{1-10}$ haloalkyl, -$C_{1-10}$ alkoxyl. In some embodiments, In some embodiments, $R^4$ in Formula (A-7) to (A-8) is selected from O, S, and N or a $C_{1-6}$ alkylene, and the heteroarylene or the a $C_{1-6}$ alkylene is optionally substituted with 1-3 substituents selected from OH, halogen, $C_{1-10}$ alkyl, $NO_2$, CN, NR'R", $C_{1-6}$ haloalkyl, -$C_{1-6}$ alkoxyl, $C_{1-6}$ haloalkoxy, $C_{3-7}$ carbocyclyl, 4-10 membered heterocyclyl, $C_{6-10}$aryl, 5-10 membered heteroaryl, -SR', COOH, or CONR'R"; wherein each R' and R" are independently H, $C_{1-10}$ alkyl, $C_{1-10}$ haloalkyl, -$C_{1-10}$ alkoxyl.

For the chemical Formula (A-1) to (A-9), each E, $E_1$ and $E_2$ independently are optionally substituted thiophene-containing moiety, optionally substituted pyrrole containing moiety, optionally substituted immidazole containing moiety, and optionally substituted amine. In some embodiments, each E, $E_1$ and $E_2$ are independently selected from the group consisting of N-methylpyrrole, N-methylimidazole, benzimidazole moiety, and 3-(dimethylamino)propanamidyl, each group optionally substituted by 1-3 substituents selected from the group consisting of H, OH, halogen, $C_{1-10}$ alkyl, $NO_2$, CN, NR'R", $C_{1-6}$ haloalkyl, -$C_{1-6}$ alkoxyl, $C_{1-6}$ haloalkoxy, ($C_{1-6}$ alkoxy)$C_{1-6}$ alkyl, $C_{2-10}$alkenyl, $C_{2-10}$alkynyl, $C_{3-7}$ carbocyclyl, 4-10 membered heterocyclyl, $C_{6-10}$aryl, 5-10 membered heteroaryl, amine, acyl, C-carboxy, O-carboxy, C-amido, N-amido, S-sulfonamido, N-sulfonamido, -SR', COOH, or CONR'R"; wherein each R' and R" are independently H, $C_{1-10}$ alkyl, $C_{1-10}$ haloalkyl, -$C_{1-10}$ alkoxyl. In some embodiments, each $E_1$ and $E_2$ independently comprises thiophene, benzthiophene, C-C linked benzimidazole/thiophene-containing moiety, or C-C linked hydroxybenzimidazole/thiophene-containing moiety, wherein each R' and R" are independently H, $C_{1-10}$alkyl, $C_{1-10}$haloalkyl, -$C_{1-10}$alkoxyl..

In some embodiments, each E, $E_1$ or $E_2$ are independently selected from the group consisting of isophthalic acid; phthalic acid; terephthalic acid; morpholine; N,N-dimethylbenzamide; N,N-bis(trifluoromethyl)benzamide; fluorobenzene; (trifluoromethyl)benzene; nitrobenzene; phenyl acetate; phenyl 2,2,2-trifluoroacetate; phenyl dihydrogen phosphate; 2H-pyran; 2H-thiopyran; benzoic acid; isonicotinic acid; and nicotinic acid; wherein one, two or three ring members in any of these end-group candidates can be independently substituted with C, N, S or O; and where any one, two, three, four or five of the hydrogens bound to the ring can be substituted with $R_5$, wherein $R_5$ may be independently selected for any substitution from H, OH, halogen, $C_{1-10}$ alkyl, $NO_2$, $NH_2$, $C_{1-10}$ haloalkyl, -$OC_{1-10}$ haloalkyl, COOH, CONR'R"; wherein each R' and R" are independently H, $C_{1-10}$ alkyl, $C_{1-10}$ haloalkyl, -$C_{1-10}$ alkoxyl.

The DNA recognition or binding moiety can include one or more subunits selected from the group consisting of:

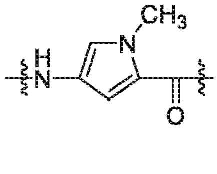 (Py), 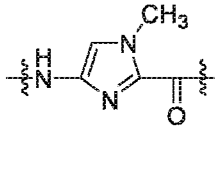 (Im), 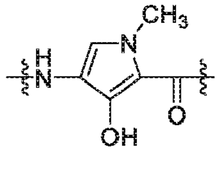 (Hp), 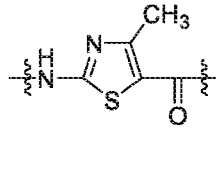 (Th),

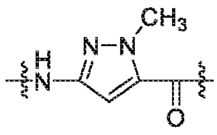 (Pz), 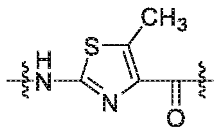 (Nt), 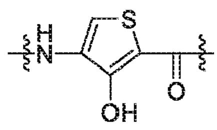 (Ht), 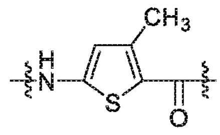 (Tn),

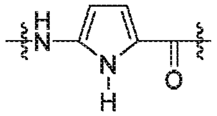 (Nh), 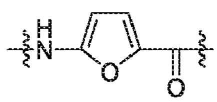 (Fr), 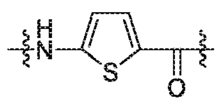 (Tp), 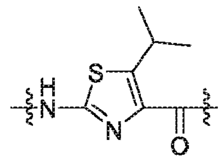 (iNt),

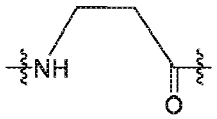 (β), 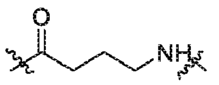 (gAB), 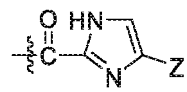 (PyT), 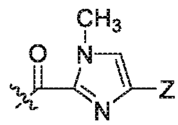 (ImT),

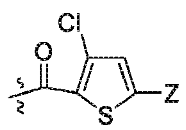 (CTh), 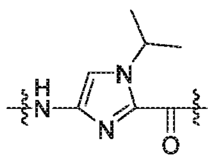 (iIm), 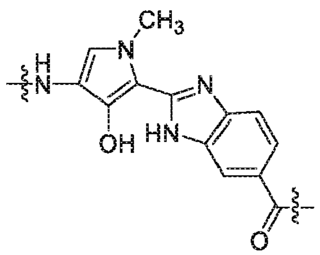 (HpBi),

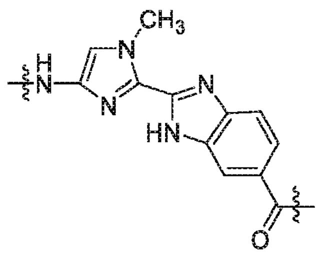 (ImBi), 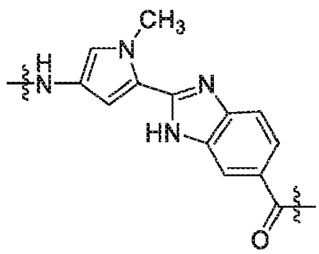 (PyBi), 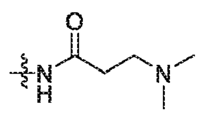 ("Dp"),

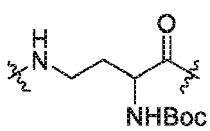, 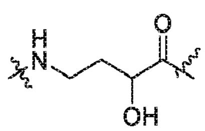, 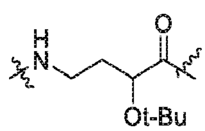, 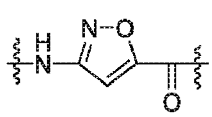, 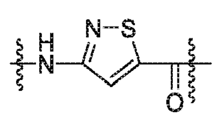,

-NH-benzopyrazinylene-CO-, -NH-phenylene-CO-, -NH-pyridinylene-CO-, -NH-piperidinylene-CO-, -NH-pyrimidinylene-CO-, -NH-anthracenylene-CO-, -NH-quinolinylene-CO-, and

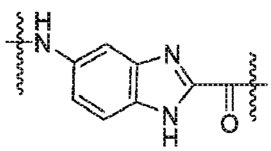,

wherein Z is H, $NH_2$, $C_{1-6}$ alkyl, or $C_{1-6}$ $alkylNH_2$.

In some sombodiments, Py is

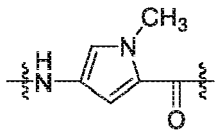

,

Im is

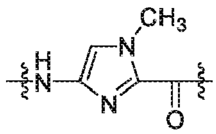

,

Hp is

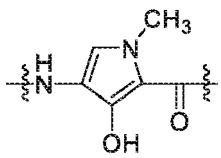

,

This

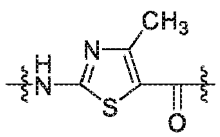

,

Pz is

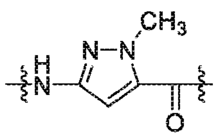

,

Nt is

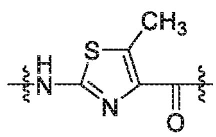

,

Tn is

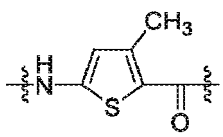

,

Nh is

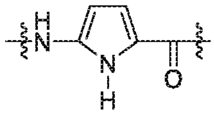

,

iNt is

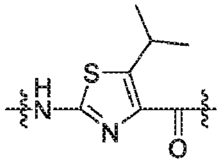

,

iIm is

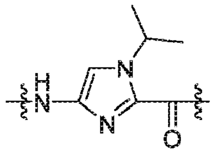

,

HpBi is

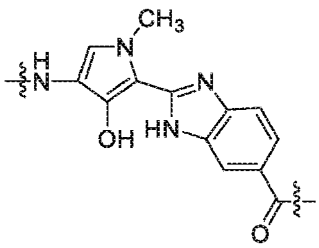

,

ImBi is

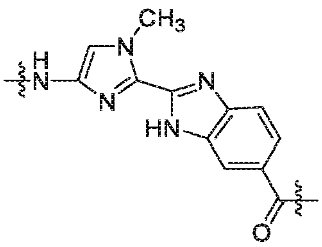

,

PyBi is

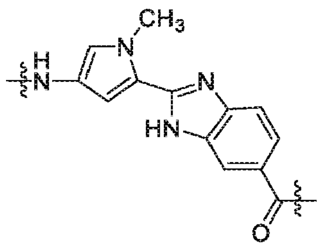

$D_P$ is

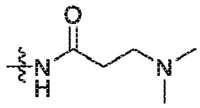

-NH-benzopyrazinylene-CO- is

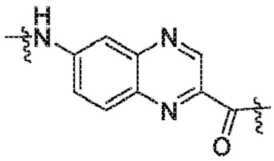

-NH-phenylene-CO- is

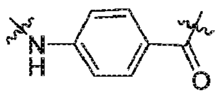

-NH-pyridinylene-CO- is

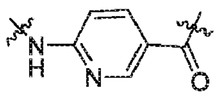

-NH-piperidinylene-CO- is

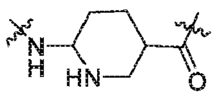

-NH-pyrazinylene-CO- is

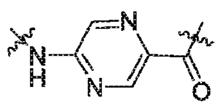

-NH-anthracenylene-CO- is

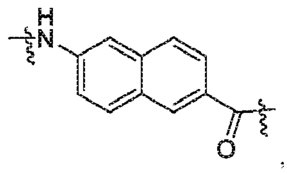

and -NH-quinolinylene-CO- is

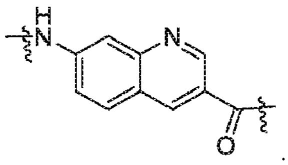

In some embodiments, the first terminus comprises one or more subunits selected from the group consisting of optionally substituted N-methylpyrrole, optionally substituted N-methylimidazole, and β-alanine (β).

In some embodiments, the first terminus does not have a structure of

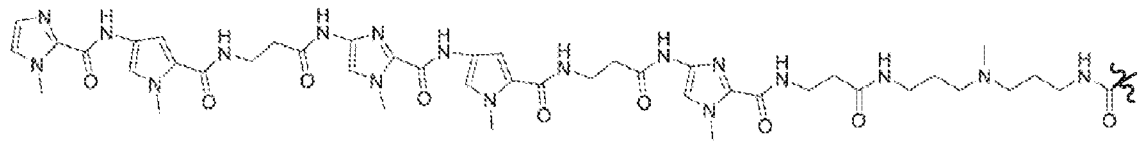

In some embodiments, the first terminus does not contain a polyamide that binds to a trinucleotide repeat CGG. In some embodiments, the first terminus does not contain a polyamide that binds to a trinucleotide repeat CTG. In some embodiments, the first terminus does not contain a polyamide that binds to a tetranucleotide repeat CCTG. In some embodiments, the first terminus does not contain a polyamide that binds to a pentanucleotide repeat TGGAA.

The polyamide composed of a pre-selected combination of subunits can selectively bind to the DNA in the minor groove. In their hairpin structure, antiparallel side-by-side pairings of two aromatic amino acids bind to DNA sequences, with a polyamide ring packed specifically against each DNA base. N-Methylpyrrole (Py) favors T, A, and C bases, excluding G; N-methylimidazole (Im) is a G-reader; and 3-hydroxyl-N-methylpyrrol (Hp) is specific for thymine base. The nucleotide base pairs can be recognized using different pairings of the amino acid subunits using the paring principle shown in Table 1A and 1B below. For example, an Im/Py pairing reads G C by symmetry, a Py/Im pairing reads C·G, an Hp/Py pairing can distinguish T·A from A·T, G·C, and C·G, and a Py/Py pairing nonspecifically discriminates both A·T and T·A from G·C and C·G.

In some embodiments, the first terminus comprises Im corresponding to the nucleotide G; Im or Nt corresponding to the nucleotide pair G; Py corresponding to the nucleotide C, wherein Im is N-alkyl imidazole, Py is N-alkyl pyrrole, Hp is 3 hydroxy N-methyl pyrrole, and β-alanine. In some embodiments, the first terminus comprises Im/Py to correspond to the nucleotide pair G/C, Py/Im to correspond to the nucleotide pair C/G, and wherein Im is N-alkyl imidazole (e.g, N-methyl imidazole), Py is N-alkyl pyrrole (e.g., N-methyl pyrrole), and Hp is 3 -hydroxy N- methyl pyrrole.

Table 1A. Base paring for single amino acid subunit (Favored (+), disfavored (-))

| Subunit | G | G | G | G | C | C |
|---|---|---|---|---|---|---|
| Py | - | - | - | - | + | + |
| Im | + | + | + | + | - | - |
| 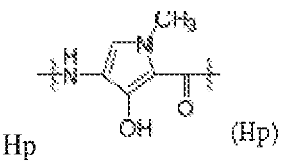 Hp (Hp) | - | - | - | - | - | - |

(continued)

| Subunit | G | G | G | G | C | C |
|---|---|---|---|---|---|---|
| 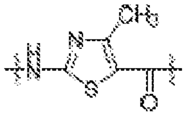 (Th), | - | - | - | - | - | - |
| 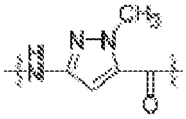 (Pz), | - | - | - | - | - | - |
| 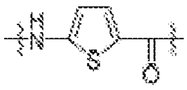 (Tp), | - | - | - | - | - | - |
| 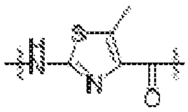 (Nt) | + | + | + | + | - | - |
| 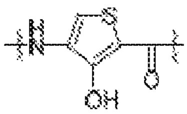 (Ht), | - | - | - | - | - | - |
| 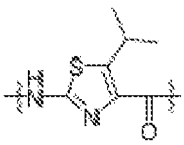 (iPTA) | + | + | + | + | - | - |
| 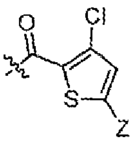 ("CTh"); | - | - | - | - | - | - |
| PEG 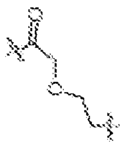 | - | - | - | - | + | + |
| iIm 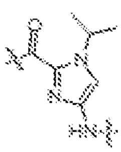 | + | + | + | + | - | - |
| 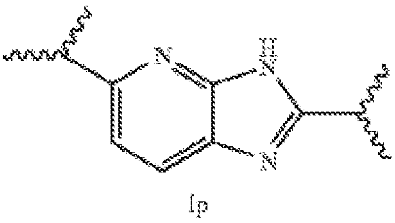 Ip | + | + | + | + | - | - |

(continued)

| Subunit | G | G | G | G | C | C |
|---|---|---|---|---|---|---|
| 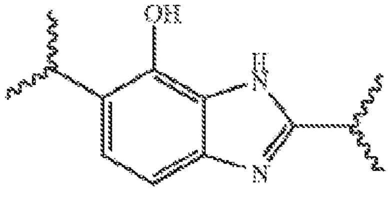 Hz | - | - | - | - | - | - |
| 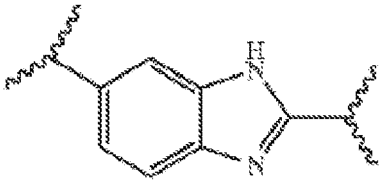 Bi | - | - | - | - | - | - |
| 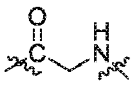 (gly) | - | - | - | - | - | - |
| 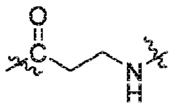 (β) | - | - | - | - | - | - |
| 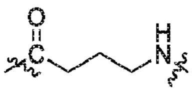 (gAB) | - | - | - | - | - | - |
| 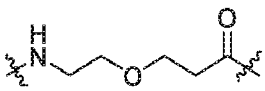 (Alx) | - | - | - | - | + | + |
| 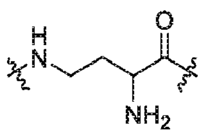 (Da) | - | - | - | - | - | - |
| 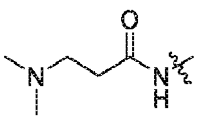 (Dp) | - | - | - | - | - | - |
| 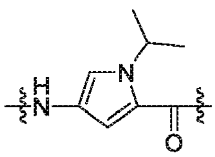 (iPP) | - | - | - | - | - | - |
| 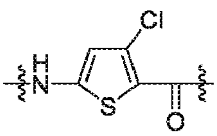 (CTh) | + | + | + | + | + | + |

(continued)

| Subunit | G | G | G | G | C | C |
|---|---|---|---|---|---|---|
| 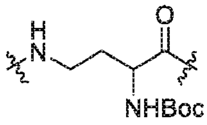 (Dab) | - | - | - | - | - | - |
| 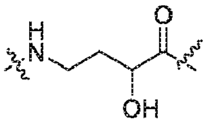 (gAH) | - | - | - | - | - | - |
| HpBi 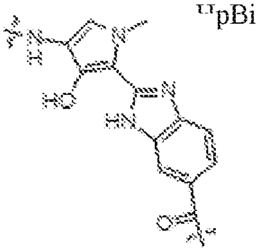 | WW* (bind to two nucleotides with same selectivity as Hp-Py) | | | | | |
| PyBi 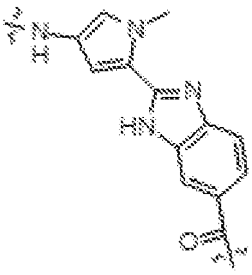 | WW* (bind to two nucleotides with same selectivity as Py-Py) | | | | | |
| ImBi 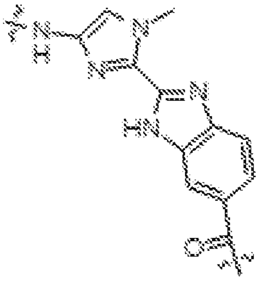 | GW* (bind to two nucleotides with same selectivity as hn-Py) | | | | | |
| *The subunit HpBi, ImBi, and PyBi function as a conjugate of two monomer subunits and bind to two nucleotides. The binding property of HpBi, ImBi, and PyBi corresponds to Hp-Py, Im-Py, and Py-Py respectively. | | | | | | |

Table 1B. Base paring for hairpin polyamide

| | G·C | G·C | G·C | G·C | C·G | C·G |
|---|---|---|---|---|---|---|
| Im/β | + | + | + | + | - | - |
| β/Im | - | - | - | - | + | + |
| Py/ β | - | - | - | - | - | - |
| β/Py | - | - | - | - | - | - |

(continued)

| | G·C | G·C | G·C | G·C | C·G | C·G |
|---|---|---|---|---|---|---|
| β/ β | - | - | - | - | - | - |
| Py/Py | - | - | - | - | - | - |
| Im/Im | - | - | - | - | - | - |
| Im/Py | + | + | + | + | - | - |
| Py/Im | - | - | - | - | + | + |
| Th/Py | - | - | - | - | - | - |
| Py/Th | - | - | - | - | - | - |
| Th/Im | + | + | + | + | - | - |
| Im/Th | - | - | - | - | + | + |
| β/Th | - | - | - | - | - | - |
| Th/β | - | - | - | - | - | - |
| Hp/Py, | - | - | - | - | - | - |
| Py/Hp, | - | - | - | - | - | - |
| Hp/Im | + | + | + | + | - | - |
| Im/Hp | - | - | - | - | + | + |
| Tn/Py | - | - | - | - | - | - |
| Py/Tn, | < | - | - | - | - | - |
| Ht/Py, | < | - | - | - | - | - |
| Py/Ht, | - | - | - | - | - | - |
| Bi/Py, | < | - | - | - | - | - |
| Py/Bi, | < | - | - | - | - | - |
| β/Bi | - | - | - | - | - | - |
| Bi/β | - | - | - | - | - | - |
| Bi/Im, | - | - | - | - | + | + |
| Im/Bi, | + | + | + | + | - | - |
| Tp/Py, | < | - | - | - | - | - |
| Py/Tp, | < | - | - | - | - | - |
| β/Tp | - | - | - | - | - | - |
| Tp/β | - | - | - | - | - | - |
| Tp/Im, | - | - | - | - | + | + |
| Im/Tp | + | + | + | + | - | - |
| Tp/Tp | - | - | - | - | - | - |
| Tp/Tn | - | - | - | - | - | - |
| Tn/Tp | - | - | - | - | - | - |
| Hz/Py, | - | - | - | - | - | - |
| Py/Hz, | - | - | - | - | - | - |
| Ip/Py | + | + | + | + | - | - |
| Py/Ip, | - | - | - | - | + | + |

(continued)

| | G·C | G·C | G·C | G·C | C·G | C·G |
|---|---|---|---|---|---|---|
| Bi/Hz, | - | - | - | - | - | - |
| Hz/Bi, | - | - | - | - | - | - |
| Bi/Bi | - | - | - | - | + | + |
| Th/Py, | - | - | - | - | - | - |
| Py/Th | - | - | - | - | - | - |
| Im/gAB | + | + | + | + | - | - |
| gAB/Im | - | - | - | - | + | + |
| Py/ gAB | + | + | + | + | - | - |
| gAB/Py | - | - | - | - | + | + |
| gAB/ β | - | - | - | - | - | - |
| β/gAB | < | - | - | - | - | - |
| Im/Dp | + | + | + | + | - | - |
| Dp/Im | - | - | - | - | + | + |
| Py/ Dp | < | - | - | - | - | - |
| Dp/Py | - | - | - | - | - | - |
| Dp/P | - | - | - | - | - | - |
| Each of HpBi, ImBi, and PyBi can bind to two nucleotides and have binding properties corresponding to Hp-Py, Im-Py, and Py-Py respectively. HpBi, ImBi, and PyBi can be paired with two monomer subunits or with themselves in a hairpin structure to bind to two nucleotide pairs. | | | | | | |

The monomer subunits of the polyamide can be strung together based on the paring principles shown in Table 1A and Table 1B. The monomer subunits of the polyamide can be strung together based on the paring principles shown in Table 1C and Table 1D.

Table 1C shows an example of the monomer subunits that can bind to the specific nucleotide. The first terminus can include a polyamide described having several monomer subunits stung together, with a monomer subunit selected from each row. For example, the polyamide can include Im-Im-Im-Im-Py-Py that binds to GGGGCC, with Im selected from the first G column, Im from the second G column, Im from the third G column, Im from the fourth G column, Py from the C column, and Py from the second C column. The polyamide can be any combinations of the subunits of GGGGCC, with a subunit selected from each column in Table 1C, wherein the subunits are strung together following the GGGGCC order. In another example, the polyamide can include Im-Im-Nt-Im-Py, with Im selected from the first G column, Im from the G column, Nt from the G column, Im from the G column, and Py from the first C column.

In addition, the polyamide can also include a partial or multiple sets of the five subunits, such as 1.5, 2, 2.5, 3, 3.5, or 4 sets of the four subunits. The polyamide can include 3, 4, 5, 6, 7, 8, 9, 10, 12, 14, and 16 monomer subunits. The multiple sets can be joined together by W. In addition to the five subunits or ten subunits, the polyamide can also include 1-4 additional subunits that can link multiple sets of the five subunits.

The polyamide can include monomer subunits that bind to 2, 3, 4, or 5 nucleotides of GGGGCC. For example, the polyamide can bind to GG, GC, CC, CG, GGG, GCC, GGC, CCG, GGGC, GGCC, GCCG, CCGG, CGGG, GGGC, GGCC, or GGGGCC. The polyamide can include monomer subunits that bind to 3, 4, 5, 6, 7, 8, 9, or 10 nucleotides of GGGGCC repeat. For example, the polyamide can bind to GG, GGG, GGGGC, GGGGCC, GGGGCCG, GGGGCCGG, GGGGCCGGGGCC. The nucleotides can be joined by W.

The monomer subunit, when positioned as a terminal unit, does not have an amine or a carboxylic acid group at the terminal. The amine or carboxylic acid group in the terminal is replaced by a hydrogen. For example, Py, when used as a terminal unit, is understood to have the structure of

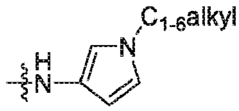

(e.g,

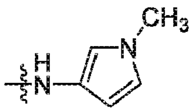

); and Im, when positioned as a terminal unit, is understood to have the structure of

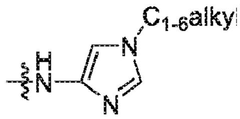

(e.g,

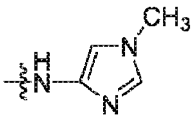

). In addition, when Py or Im is used as a terminal unit, Py and Im can be respectively replaced by PyT

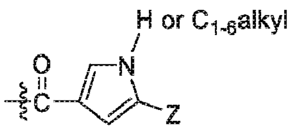

(e.g.,

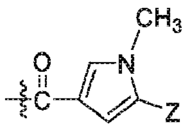

) and ImT

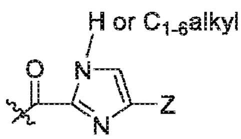

(e.g.,

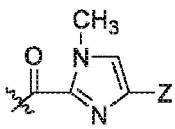

).

The linear polyamide can have nonlimiting examples including but not limited to Im-Im-Im, Im-Im-Im-Py, Im-Im-Py-Py, Im-Im-Im-Im-Py-Py, Im-Im-Im-Py-Py, Im-Im-Im-Py-Py-Im, and any combinations thereof.

Table 1C. Examples of monomer subunits in a linear polyamide that binds to GGGGCC.

| Nucleotide | G | G | G | G | C | C |
|---|---|---|---|---|---|---|
| Subunit that selectively binds to nucleotide | Im or ImT | Im or ImT | Im or ImT | Im or ImT | Py or PyT | Py or PyT |
| | iIm or iImT | iIm or iImT | iIm or iImT | iIm or iImT | iIm or iImT | iIm or iImT |
| | PEG | PEG | PEG | PEG | PEG | PEG |
| | CTh | CTh | CTh | CTh | CTh | CTh |
| | Nt | Nt | Nt | Nt | Alx | Alx |
| | iPTA | iPTA | iPTA | iPTA | | |
| | Ip | Ip | Ip | Ip | | |
| | CTh | CTh | CTh | CTh | | |

The DNA-binding moiety can also include a hairpin polyamide having subunits that are strung together based on the pairing principle shown in Table 1B. Table 1D shows some examples of the monomer subunit pairs that selectively bind to the nucleotide pair. The hairpin polyamide can include 2n monomer subunits (n is an integer in the range of 2-8), and the polyamide also includes a W in the center of the 2n monomer subunits. W can be $-(CH_2)_a-NR^1-(CH_2)_b-$, $-(CH_2)_a-$, $-(CH_2)_a-O-(CH_2)_b-$, $-(CH_2)_a-CH(NHR^1)-$, - $(CH_2)_a-CH(NHR^1)-$, $-(CR^2R^3)_a$ -or $-(CH_2)_a-CH(NR^1{}_3)^+-(CH_2)_b-$, wherein each a is independently an integer between 2 and 4; $R^1$ is H, an optionally substituted $C_{1-6}$ alkyl, an optionally substituted $C_{3-10}$ cycloalkyl, an optionally substituted $C_{6-10}$ aryl, an optionally substituted 4-10 membered heterocyclyl, or an optionally substituted 5-10 membered heteroaryl; each $R^2$ and $R^3$ are independently H, halogen, OH, NHAc, or $C_{1-4}$ alky. In some embodiments, W is $-(CH_2)-CH(NH_3)^+-(CH_2)-$ or $-(CH_2)-CH_2CH(NH_3)^+-$. In some embodiments, $R^1$ is H. In some embodiments, $R^1$ is $C_{1-6}$ alkyl optionally substituted by 1-3 substituents selected from -C(O)-phenyl. In some embodiments, W is $-(CR^2R^3)-(CH_2)a-$ or $-(CH_2)_a-(CR^2R^3)-(CH_2)_b-$, wherein each a is independently 1-3, b is 0-3, and each $R^2$ and $R^3$ are independently H, halogen, OH, NHAc, or $C_{1-4}$ alky. W can be an aliphatic amino acid residue shown in Table 4 such as gAB.

When n is 2, the polyamide includes 4 monomer subunits, and the polyamide also includes a W joining the first set of two subunits with the second set of two subunits, Q1-Q2-W-Q3-Q4, and Q1/Q4 correspond to a first nucleotide pair on the DNA double strand, Q2/Q3 correspond to a second nucleotide pair, and the first and the second nucleotide pair is a part of the GGGGCC repeat. When n is 3, the polyamide includes 6 monomer subunits, and the polyamide also includes a W joining the first set of three subunits with the second set of three subunits, Q1-Q2-Q3-W-Q4-Q5-Q6, and Q1/Q6 correspond to a first nucleotide pair on the DNA double strand, Q2/Q5 correspond to a second nucleotide pair, Q3/Q4 correspond to a third nucleotide pair, and the first and the second nucleotide pair is a part of the A repeat. When n is 4, the polyamide includes 8 monomer subunits, and the polyamide also includes a W joining the first set of four subunits with the second set of four subunits, Q1-Q2-Q3-Q4-W-Q5-Q6-Q7-Q8, and Q1/Q8 correspond to a first nucleotide pair on the DNA double strand, Q2/Q7 correspond to a second nucleotide pair, Q3/Q6 correspond to a third nucleotide pair, and Q4/Q5 correspond to a fourth nucleotide pair on the DNA double strand. When n is 5, the polyamide includes 10 monomer subunits, and the polyamide also includes a W joining a first set of five subunits with a second set of five subunits, Q1-Q2-Q3-Q4-Q5-W-Q6-Q7-Q8-Q9-Q10, and Q1/Q10, Q2/Q9, Q3/Q8, Q4/Q7, Q5/Q6 respectively correspond to the first to the fifth nucleotide pair on the DNA double strand. When n is 6, the polyamide includes 12 monomer subunits, and the polyamide also includes a W joining a first set of six subunits with a second set of six subunits, Q1-Q2-Q3-Q4-Q5-Q6-W-Q7-Q8-Q9-Q10-Q11-Q12, and Q1/Q12, Q2/Q11, Q3/Q10, Q4/Q9, Q5/Q8, Q6/Q7 respectively correspond to the first to the six nucleotide pair on the DNA double strand. When n is 8, the polyamide includes 16 monomer subunits, and the polyamide also includes a W joining a first set of eight subunits with a second set of eight subunits, Q1-Q2-Q3-Q4-Q5-Q6-Q7-Q8-W-Q9-Q10-Q11-Q12-Q13-Q14-Q15-Q16, and Q1/Q16, Q2/Q15, Q3/Q14, Q4/Q13, Q5/Q12, Q6/Q11, Q7/Q10, and Q8/Q9 respectively correspond to the first to the eight nucleotide pair on the DNA double strand. When n is 9, the polyamide includes 18 monomer subunits, and the polyamide also includes a W joining a first set of eight subunits with a second set of eight subunits, Q1-Q2-Q3-Q4-Q5-Q6-Q7-Q8-Q9-W-Q10-Q11-Q12-Q13-Q14-Q15-Q16-Q17-Q18, and Q1/Q18, Q2/Q17, Q3/Q16, Q4/Q15, Q5/Q14, Q6/Q13, Q7/Q12, Q8/Q11, and Q9/Q10 respectively correspond to the first to the eight nucleotide pair on the DNA double strand. When n is 10, the

polyamide includes 20 monomer subunits, and the polyamide also includes a W joining a first set of eight subunits with a second set of eight subunits, Q1-Q2-Q3-Q4-Q5-Q6-Q7-Q8-Q9-Q10-W-Q11-Q12-Q13-Q14-Q15-Q16-Q17-Q18-Q19-Q20, and Q1/Q20, Q2/Q19, Q3/Q18, Q4/Q17, Q5/Q16, Q6/Q15, Q7/Q14, Q8/Q13, Q9/Q12, and Q10/Q11 respectively correspond to the first to the eight nucleotide pair on the DNA double strand. Q1-Q2-Q3-Q4-Q5-Q6-Q7-Q8-Q9-Q10-Q11-W-Q12-Q13-Q14-Q15-Q16-Q17-Q18-Q19-Q20-Q21-Q22, and Q1/Q22, Q2/Q21, Q3/Q20, Q4/Q19, Q5/Q18, Q6/Q17, Q7/Q16, Q8/Q15, Q9/Q14, Q10/Q13, and Q11/Q12 respectively correspond to the first to the eight nucleotide pair on the DNA double strand. Q1-Q2-Q3-Q4-Q5-Q6-Q7-Q8-Q9-Q10- Q11-Q12-W-Q13-Q14-Q15-Q16-Q17-Q18-Q19-Q20-Q21-Q22-Q23-Q24, and Q1/Q24, Q2/Q23, Q3/Q22, Q4/Q21, Q5/Q20, Q6/Q19, Q7/Q18, Q8/Q17, Q9/Q16, Q10/Q15, Q11/Q14, Q12/Q13 respectively correspond to the first to the eight nucleotide pair on the DNA double strand. W can be an aliphatic amino acid residue such as gAB or other appropriate spacers as shown in Table 4.

Because the target gene can include multiple repeats of GGGGCC, the subunits can be strung together to bind at least two, three, four, five, six, seven, eight, nine, or ten nucleotides in one or more GGGGCC repeat (e.g., GGGGCCGGGGCC). For example, the polyamide can bind to the GGGGCC repeat by binding to a partial copy, a full copy, or a multiple repeats of GGGGCC such as GG, GC, CC, GGG, GCC, CCG, CGG, GGGG, GGGC, GGCC, GCCG, CCGG, CGGG, GGGGC, GGGCC, GGCCG, . For example, the polyamide can include Im-Im-Im-Im-β-β-W-Im-Im-β-Py-β-Py that binds to GGGGCC and its complementary nucleotides on a double strand DNA, in which the Im/Py pair binds to the G-C, the Im/β pair binds to G·C, the Im/Py pair binds to G·C, the Im/β binds to G·C, and β/Im binds to C·G; and β/Im binds to C·G. In another example Im-Im-β-β-W-Im-Im-β-Py that binds to GGCC and its complementary nucleotides on a double strand DNA, in which Im/Py pair binds to G·C, Im/β pair binds to G·C, β/Im pair binds to C·G, β/Im pair binds to C·G. W can be an aliphatic amino acid residue such as gAB or other appropriate spacers as shown in Table 4.

Some additional examples of the polyamide include but are not limited to Im-Im-Im-W-Py-β-Py, Im-Im-lm-W-Py-Py-Py, Th-Im-Im-β-W-Im-Py-β-Im, and Im-Im-β-βW-Im-Im-β-Py.

Table 1D. Examples of monomer pairs in a hairpin or H-pin polyamide that binds to GGGGCC.

| Nucleotide | G·C | G·C | G·C | G·C | C·G | C·G |
|---|---|---|---|---|---|---|
| Subunit pairs that selectively binds to nucleotide | Im/β | Im/β | Im/β | Im/β | β/Im | β/Im |
| | Im/Py | Im/Py | Im/Py | Im/Py | Py/Im | Py/Im |
| | Th/Im | Th/Im | Th/Im | Th/Im | Im/Th | Im/Th |
| | Hp/Im | Hp/Im | Hp/Im | Hp/Im | Im/Hp | Im/Hp |
| | Im/Bi | Im/Bi | Im/Bi | Im/Bi | Bi/Im | Bi/Im |
| | Im/Tp | Im/Tp | Im/Tp | Im/Tp | Tp/Im | Tp/Im |
| | Ip/Py | Ip/Py | Ip/Py | Ip/Py | Py/Ip | Py/Ip |
| | Im/gAB | Im/gAB | Im/gAB | Im/gAB | Bi/Bi | Bi/Bi |
| | Py/gAB | Py/gAB | Py/gAB | Py/gAB | gAB/Im | gAB/Im |
| | Im/Dp | Im/Dp | Im/Dp | Im/Dp | gAB/Py | gAB/Py |
| | | | | | Dp/Im | Dp/Im |

Recognition of a nucleotide repeat or DNA sequence by two antiparallel polyamide strands depends on a code of side-by-side aromatic amino acid pairs in the minor groove, usually oriented N to C with respect to the 5' to 3' direction of the DNA helix. Enhanced affinity and specificity of polyamide nucleotide binding is accomplished by covalently linking the antiparallel strands. The "hairpin motif' connects the N and C termini of the two strands with a W (e.g., gamma-aminobutyric acid unit (gamma-turn)) to form a folded linear chain. The "H-pin motif' connects the antiparallel strands across a central or near central ring/ring pairs by a short, flexible bridge.

The DNA-binding moiety can also include a H-pin polyamide having subunits that are strung together based on the pairing principles shown in Table 1A and/or Table 1B. Table 1C shows some examples of the monomer subunit that selectively binds to the nucleotide, and Table 1D shows some examples of the monomer subunit pairs that selectively bind to the nucleotide pair. The h-pin polyamide can include 2 strands and each strand can have a number of monomer subunits (each strand can include 2-8 monomer subunits), and the polyamide also includes a bridge $L_1$ to connect the two strands in the center or near the center of each strand. At least one or two of the monomer subunits on each strand are paired with the corresponding monomer subunits on the other stand following the paring principle in Table ID to favor binding of either GC or C.G pair, and these monomer subunit pairs are often positioned in the center, close to center region, at or close to the bridge that connects the two strands. In some instances, the H-pin polyamide can have

all of the monomer subunits be paired with the corresponding monomer subunits on the antiparallel strand based on the paring principle in Table 1B and 1D to bind to the nucleotide pairs on the double strand DNA. In some instances, the H-pin polyamide can have a part of the monomer subunits (2, 3, 4, 5, or 6) be paired with the corresponding monomer subunits on the antiparallel strand based on the binding principle in Table 1B and 1D to bind to the nucleotide pairs on the double strand DNA, while the rest of the monomer subunit binds to the nucleotide based on the binding principle in Table 1A and 1C but does not pair with the mononer subunit on the antiparallel strand. The h-pin polyamide can have one or more overhanging monomer subunit that binds to the nucleotide but does not pair with the nomoner subunit on the antiparrallel strand.

Another polyamide structure that derives from the h-pin structure is to connect the two antiparallel strands at the end through a bridge, while only the two mononer subunits that are connected by the bridge form a pair that bind to the nucleotide pair G·C or C·G based on the binding principle in Table 1B/1D, but the rest of the monomer subunits on the strand form an overhang, bind to the nucleotide based on the binding principle in Table 1A and/or 1C and do not pair with the monomer subunit on the other strand.

The bridge can be is a bivalent or trivalent group selected from

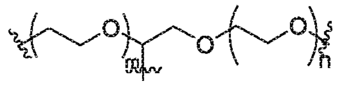,

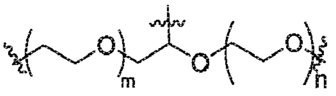, 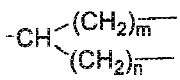, 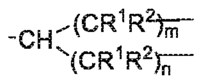, 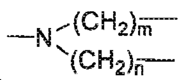, 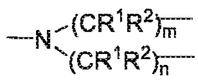,

a $C_{1-10}$ alkylene, -NH-$C_{0-6}$ alkylene-C(O)-, -N($CH_3$)-$C_{0-6}$ alkylene, and

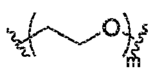,

-$(CH_2)_a$-$NR^1$-$(CH_2)_b$-, - $(CH_2)_a$-, -$(CH_2)_a$O-$(CH_2)_b$-, -$(CH_2)_a$-CH($NHR^1$)-, -$(CH_2)_a$-CH($NHR^1$)-, -$(CR^2R^3)_a$- or -$(CH_2)_a$-CH($NR^1{}_3$)$^+$-$(CH_2)_b$-, wherein m is an integer in the range of 0 to 10; n is an integer in the range of 0 to 10; each a is independently an integer between 2 and 4; $R^1$ is H, an optionally substituted $C_{1-6}$ alkyl, an optionally substituted $C_{3-10}$ cycloalkyl, an optionally substituted $C_{6-10}$ aryl, an optionally substituted 4-10 membered heterocyclyl, or an optionally substituted 5-10 membered heteroaryl; each $R^2$ and $R^3$ are independently H, halogen, OH, NHAc, or $C_{1-4}$ alky. In some embodiments, W is -($CH_2$)-CH($NH_3$)$^+$-($CH_2$)- or -($CH_2$)-$CH_2$CH($NH_3$)$^+$-. In some embodiments, $R^1$ is H. In some embodiments, $R^1$ is $C_{1-6}$ alkyl optionally substituted by 1-3 substituents selected from -C(O)-phenyl. In some embodiments, $L_1$ is -($CR^2R^3$)-$(CH_2)_a$- or -$(CH_2)_a$-($CR^2R^3$)-$(CH_2)_b$-, wherein each a is independently 1-3, b is 0-3, and each $R^2$ and $R^3$ are independently H, halogen, OH, NHAc, or $C_{1-4}$ alky. $L_1$ can be a $C_{2-9}$ alkylene or $(PEG)_{2-8}$.

When n is 3, the polyamide includes 6 monomer subunits, and the polyamide also includes a bridge $L_1$ joining the first set of three subunits with the second set of three subunits, and Q1-Q2-Q3 can be joined to Q4-Q5-Q6 through $L_1$ at the center Q2 and Q5, and Q1/Q4 correspond to a first nucleotide pair on the DNA double strand, Q2/Q5 correspond to a second nucleotide pair, Q3/Q6 correspond to a third nucleotide pair. When n is 4, the polyamide includes 8 monomer subunits, and the polyamide also includes a bridge $L_1$ joining the first set of four subunits with the second set of four subunits, Q1 -Q2-Q3-Q4 can be joined to Q5-Q6-Q7-Q8 through $L_1$ at Q2 and Q6 Q2 and Q7, Q3 and Q6, or Q3 and Q7 positions; Q1/Q5 may correspond to a nucleotide pair on the DNA double strand, and Q3/Q8 may correspond to another nucleotide pair; or Q1 and Q8 form overhangs on each strand, or Q and Q5 form overhangs on each strand. When n is 5, the polyamide includes 10 monomer subunits, and the polyamide also includes a bridge $L_1$ joining a first set of five subunits with a second set of five subunits, and Q1-Q2-Q3-Q4-Q5 can be joined to Q6-Q7-Q8-Q9-Q10 through a bridge $L_1$ at non-terminal positions (any position except for Q1, Q5, Q6 and Q10); if the two strands are linked at Q3 and Q8 by the bridge, Q1/Q6, Q2/Q7, Q3/Q8, Q4/Q9, and Q5/Q10 can be paired to bind to the nucleotide pairs; if the two strands are linked at Q2 and Q9 by the bridge, then Q1/Q8, Q3/Q10 can be paired to bind to the nucleotide pairs, Q4 and Q5 form an overhang on one strand and Q6 and Q7 form an overhang on the other strand.

In some embodiments, the monomer subunit at the central or near the central (n/2, (N±1)/2) on one strand is paired with the corresponding one on the other strand to bind to the nucleotide pairs on the double stranded DNA. In some embodiments, the monomer subunit at the central or near the central (n/2, (N±1)/2) on one strand is connected with the corresponding one on the other strand through a bridge $L_1$.

When n is 4, the polyamide includes 8 monomer subunits, and the polyamide also includes a bridge $L_1$ joining the first set of four subunits with the second set of four subunits, Q1-Q2-Q3-Q4 can be joined to Q5-Q6-Q7-Q8 at the end Q4 and Q5 through $L_{1;}$ while Q4/Q5 can be paired to bind to the nucleotide pairs, Q1-Q2-Q3 form an overhang on one strand and Q6-Q7-Q8 form an overhang on the other strand.

Some additional examples of the polyamide include but are not limited to Im-Im-Im-Py-Py-Im (Linked in the middle - either position 3 or 4) to Py-Im-Im-Py-Py-Py, Im-Im-Im-Py-Py (Linked in the middle - position 3) to Im-Im-Py-Py-Py, Im-Im-Im-Py (Linked in the middle - either position 2 or 3) to Im-Py-Py-Py, Im-Im-Py-Py-Im-Im (Linked in the middle - either position 3 or 4) to Py-Py-Im-Im-Py-Py, Im-Im-Py-Py-Im (Linked in the middle - position 3) to Py-Im-Im-Py-Py, Im-Im-Py-Py (Linked in the middle - either position 2 or 3) to Im-Im-Py-Py.

***Second Terminus -Regulatory protein binding moiety***

In certain embodiments, the regulatory molecule is chosen from a nucleosome remodeling factor (NURF), a bromodomain PHD finger transcription factor (BPTF), a ten-eleven translocation enzyme (TET), methylcytosine dioxygenase (TET1), a DNA demethylase, a helicase, an acetyltransferase, and a histone deacetylase ("HDAC").

The binding affinity between the regulatory protein and the second terminus can be adjusted based on the composition of the molecule or type of protein. In some embodiments, the second terminus binds the regulatory molecule with an affinity of less than about 600 nM, about 500 nM, about 400 nM, about 300 nM, about 250 nM, about 200 nM, about 150 nM, about 100 nM, or about 50nM. In some embodiments, the second terminus binds the regulatory molecule with an affinity of less than about 300 nM. In some embodiments, the second terminus binds the regulatory molecule with an affinity of less than about 200 nM. In some embodiments, the polyamide is capable of binding the DNA with an affinity of greater than about 200 nM, about 150 nM, about 100 nM, about 50 nM, about 10 nM, or about 1 nM. In some embodiments, the polyamide is capable of binding the DNA with an affinity in the range of about 1-600 nM, 10-500 nM, 20-500 nM, 50400 nM, 100-300 nM, or 50-200 nM.

In some embodiments, the second terminus comprises one or more optionally substituted $C_{6-10}$ aryl, optionally substituted $C_{4-10}$ carbocyclic, optionally substituted 4 to 10 membered heterocyclic, or optionally substituted 5 to 10 membered heteroaryl.

In some embodiments, the protein-binding moiety binds to the regulatory molecule that is selected from the group consisting of a CREB binding protein (CBP), a P300, an O-linked β-N-acetylglucosamine-transferase- (OGT-), a P300-CBP-associated-factor- (PCAF-), histone methyltransferase, histone demethylase, chromodomain, a cyclin-dependent-kinase-9- (CDK9-), a nucleosome-remodeling-factor-(NURF-), a bromodomain-PHD-finger-transcription-factor-(BPTF-), a ten-eleven-translocation-enzyme-(TET-), a methylcytosine-dioxygenase- (TET1-), histone acetyltransferase (HAT), a histone deacetalyse (HDAC), , a host-cell-factor-1(HCF1-), an octamer-binding-transcription factor- (OCT1-), a P-TEFb-, a cyclin-T1-, a PRC2-, a DNA-demethylase, a helicase, an acetyltransferase, a histone-deacetylase, methylated histone lysine protein.

In some embodiments, the second terminus comprises a moiety that binds to an O-linked β-N-acetylglucosamine-transferase(OGT), or CREB binding protein (CBP). In some embodiments, the protein binding moiety is a residue of a compound that binds to an O-linked β-N-acetylglucosamine-transferase(OGT), or CREB binding protein (CBP).

In some embodiments, the second terminus does not comprises JQ1, iBET762, OTX015, RVX208, or AU1. In some embodiments, the second terminus does not comprises JQ1. In some embodiments, the second terminus does not comprises a moiety that binds to a bromodomain protein.

In some embodiments, the second terminus comprises a diazine or diazepine ring, wherein the diazine or diazepine ring is fused with a $C_{6-10}$ aryl or a 5-10 membered heteroaryl ring comprising one or more heteroatom selected from S, N and O.

In some embodiments, the second terminus comprises an optionally substituted bicyclic or tricyclic structure. In some embodiments, the optionally substituted bicyclic or tricyclic structure comprises a diazepine ring fused with a thiophene ring.

In some embodiments, the second terminus does not comprise an optionally substituted bicyclic structure, wherein the bicyclic structure comprises a diazepine ring fused with a thiophene ring.

In some embodiments, the second terminus does not comprise an optionally substituted tricyclic structure, wherein the tricyclic structure is a diazephine ring that is fused with a thiophene and a triazole.

In some embodiments, the second terminus does not comprise an optionally substituted diazine ring.

In some embodiments, the second terminus does not comprise a structure of Formula (C-11):

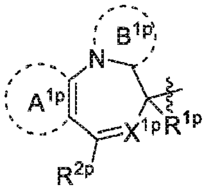

(C-11),

wherein:

- each of $A^{1p}$ and $B^{1p}$ is independently an optionally substituted aryl or heteroaryl ring;
- $X^{1p}$ is CH or N;
- $R^{1p}$ is hydrogen, halogen, or an optionally substituted $C_{1-6}$ alkyl group; and
- $R^{2p}$ is an optionally substituted $C_{1-6}$ alkyl, cycloalkyl, $C_{6-10}$ aryl, or heteroaryl.

In some embodiments, $X^{1p}$ is N. In some embodiments, $A^{1p}$ is an aryl or heteroaryl substituted with one or more substituents. In some embodiments, $A^{1p}$ is an aryl or heteroaryl substituted with one or more substituents selected from halogen, $C_{1-6}$alkyl, hydroxyl, $C_{1-6}$alkoxy, and $C_{1-6}$haloalkyl. In some embodiments, $B^{1p}$ is an optionally substituted aryl or heteroaryl substituted with one or more substituents selected from halogen, $C_{1-6}$alkyl, hydroxyl, $C_{1-6}$alkoxy, and $C_{1-6}$haloalkyl.

In some embodiments, $A^{1p}$ is an optionally substituted thiophene or phenyl. In some embodiments, $A^{1p}$ is a thiophene or phenyl, each substituted with one or more substituents selected from halogen, $C_{1-6}$ alkyl, hydroxyl, $C_{1-6}$ alkoxy, and $C_{1-6}$ haloalkyl. In some embodiments, $B^{1p}$ is an optionally substituted triazole. In some embodiments, $B^{1p}$ is a triazole substituted with one or more substituents selected from halogen, $C_{1-6}$alkyl, hydroxyl, $C_{1-6}$alkoxy, and $C_{1-6}$haloalkyl.

In some embodiments, the protein binding moiety is not

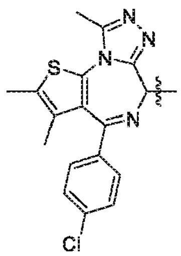

,

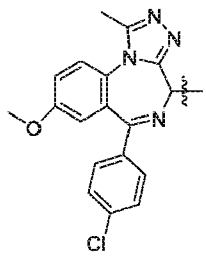

,

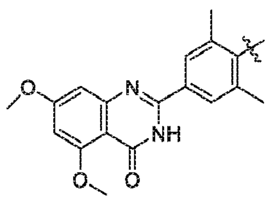

,

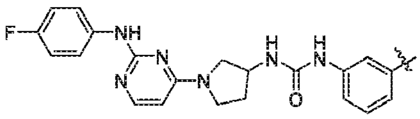

, or

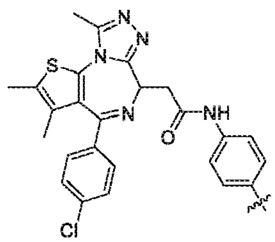

.

In some embodiments, the protein binding moiety is not

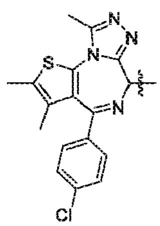

.

In some embodiments, the protein binding moiety does not have the structure of Formula (C-12): wherein:

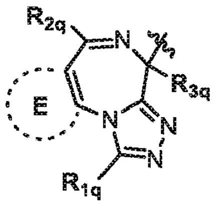

(C-12),

$R_{1q}$ is a hydrogen or an optionally substituted alkyl, hydroxyalkyl, aminoalkyl, alkoxyalkyl, halogenated alkyl, hydroxyl, alkoxy, or $-COOR_{4q}$;
$R_{4q}$ is hydrogen, or an optionally substituted aryl, aralkyl, cycloalkyl, heteroaryl, heteroaralkyl, heterocycloalkyl, alkyl, alkenyl, alkynyl, or cycloalkylalkyl group, optionally containing one or more heteroatoms;
$R_{2q}$ is an optionally substituted aryl, alkyl, cycloalkyl, or aralkyl group;
$R_{3q}$ is hydrogen, halogen, or an optionally substituted alkyl group, preferably $(CH_2)_x$ - $C(O)N(R_{20})(R_{21})$, or $(CH_2)_x$-$N(R_{20})$-$C(O)R_{21}$; or halogenated alkyl group;
wherein x is an integer from 1 to 10; and $R_{20}$ and $R_{21}$ are each independently hydrogen or $C_1$-$C_6$ alkyl group, preferably $R_{20}$ is hydrogen and $R_{21}$ ismethyl; and
Ring E is an optionally substituted aryl or heteroaryl group.

The protein binding moiety can include a residue of a compound that binds to a regulatory protein. In some embodiments, the protein binding moiety can be a residue of a compound shown in Table 2. Exemplary residues include, but are not limited to, amides, carboxylic acid esters, thioesters, primary amines, and secondary amines of any of the compounds shown in Table 2.

Table 2. A list of compounds that bind to regulatory proteins.

| Target protein | Compound |
|---|---|
| p300/ CBP HAT (histone acetyltransferase) | 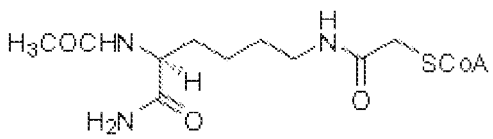 Lys-CoA |
| p300/ CBP HAT (histone acetyltransferase) | 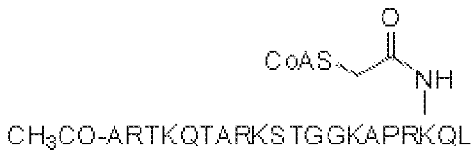 H3-CoA-20 |
| p300/ CBP HAT (histone acetyltransferase) | 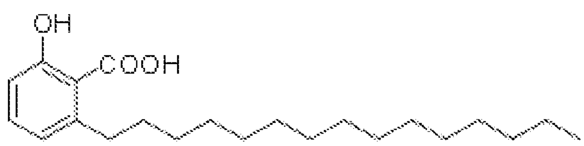 anacardic acid (AA) |
| p300/ CBP HAT (histone acetyltransferase) | 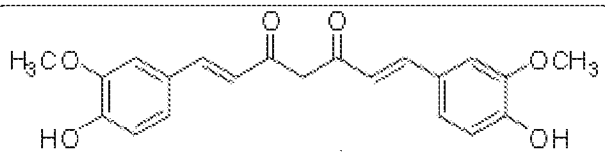 curcumin |

| | |
|---|---|
| p300/ CBP HAT (histone acetyltransferase) | 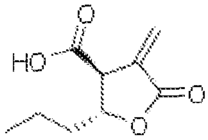 MB-3 |
| p300/ CBP HAT (histone acetyltransferase) | 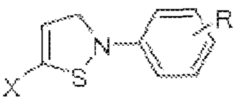 X = H, Cl<br>R = $NO_2$, Cl, $CF_3$, $OCH_3$, $COOC_2H_5$<br>isothiazolones |
| p300/ CBP HAT (histone acetyltransferase) | 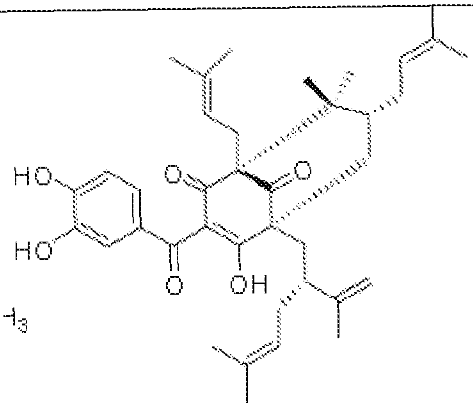 garcinol |
| p300/ CBP HAT (histone acetyltransferase) | 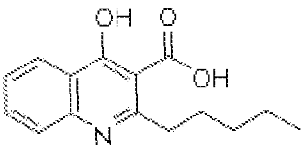 MC1823 (**4**) |
| p300/ CBP HAT (histone acetyltransferase) | 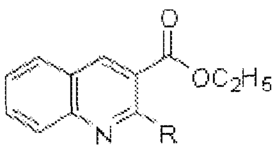 MC1626 (R = $CH_3$)<br>MC1752 (R = H) |
| p300/ CBP HAT (histone acetyltransferase) | 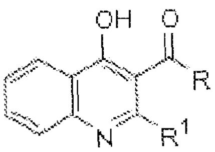 **1** (R = $OC_2H_5$; $R^1$ = $CH_3$)<br>**2** (R = OH; $R^1$ = $CH_3$)<br>**3** (R = $OC_2H_5$; $R^1$ = $C_5H_{11}$)<br>**5** (R = $OC_2H_5$; $R^1$ = $C_{10}H_{21}$)<br>**6** (R = OH; $R^1$ = $C_{10}H_{21}$)<br>**7** (R = $OC_2H_5$; $R^1$ = $C_{15}H_{31}$)<br>**8** (R = OH; $R^1$ = $C_{15}H_{31}$) |

| | |
|---|---|
| p300/ CBP HAT (histone acetyltransferase) | CBP30 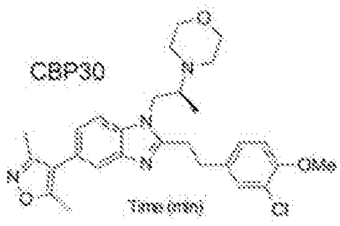 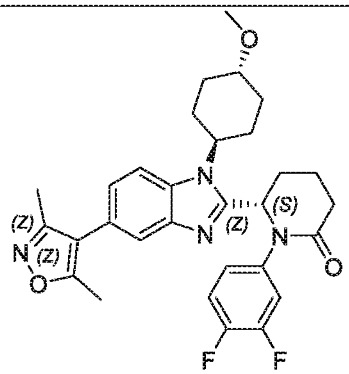 |
| p300/ CBP HAT (histone acetyltransferase) | 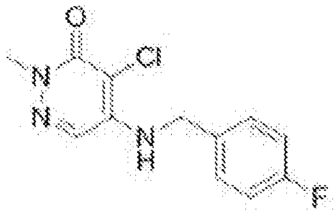 |
| p300/ CBP HAT (histone acetyltransferase) | 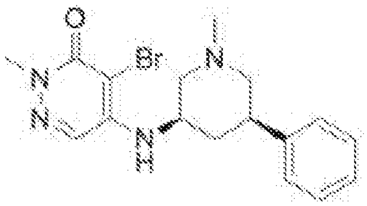 |
| p300/ CBP HAT (histone acetyltransferase) | 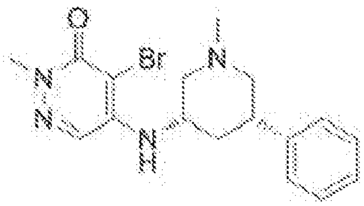 |
| p300/ CBP HAT (histone acetyltransferase) | 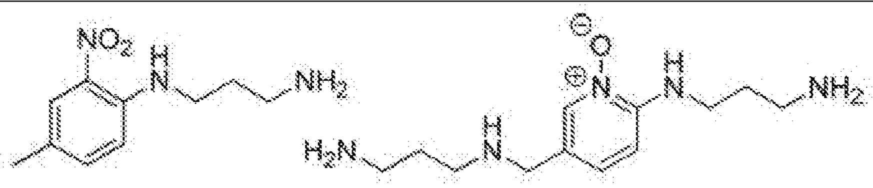 |
| p300/ CBP HAT (histone acetyltransferase) | 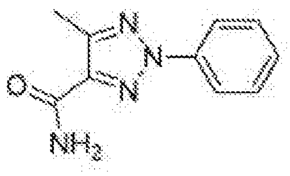 |

| | |
|---|---|
| p300/ CBP HAT (histone acetyltransferase) | 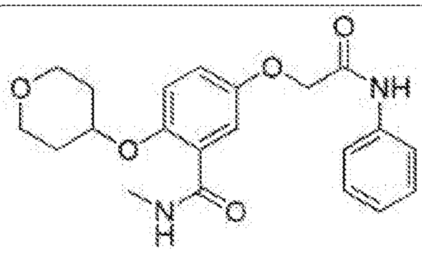 |
| p300/ CBP HAT (histone acetyltransferase) | 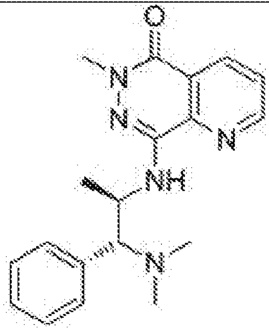 |
| p300/ CBP HAT (histone acetyltransferase) | 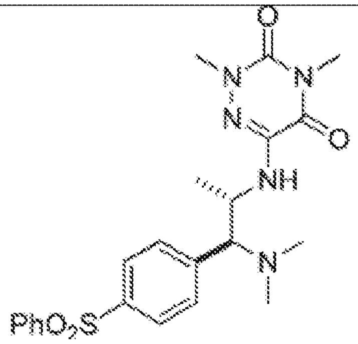 |
| p300/ CBP HAT (histone acetyltransferase) | 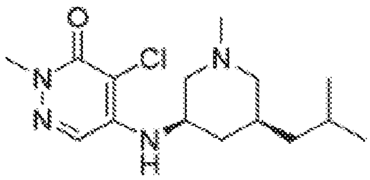 |
| p300/ CBP HAT (histone acetyltransferase) | 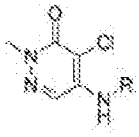 R 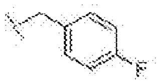 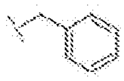 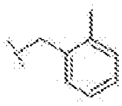 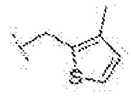 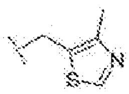 Ph<br>Me<br>*i*-Pr |
| p300/ CBP HAT (histone acetyltransferase) | 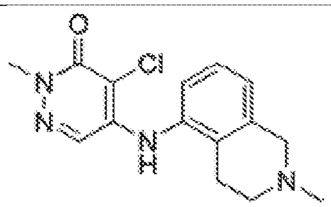 |

| | |
|---|---|
| p300/ CBP HAT (histone acetyltransferase) | 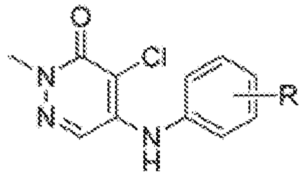 R<br>H<br>3-Me<br>2-$CH_2NH_2$<br>see above |
| p300/ CBP HAT (histone acetyltransferase) | 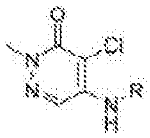<br>R: 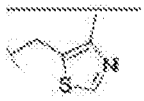, Ph, *i*-Pr<br>R: *i*-Pr, 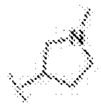, 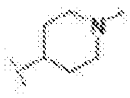<br>R: 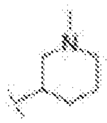, 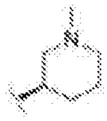, 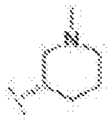 |
| p300/ CBP HAT (histone acetyltransferase) | 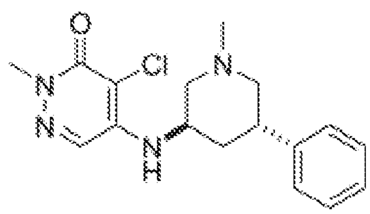 |
| p300/ CBP HAT (histone acetyltransferase) | 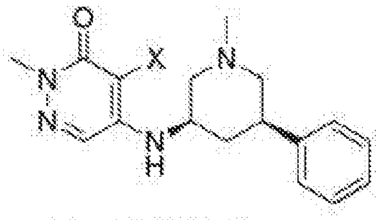 X = Cl, **(*R*,*R*)-31**<br>X = Br, **(*R*,*R*)-32**<br>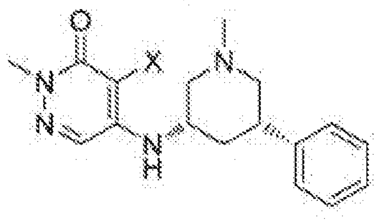 X = Cl, **(*S*,*S*)-31**<br>X = Br, **(*S*,*S*)-32** |
| p300/ CBP HAT (histone acetyltransferase) | 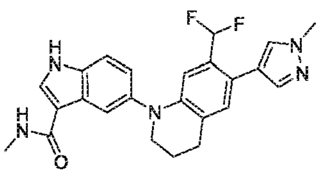 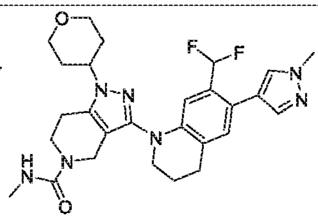 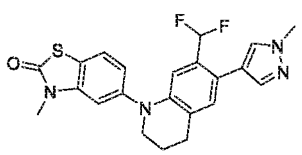 |
| p300/ CBP HAT (histone acetyltransferase) | 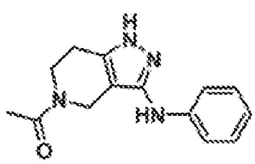 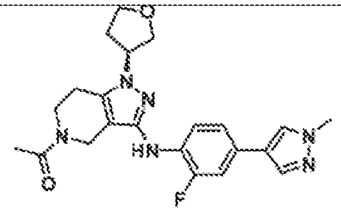 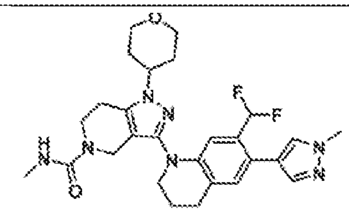 |

| | |
|---|---|
| p300/ CBP HAT (histone acetyltransferase) | 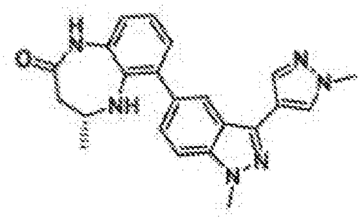 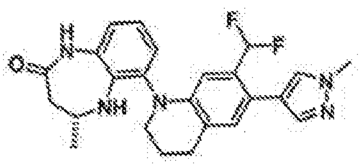 |
| p300/ CBP HAT (histone acetyltransferase) | 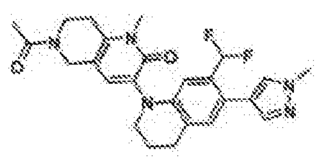 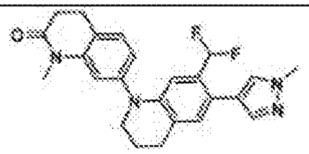 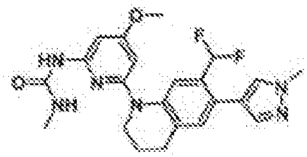 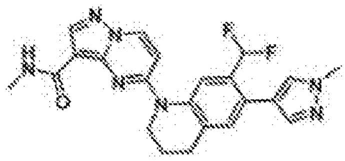 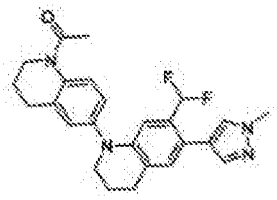 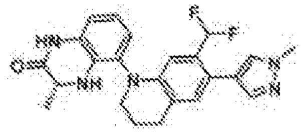 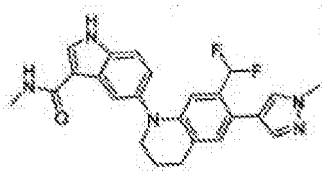 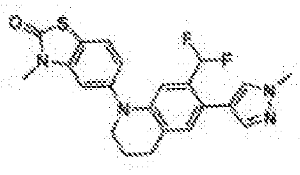 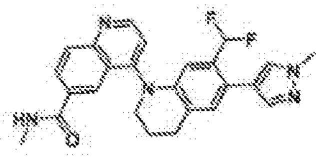 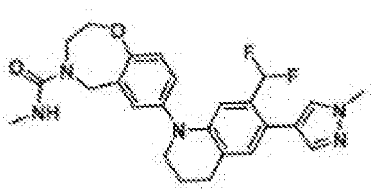 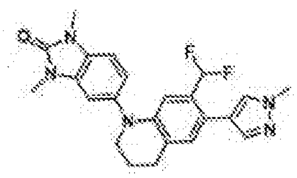 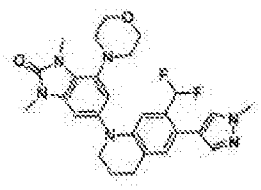 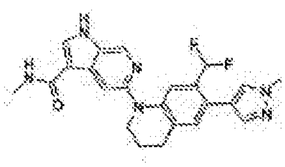 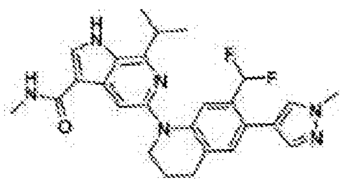 |
| p300/ CBP HAT (histone acetyltransferase) | 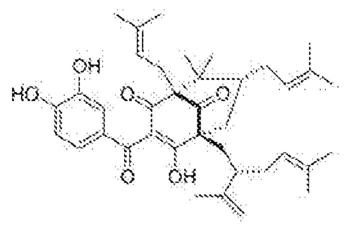 Garcinol 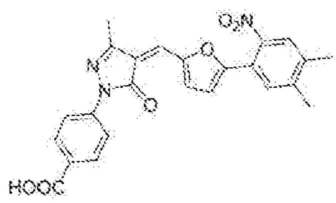 C646 |

| | |
|---|---|
| p300/ CBP HAT (histone acetyltransferase) | 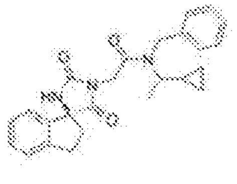 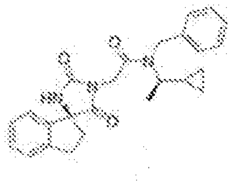 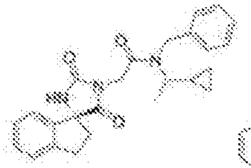 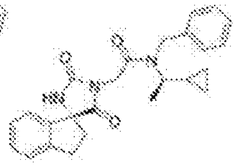 |
| p300/ CBP HAT (histone acetyltransferase) | 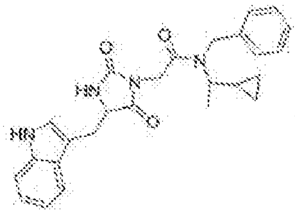 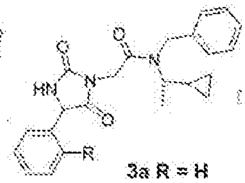 3a R = H 3b R = Me 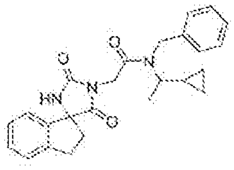 4 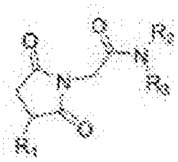 |
| p300/ CBP HAT (histone acetyltransferase) | 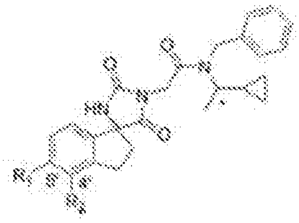 <br> *stereochemistry / R1 / R2: <br> R,S / H / H <br> R,S / CN / H <br> R,S / H / CN <br> R,S / $CONH_2$ / H <br> R,S / H / $CONH_2$ <br> R,S / OMe / H <br> R,S / $CH_2OH$ / H <br> R,S / cyclopropyl / H <br> R,S / NHCOMe / H <br> S / $NHCO_2Me$ / H <br> S / $NHSO_2Me$ / H <br> S / NHCONH-Me / H |
| p300/ CBP HAT (histone acetyltransferase) | 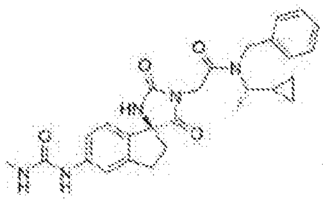 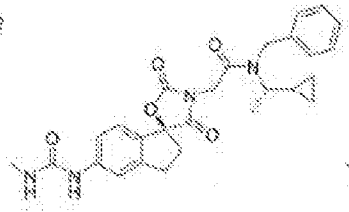 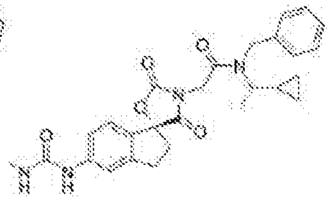 |
| p300/ CBP HAT (histone acetyltransferase) | 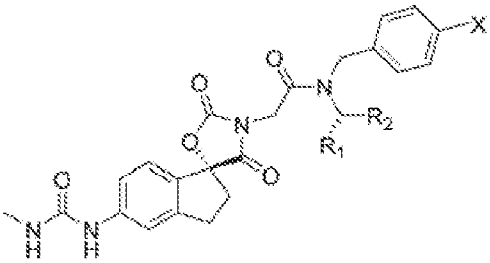 <br> compd / R1 / R2 / X: <br> 22 / Me / cyclopropyl / H <br> 23 / $CF_3$ / cyclopropyl / F <br> 24 / Me / $CF_3$ / F |

| | |
|---|---|
| p300/ CBP HAT | 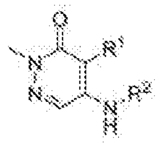<br>$R^1$ $R^2$ $R^1$ $R^2$<br>Cl 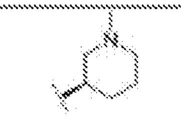 Cl 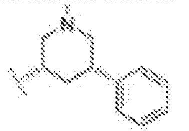<br>Cl 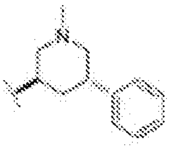 Br 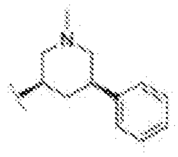<br>Cl 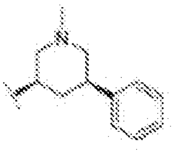 Br 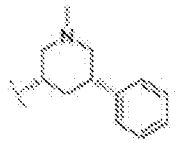 |
| OGT | 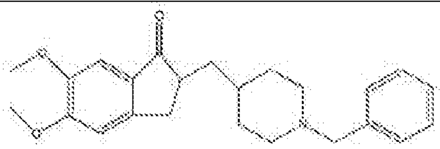 |
| OGT | 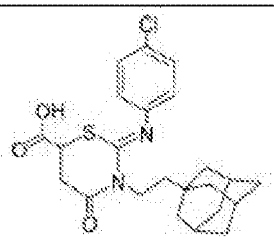 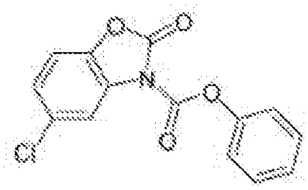 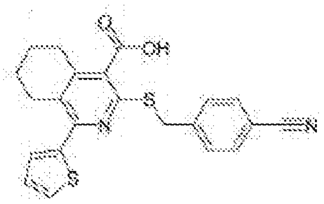 |
| OGT | 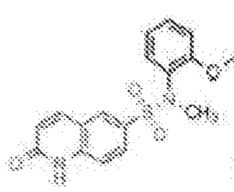 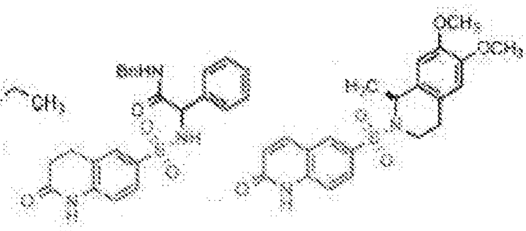 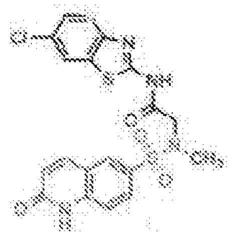 |
| OGT | 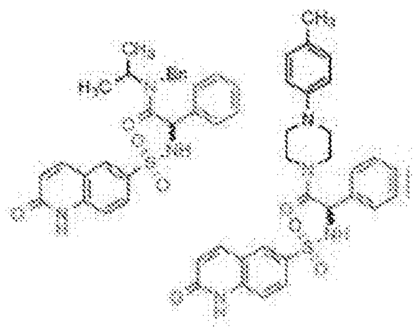 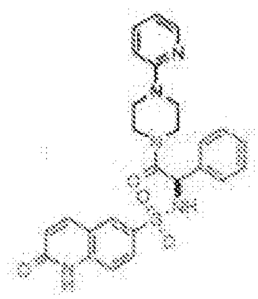 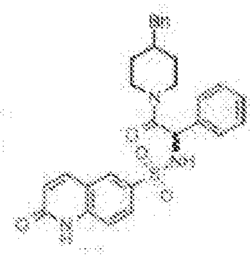 |

| | |
|---|---|
| OGT | 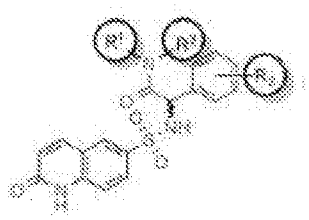 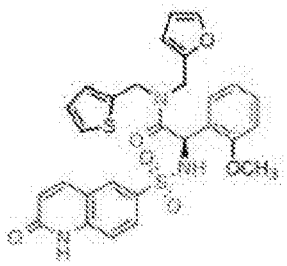 |
| OGT | (d) 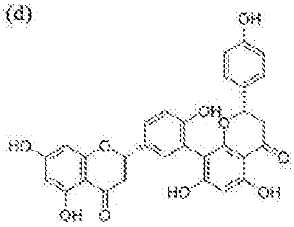 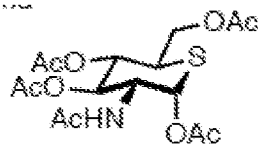 Ac4-5SGlcNAc |
| LFA-1/ICAM-1 | 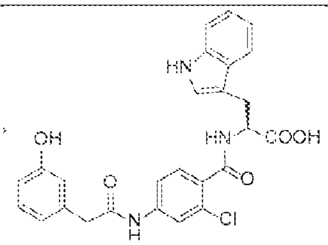 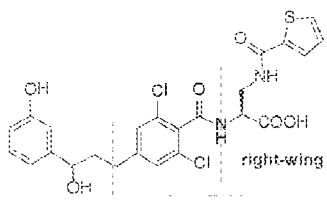 |
| LFA-1/ICAM-1 | 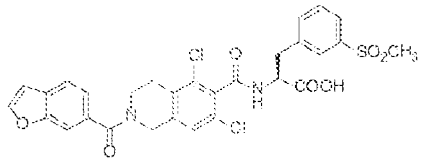 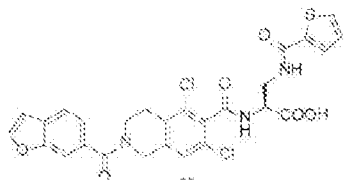 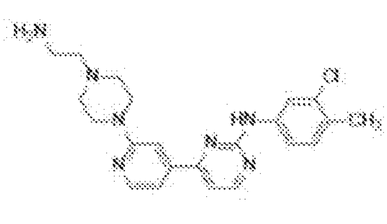 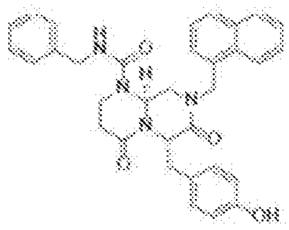 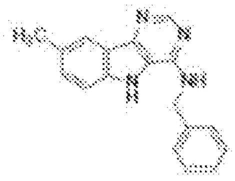 |
| LFA-1/ICAM-1 | 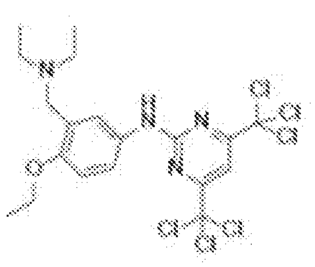 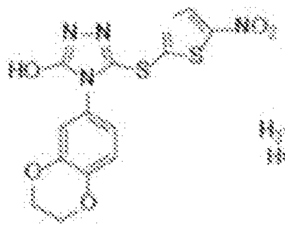 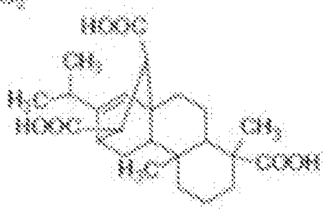 |

| | |
|---|---|
| LFA-1/ICAM-1 | 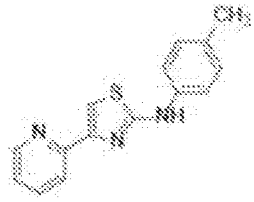 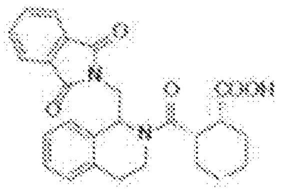 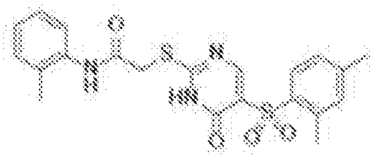 |
| LFA-1/ICAM-1 | 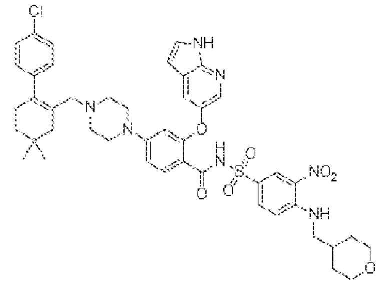 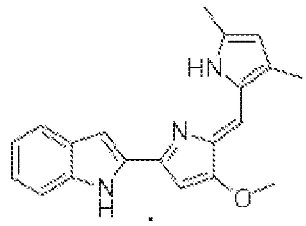 $CH_3SO_3H$ 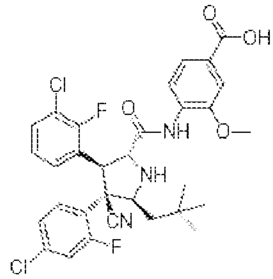 |
| Methyllysine binding/ L3MBTL1 | 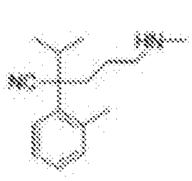 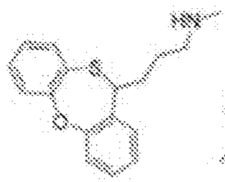 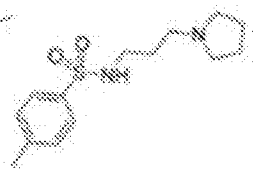<br>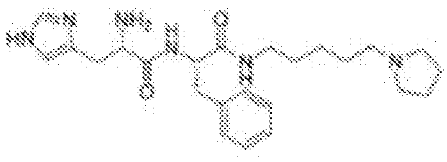 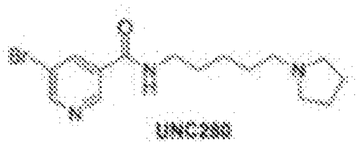 UNC288<br>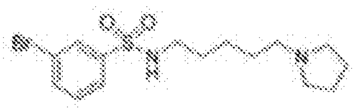 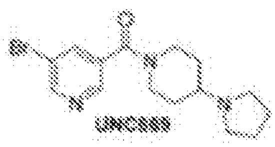 UNC669<br>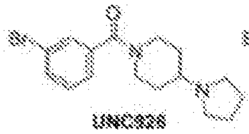 UNC926 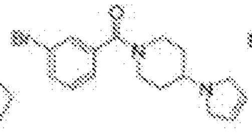 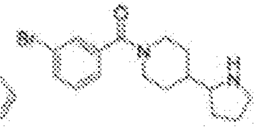 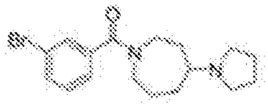 |

| | |
|---|---|
| | 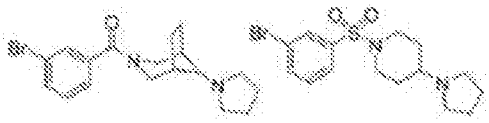 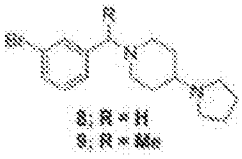<br>8: R = H<br>9: R = Me<br>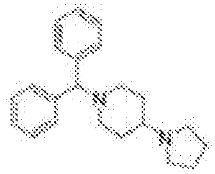 |
| Methyllysine binding/ L3MBTL3 | 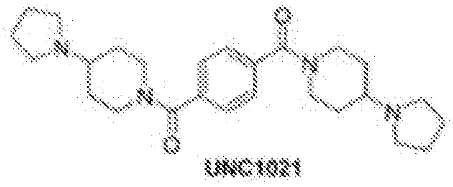<br>UNC1021<br>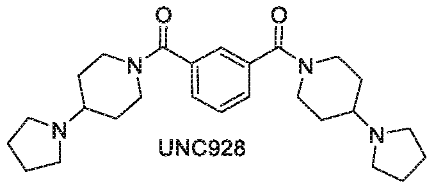<br>UNC928 |
| Methyllysine binding/ L3MBTL3 | 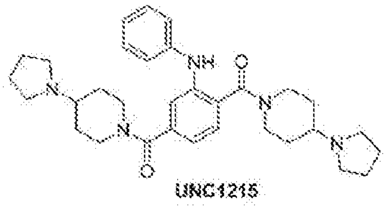<br>UNC1215<br>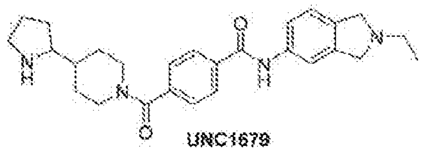<br>UNC1679<br>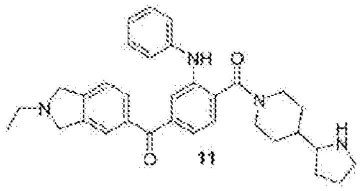<br>11<br>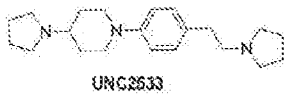<br>UNC2533<br>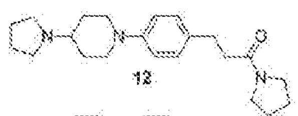<br>12<br>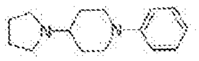<br>13<br>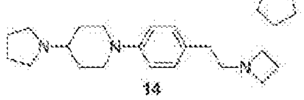<br>14<br>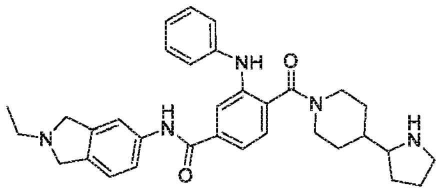 |
| Methyllysine binding/ L3MBTL3 | 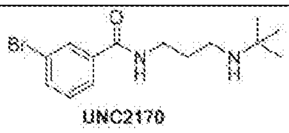<br>UNC2170<br>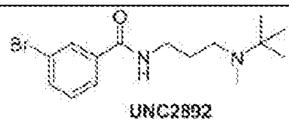<br>UNC2892<br>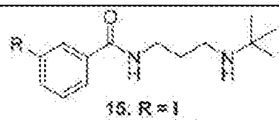<br>15: R = I<br>16: R = *i*-Pr<br>17: R = $CF_3$ |
| Methyllysine binding/ L3MBTL3 | 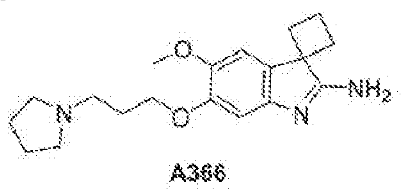<br>A366<br>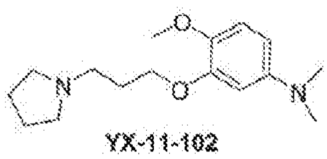<br>YX-11-102 |

| | |
|---|---|
| Chromodomain | 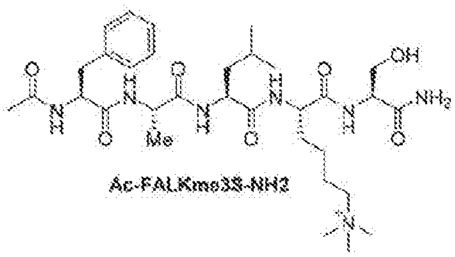 Ac-FALKme3S-NH2 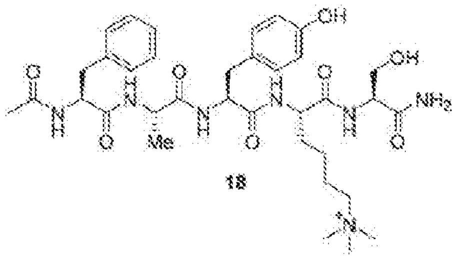 18 |
| Chromodomain | 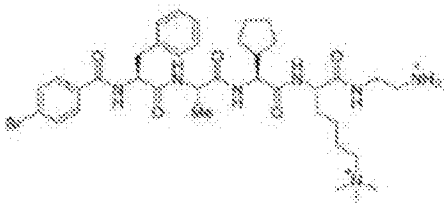 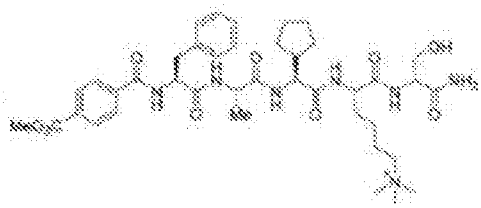 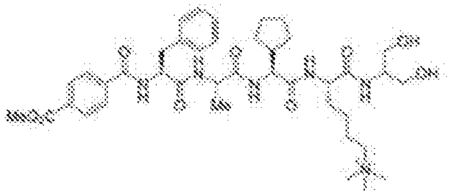 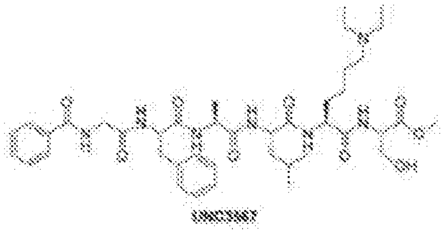 UNC3567 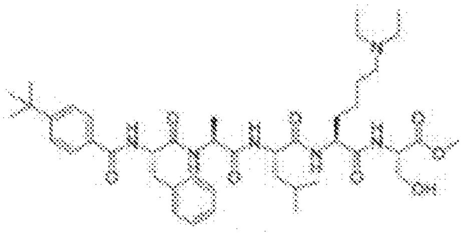 [illegible] 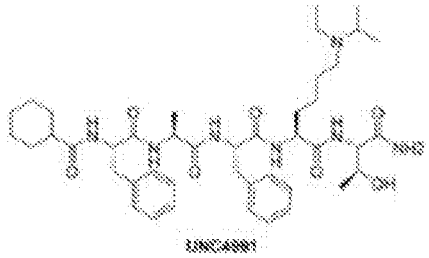 [illegible] |
| Chromodomain | 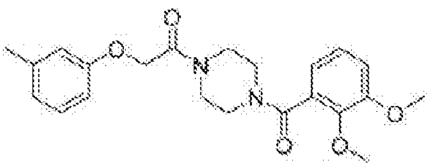 MS37452 (MS452) 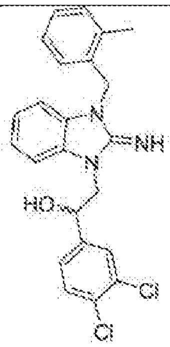 MS351 |

| | |
|---|---|
| Chromodomain | 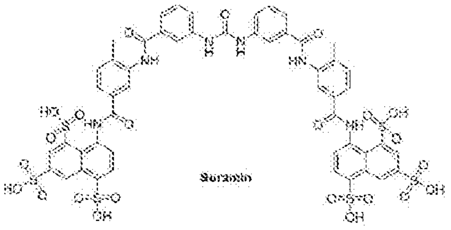 Suramin 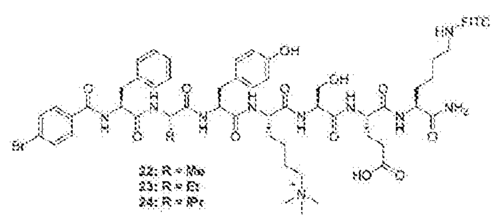 22: R = Me 23: R = Et 24: R = iPr |
| Chromodomain | 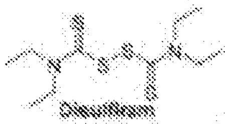 Disulfiram 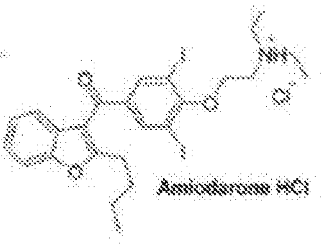 Amiodarone HCl 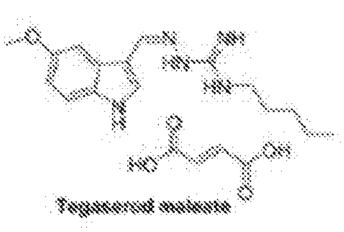 Tegaserod maleate 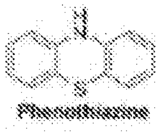 Phenothiazine <br> 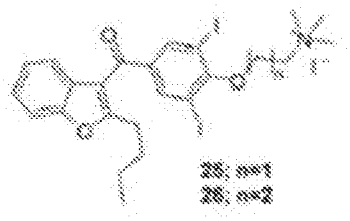 25; n=1 26; n=2 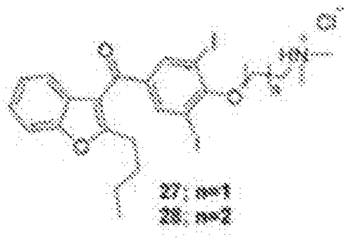 27; n=1 28; n=2 <br> 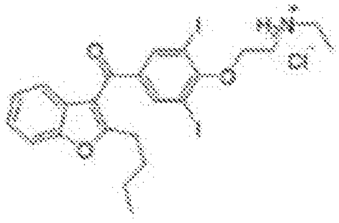 Desethylamiodarone 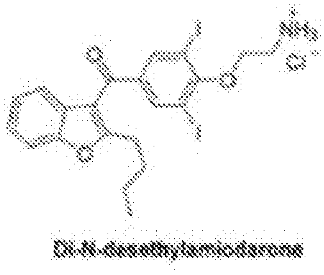 Di-N-desethylamiodarone |
| Chromodomain | 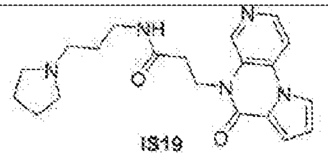 IS19 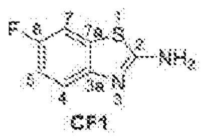 CF1 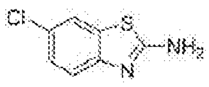 CF2 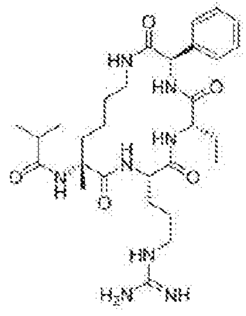 MM-401 <br> 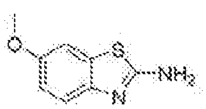 CF4 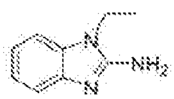 CF16 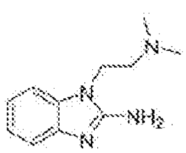 CF18 |

| | |
|---|---|
| | 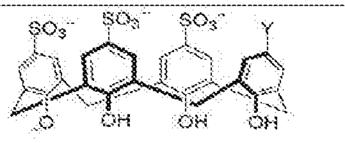<br>Y= 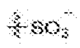 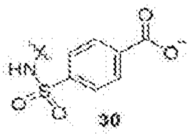 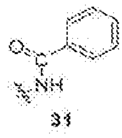<br>29 30 31<br>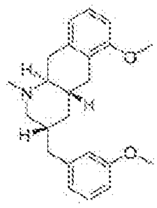 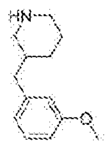 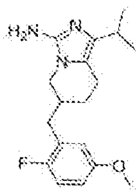<br>32 33 34 |
| Chromodomain/CBX7 | 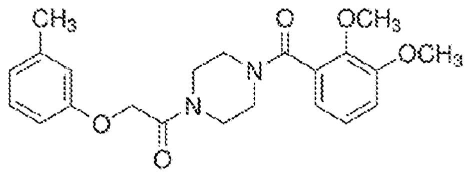 |
| Chromodomain | 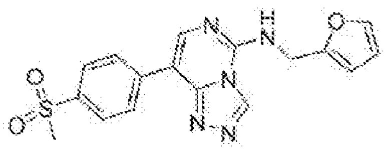<br>EED226<br>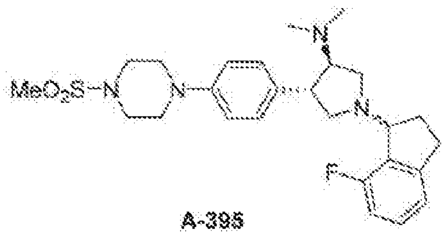<br>A-395 |
| Chromodomain | 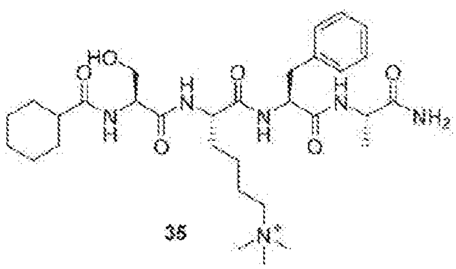<br>35<br>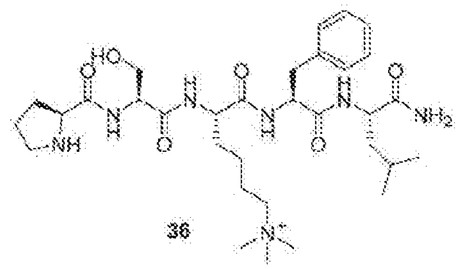<br>36 |
| Chromodomain | 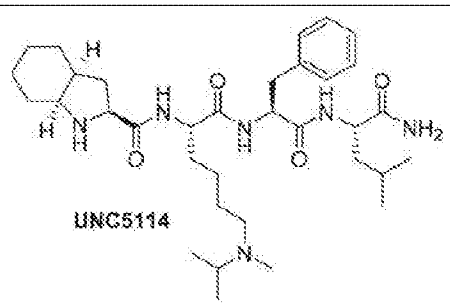<br>UNC5114<br>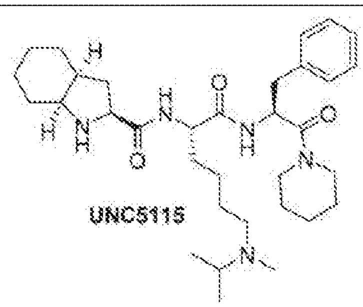<br>UNC5115 |

| | |
|---|---|
| | 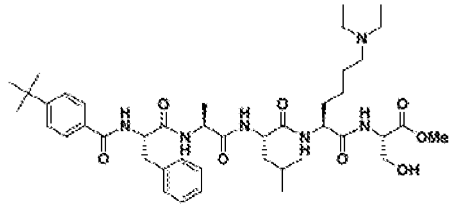<br>UNC3866 |
| Methyl transferase | DOT1L — EPZ004777 (ref. 21), EPZ-5676 (ref. 24), SGC0946 (ref. 86)<br>EZH2 — GSK126 (ref. 37), GSK343 (refs 87, 88), EPZ005687 (ref. 38), EPZ-6438 (ref. 44), EI1 (ref. 39), UNC1999 (ref. 89)<br>G9A — BIX01294 (ref. 90), UNC0321 (ref. 91), UNC0638 (ref. 92), NC0642 (ref. 88), BRD4770 (ref. 93)<br>PRMT3 — 14u (ref. 94)<br>PRMT4 (CARM1) — 17b (Bristol-Myers Squibb) (refs 95, 96), MethylGene (ref. 97) |
| Methyl transferase | BAZ2B — GSK2801 (ref. 88)<br>**Chromodomains**<br>L3MBTL1 — UNC669 (ref. 100)<br>L3MBTL3 — UNC1215 (ref. 101)<br>**Histone demethylases**<br>LSD1 — Tranylcypromine (ref. 62), ORY-1001 (ref. 63) |
| Methyl transferase | **EPZ004777** 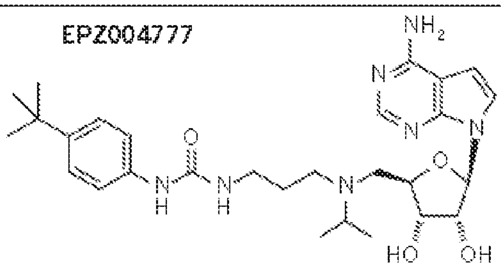 **Br-SAH** 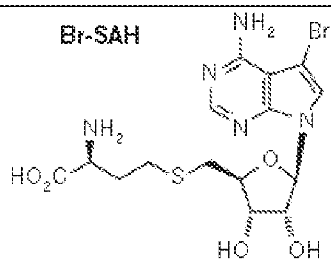 |
| Methyl transferase | **Hybrid** 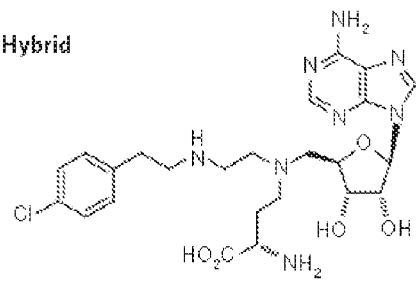 **DZNep** 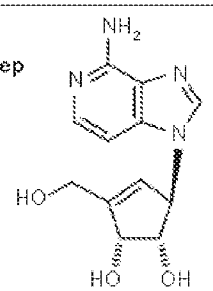 |

| | |
|---|---|
| Methyl transferase | 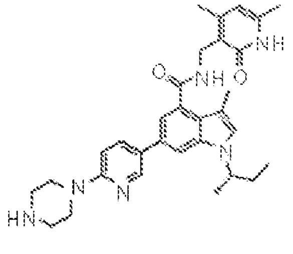 GSK126 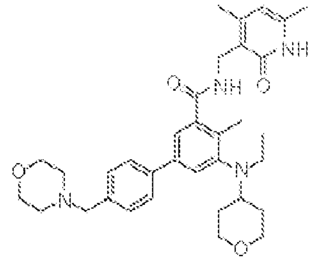 EPZ-6438 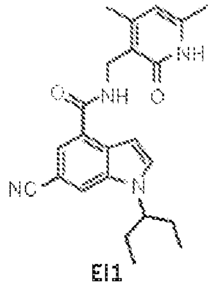 EI1 |
| Methyl transferase | 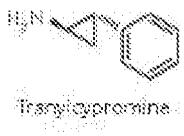 Tranylcypromine 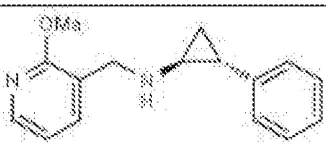 Oryzon LSD1 inhibitor |
| Chormodomain | a) 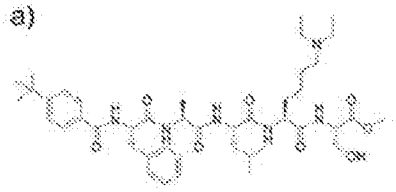 UNC3866 b) 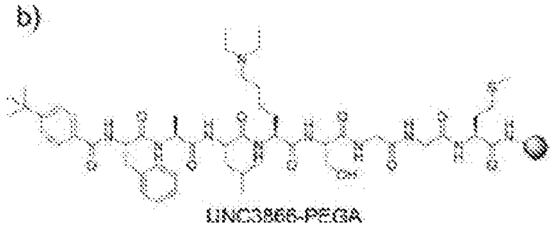 UNC3866-PEGA 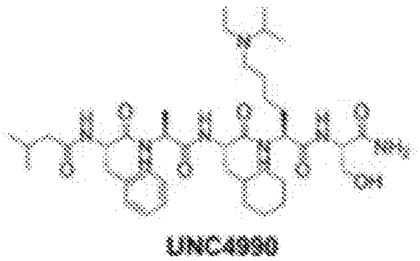 UNC4990 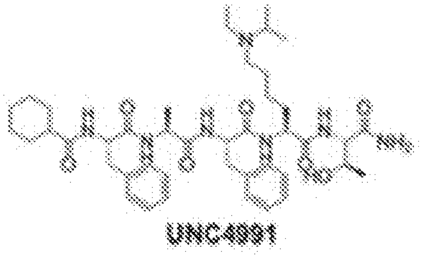 UNC4991 |

| | |
|---|---|
| Chormodomain | a)<br>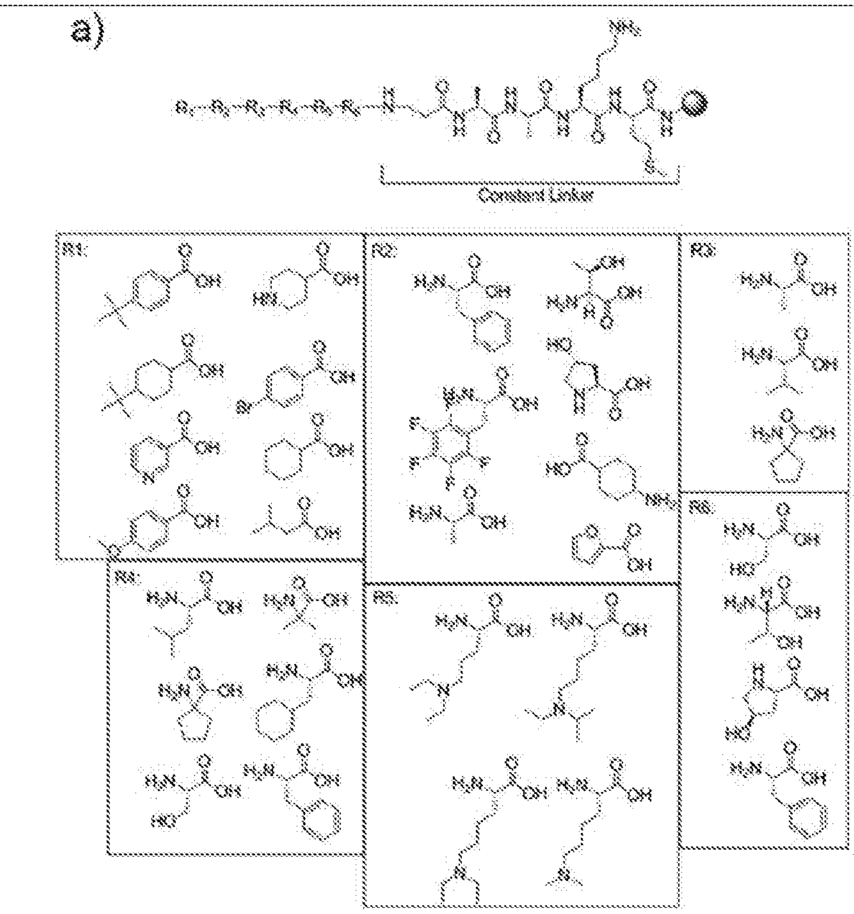<br> |
| Chormodomain | 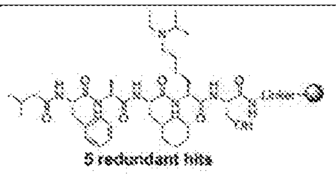<br>5 redundant hits<br>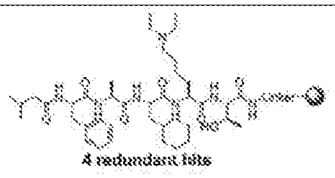<br>4 redundant hits<br>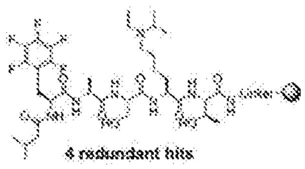<br>4 redundant hits<br>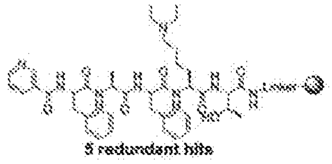<br>5 redundant hits<br>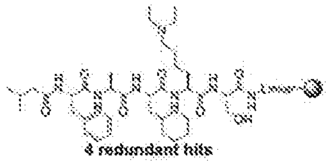<br>4 redundant hits<br>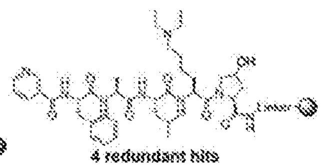<br>4 redundant hits<br>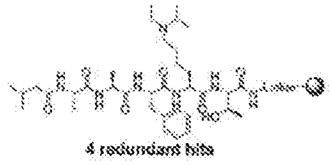<br>4 redundant hits<br>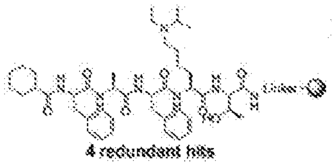<br>4 redundant hits |

| | |
|---|---|
| Chormodomain | a) 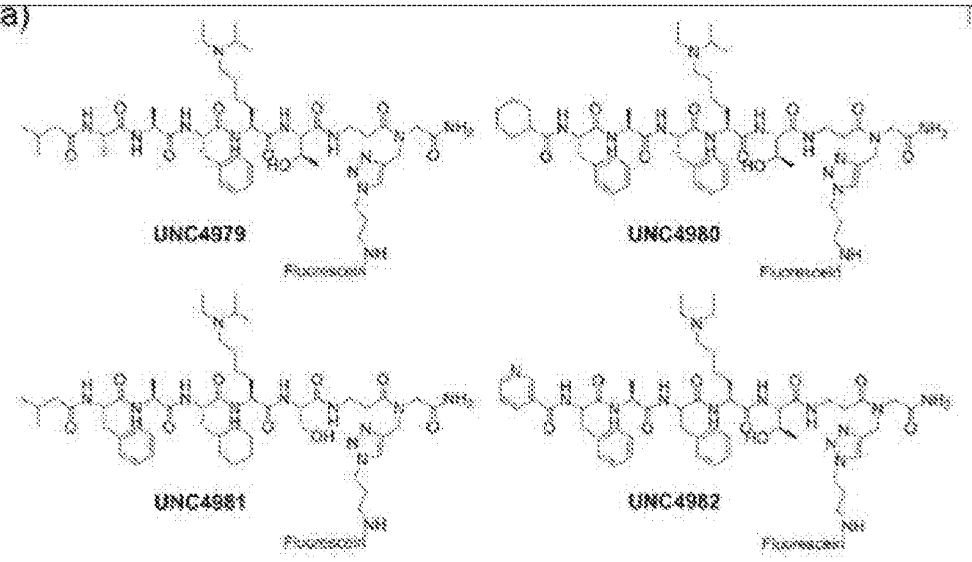 UNC4979 UNC4980 UNC4981 UNC4982 |
| Chormodomain | 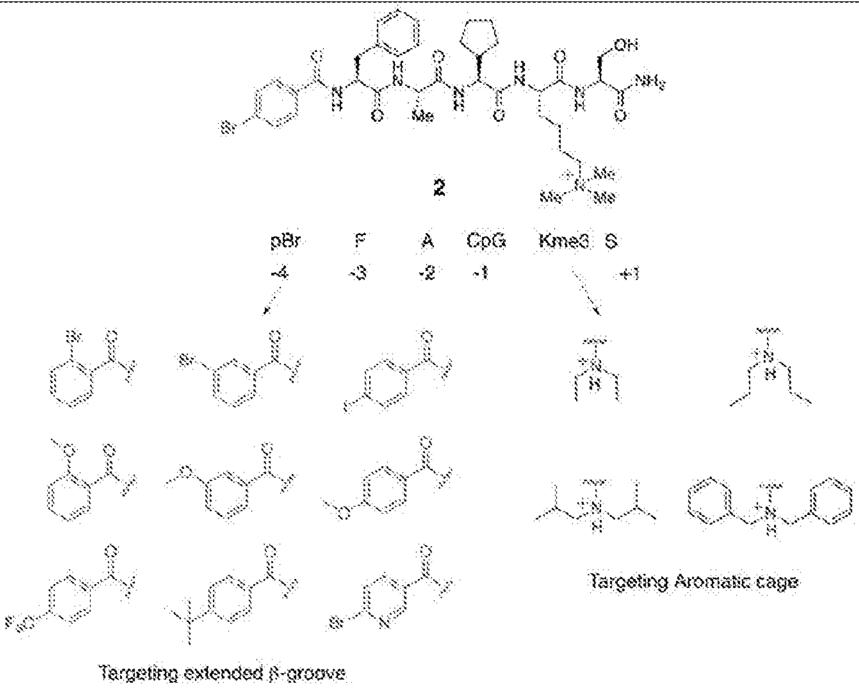  |

| | |
|---|---|
| Chormodomain | 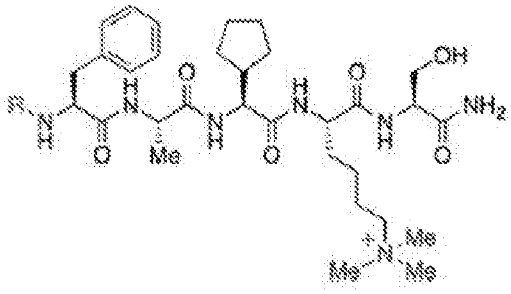 R = 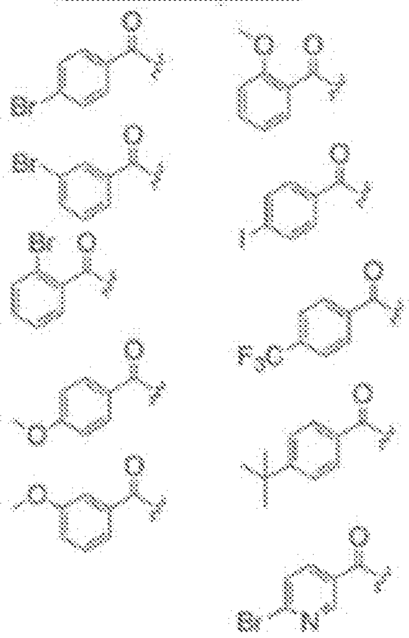 |
| Chormodomain | $NR_3^+$ 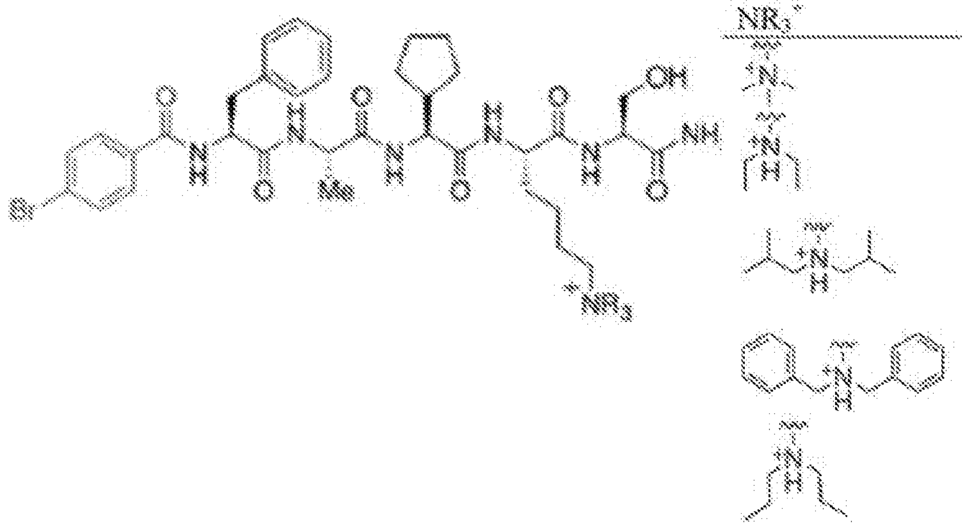 |
| Chormodomain | a) b 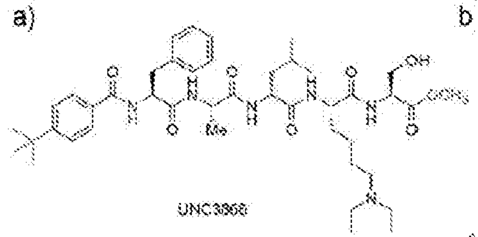 UNC3866 b 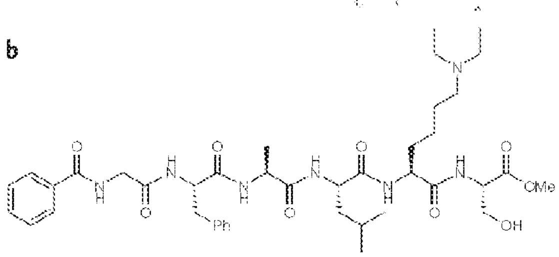 UNC3567 (1) |

| | |
|---|---|
| Chormodomain | UNC4219 (3) 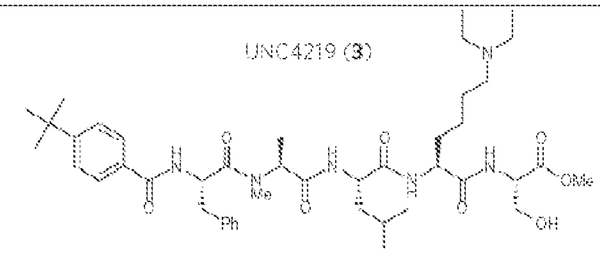 |
| Chormodomain | c 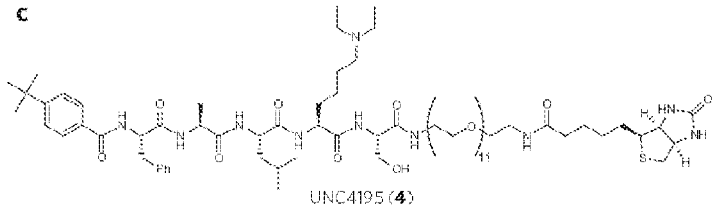 UNC4195 (4) |

| | |
|---|---|
| Methyl lysine binding domain | 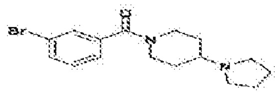 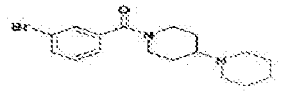 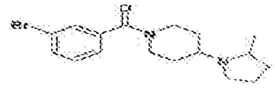 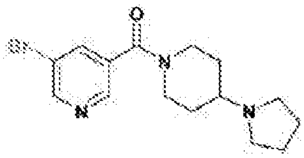 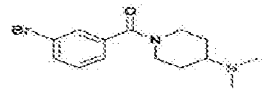 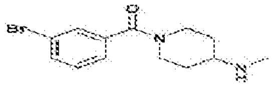 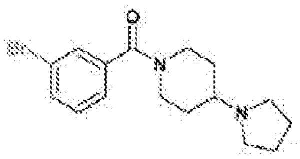 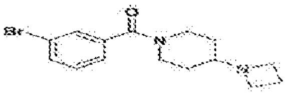 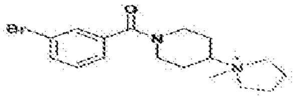 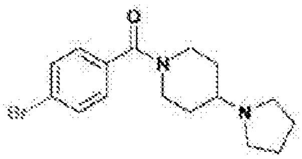 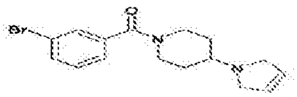 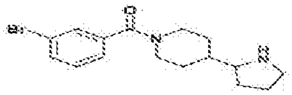 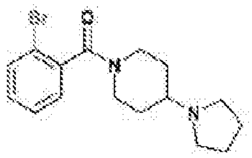 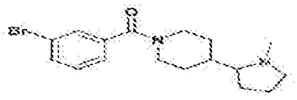 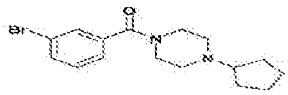 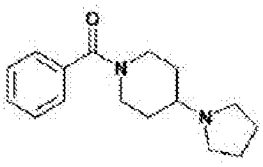 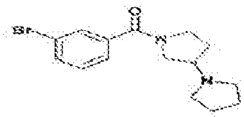 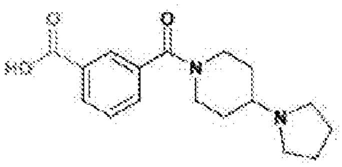 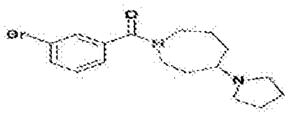 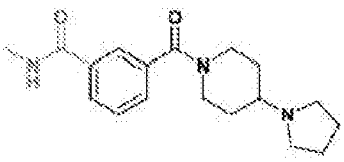 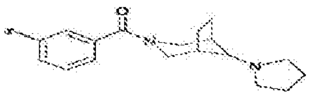 |

| | |
|---|---|
| | 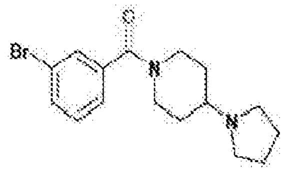<br>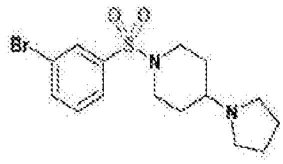<br>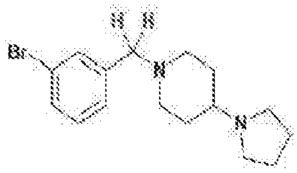<br>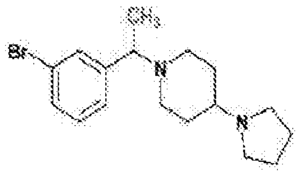<br>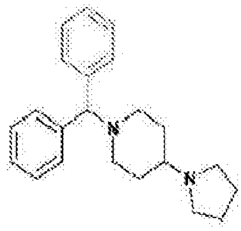 |
| Methyl lysine binding domain | 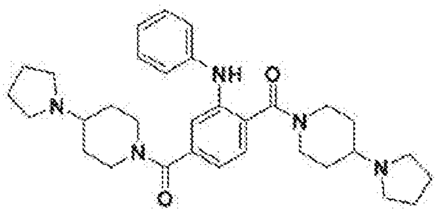<br>UNC1215<br>UNC2533 (1) 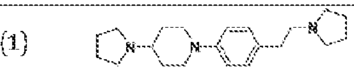<br>UNC669 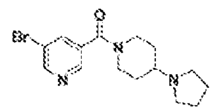<br>UNC1079 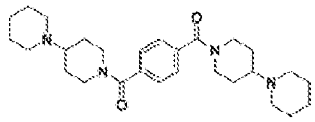<br>UNC1215 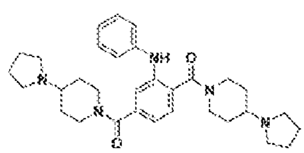 |

| | |
|---|---|
| Methyl lysine binding domain | 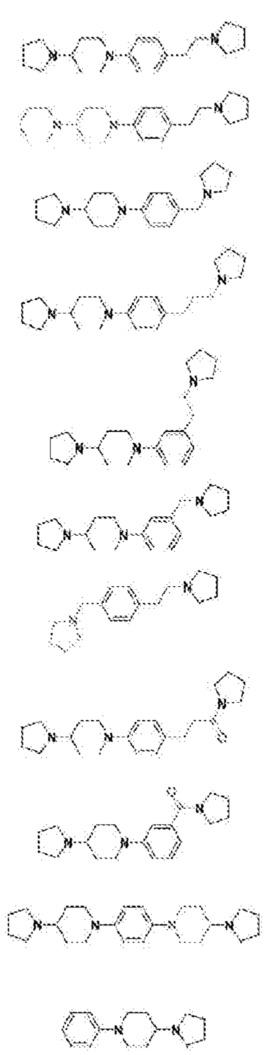 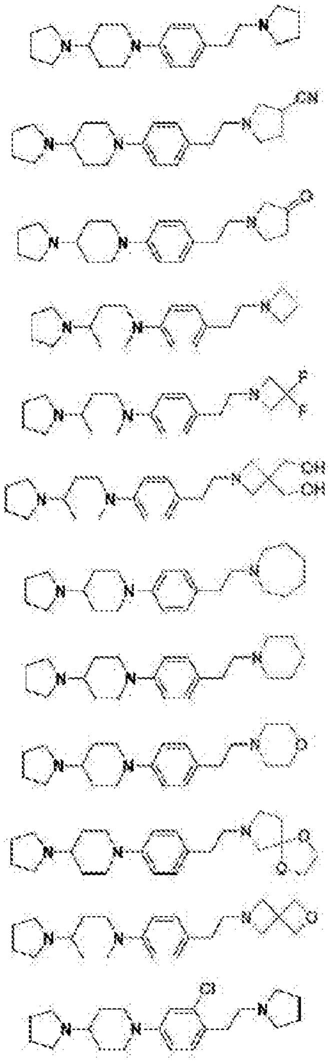 |
| Methyl lysine binding domain | 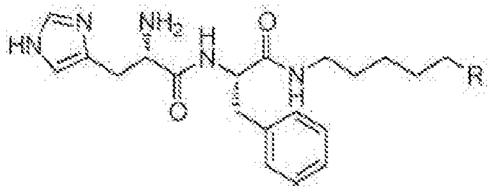 R 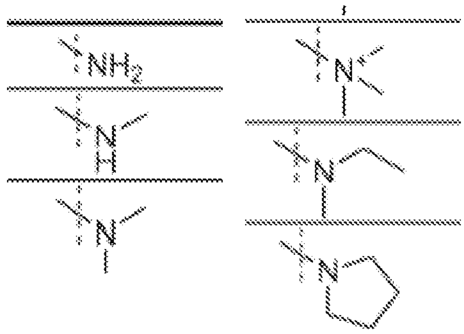 |

| | |
|---|---|
| Methyl lysine binding domain | 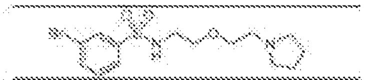 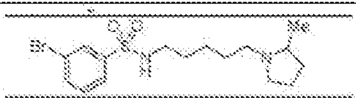 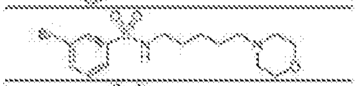 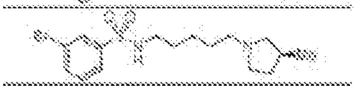 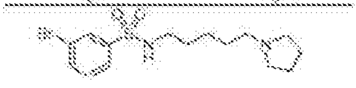 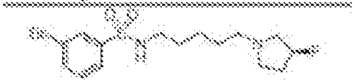 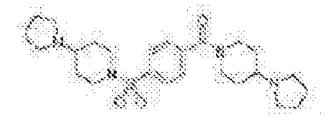 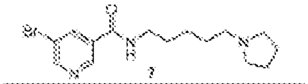 7 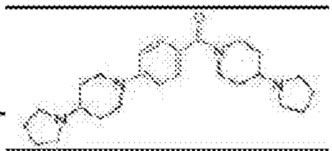 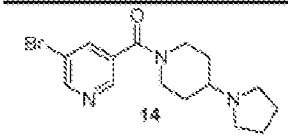 14 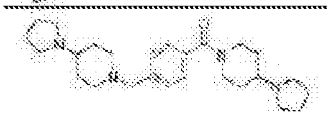 |
| Methyl lysine binding domain | 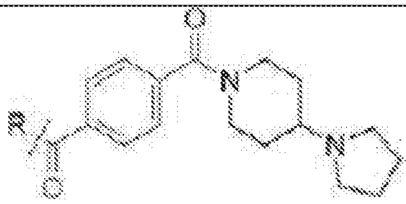 R 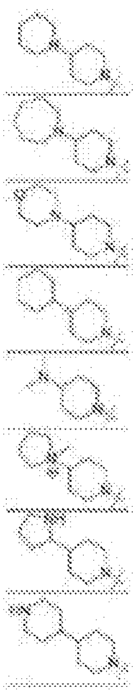 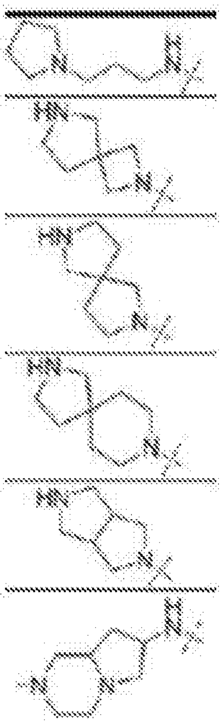 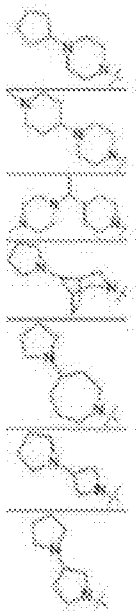 |

| | |
|---|---|
| Methyl lysine binding domain | 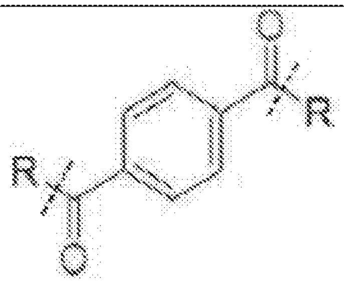 R 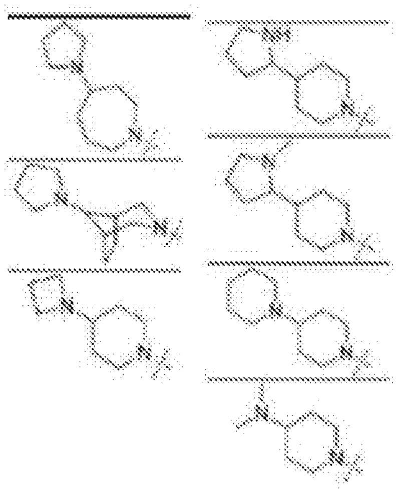 |
| Methyl lysine binding domain | 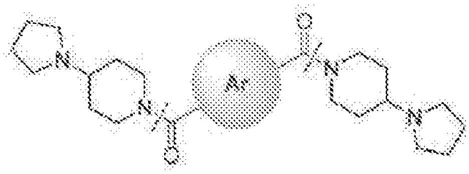 Ar 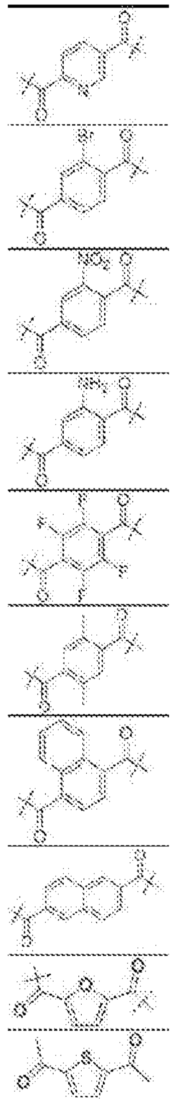 Ar 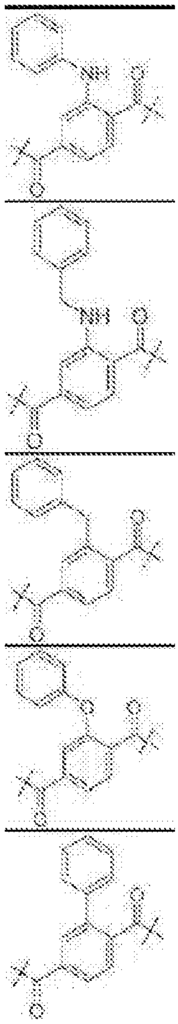 |

| | |
|---|---|
| Methyl lysine binding domain | 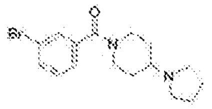 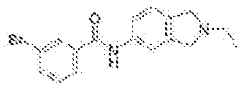 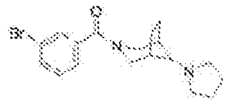 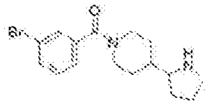 |
| Methyl lysine binding domain | 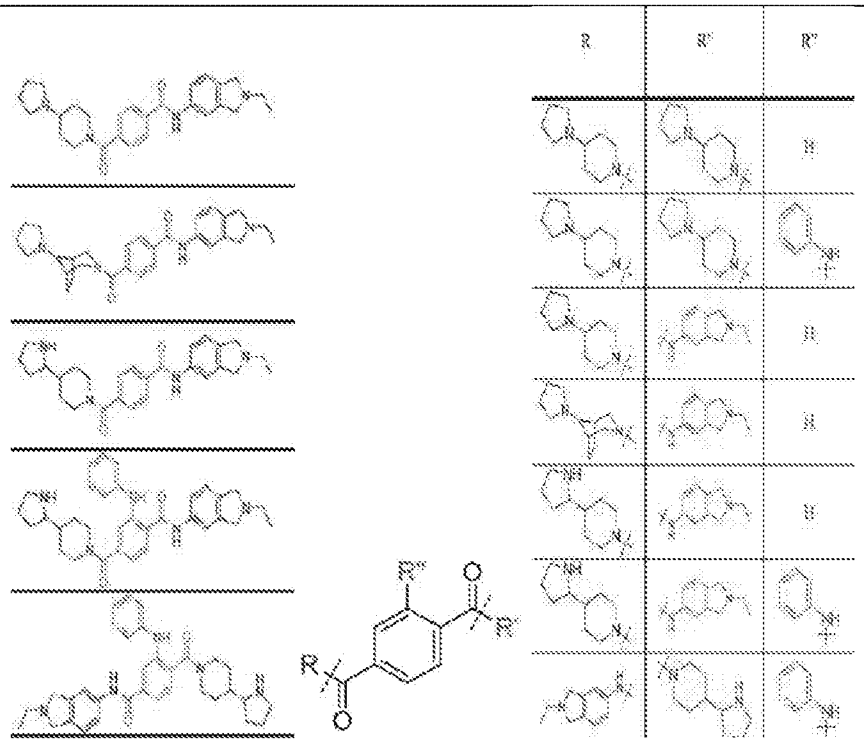 |
| Methyl lysine binding domain | 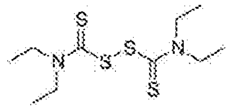<br>Disulfiram<br>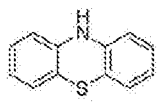<br>Phenothiazine<br>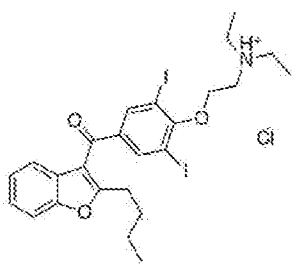<br>Amiodarone HCl<br>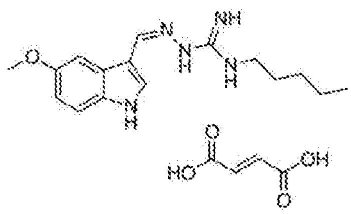<br>Tegaserod maleate |
| Methyl lysine binding domain | 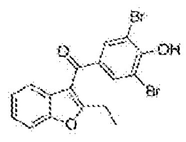<br>Benzbromarone<br>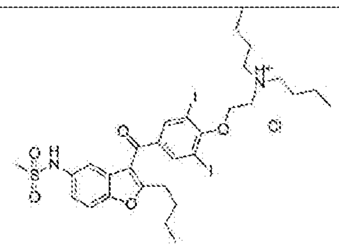<br>Dronedarone<br>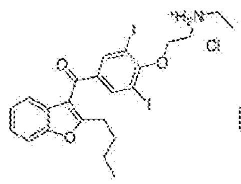<br>Desethylamiodarone<br>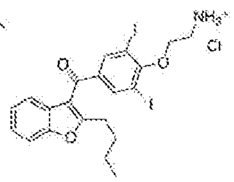<br>Di-N-desethylamiodarone |

| | |
|---|---|
| Methyl lysine binding domain | 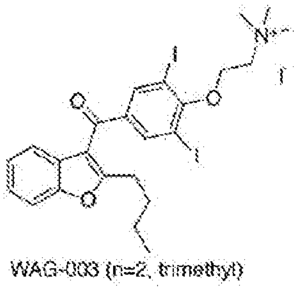 WAG-003 (n=2, trimethyl) 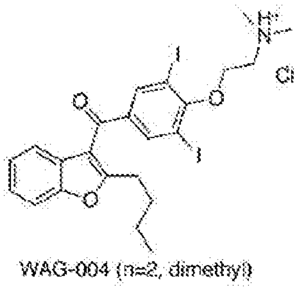 WAG-004 (n=2, dimethyl) 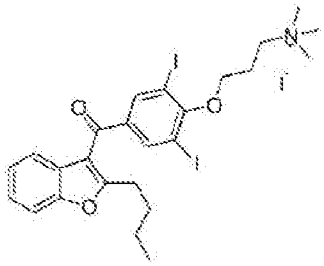 WAG-005 (n=3, trimethyl) 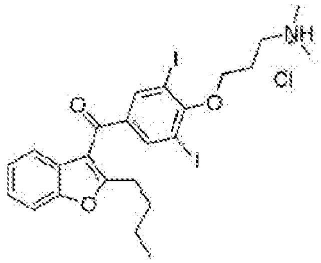 WAG-006 (n=3, dimethyl) |
| Methyl lysine binding domain | IS1 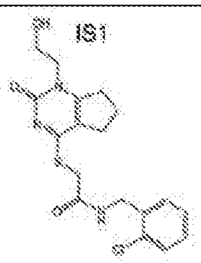 IS2 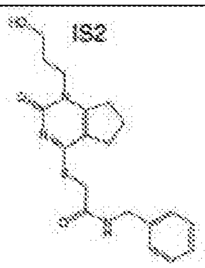 IS3 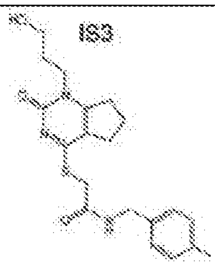 IS5 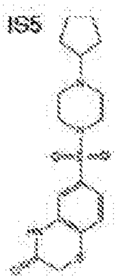 IS12 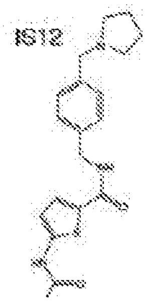 IS15 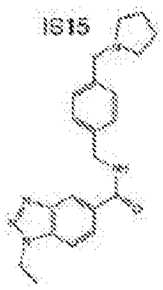 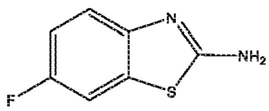 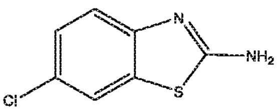 |
| Methyl lysine binding domain | 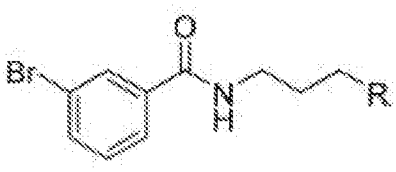 R 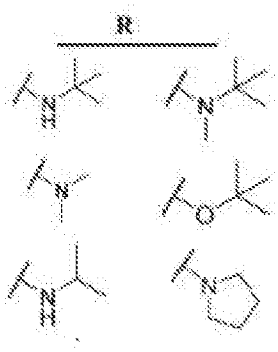 |

| | |
|---|---|
| | 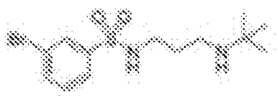 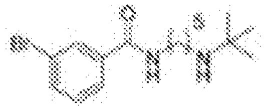 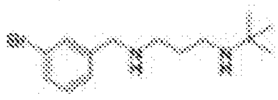 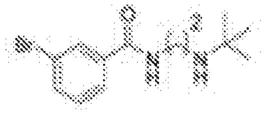 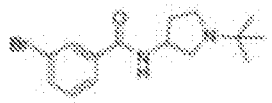 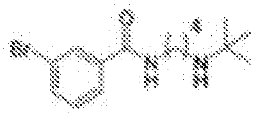 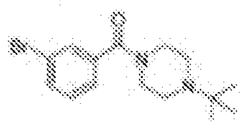 |
| Methyl lysine binding domain | 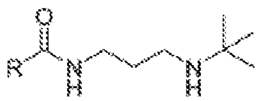 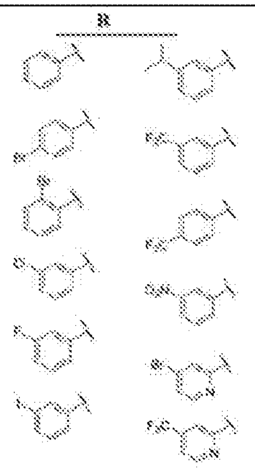 |
| Methyl lysine binding domain | 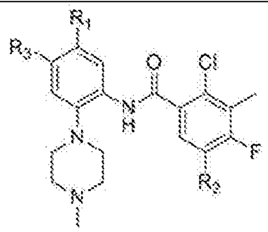 group 1-3 (b) 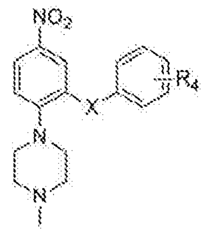 group 4 |
| Methyl lysine binding domain | 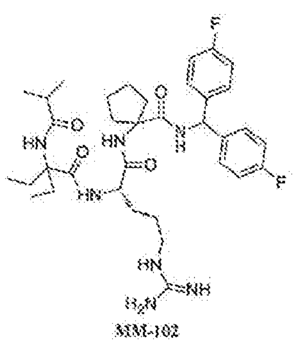 MM-102 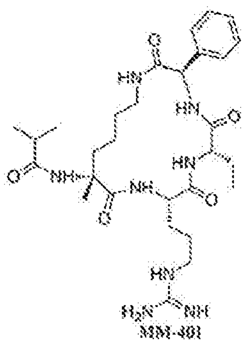 MM-401 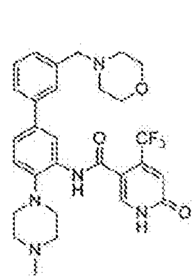 OICR-9429 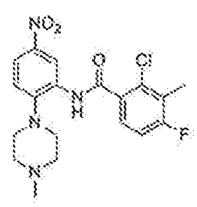 WDR5-47 |

<table>
<tr><td>Methyl lysine binding domain</td><td>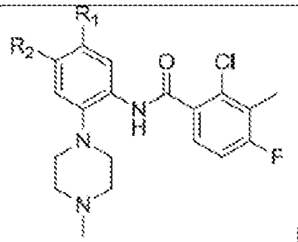<br>18 $R_1$=3-COOH-Ph $R_2$=-H<br>19 $R_1$=4-COOH-Ph $R_2$=-H<br>20 $R_1$=4-CN-Ph $R_2$=-H<br>21 $R_1$=-Ph $R_2$=-H<br>22 $R_1$=4-F-Ph $R_2$=-H<br>23 $R_1$=4-Pyridinyl $R_2$=-H<br>24 $R_1$=5-Pyrimidyl $R_2$=-H<br>25 $R_1$=4-$NO_2$-Ph $R_2$=-H<br>h<br>26 $R_1$=4-$NH_2$-Ph $R_2$=-H<br>29 $R_2$=4-COOH-Ph $R_1$=-H<br>30 $R_2$=4-Pyridinyl $R_1$=-H<br>31 $R_2$=4-$NO_2$-Ph $R_1$=-H<br>h<br>32 $R_2$=4-$NH_2$-Ph $R_1$=-H</td></tr>
<tr><td>Methyl lysine binding domain</td><td>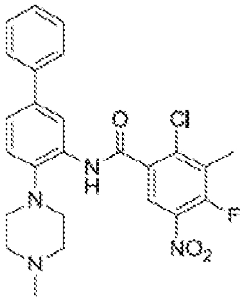 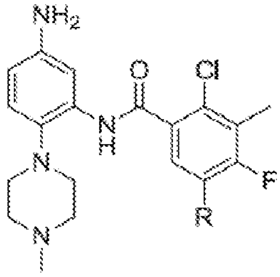 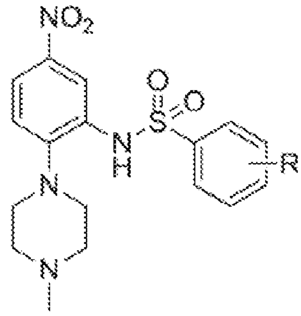<br>33 R=4-fluoro<br>34 R=4-methoxyl<br>35 R=3,4-dimethoxyl<br>36 R=2,4,6-trimethyl</td></tr>
<tr><td>Methyl lysine binding domain</td><td>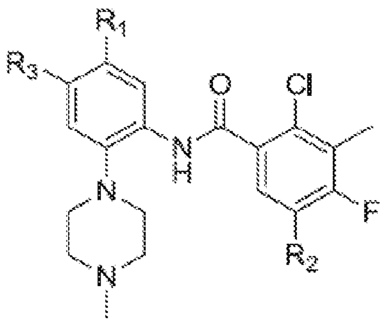
<table>
<tr><th>$R_1$</th><th>$R_2$</th><th>$R_3$</th></tr>
<tr><td>$-NH_2$</td><td>-H</td><td>-H</td></tr>
<tr><td>3-COOH-Ph</td><td>-H</td><td>-H</td></tr>
<tr><td>4-COOH-Ph</td><td>-H</td><td>-H</td></tr>
<tr><td>4-CN-Ph</td><td>-H</td><td>-H</td></tr>
<tr><td>-Ph</td><td>-H</td><td>-H</td></tr>
<tr><td>4-F-Ph</td><td>-H</td><td>-H</td></tr>
<tr><td>4-Pyridyl</td><td>-H</td><td>-H</td></tr>
<tr><td>5-Pyrimidyl</td><td>-H</td><td>-H</td></tr>
<tr><td>4-$NO_2$-Ph</td><td>-H</td><td>-H</td></tr>
<tr><td>4-$NH_2$-Ph</td><td>-H</td><td>-H</td></tr>
<tr><td>-Ph</td><td>$-NO_2$</td><td>-H</td></tr>
<tr><td>$-NO_2$</td><td>$-NO_2$</td><td>-H</td></tr>
<tr><td>-H</td><td>-H</td><td>4-COOH-Ph</td></tr>
<tr><td>-H</td><td>-H</td><td>4-Pyridyl</td></tr>
<tr><td>-H</td><td>-H</td><td>4-$NO_2$-Ph</td></tr>
<tr><td>-H</td><td>-H</td><td>4-$NH_2$-Ph</td></tr>
<tr><td>$-NO_2$</td><td>-H</td><td>-H</td></tr>
</table></td></tr>
<tr><td>Methyl lysine binding domain</td><td>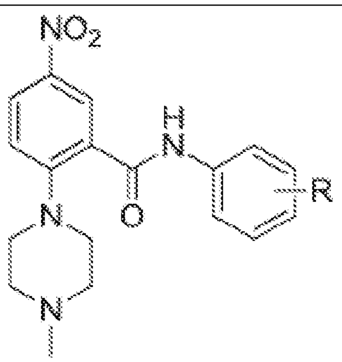</td></tr>
</table>

| | |
|---|---|
| | 37a R=4-fluoro-2-chloro-3-methyl 37 R=4-fluoro-2-chloro-3-methyl<br>38a R=3-methoxyl 38 R=3-methoxyl<br>39a R=2,4-difluoro 39 R=2,4-difluoro<br>40a R=2-chloro 40 R=2-chloro |
| Methyl lysine binding domain | 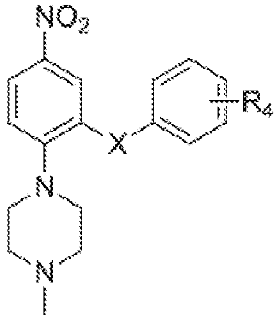<br>X — $R_4$<br>$-NHSO_2-$ — 4-fluoro<br>$-NHSO_2-$ — 4-methoxyl<br>$-NHSO_2-$ — 3,4-dimethoxyl<br>$-NHSO_2-$ — 2,4,6-trimethyl<br>$-CONH-$ — 4-fluoro-2-chloro-3-methyl<br>$-CONH-$ — 3-methoxyl<br>$-CONH-$ — 2,4-difluoro<br>$-CONH-$ — 2-chloro<br>$-NHCO-$ — 4-fluoro-2-chloro-3-methyl |
| Methyl lysine binding domain | 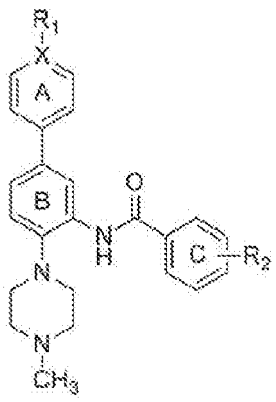 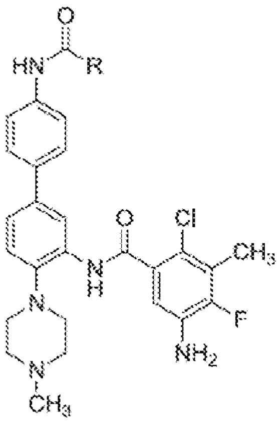<br>$R=-CH_3$<br>R= 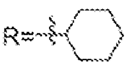<br>R=-Ph<br>$R=-CH_2CH_3$<br>$R=-CH(CH_3)_2$<br>$R=-CH_2CH_2CH_3$<br>$R=-CH_2NH$-Boc<br>$R=-CH(CH_3)NH$-Boc<br>$R=-CH_2CH_2NH$-Boc<br>$R=-C(CH_3)_2NH$-Boc<br>R= 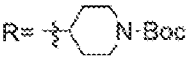<br>$R=-(CH_2)_3NH$-Boc<br>$R=-CH_2CH(CH_3)_2$<br>R= 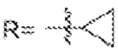 |
| Methyl lysine binding domain | 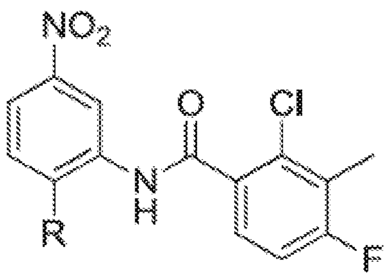<br>R<br>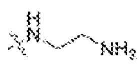<br>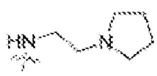<br>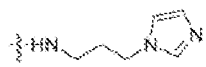<br>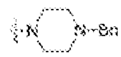<br>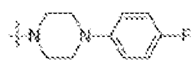<br>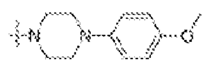<br>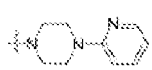<br>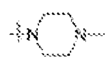 |

| | | | |
|---|---|---|---|
| Methyl lysine binding domain | 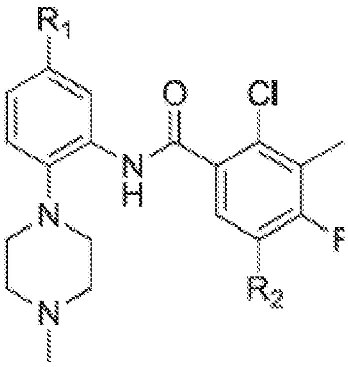 | $R_1$ | $R_2$ |
| | | $-Ph$ | $-H$ |
| | | 4-Pyridyl | $-H$ |
| | | $4-NH_2-Ph$ | $-H$ |
| | | $-Ph$ | $-NO_2$ |
| | | $4-NO_2-Ph$ | $-NHCOCH_3$ |
| | | 4-Pyridyl | $-NO_2$ |
| | | $4-COOCH_3-Ph$ | $-NO_2$ |
| | | $-Ph$ | $-NH_2$ |
| | | 4-Pyridyl | $-NH_2$ |
| | | $4-COOCH_3-Ph$ | $-NH_2$ |
| | | $4-NH_2-Ph$ | $-NHCOCH_3$ |
| | | 4-Pyridyl | $-NHCOCH_3$ |
| | | $4-NO_2-Ph$ | $-NO_2$ |
| | | $4-NH_2-Ph$ | $-NH_2$ |
| Methyl lysine binding domain | 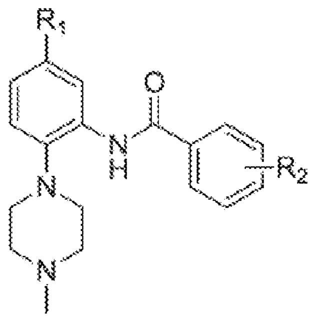 | $R_1$ | $R_2$ |
| | | $4-NO_2-Ph$ | $4-F-3-NO_2$ |
| | | $4-NO_2-Ph$ | $3-NO_2$ |
| | | $4-NH_2-Ph$ | $4-F-3-NH_2$ |
| | | $4-NH_2-Ph$ | $3-NH_2$ |
| | | 4-Pyridyl | $4-F-3-NO_2$ |
| | | 4-Pyridyl | $4-F-3-NH_2$ |
| Methyl lysine binding domain | 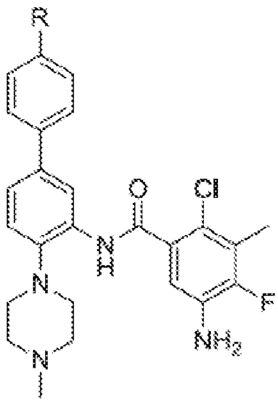 | R | |
| | | $-NHCOCH_3$ | |
| | | 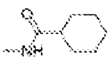 | |
| | | $-NHCOPh$ | |
| | | $-NHCOCH_2CH_3$ | |
| | | $-NHCOCH(CH_3)_2$ | |
| | | $-NHCOCH_2CH_2CH_3$ | |
| | | $-NHCOCH_2NH_2$ | |
| | | $-NHCOCH_2NHBoc$ | |
| | | $-NHCOCH(CH_3)NH_2$ | |
| | | $-NHCOCH(CH_3)NHBoc$ | |

| | |
|---|---|
| | R<br>$-NHCOCH_2CH_2NH_2$<br>$-NHCOCH_2CH_2NHBoc$<br><br>$-NHCOCH(i\text{-}Pro)NH_2$<br>$-NHCOCH(i\text{-}Pro)NHBoc$<br>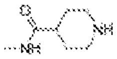<br>$-NHCO(CH_2)_3NH_2$<br>$-NHCO(CH_2)_3NHBoc$<br>$-NHCOCH_2CH(CH3)_2$<br>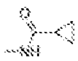 |
| Methyl lysine binding domain | 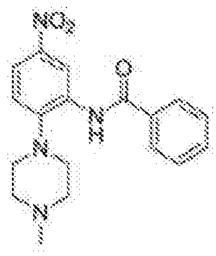 WDR5-0101 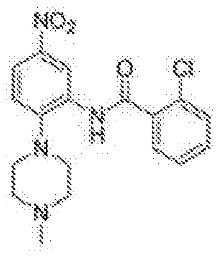 WDR5-0102 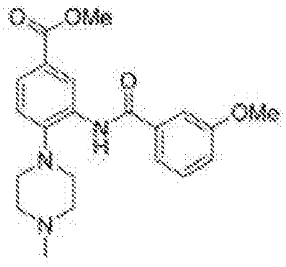 WDR5-0103 |
| Methyl lysine binding domain | 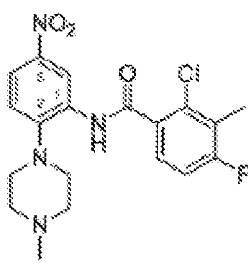 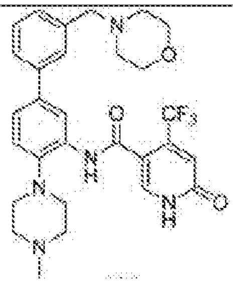 |
| Methyl lysine binding domain | A 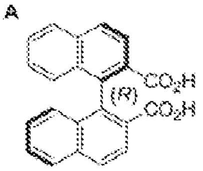 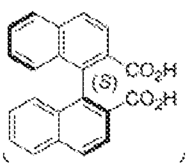 B 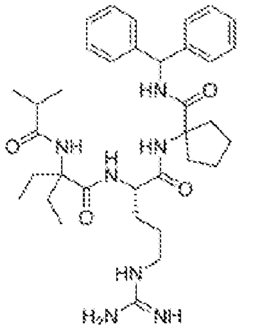 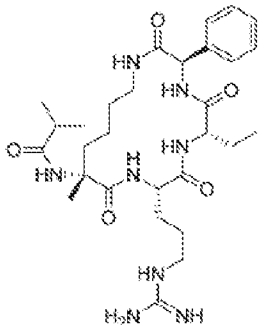 |

| | |
|---|---|
| Methyl lysine binding domain | 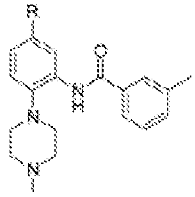 R 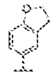 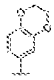 Br 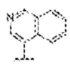 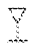 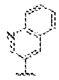 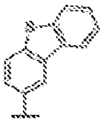 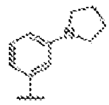 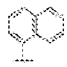 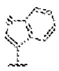 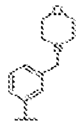 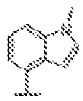 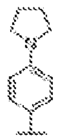 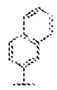 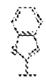 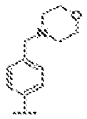 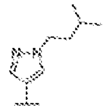 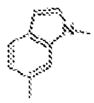 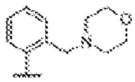 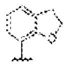 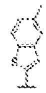 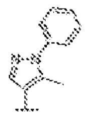 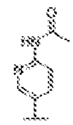 |
| Methyl lysine binding domain | 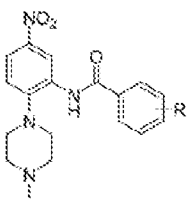 R<br>2-$CF_3$, 5-F<br>2-$CF_3$, 4-OH<br>2-Cl, 4-$CF_3$<br>2-Cl, 5-$CF_3$<br>2-Cl, 5-Me<br>2-Cl, 6-F<br>3-$CF_3$, 4-OMe<br>3-Me, 5-Me<br>3-Me, 5-$CF_3$<br>3-F, 5-$CF_3$<br>3-Cl, 5-Cl<br>3-OH, 5-$CF_3$<br>2-F, 5-$SO_2NH_2$<br>2-F, 3-F, 5-OH<br>2-F, 3-Cl, 5-$CF_3$<br>2-Cl, 3-Me, 6-F<br>2-F, 3-Me, 4-F<br>2-Me, 3-F, 5-F<br>3-Me, 4-F, 5-Me<br>2-F, 3-Me, 4-F, 5-Me, 6-F |

| | |
|---|---|
| Methyl lysine binding domain | 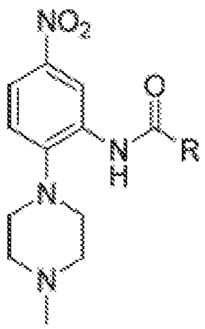 R 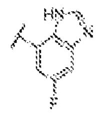 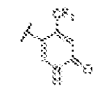 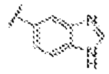 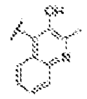 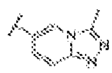 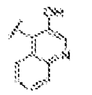 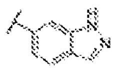 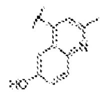 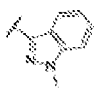 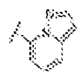 |

| | |
|---|---|
| Methyl lysine binding domain | 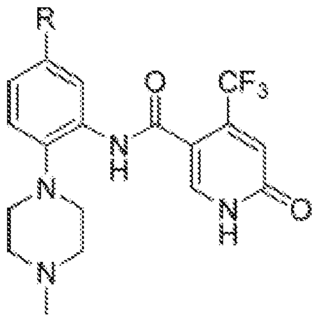<br>**R**<br>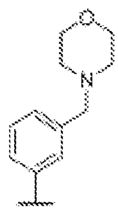<br>$NO_2$<br>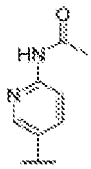<br>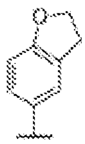<br>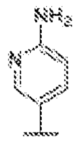<br>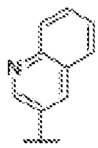<br>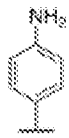<br>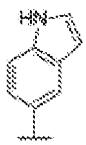<br>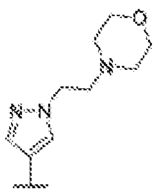 |
| Methyl lysine binding domain | 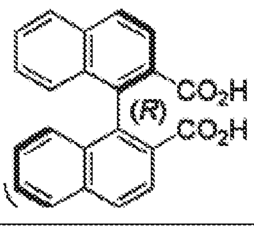 , 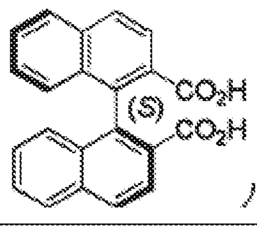 , 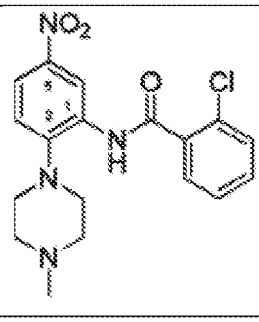 |

| | |
|---|---|
| Methyl lysine binding domain | 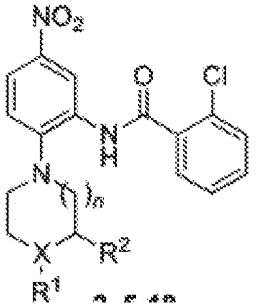<br>X = N, $R^1$ = Me, $R^2$ = H, *n* = 1<br>X = N, $R^1$ = Me, $R^2$ = Me, *n* = 1<br>X = N, $R^1$ = Me, $R^2$ = H, *n* = 2<br>X = O, $R^2$ = H, *n* = 1<br>X = $CH_2$, $R^2$ = H, *n* = 1<br>X = N, $R^1$ = Et, $R^2$ = H, *n* = 1<br>X = CH, $R^1$ = $NMe_2$, $R^2$ = H, *n* = 0<br>X = CH, $R^1$ = $NMe_2$, $R^2$ = H, *n* = 1<br>X = N, $R^1$ = Boc, $R^2$ = H, *n* = 1<br>X = N, $R^1$ = H, $R^2$ = H, *n* = 1<br>X = CH, $R^1$ = NHBoc, $R^2$ = H, *n* = 0<br>X = CH, $R^1$ = $NH_2$, $R^2$ = H, *n* = 0<br>X = CH, $R^1$ = NHBoc, $R^2$ = H, *n* = 1<br>X = CH, $R^1$ = $NH_2$, $R^2$ = H, *n* = 1<br>X = NMe, $R^1$ = Me, $R^2$ = H, *n* = 1 |
| Methyl lysine binding domain | 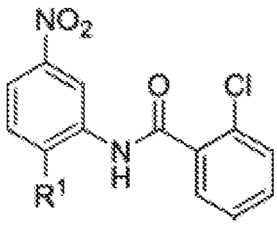<br>$R^1$ (2° amine)<br>1-methylpiperazine<br>F<br>1,2-dimethylpiperazine<br>1-methyl-1,4-diazepane<br>morpholine<br>piperidine<br>1-ethylpiperazine<br>$N^{1,1}$-dimethylpyrrolidin-3-amine<br>$N^{1,1}$-dimethylpiperidin-4-amine<br>piperazine<br>pyrrolidin-3-amine<br>piperidin-4-amine<br>$N^{1,1,2}$-trimethylethane-1,2-diamine |
| Methyl lysine binding domain | 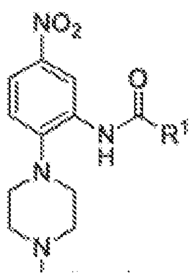<br>$R^1$ = Me<br>$R^1$ = 3-Cl-Ph<br>$R^1$ = 3-Me-Ph<br>$R^1$ = 2-Cl,3-Me-Ph<br>$R^1$ = 3-OH-Ph<br>$R^1$ = 3-OMe-Ph<br>$R^1$ = 4-F-Ph<br>$R^1$ = 2-Cl,4-F-Ph<br>$R^1$ = 3-Me,4-F-Ph<br>**46** $R^1$ = 3-OMe,4-F-Ph<br>**47** $R^1$ = 2-Cl,3-Me,4-F-Ph<br>**48** $R^1$ = phenyl<br>**49** $R^1$ = cyclohexyl<br>**50** $R^1$ = 1-naphthyl<br>**51** $R^1$ = 5-quinolyl<br>**52** $R^1$ = benzyl<br>**53** $R^1$ = 3-pyridyl<br>**54** $R^1$ = 2-furanyl |

| | |
|---|---|
| | $R^1$<br>2-Cl-phenyl<br>Me<br>3-Cl-phenyl<br>3-Me-phenyl<br>2-Cl-3-Me-phenyl<br>3-OH-phenyl<br>3-OMe-phenyl<br>4-F-phenyl<br>2-Cl-4-F-phenyl<br>3-Me-4-F-phenyl<br>3-OMe-4-F-phenyl<br>2-Cl-3-Me-4-F-phenyl<br>phenyl<br>cyclohexyl<br>1-naphthyl<br>5-quinolyl<br>benzyl<br>3-pyridyl<br>2-furanyl |
| Methyl lysine binding domain | 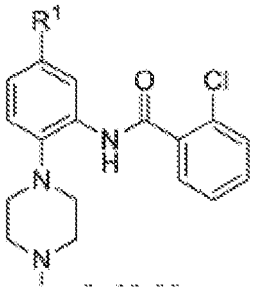<br>$R^1$ = $NO_2$<br>$R^1$ = $NH_2$<br>$R^1$ = $CO_2Me$<br>$R^1$ = $CO_2H$<br>$R^1$ = $CF_3$<br>$R^1$ = Br<br>$R^1$ = cyclopropyl<br>$R^1$ = 2-furanyl<br>$R^1$ = 4-pyridyl<br><br>$R^1$<br>$NO_2$<br>$CO_2Me$<br>$CF_3$<br>Br<br>$NH_2$<br>$CO_2H$<br>cyclopropyl<br>2-furanyl<br>4-pyridyl |
| Methyl lysine binding domain | 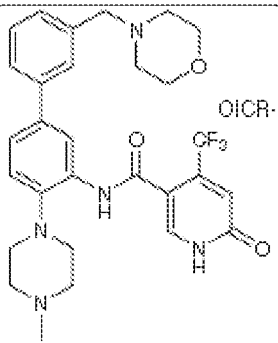<br>OICR- |

| | |
|---|---|
| CDK2 | 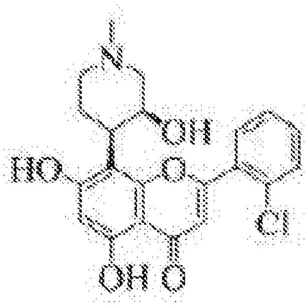 |
| CDK2 | 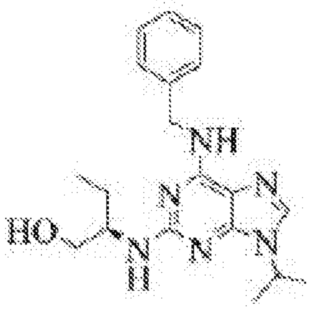 |
| CDK2 | 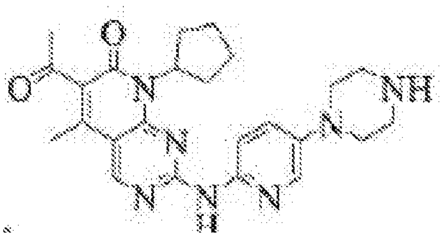 |
| CDK2 | 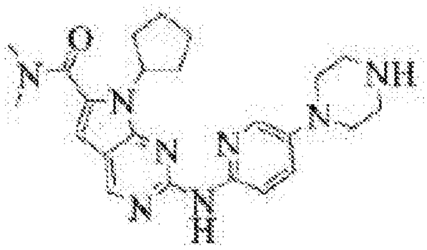 |
| CDK2 | 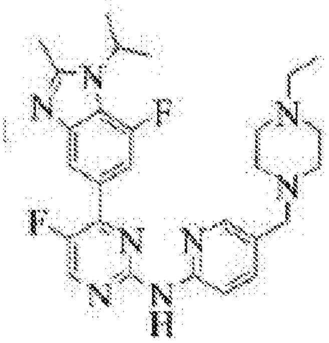 |

| | |
|---|---|
| CDK2 | 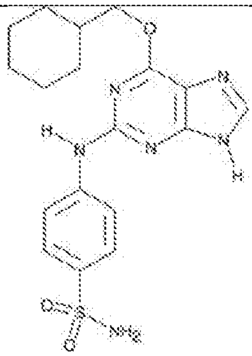 |
| CDK1, 2, or 4 | 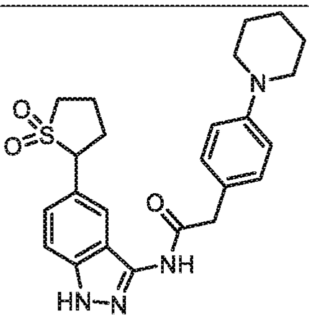 |
| CDK2, CDK1, or CDK5 | 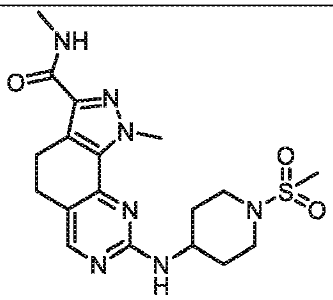 |
| CDK2, CDK4, CDK5, CDK1, CDK7 | 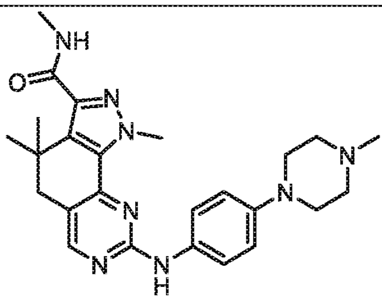 |
| CDK2, CDK1, CDK4 | 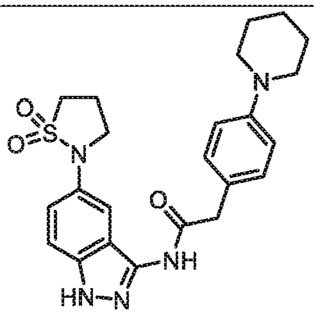 |

| | |
|---|---|
| CDK2, CDK4, CDK5, or CDK1 | 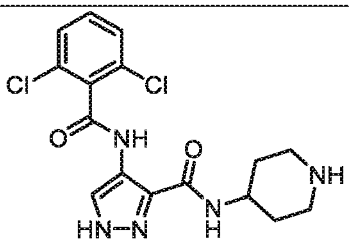 |
| CDK2, CDK5, or CDK7 | 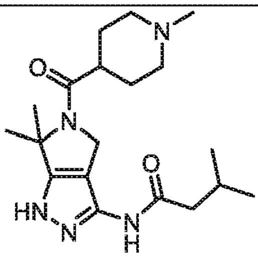 |
| CDK2 or CDK4 | 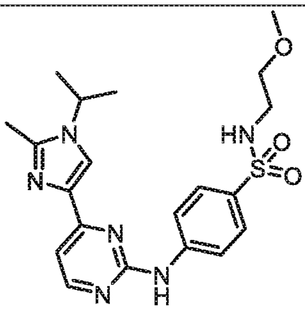 |
| CDK2 | 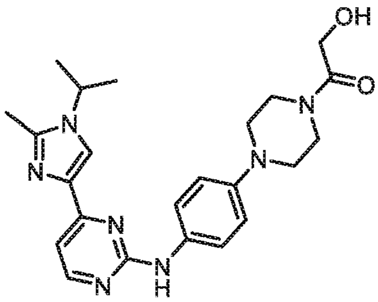 |
| CDK2 or CDK1 | 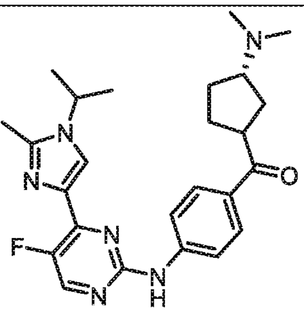 |
| CDK1, CDK2, CDK4 or CDK9 | 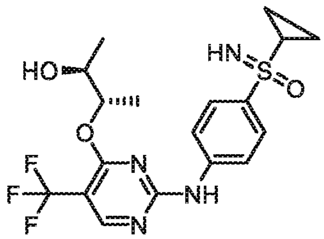 |

| | |
|---|---|
| CDK2 | 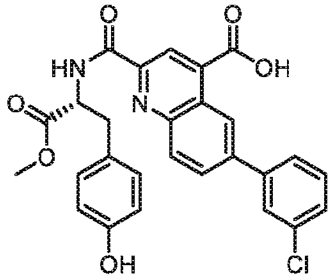 |
| CDK1 or CDK2 | 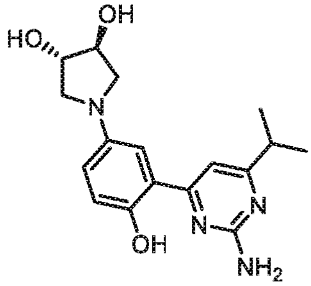 |
| CDK1 or CDK2 | 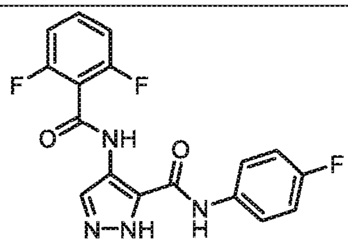 |
| CDK5 or GSK3beta | 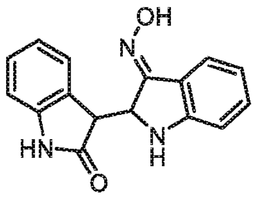 |
| CDK1, CDK5, or GSK3 alpha/beta | 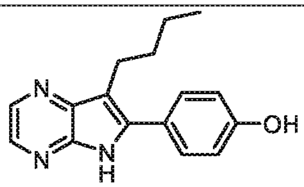 |
| CDK4 or FLT3 | 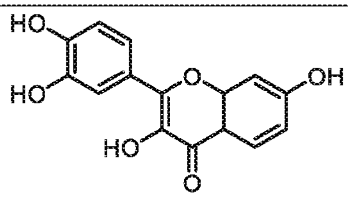 |
| CDK8 | 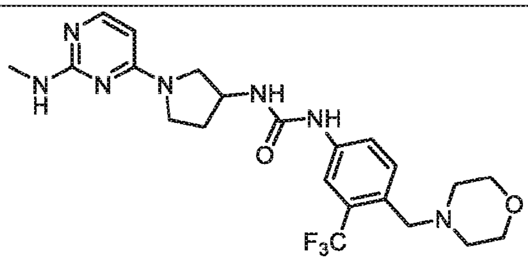 |

| | |
|---|---|
| CDK8 | 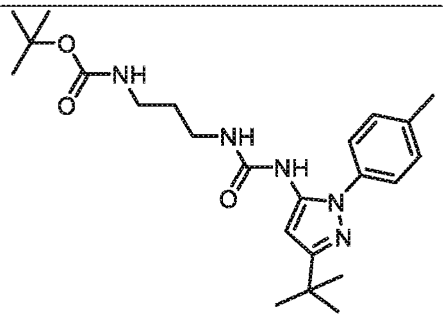 |
| CDK8 or CDK19 | 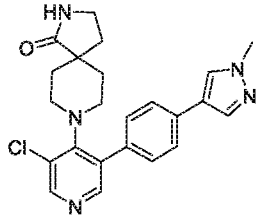 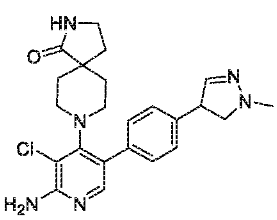 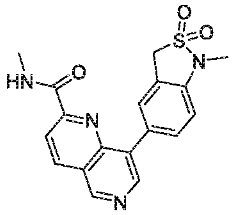 |
| CDK8 | 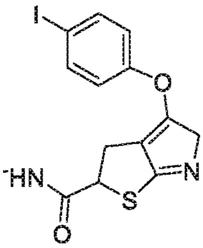 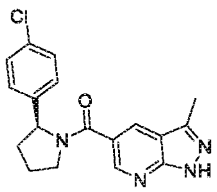 |
| CDK8 | 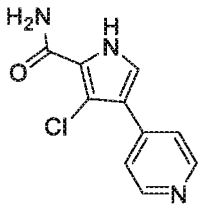 |
| CDK8 or CDK19 | 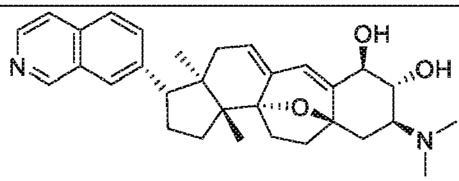 |
| CDK9 | 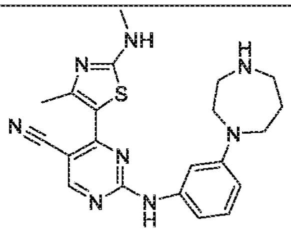 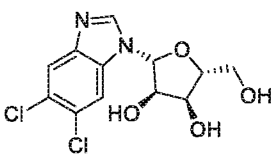 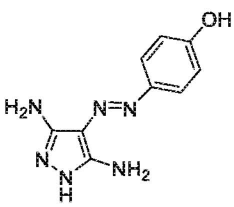 |
| CDK7/9 | 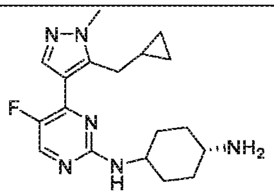 |

| | |
|---|---|
| CDK9 | 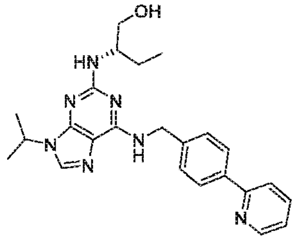 |
| CDK12/13 | 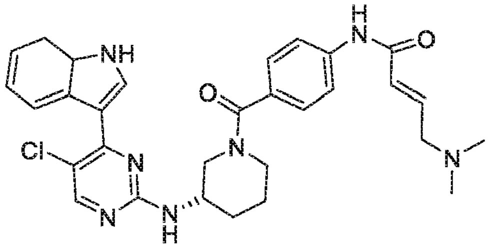 |
| CDK12 | 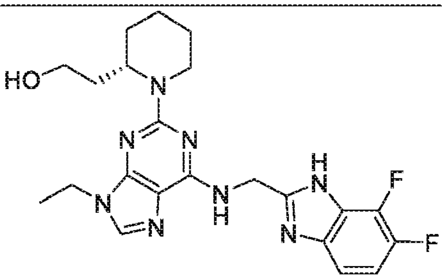 |
| CDK12/2 | 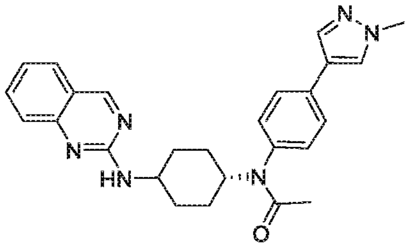 |
| CDK1/2/5/9 (Dinaciclib) | 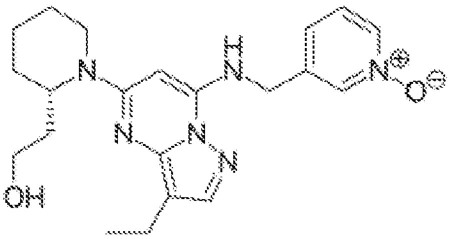 |
| CDK9/4/1/2/6 (P276-00) | 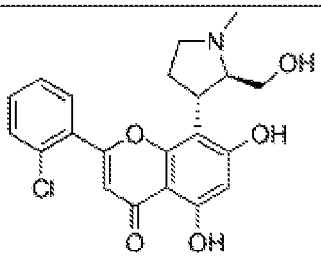 HCl |

| | |
|---|---|
| CDK9 (voruciclib) | 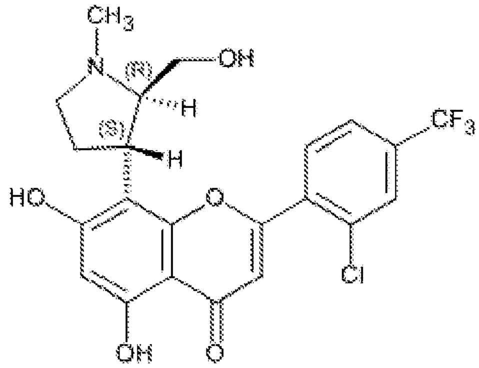 |
| CDK1/2/4/5/9 (AT7519M) | 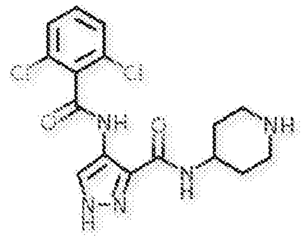 |
| CDK9/2/7/GSK3alpha (SNS-032) | 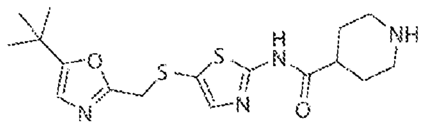 |
| CDK2 | 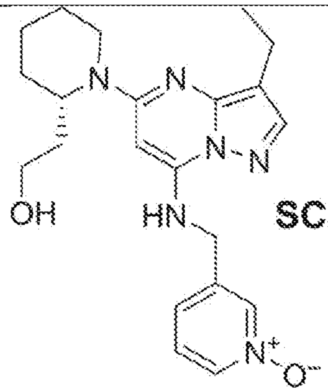 SCH 727965 |

| | |
|---|---|
| CDK1/2/4 | 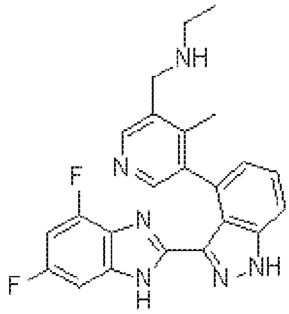 |
| CDK1/2/7/9 | 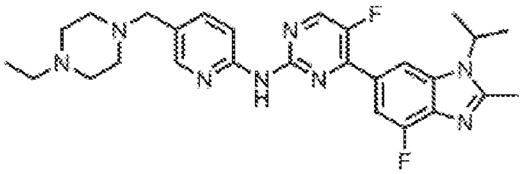 |
| CDK1/2/4/7/9 | 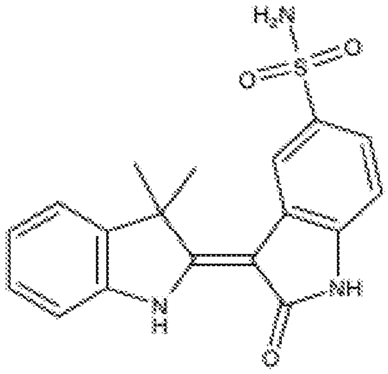 |
| CDK12/13 (THZ531) | 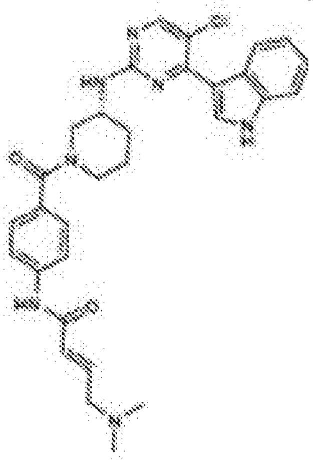 |
| CDK9/2/7/GSK3alpha | 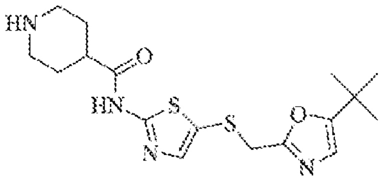 |

| | |
|---|---|
| CDK2 (roscovitine) | 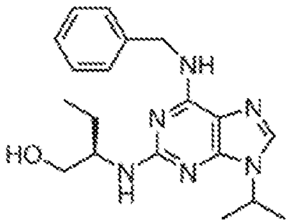 |
| CDK2 (NU2058) | 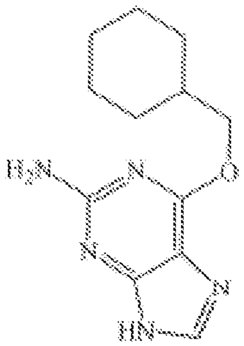 |
| CDK2 (R457) | 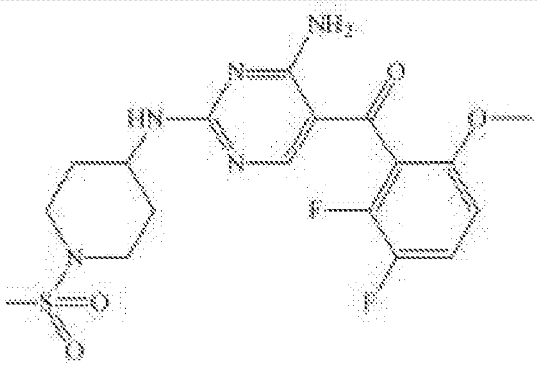 |
| CDK2 (Flavopiridol) | 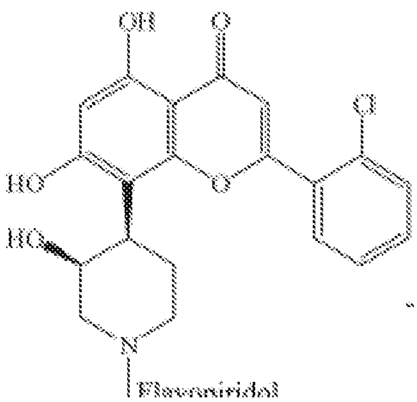 Flavopiridol |
| CDK1/2/4/5/7 /9 (R547) | 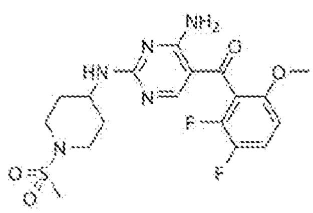 |

| | |
|---|---|
| H3K4 lysine methyltransferase KMT7 (PFI-2) | 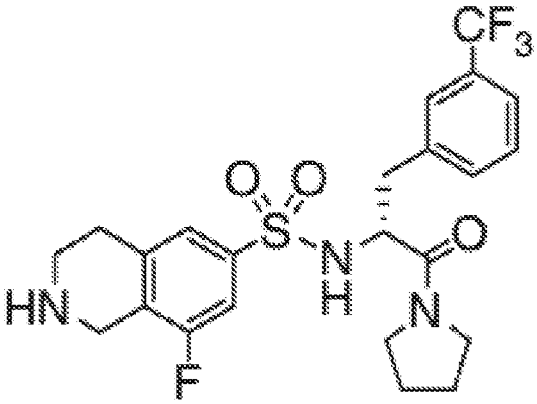 |
| H3K4 lysine methyltransferase KMT7 (cyprohepatadiene) | 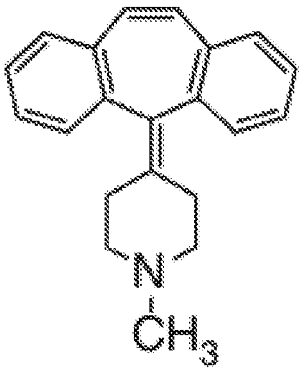 |
| KDM1A/B (RN1) | 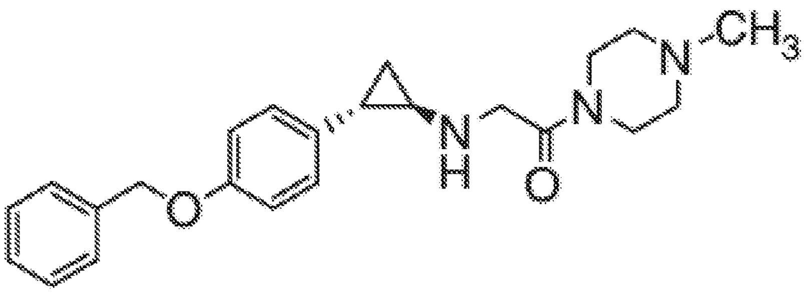 |
| KDM1A (GSK2879552) | 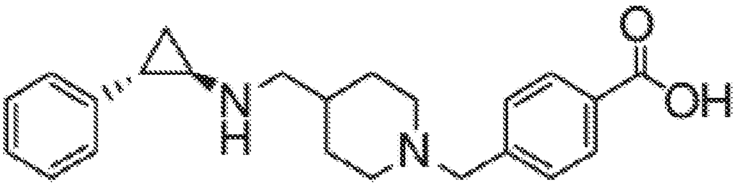 |
| KDM5 (CPI-455) | 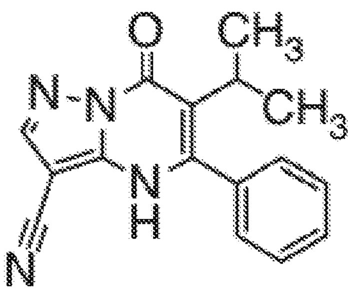 |

| | |
|---|---|
| KDM5 (KDM-C49) | 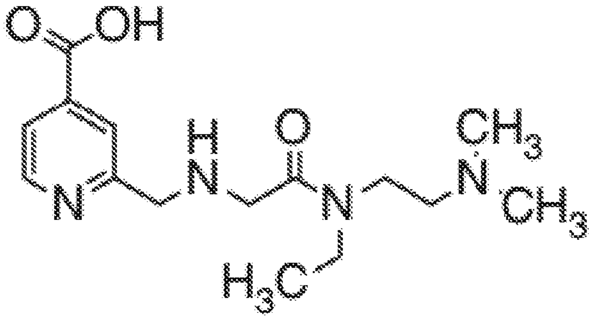 |
| KDM5 (amiodarone) | 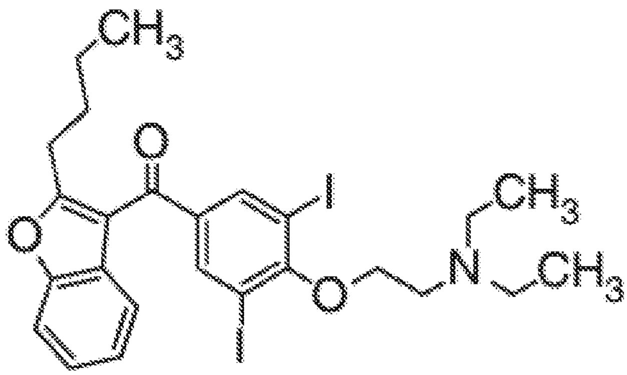 |
| KDM5 (Disulfuram) | 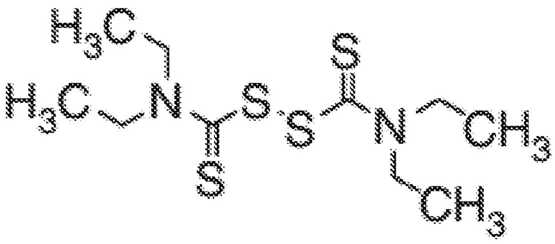 |
| EHMT2 aka G9a | 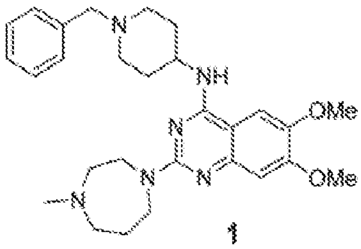 1 BIX-01294 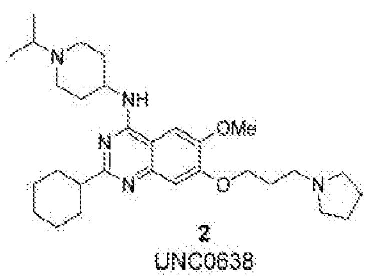 2 UNC0638 |

| | |
|---|---|
| | 3 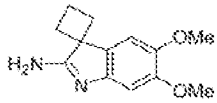<br>4 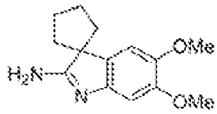<br>5 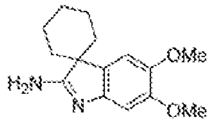<br>6 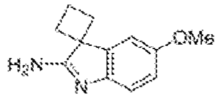 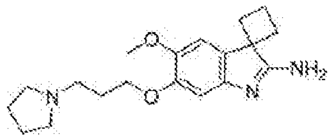<br>7 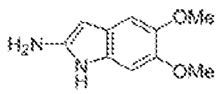 **12** **(A-366)** |
| EHMT2 aka G9a | 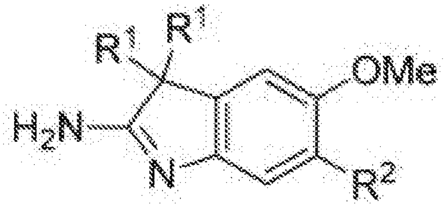 |

| | | |
|---|---|---|
| | $R^1$ | $R^2$ |
| | 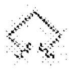 | 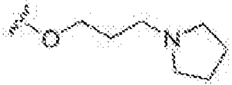 |
| | 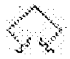 | 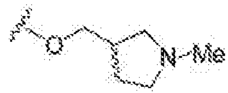 |
| | 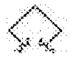 | 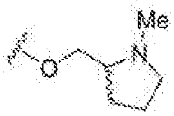 |
| | 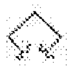 | 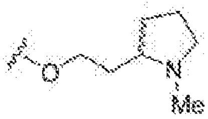 |
| | 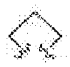 | 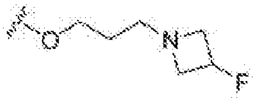 |
| | 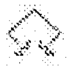 | 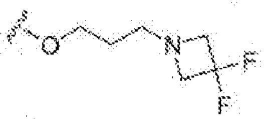 |
| | 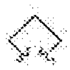 | 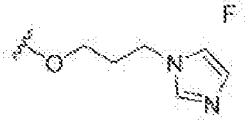 |
| | 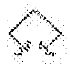 | 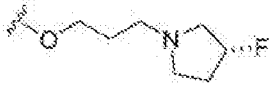 |
| | 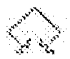 | 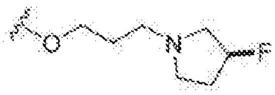 |
| | 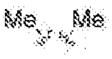 | 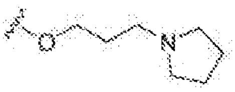 |
| | 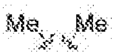 | 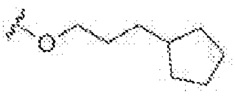 |
| EHMT2 or GLP methyltransferase | 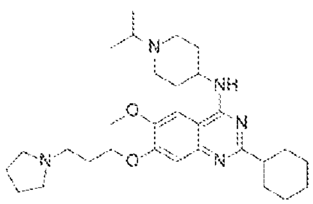 (UNC0638) | |

| | | |
|---|---|---|
| G9a or HDAC | 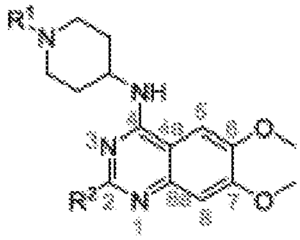 | |
| | R¹ | R² |
| | 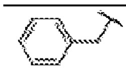 | 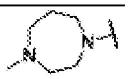 |
| | 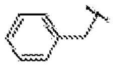 | 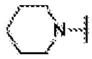 |
| | 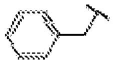 | 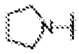 |
| | 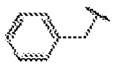 | 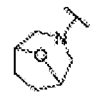 |
| | 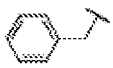 | 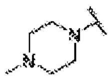 |
| | Me | 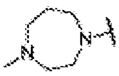 |
| | Me | 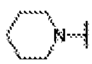 |
| | 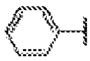 | 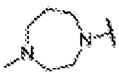 |
| | 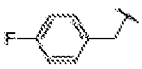 | 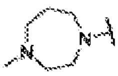 |
| | 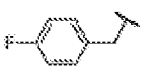 | 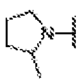 |
| SMYD2 | LLY-507<br>SMYD2<br>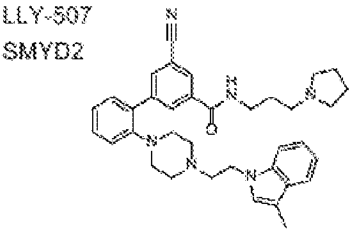 | |

| | |
|---|---|
| DOT1L | EPZ-5676<br>DOT1L<br>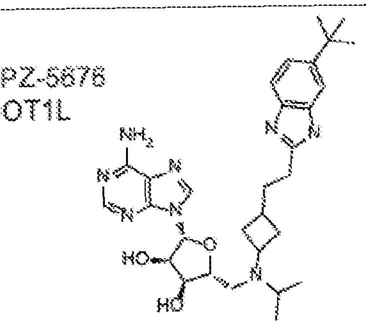 |
| DOT1L | 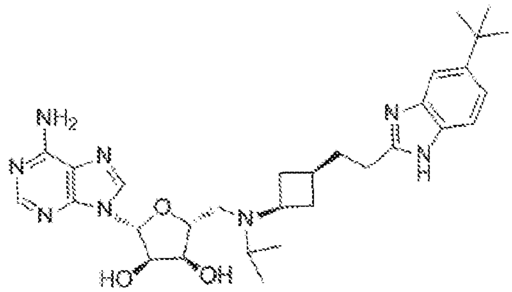<br>(pinometostat) |
| PRMT5 | EPZ015666 (GSK3235025)<br>PRMT5<br>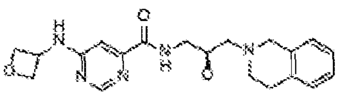 |
| Pan-jmjC | Methylstat<br>pan-jmjC<br>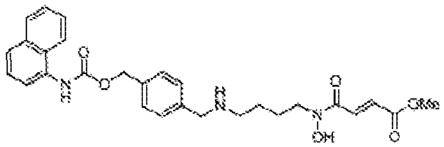 |
| JMJD3/UTX/ JARID | GSK-J1<br>JMJD3/<br>UTX/<br>JARID<br>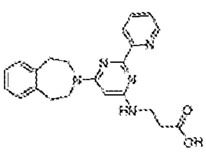 |
| JARID | KDM5-C49<br>JARID<br>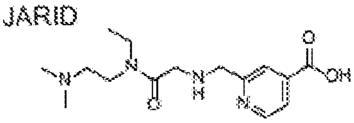 |

| | |
|---|---|
| LSD1 | ORY-1001<br>LSD1<br>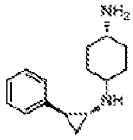 |
| LSD1 | 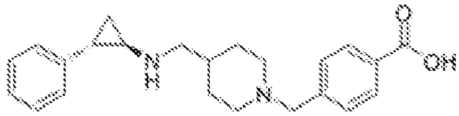 |
| OGT | 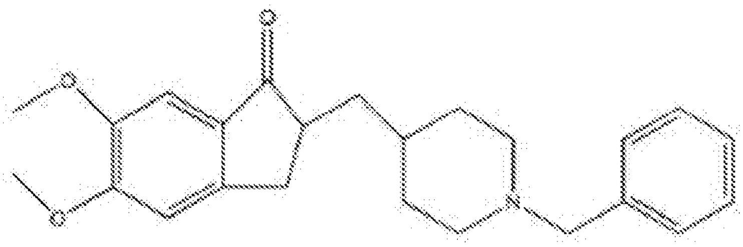 |
| OGT | 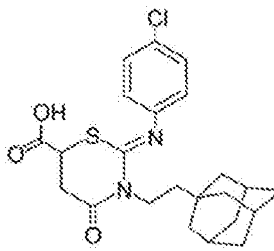 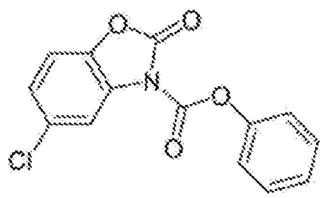 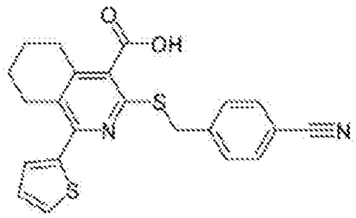 |
| OGT | 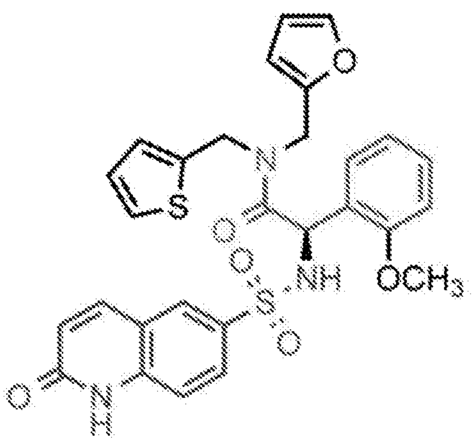 |
| OGT | 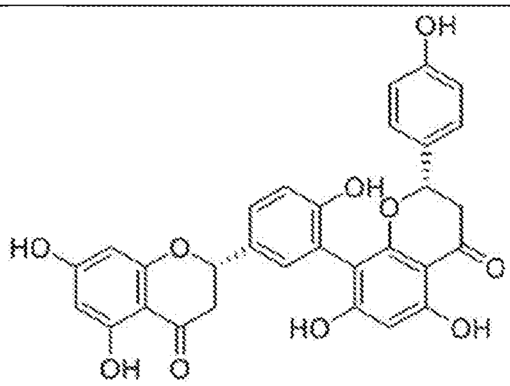 |

| | |
|---|---|
| TET1, TET2 | 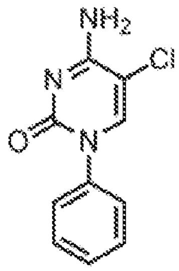 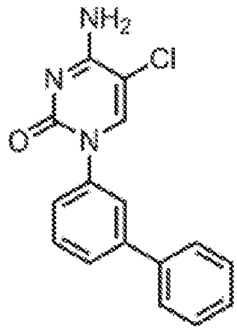 |
| TET1 | 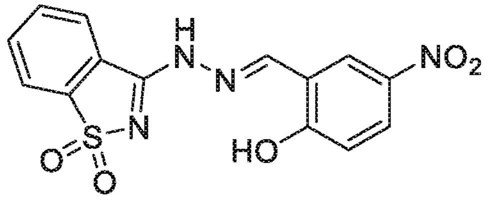 |
| TET1 | 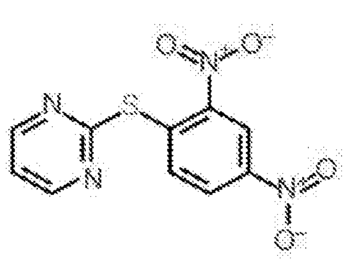 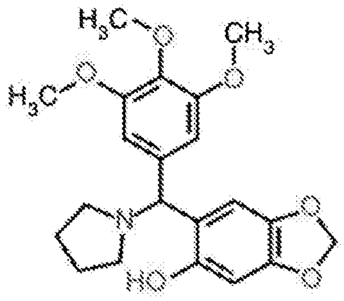 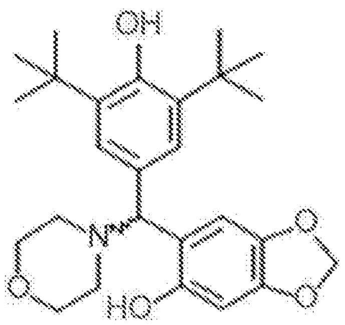 |

| | |
|---|---|
| CBP BRD | 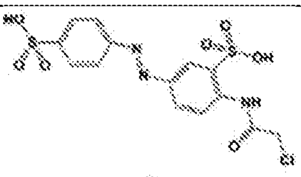 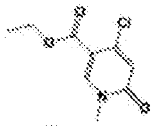 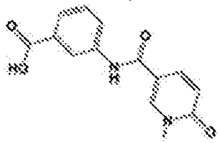 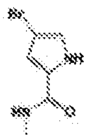 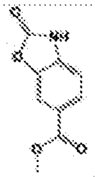 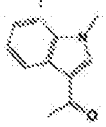 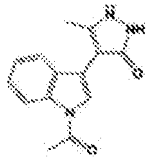 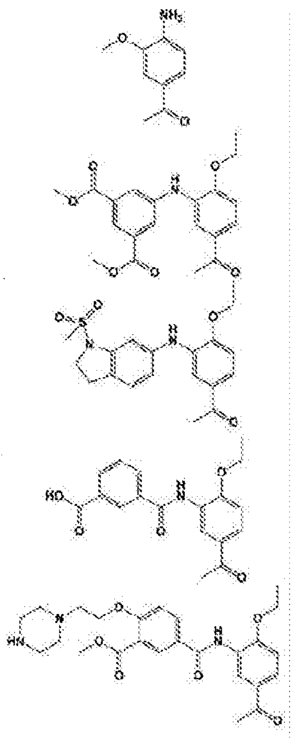 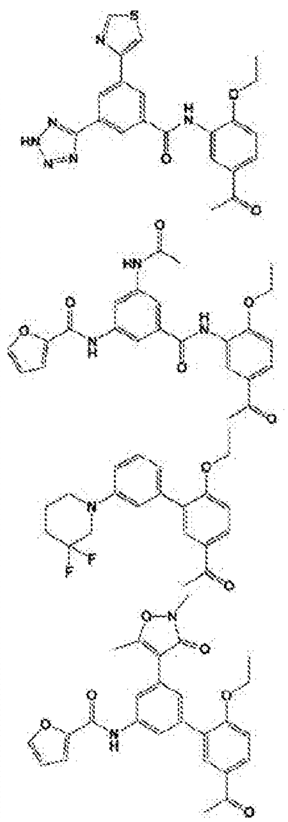 |

| | |
|---|---|
| | 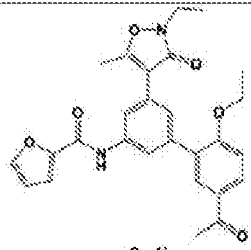 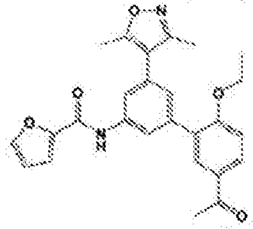 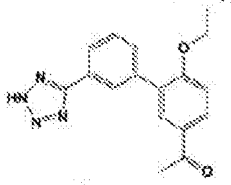 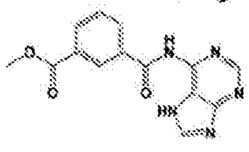 |
| CBP BRD | 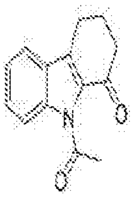 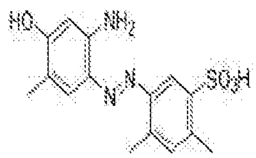 Ac-NH-C-W-K-K(Ac)-L-C-F-K-$CONH_2$ 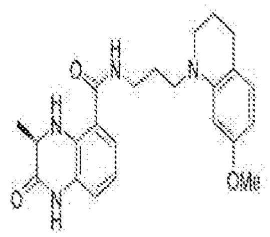 |
| CBP BRD | 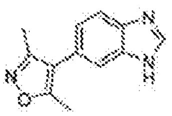 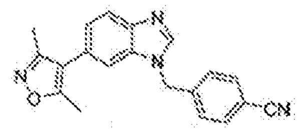 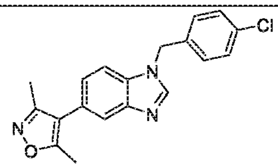 |
| CBP BRD | 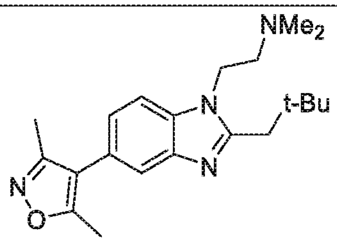 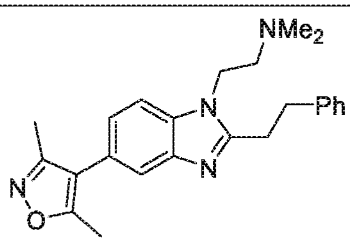 |

| CBP BRD | R |
|---|---|
| | 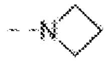 |
| | 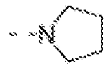 |
| | 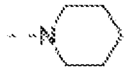 |
| | 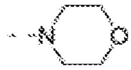 |
| | 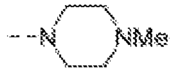 |
| | 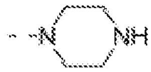 |
| | 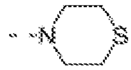 |
| | 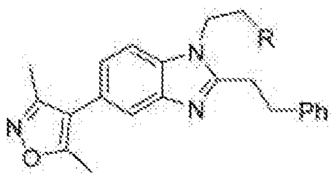 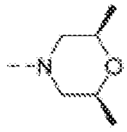 |

| | |
|---|---|
| CBP BRD | R<br>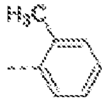<br>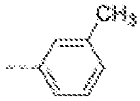<br>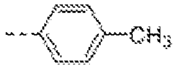<br>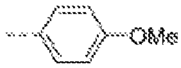<br>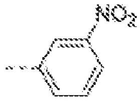<br>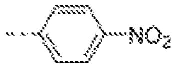<br>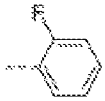<br>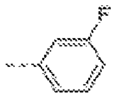<br>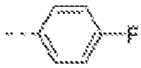<br>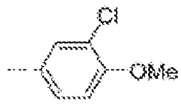<br>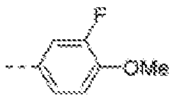<br>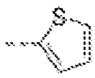<br>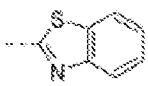<br>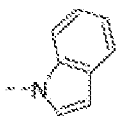<br>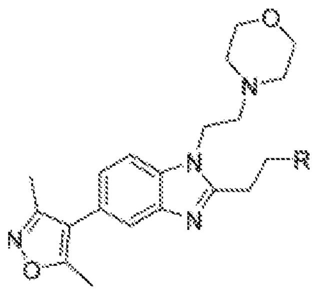 |

| | |
|---|---|
| CBP BRD | 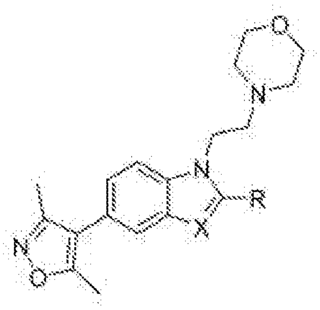 R 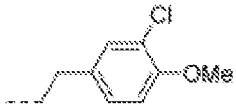 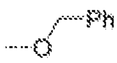 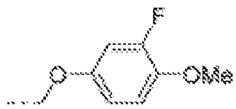 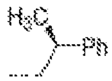 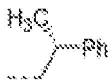 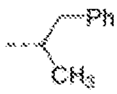 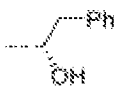 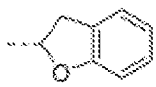 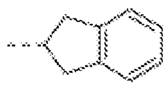 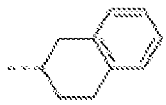 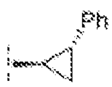 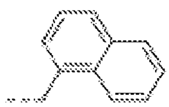 |
| CBP BRD | 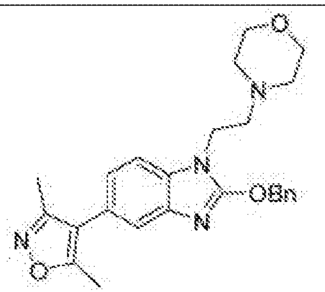 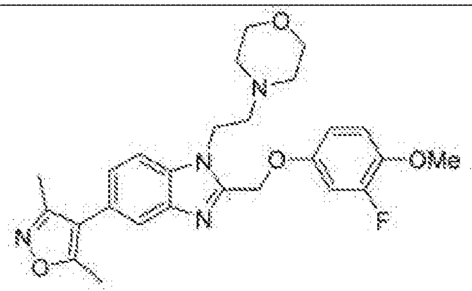 |

| | |
|---|---|
| CBP BRD | R<br>1 2 3<br>A $CH_3$ H<br>A H $CH_3$<br>B $CH_3$ H<br>B H $CH_3$<br>A H (*R*)-$CH_3$<br>A H (*S*)-$CH_3$<br>B H (*R*)-$CH_3$<br>B H (*S*)-$CH_3$<br>C H (*R*)-$CH_3$<br>C H (*S*)-$CH_3$<br>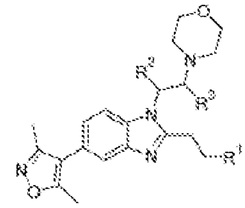<br>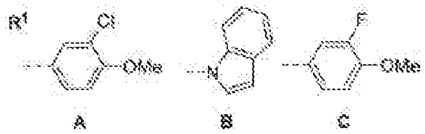<br>A B C |
| HDAC | 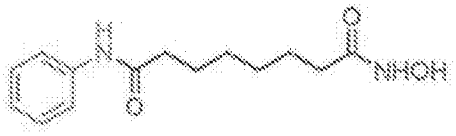 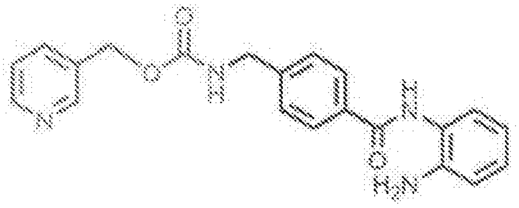<br>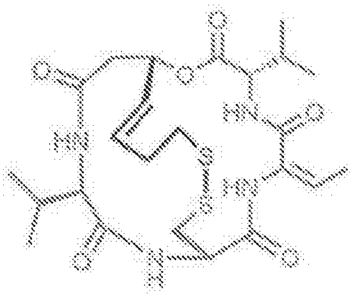 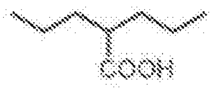<br>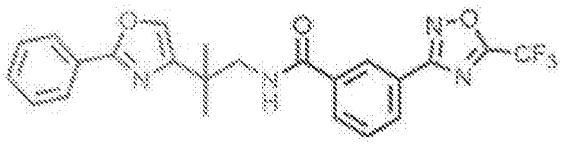<br>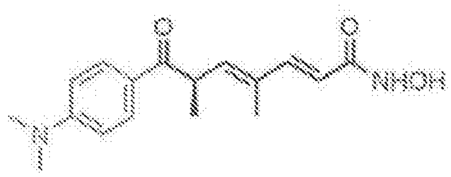 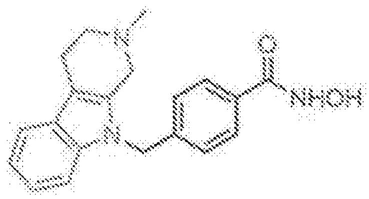 |
| HDAC | 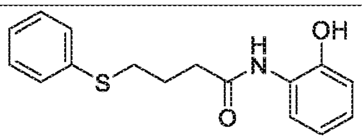 |
| HDAC | 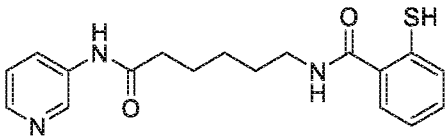 |

| | |
|---|---|
| HDAC1, HDAC2, HDAC3 | 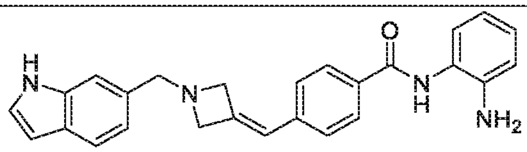 |
| HDAC2, HDAC3 | 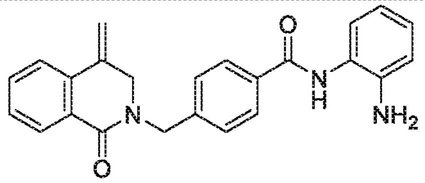 |
| HDAC1, HDAC3 | 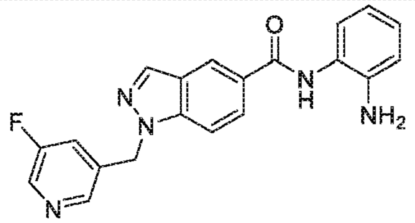 |
| HDAC | 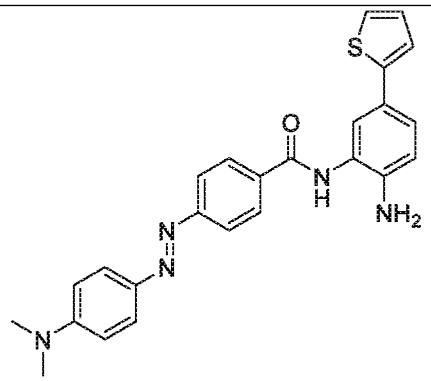 |
| HDAC1, HDAC2, HDAC3 | 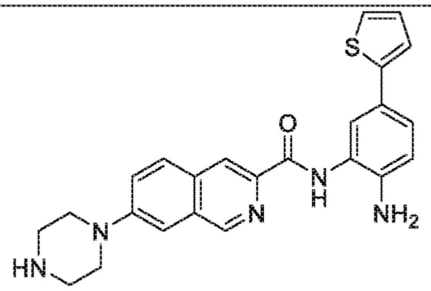 |
| HDAC1, HDAC2, HDAC3 | 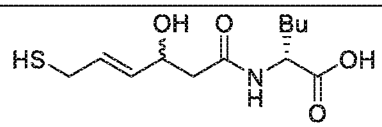 |
| HDAC6, HDAC8 | 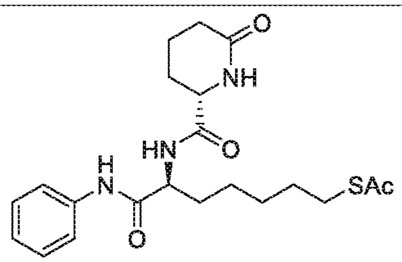 |

| | |
|---|---|
| HDAC6 | 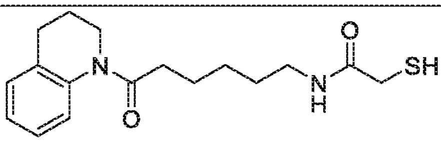 |
| HDAC6 | 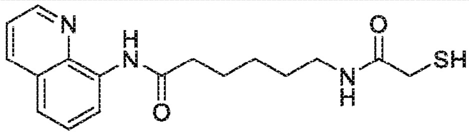 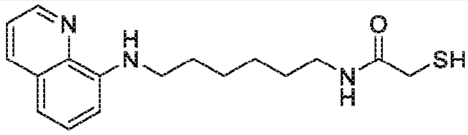 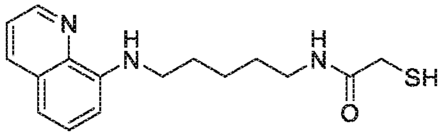 |
| HDAC | 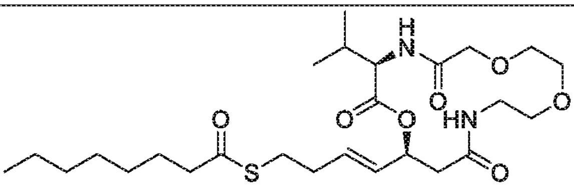 |
| HDAC6 | 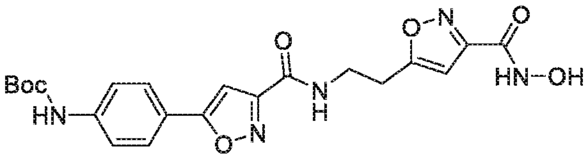 |
| HDAC1, HDAC2, HDAC3, HDAC6 | 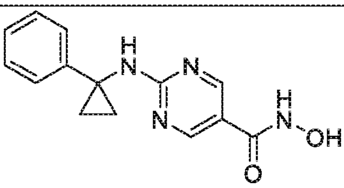 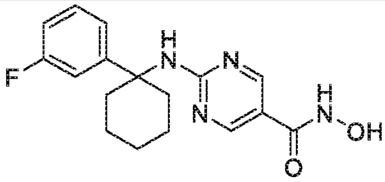 |
| HDAC1, HDAC2, HDAC3, HDAC6 | 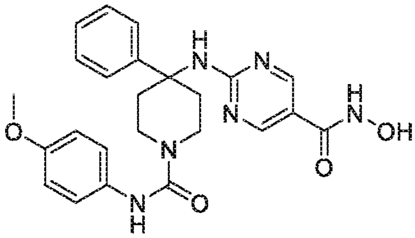 |
| HDAC4 | 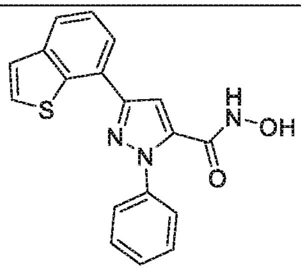 |
| HDAC6, HDAC8 | 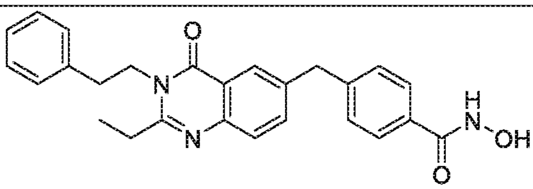 |

| | |
|---|---|
| HDAC6 | 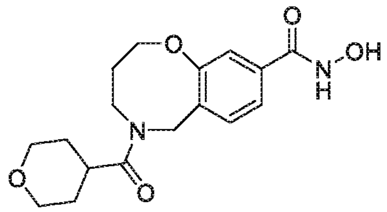 |
| HDAC6 | 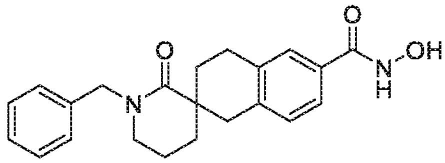 |
| HDAC | 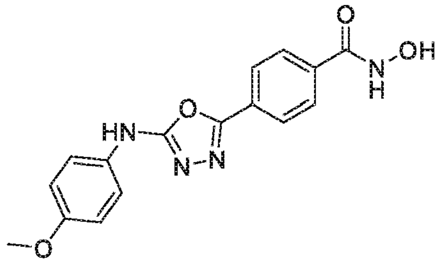 |
| HDAC6 | 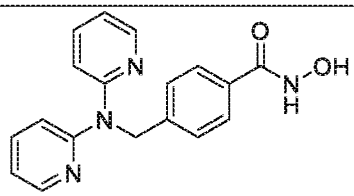 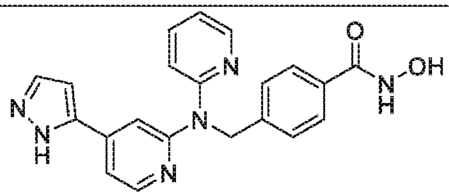 |
| HDAC6 | 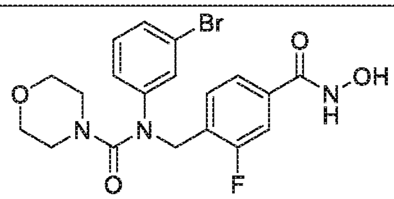 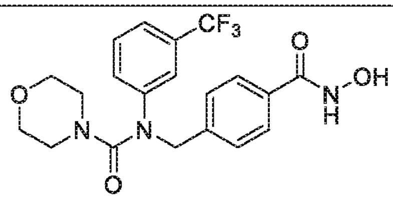 |
| HDAC1, HDAC6 | 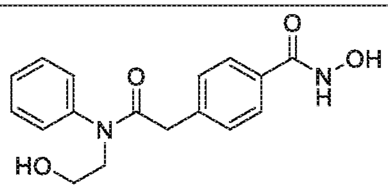 |
| HDAC6, HDAC8 | 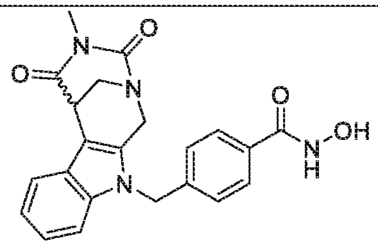 |

| | |
|---|---|
| HDAC1, HDAC6 | 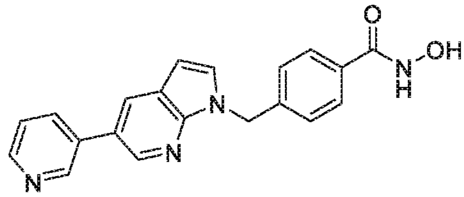 |
| HDAC5, HDAC5, HDAC6, HDAC8 | 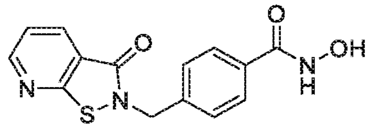 |
| HDAC6 | 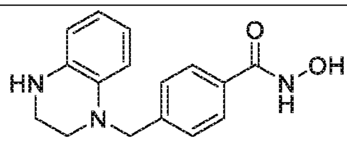 |
| HDAC1, HDAC6 | 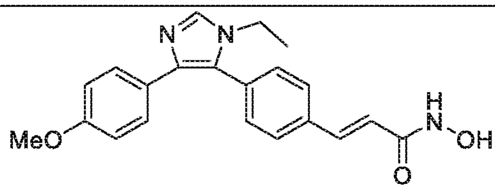 |
| HDAC1, HDAC6 | 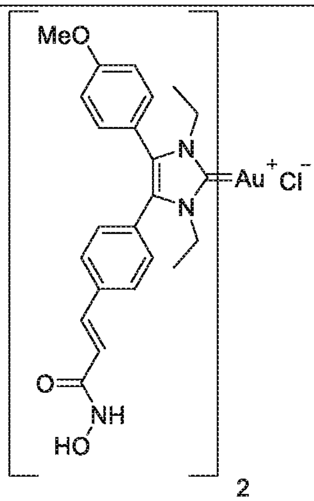 |
| HDAC | 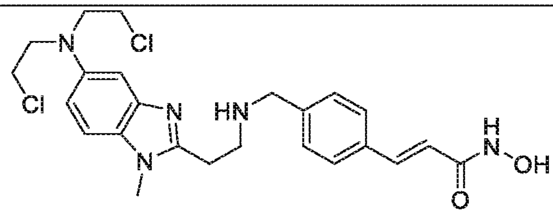 |
| HDAC1, HDAC2, HDAC3, HDAC5, HDAC 6 | 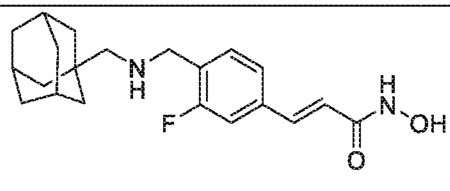 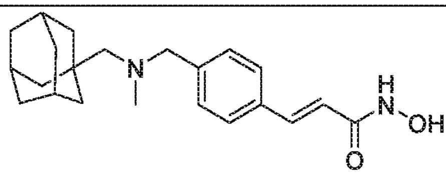 |

| | |
|---|---|
| HDAC1, HDAC6 | 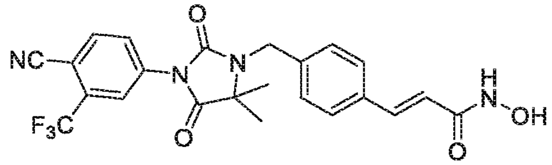 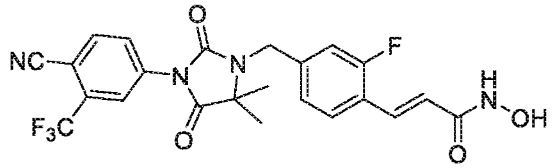 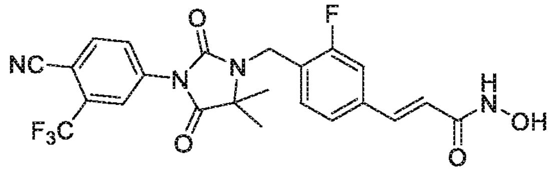 |
| HDAC8, HDAC11 | 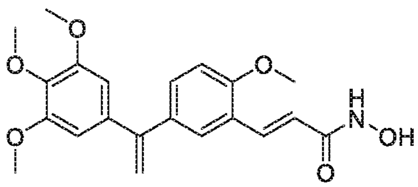 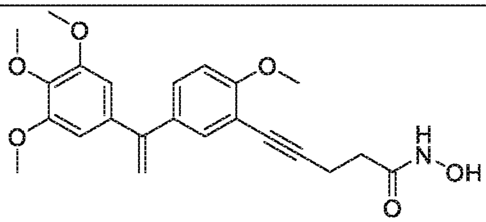 |
| HDAC8 | 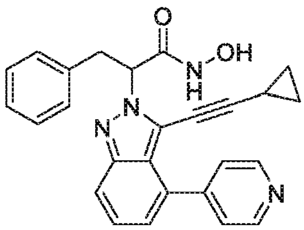 |
| HDAC1, HDAC6 | 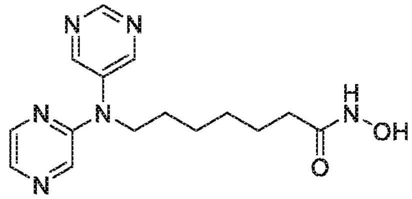 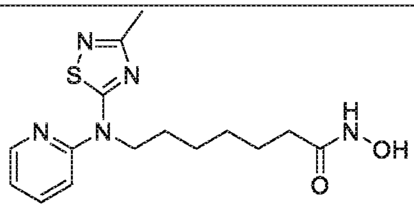 |
| HDAC1, HDAC6 | 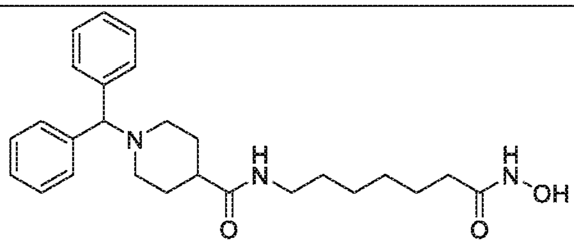 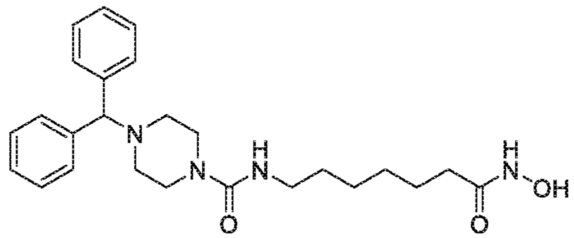 |

| | |
|---|---|
| HDAC | 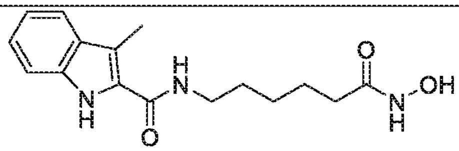 |
| HDAC1 | 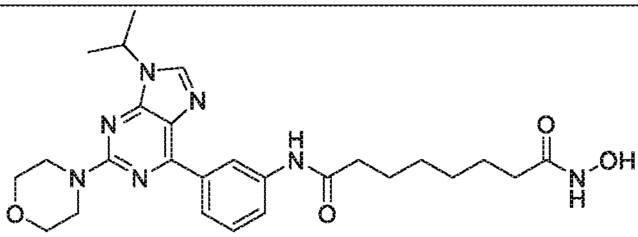 |
| HDAC1, HDAC2, HDAC3, HDAC6, HDAC8, HDAC10, HDAC11 | 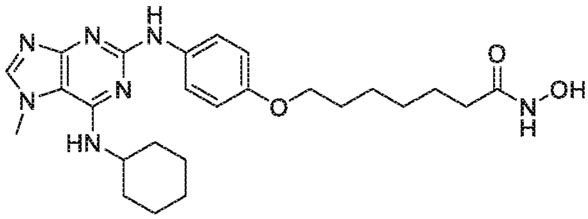 |
| HDAC1, HDAC 2, HDAC3, HDAC6, HDAC8, HDAC10, HDAC11 | 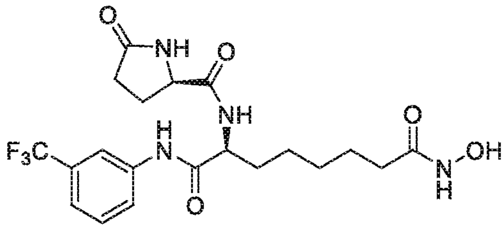 |
| HDAC4, HDAC5, HDAC7, HDAC9 | 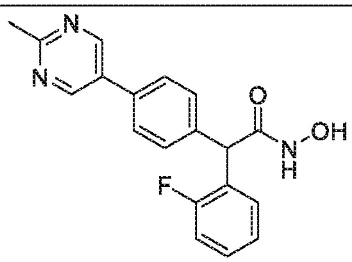 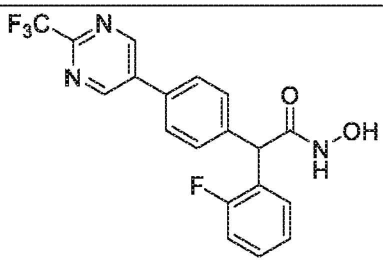 |
| HDAC4 | 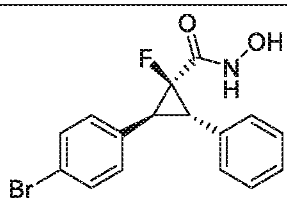 |

| | |
|---|---|
| HDAC4 | 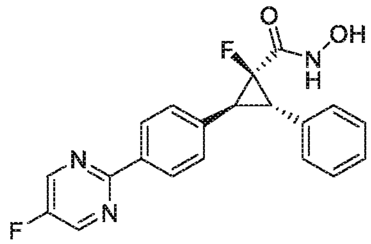 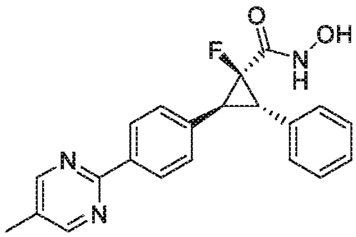 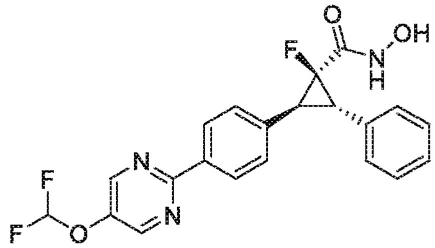 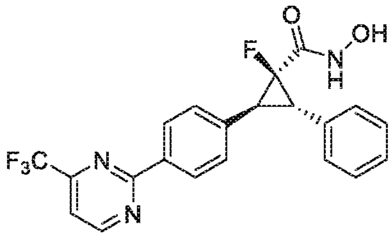 |
| HDAC4 | 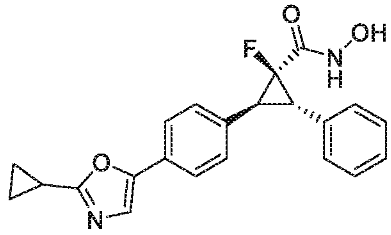 |
| HDAC4 | 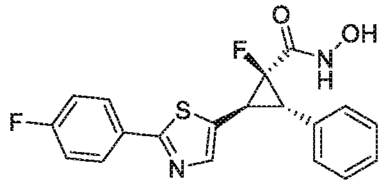 |
| HDAC4 | 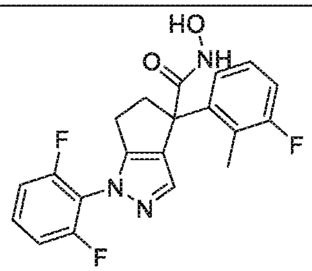 |
| HDAC4 | 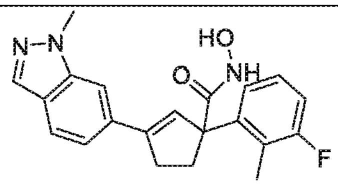 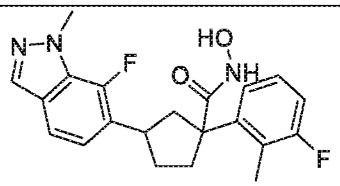 |
| HDAC5, HDAC8 | 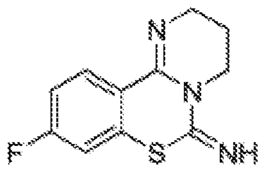 |

| | |
|---|---|
| HDAC4, HDAC8 | 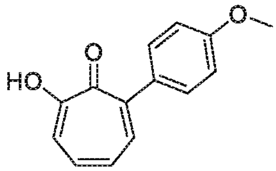 |
| HDAC | 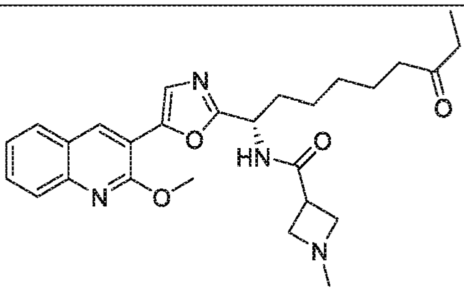 |
| HDAC4 | 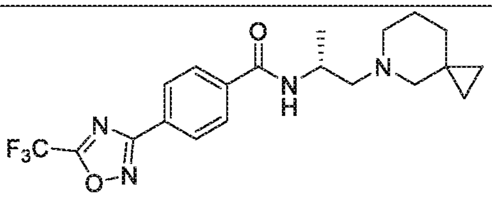 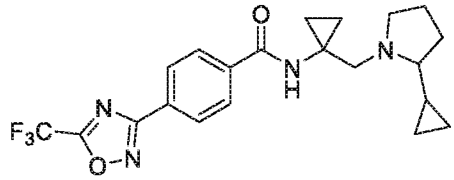 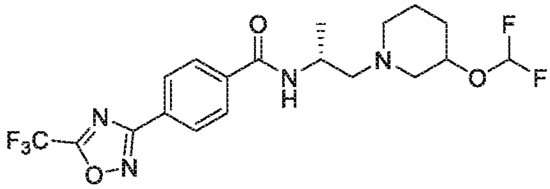 |
| HDAC1, HDAC6, HDAC9 | 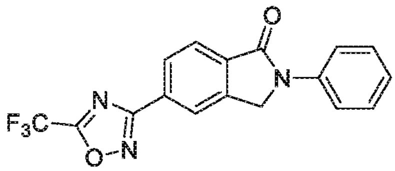 |
| HDAC2, HDAC6 | 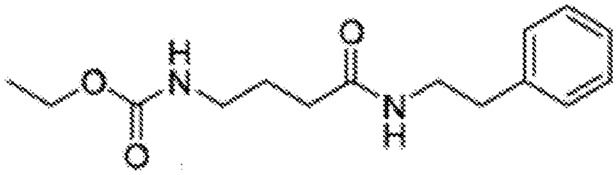 |
| P300/CBP | 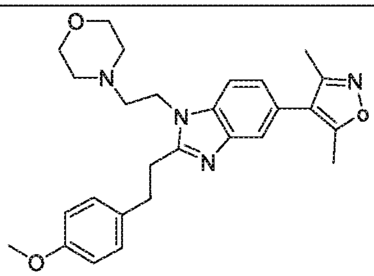 |
| p300, PCAF | 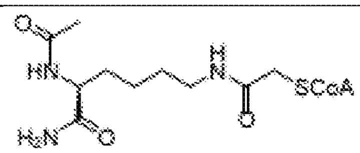 |

| | |
|---|---|
| p300, PCAF | 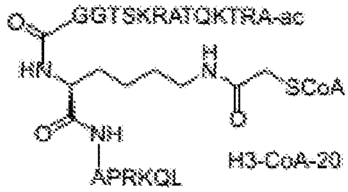 H3-CoA-20 |
| p300 | 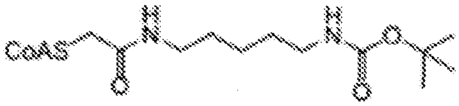 |
| HAT | 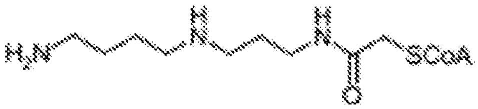 |
| Tip60 | 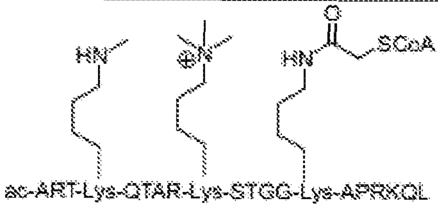 |
| p300/CBP, PCAF, Tip60 | 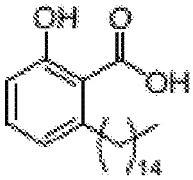 |
| p300 activator | 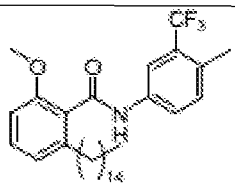 |
| PCAF | 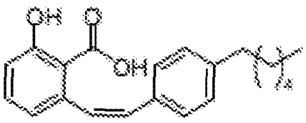 |
| Tip60 | 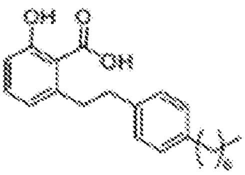 |
| PCAF | 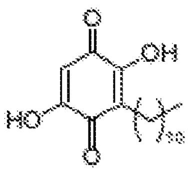 |

| | |
|---|---|
| p300 | 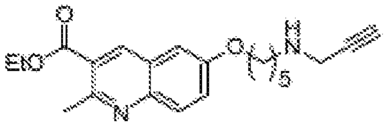 |
| p300, PCAF | 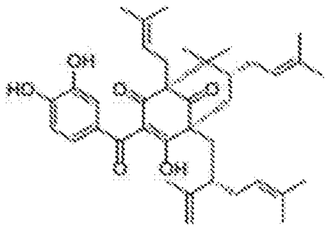 |
| p300 | 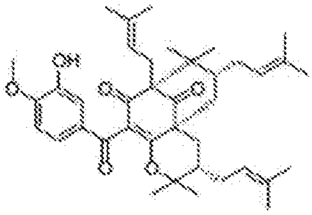 |
| p300 | 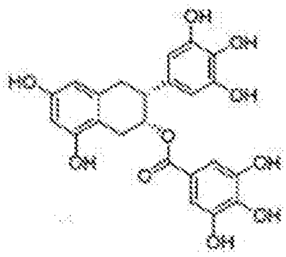 |
| p300/CBP | 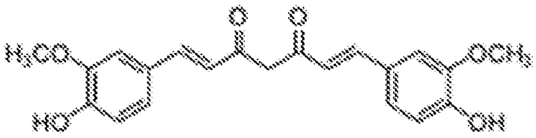 |
| p300 | 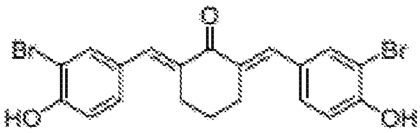 |
| p300 | 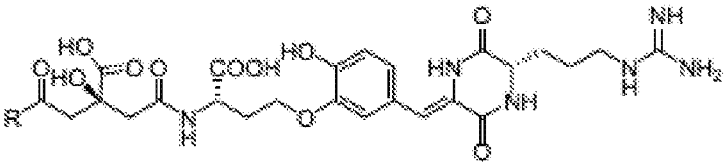 |
| p300 | 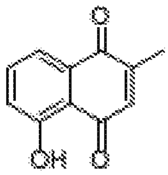 |

| | |
|---|---|
| p300/CBP | 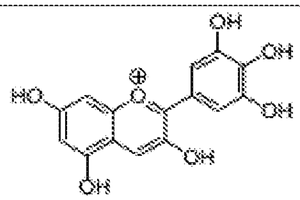 |
| PCAF | 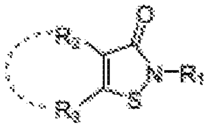 |
| GCN5 | 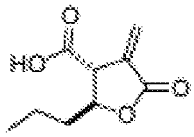 |
| p300 | 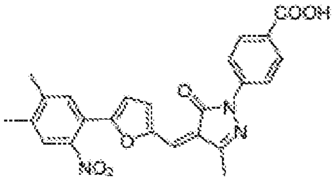 |
| Tip60 | 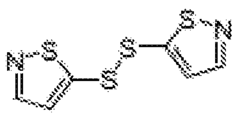 |
| Tip60 | 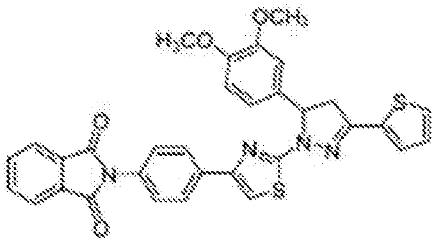 |
| p300 | 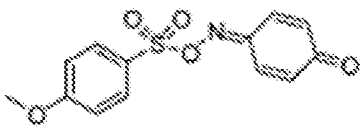 |
| Tip60 | 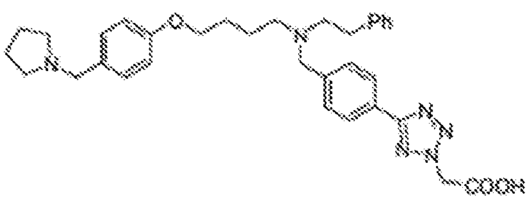 |
| HDAC1, HDAC2, HDAC3, HDAC8 | 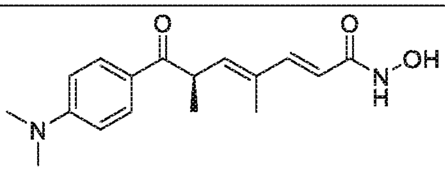 |

| | |
|---|---|
| HDAC1, HDAC2, HDAC3, HDAC8 | 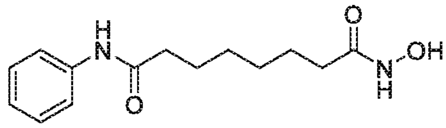 |
| HDAC1, HDAC2, HDAC3, HDAC8 | 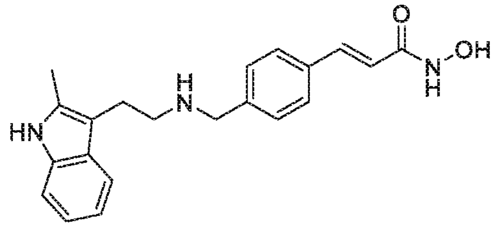 |
| HDAC1, HDAC2, HDAC3 | 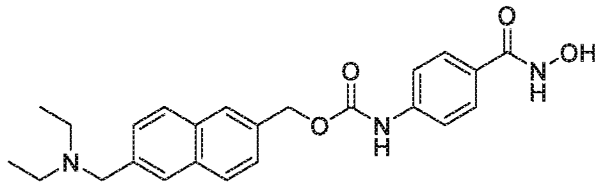 |
| HDAC1, HDAC2, HDAC3, HDAC8 | 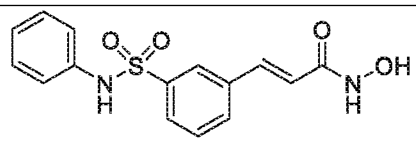 |
| HDAC1, HDAC2, HDAC3, HDAC8 | 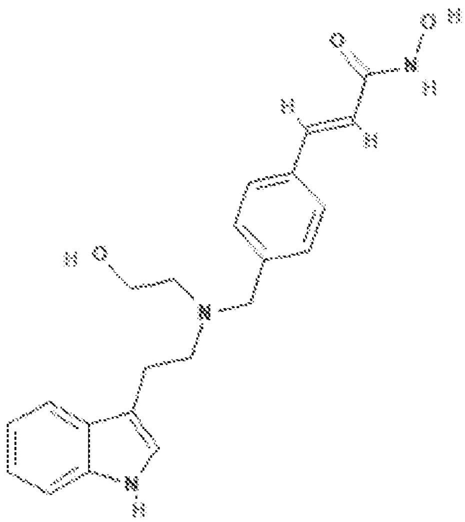 |
| HDAC1, HDAC2, HDAC3, HDAC8 | 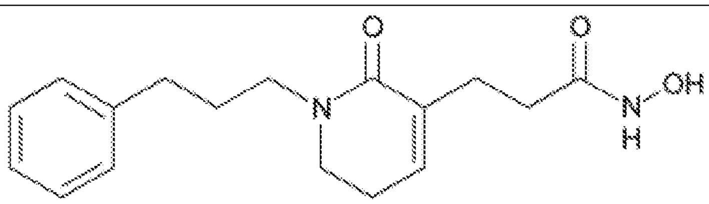 |

| | |
|---|---|
| HDAC1,<br>HDAC2,<br>HDAC3 | 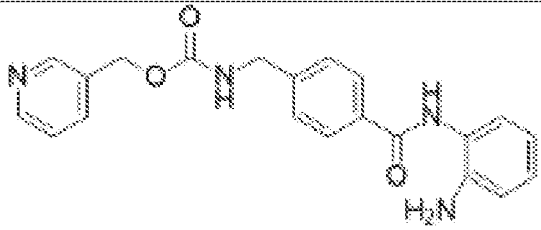 |
| HDAC1,<br>HDAC2,<br>HDAC3 | 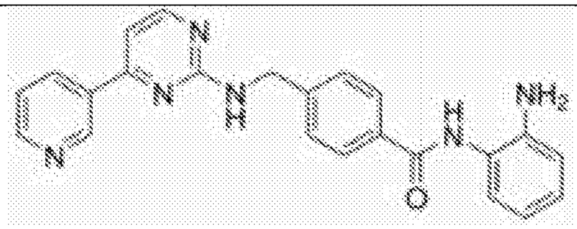 |
| HDAC2,<br>HDAC3 | 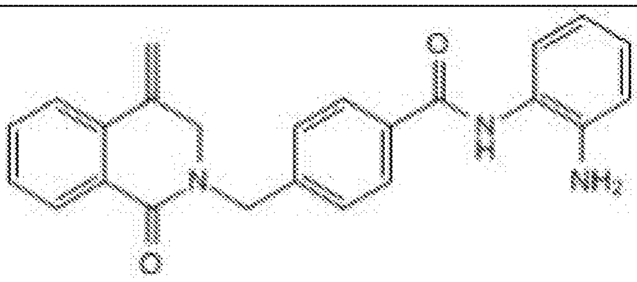 |
| CDK2 | 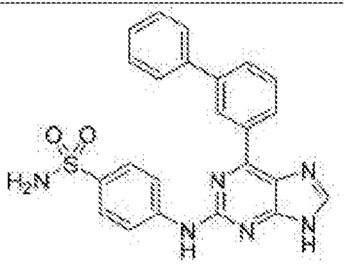 |
| CDK2 | 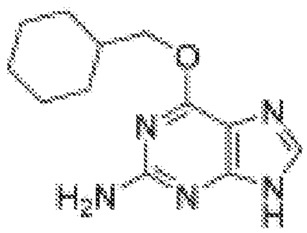 |
| CDK2 | 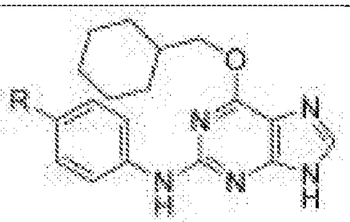<br>2: R = H<br>3: R = $SO_2NH_2$ |

| | |
|---|---|
| CDK2 | 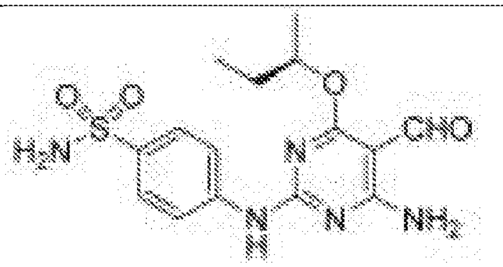 |
| CDK2, CDK7, CDK9 | 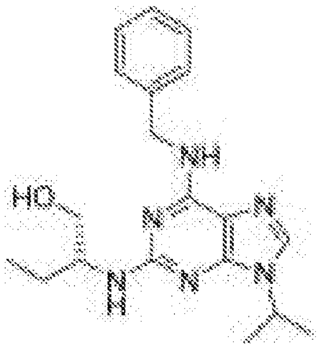 |
| CDK2, CDK7, CDK9 | 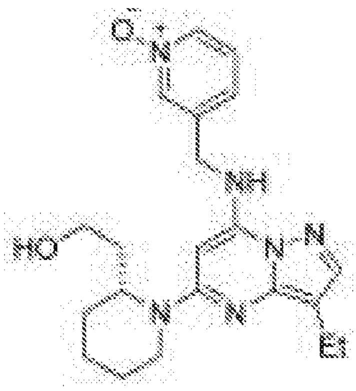 |
| CDK2, CDK7, CDK9 | 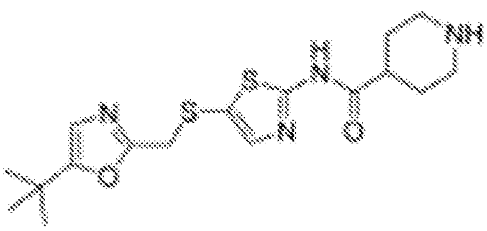 |

| | | $R^1$ | $R^2$ |
|---|---|---|---|
| CDK2 | | 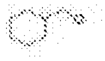 | – |
| | | 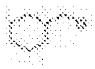 | H |
| | | 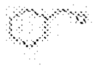 | $SO_2NH_2$ |
| | | H | – |
| | | H | H |
| | | H | $SO_2NH_2$ |
| | | OH (C=O)[a] | H |
| | | OH (C=O)[a] | $SO_2NH_2$ |
| | | OEt | $SO_2NH_2$ |
| | | 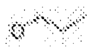 | $SO_2NH_2$ |
| | | 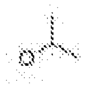 | $SO_2NH_2$ |
| | | 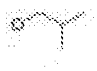 | $SO_2NH_2$ |
| | | 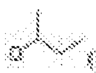 (rac) | $SO_2NH_2$ |
| | | 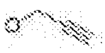 | $SO_2NH_2$ |
| | | $C{\equiv}CSi(i\text{-}Pr)_3$ | $SO_2NH_2$ |
| | 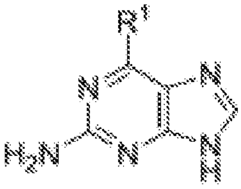 | C≡CH | H |

| | | | |
|---|---|---|---|
| CDK2 | 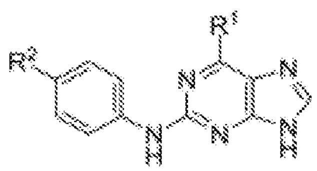 | $R^1$ | $R^2$ |
| | | C≡CH | $SO_2NH_2$ |
| | | C≡CMe | H |
| | | C≡CPh | H |
| | | Et | $SO_2NH_2$ |
| | | 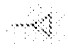 | $SO_2NH_2$ |
| | | Ph | $SO_2NH_2$ |
| | | 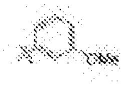 | $SO_2NH_2$ |
| | | 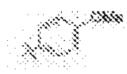 | $SO_2NH_2$ |
| | | 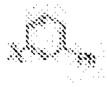 | $SO_2NH_2$ |
| | | 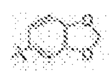 | $SO_2NH_2$ |
| | | 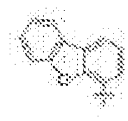 | $SO_2NH_2$ |
| | | 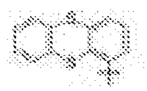 | $SO_2NH_2$ |

| | |
|---|---|
| CDK2 | R |
| | 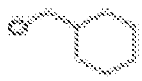 |
| | H |
| | 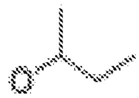 |
| | 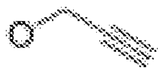 |
| | Ph |
| | 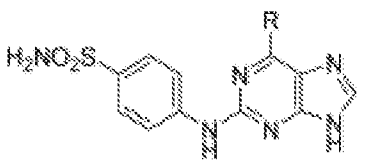 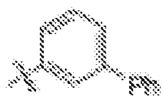 |

| | Structure | R |
|---|---|---|
| CDK2 | 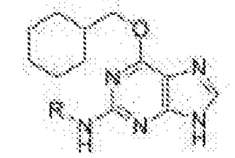 | H<br>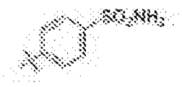 |
| | 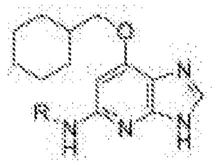 | H<br>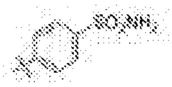 |
| | 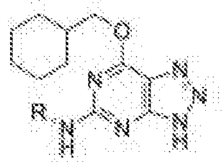 | H<br>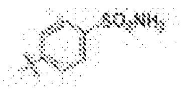 |
| | 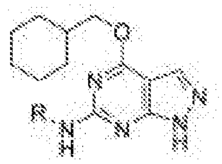 | H<br>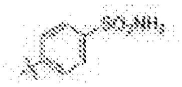 |
| | 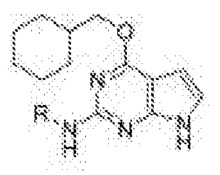 | H<br>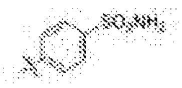 |
| | 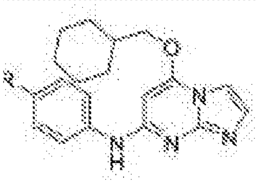 | H<br>$SO_2NH_2$ |
| CDK | 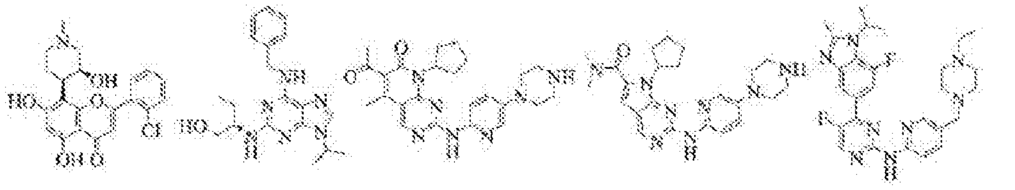 | |

| | |
|---|---|
| PCAF BRD, L3MBTL3 | 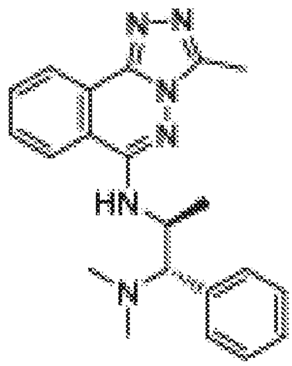 |
| PCAF BRD, L3MBTL3 | 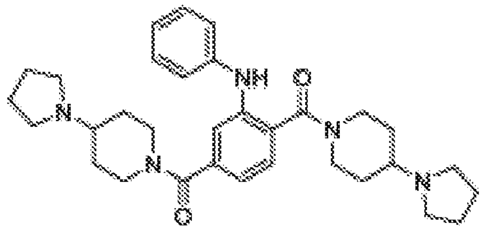 |
| CBP/p300 | 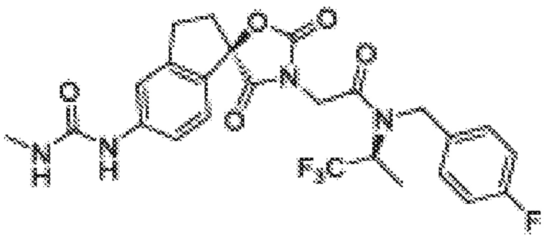 |
| PRMT5 | 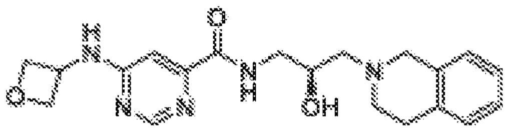 |
| HDAC | 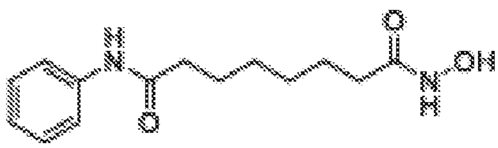 |
| 2-oxoglutarate dependent KDM5 demethylases | 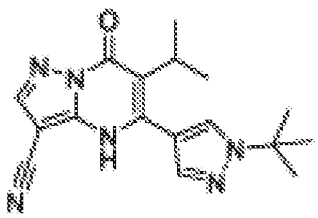 |

| | |
|---|---|
| CDK4, CDK6 | 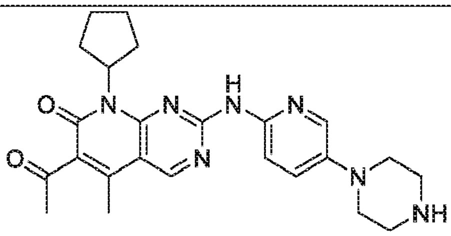 |
| CDK4, CDK6 | 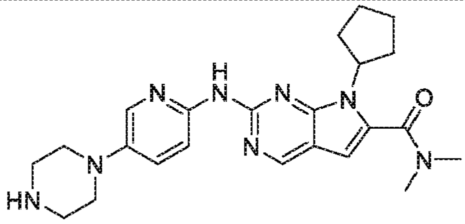 |
| CDK4, CDK6 | 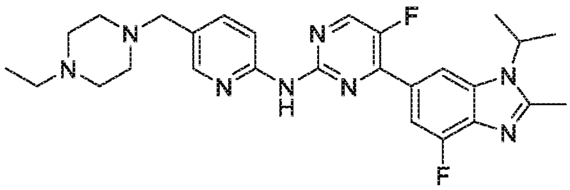 |
| HDAC | 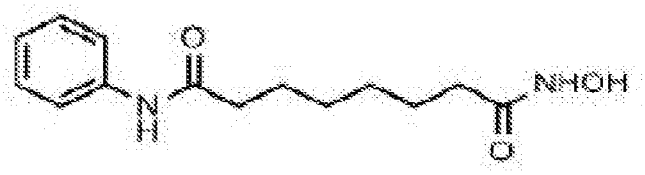 |
| HDAC | 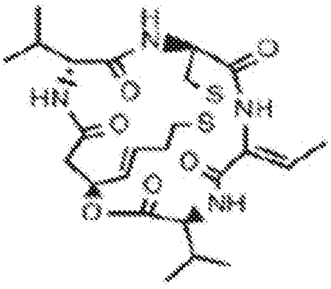 |
| HDAC | 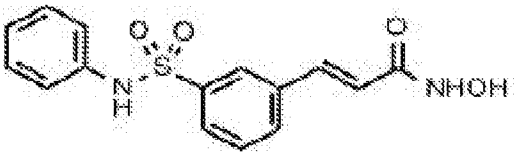 |
| Pan-HDAC | 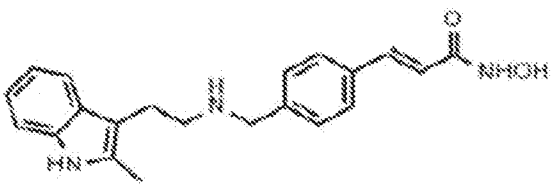 |

| | |
|---|---|
| HDAC | 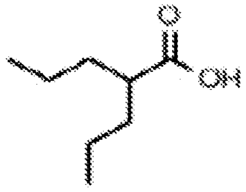 |
| HDAC1, HDAC3 | 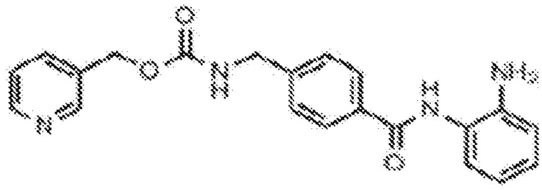 |
| HDAC | 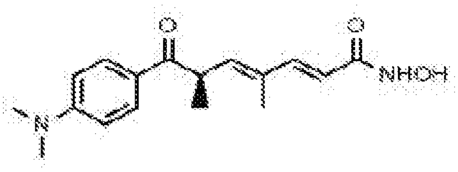 |
| Pan-HDAC | 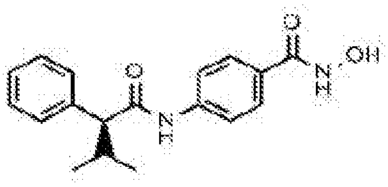 |
| HDAC6 | 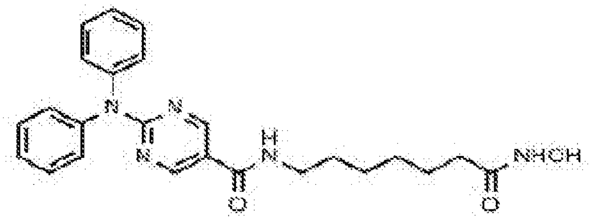 |
| Class I HDAC | 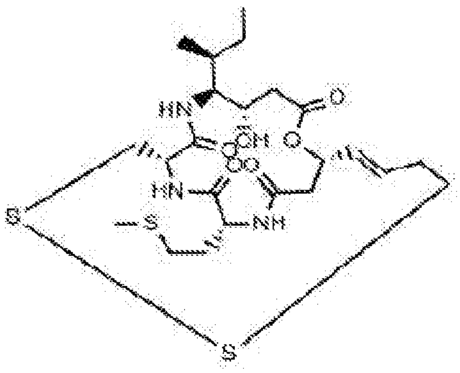 |

| | |
|---|---|
| Class I HDAC | 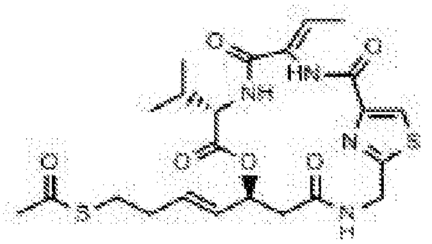 |
| Class I HDAC | 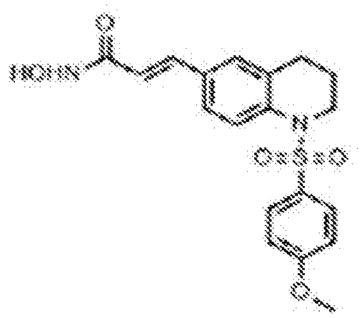 |
| Class IIa HDAC | 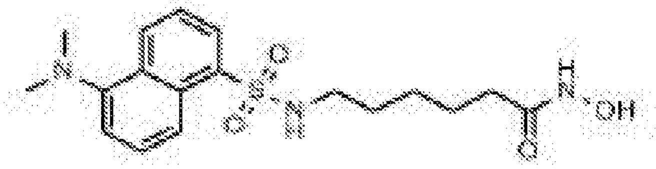 |
| HDAC3 | 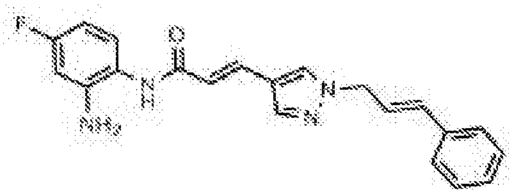 |
| HDAC3 | 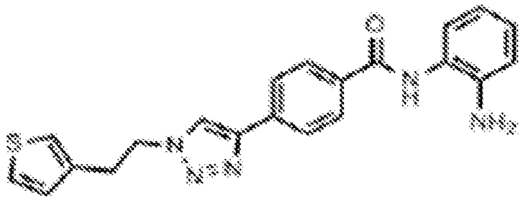 |
| HDAC6 | 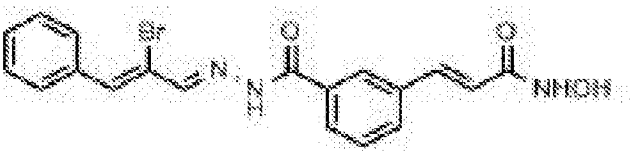 |
| HDAC6 | 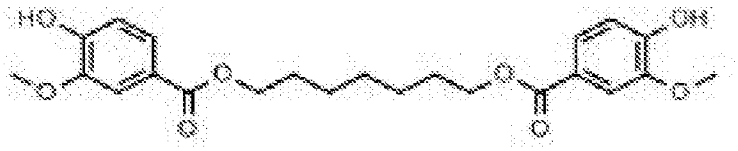 |

| | |
|---|---|
| HDAC6 | 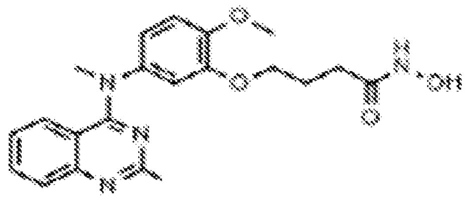 |
| HDAC8 | 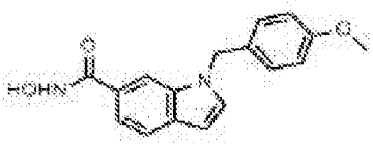 |
| HDAC8 | 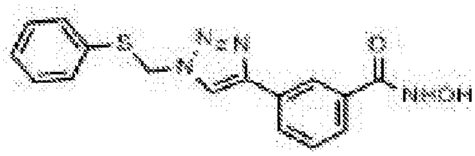 |
| HDAC1, HDAC2 | 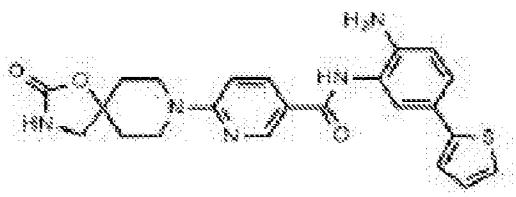 |
| HDAC1, HDAC2 | 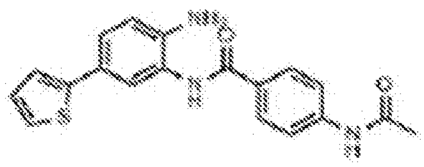 |
| HDAC1 | 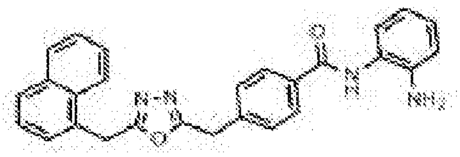 |
| HDAC | 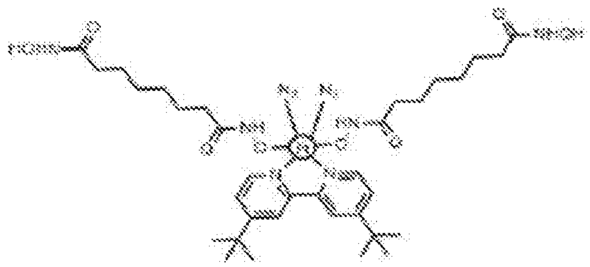 |
| HDAC, PI3K | 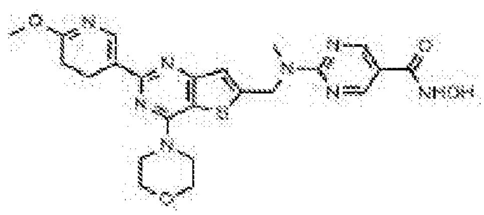 |

| | |
|---|---|
| HDAC, EGFR, HER2 | 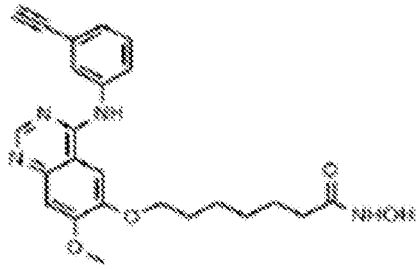 |
| HDAC | 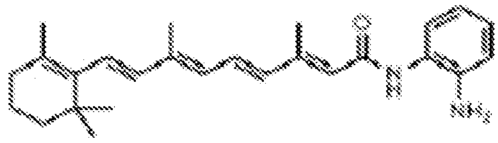 |
| HDAC1, HDAC6, ER | 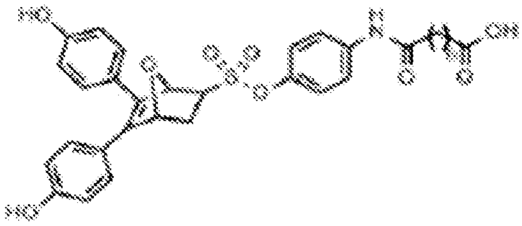 |
| Class I HDACs, ZEB1 | 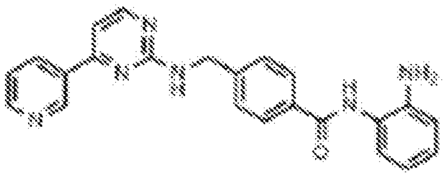 |
| HDAC, Akt | 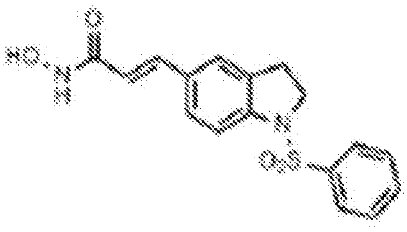 |
| HDAC | 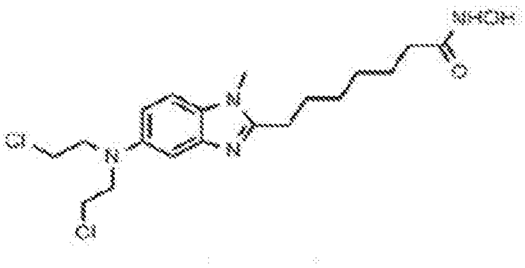 |
| HDAC | 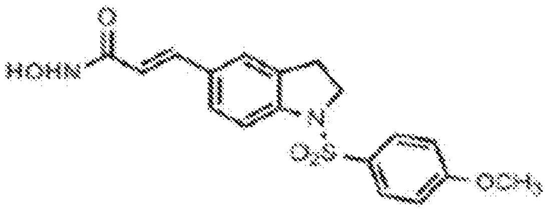 |

| | |
|---|---|
| HDAC1 | 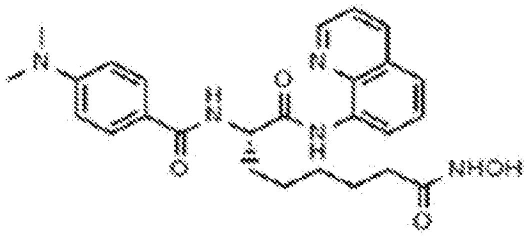 |
| Class I HDACs | 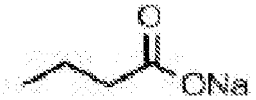 |
| HDAC6 | 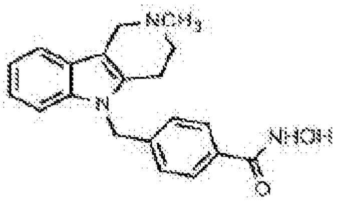 |
| HDAC6 | 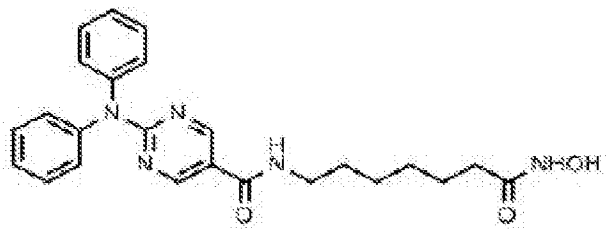 |
| HDAC3, HDAC6, HDAC8 | 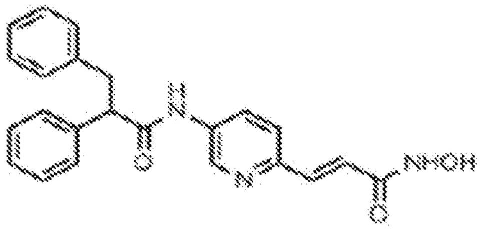 |
| HDAC6 | 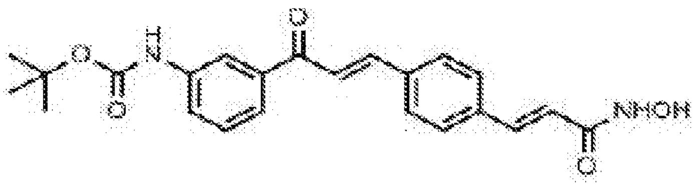 |
| HDAC2 | 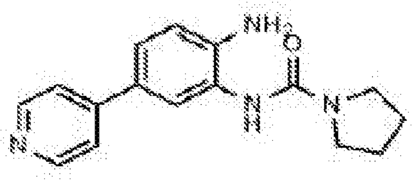 |
| HDAC2 | 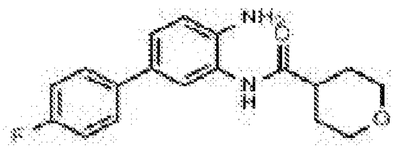 |

| | |
|---|---|
| HDAC4 | 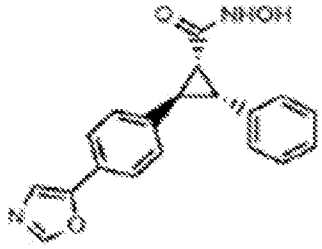 |
| HDAC1, HDAC2 | 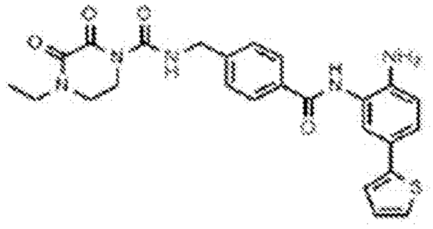 |
| Pan-HDAC | 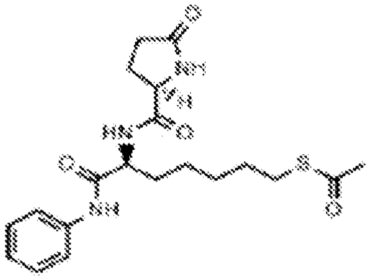 |
| HDAC4 | 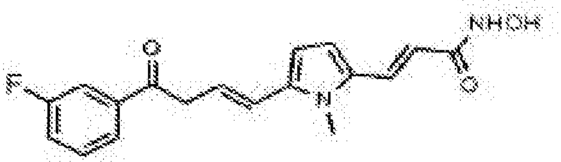 |
| HDAC6 | 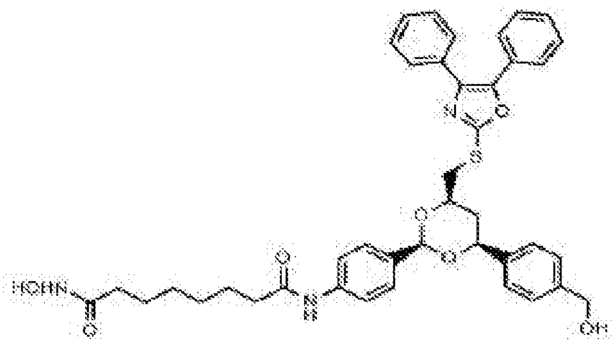 |
| G9a, GLP | 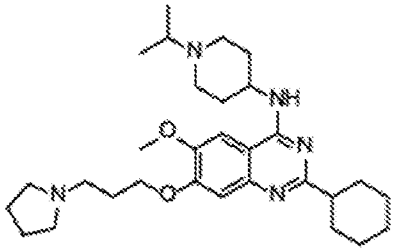 |

| | |
|---|---|
| SMYD2 | 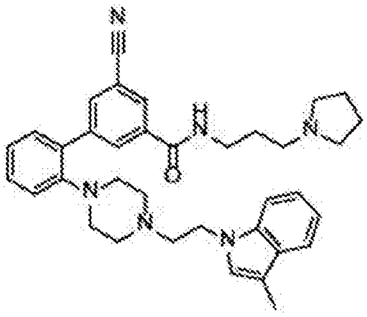 |
| EZH2 | 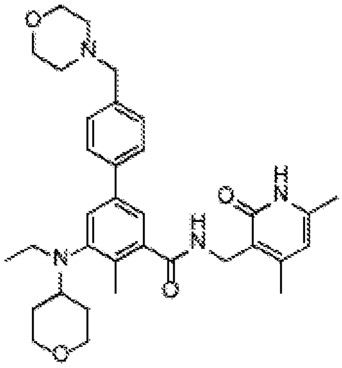 |
| DOT1L | 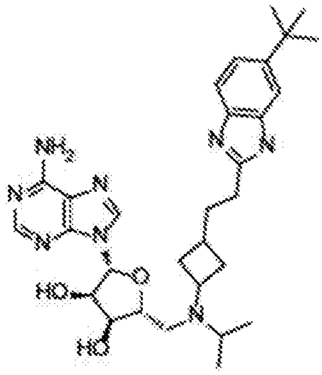 |
| PRMT5 | 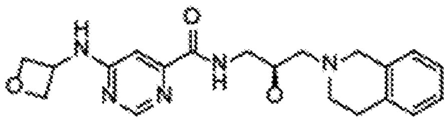 |
| Pan-jmjC | 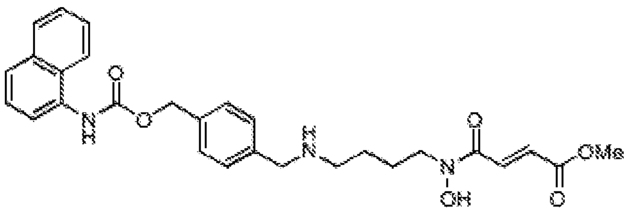 |
| JARID | 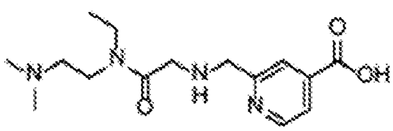 |

| | |
|---|---|
| JMJD3, UTX, JARID | 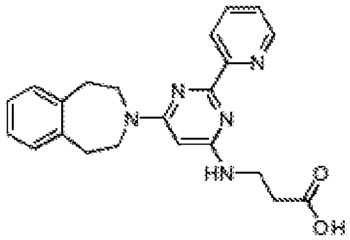 |
| LSD1 | 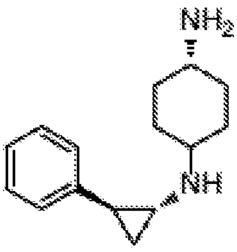 |
| L3MBTL1-MBT | 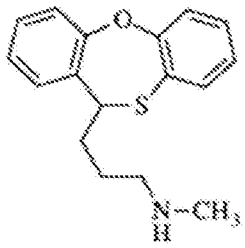 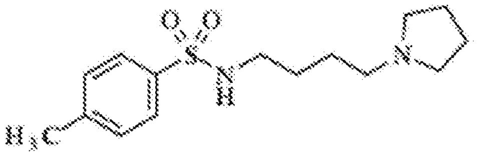 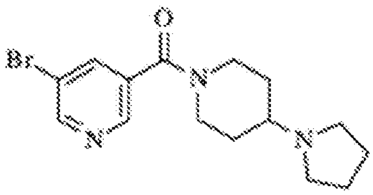 |

| | |
|---|---|
| L3MBTL1-MBT | 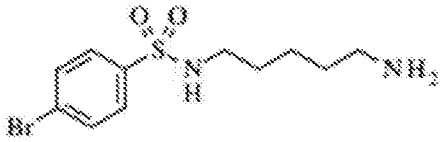 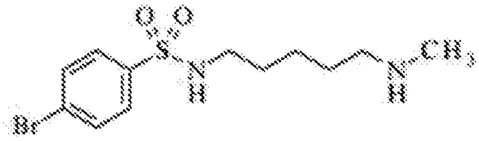 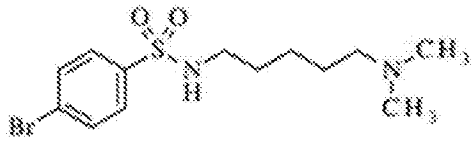 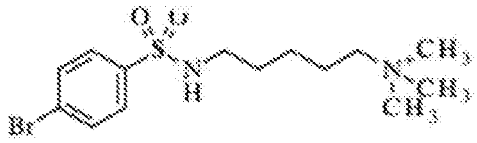 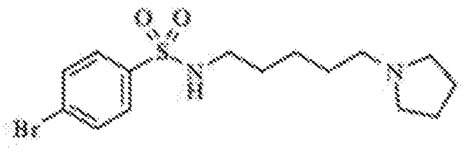 |
| L3MBTL3-MBT | 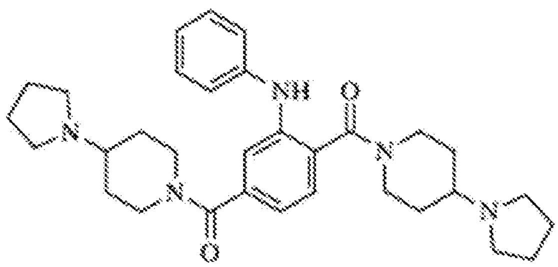 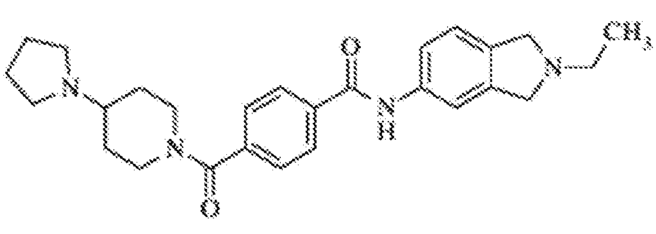 |

| | |
|---|---|
| CBX7 | 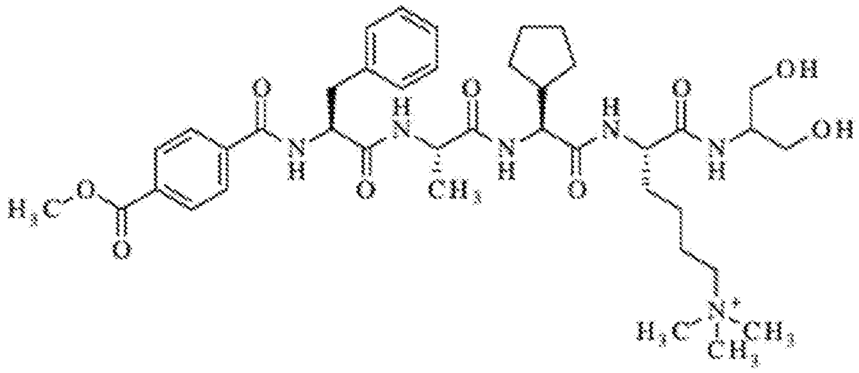 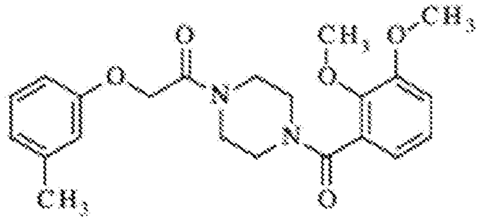 |
| 53BP1 | 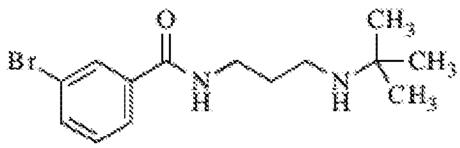 |
| JARID1A-PHD3 | 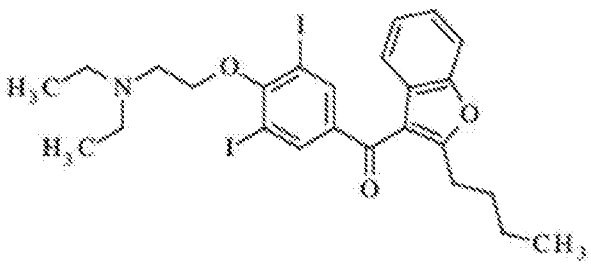 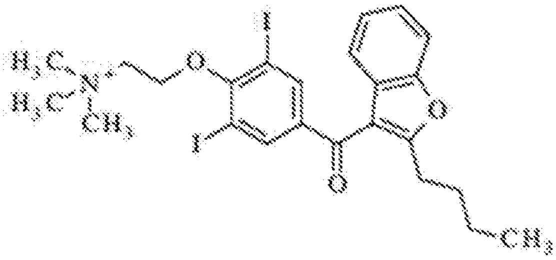 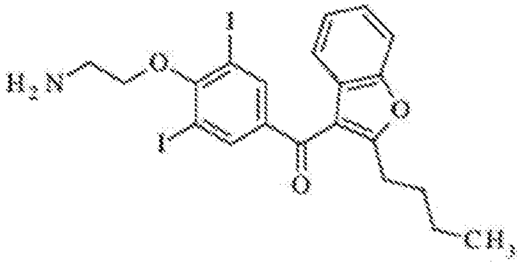 |

| | |
|---|---|
| Pygo-PHD | 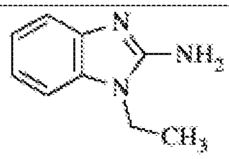 |
| WDR5-MML | 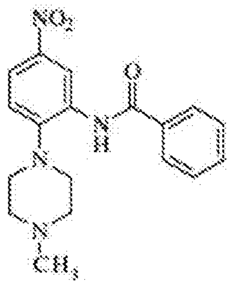 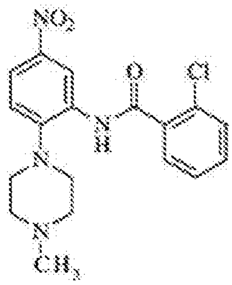 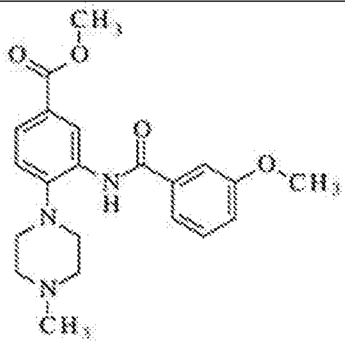 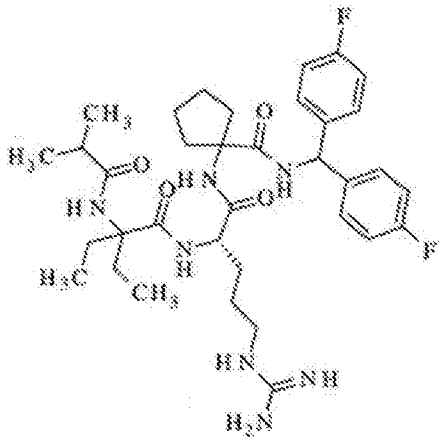 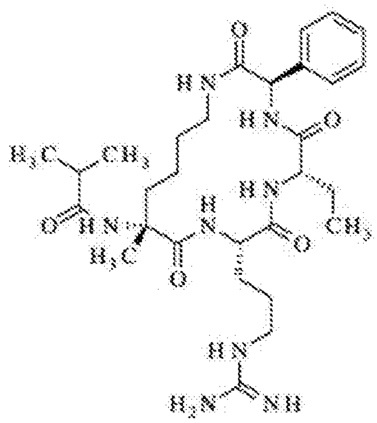 |
| CDK1,<br>CDK2,<br>CDK4,<br>CDK5,<br>CDK6,<br>CDK7, CDK9 | 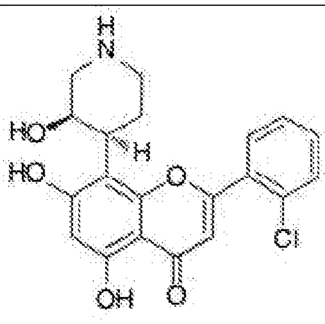 |
| CDK1,<br>CDK2,<br>CDK4,<br>CDK6, CDK9 | 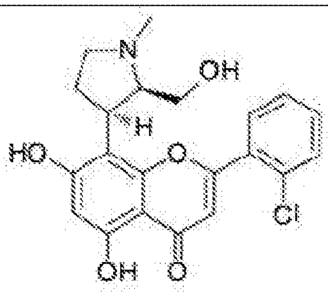 |

| | |
|---|---|
| CDK1, CDK2, CDK5, CDK7 | 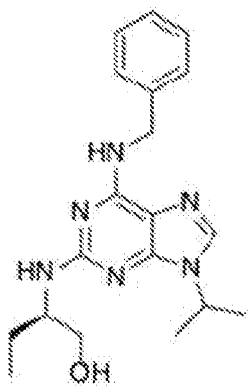 |
| CDK1, CDK2, CDK5, CDK9 | 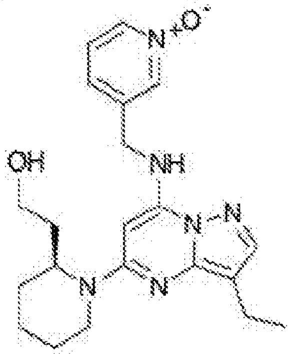 |
| CDK1, CDK2, CDK4, CDK5, CDK6, CDK7 | 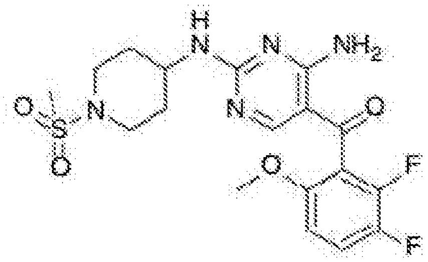 |
| CDK1, CDK2, CDK4, CDK5, CDK7, CDK9 | 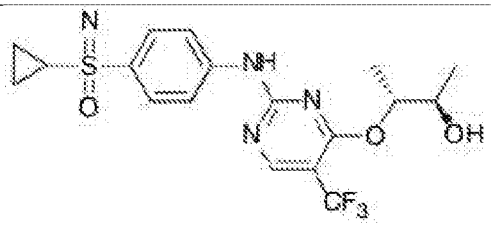 |
| CDK1, CDK2, CDK5, CDK7, CDK9 | 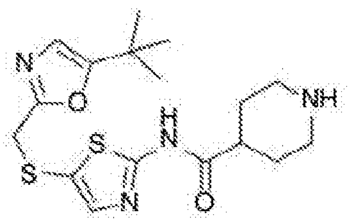 |

| | |
|---|---|
| CDK4, CDK6 | 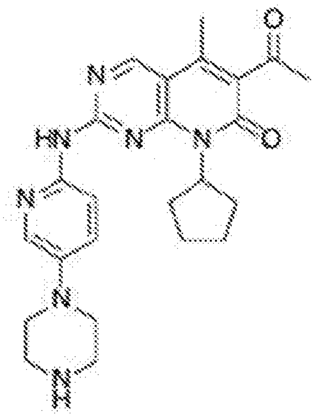 |
| CDK1, CDK2, CDK4, CDK5 | 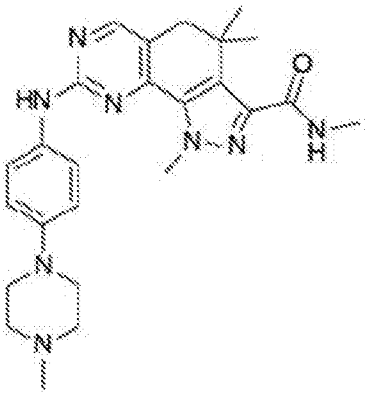 |
| CDK4, CDK6 | 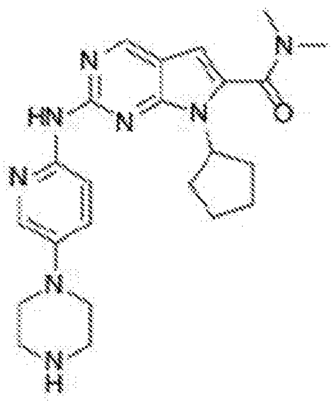 |
| CDK1, CDK2, CDK5, CDK6, CDK7, CDK9 | 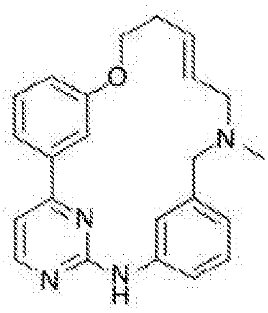 |

| | |
|---|---|
| CDK2, CDK4, CDK5, CDK6, CDK9 | 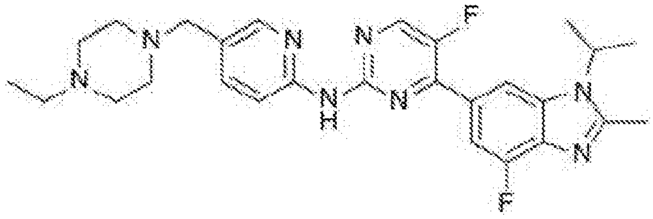 |
| CDK1, CDK2, CDK4, CDK7, CDK9 | 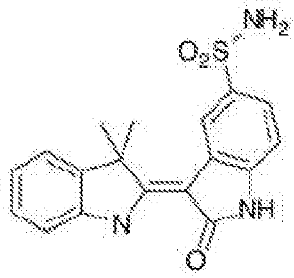 |
| CDK1, CDK2, CDK4, CDK5, CDK6, CDK9 | 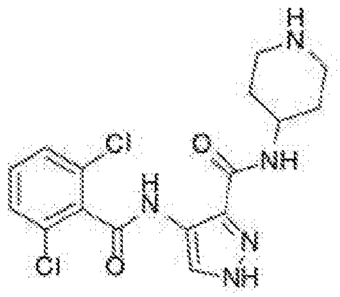 |
| CDK4 | 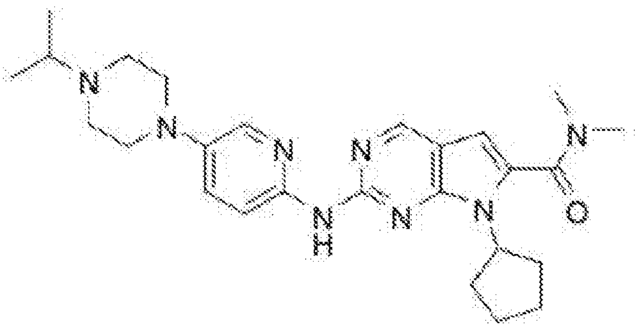 |
| CDK1, CDK4 | 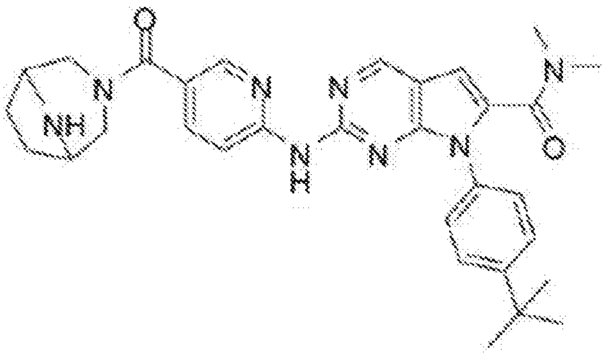 |

| | |
|---|---|
| CDK4, CDK6 | 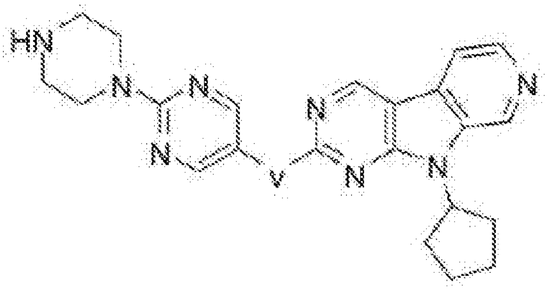 |
| CDK4 | 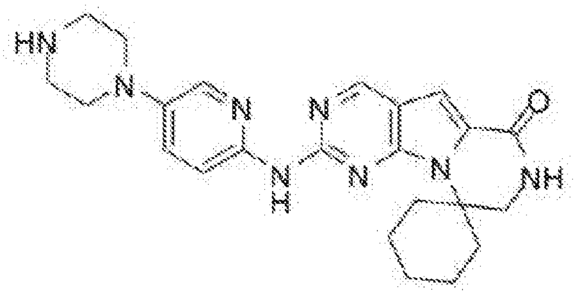 |
| CDK2, CDK9 | 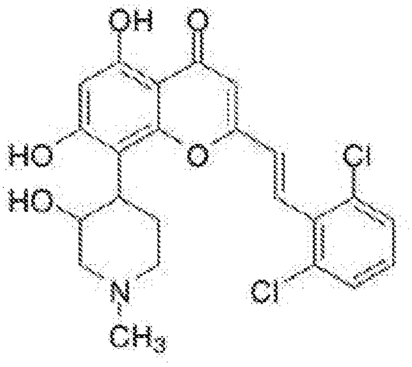 |
| CDK5 | 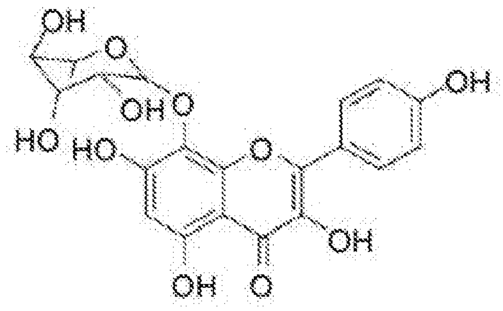 |
| CDK8 | 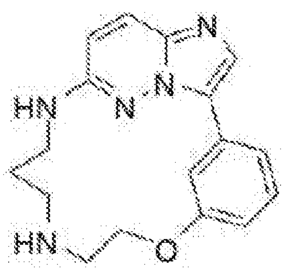 |

| | |
|---|---|
| CDK1, CDK2, CDK5, CDK7, CDK9 | 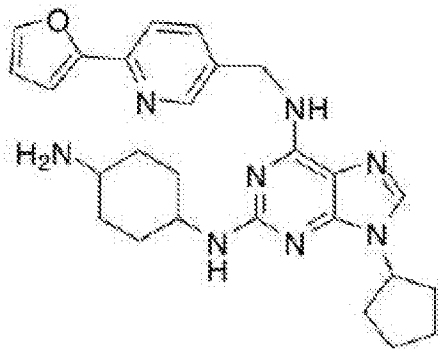 |
| CDKs | 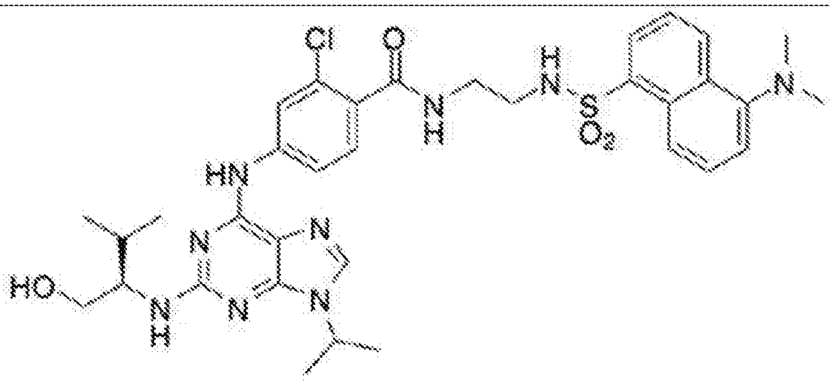 |
| | 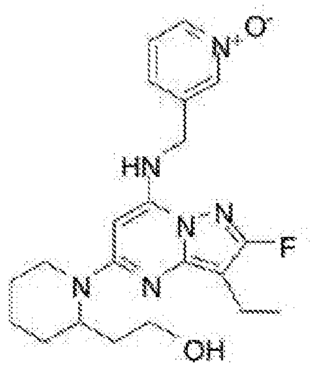 |
| CDKs | 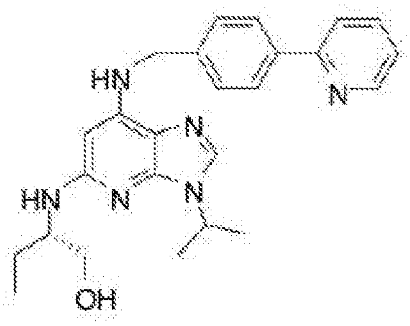 |

| | |
|---|---|
| CDK1, CDK2, CDK5, CDK9 | 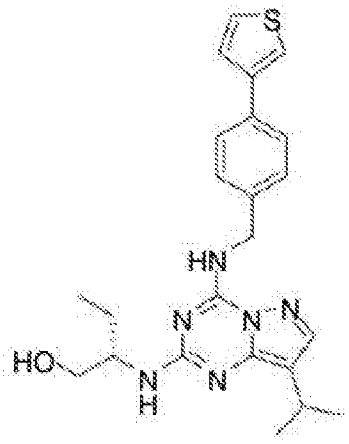 |
| CDK7 | 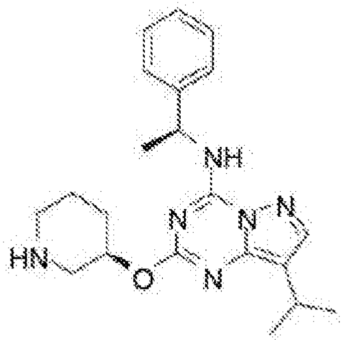 |
| CDK7 | 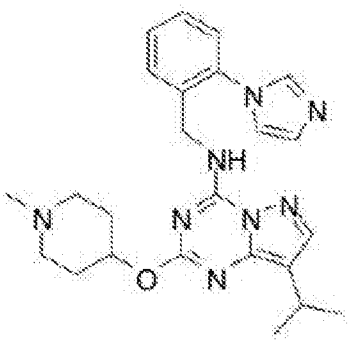 |
| CDK2 | 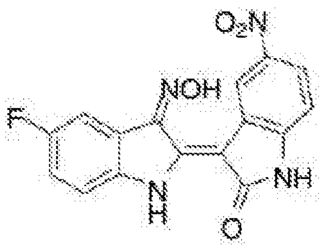 |
| CDK2, HDAC | 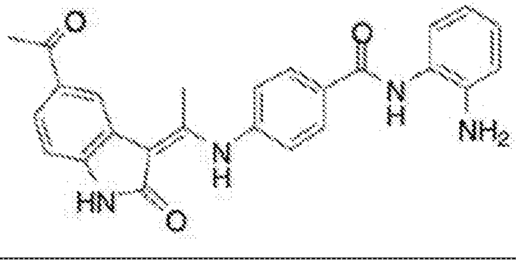 |

| | |
|---|---|
| CDK3 | 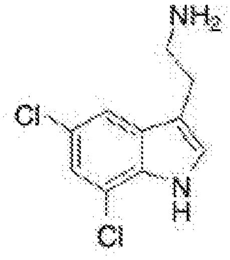 |
| CDK5 | 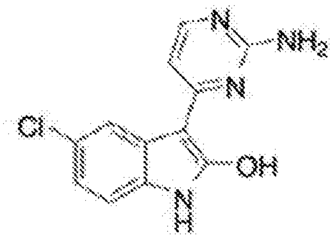 |
| CDK4 | 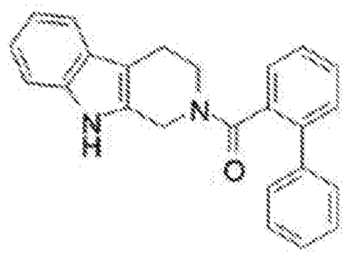 |
| CDK4 | 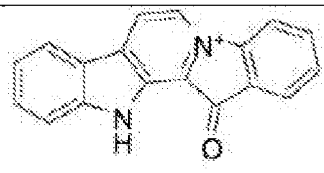 |
| CDK8 | 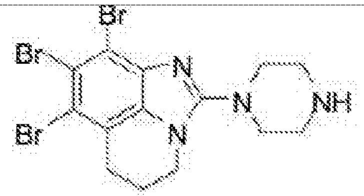 |
| CDK4 | 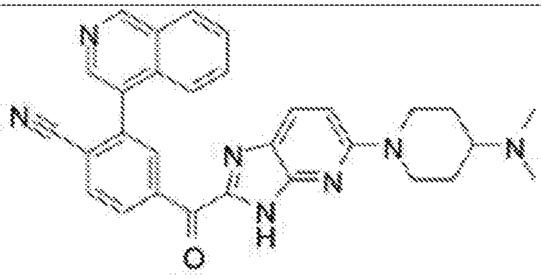 |
| CDK2, CDK9 | 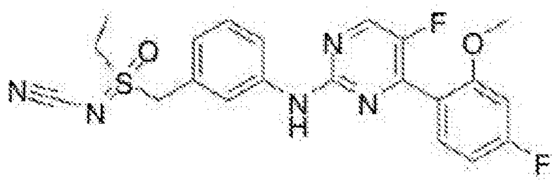 |

| | |
|---|---|
| CDK2, CDK9 | 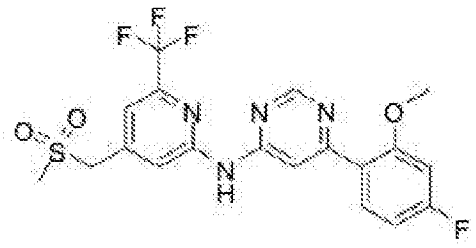 |
| CDK2, CDK9 | 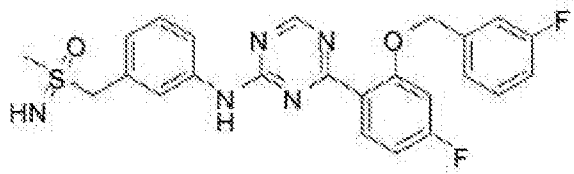 |
| CDK2, CDK9 | 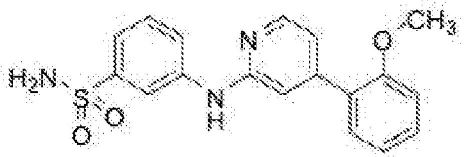 |
| CDK2, CDK9 | 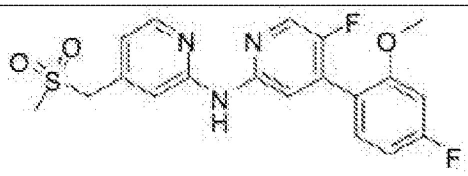 |
| CDK2, CDK9 | 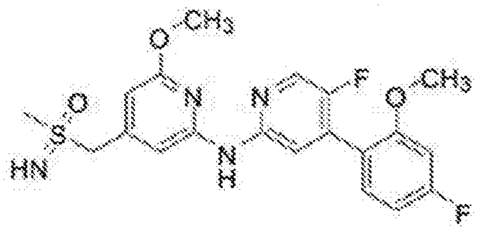 |
| CDK2, CDK9 | 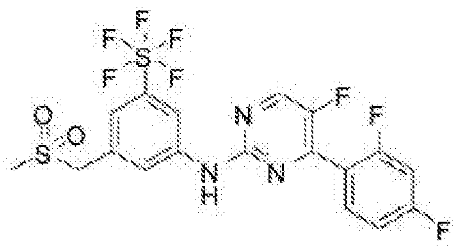 |
| CDK2, CDK9 | 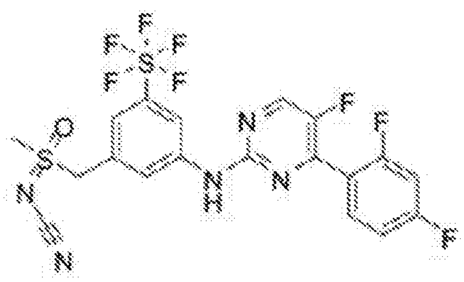 |

| | |
|---|---|
| CDK2, CDK9 | 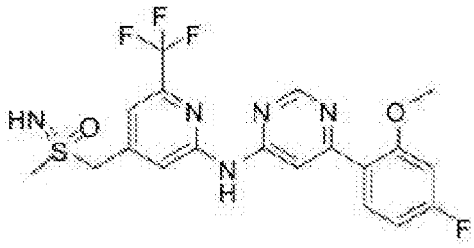 |
| CDK9 | 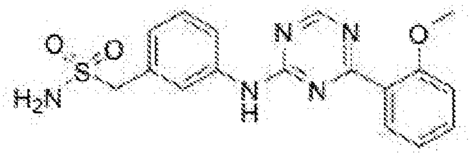 |
| CDK2, HDAC | 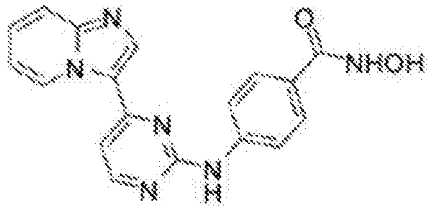 |
| CDK7 | 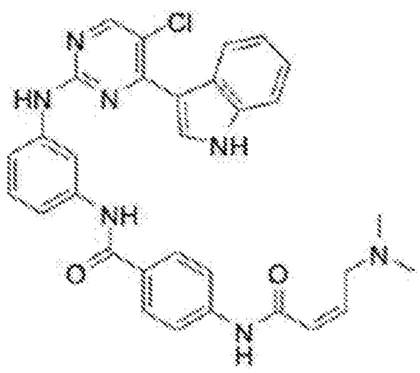 |
| CDK2, CDK9 | 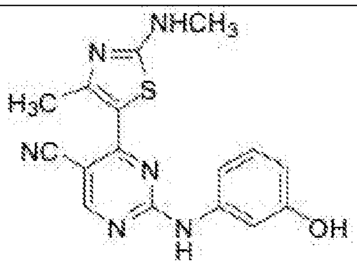 |
| CDK1, CDK2, CDK5, CDK9 | 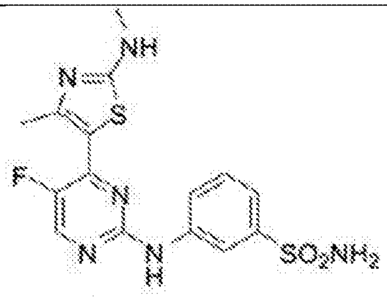 |

| | |
|---|---|
| CDK2, HDAC1 | 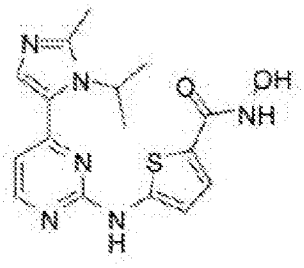 |
| CDK9 | 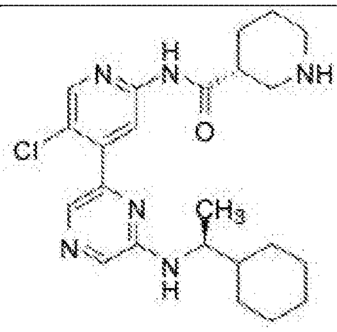 |
| CDK9 | 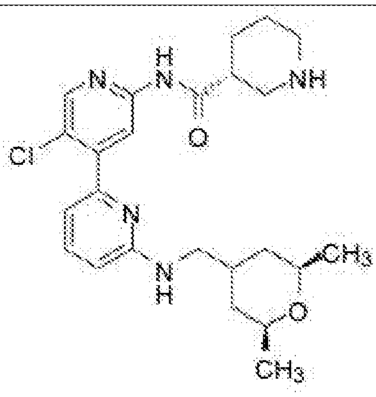 |
| CDK9 | 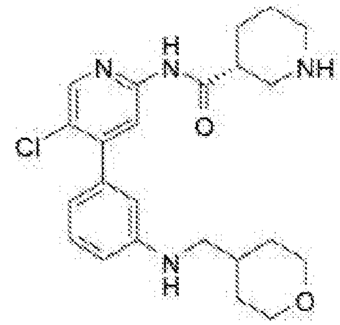 |
| CDK9 | 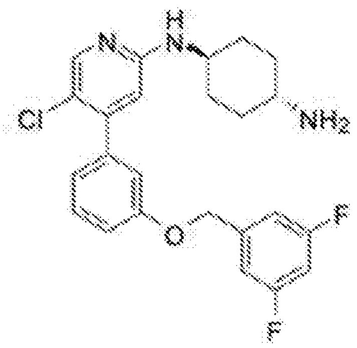 |

| | |
|---|---|
| CDK9 | 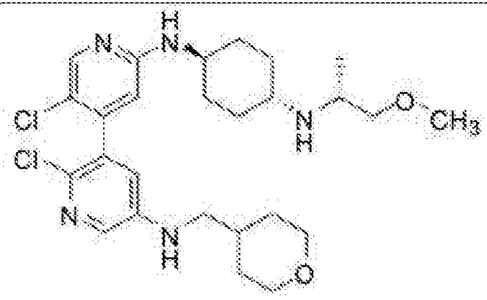 |
| CDK9 | 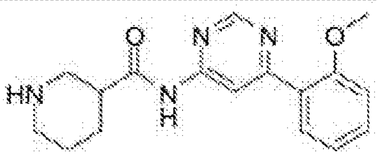 |
| CDK, CDC7 | 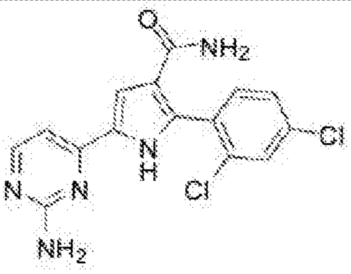 |
| CDK8, CDK19 | 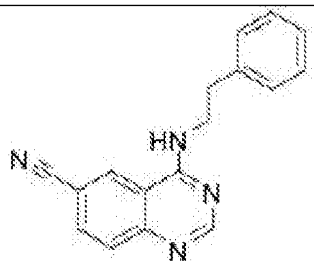 |
| CDK8, CDK19 | 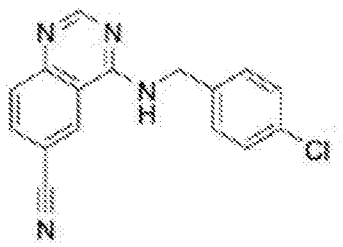 |
| CDK8, CDK19, MAP4K2, YSK4 | 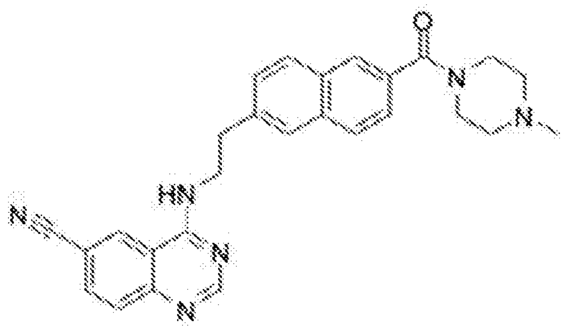 |

| | |
|---|---|
| CDK8, CDK19 | 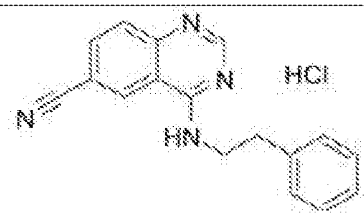 |
| CDK4, CDK6 | 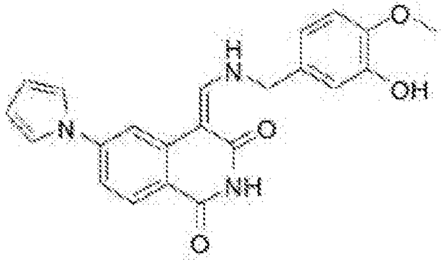 |
| CDK9, CK2, PIM1 | 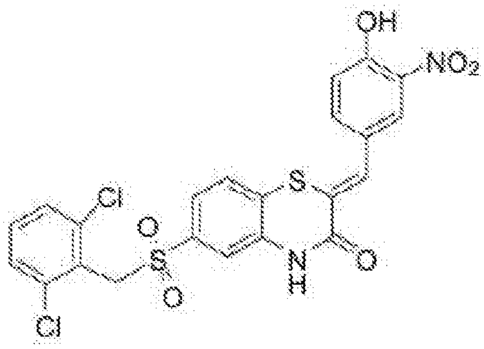 |
| CDK1, CDK2, CDK5 | 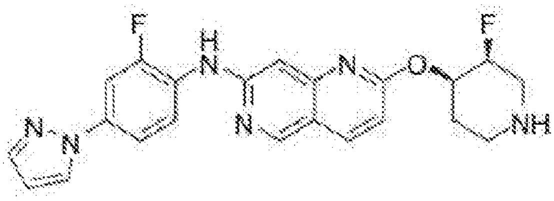 |
| CDK1, CDK2, CDK3, CDK4, CDK6, CDK7, CDK9, HDAC | 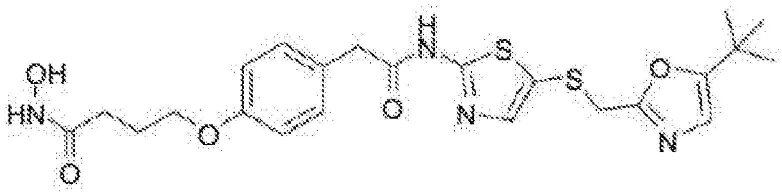 |
| CDK2 | 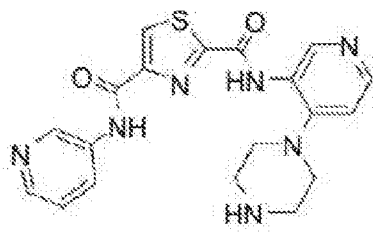 |

| | |
|---|---|
| CDK2 | 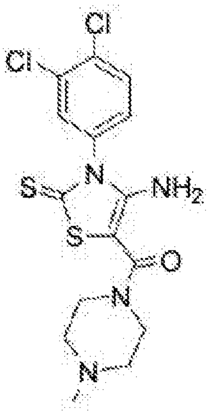 |
| | 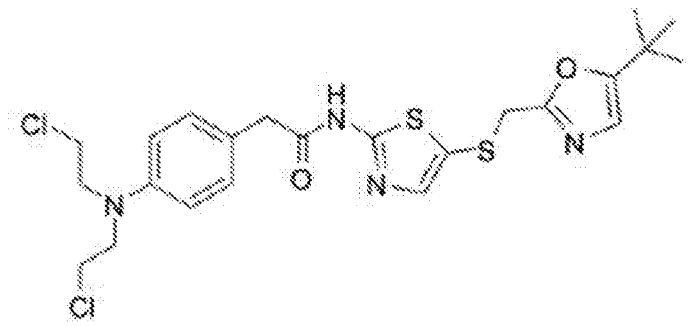 |

In some embodiments, the second terminus does not comprises JQ1, JQ-1, OTX015, RVX208 acid, or RVX208 hydroxyl.

In certain embodiments, the the protein binding moiety is a residue of a compound having a structure of Formula (C-1):

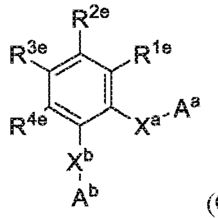

(C-1),

wherein:

$X^a$ is NHC(O)-, -C(O)-NH-, -NHSO$_2$-, or -SO$_2$NH-;

$A^a$ is selected from an optionally substituted -$C_{1-12}$ alkyl, optionally substituted -$C_{2-10}$ alkenyl, optionally substituted -$C_{2-10}$ alkynyl, optionally substituted -$C_{1-12}$ alkoxyl, optionally substituted -$C_{1-12}$ haloalkyl, optionally substituted $C_{6-10}$ aryl, optionally substituted $C_{3-7}$ cycloalkyl, optionally substituted 5- to 10 membered heteroaryl, and optionally substituted 5- to 10-membered heterocycloalkyl;

$X^b$ is a bond, NH, NH-$C_{1-10}$alkylene, -$C_{1-12}$ alkyl, NHC(O)-, or -C(O)-NH-;

$A^b$ is selected from an optionally substituted -$C_{1-12}$ alkyl, optionally substituted -$C_{2-10}$ alkenyl, optionally substituted -$C_{2-10}$ alkynyl, optionally substituted -$C_{1-12}$ alkoxyl, optionally substituted -$C_{1-12}$haloalkyl, optionally substituted $C_{6-10}$ aryl, optionally substituted $C_{3-7}$ cycloalkyl, optionally substituted 5- to 10 membered heteroaryl, and optionally substituted 4- to 10-membered heterocycloalkyl; and

each $R^{1e}$, $R^{2e}$, $R^{3e}$, $R^{4e}$ are independently selected from the group consisting of H, OH, - NO$_2$, halogen, amine, COOH, COOC$_{1-10}$alkyl, -NHC(O)-optionally substituted -$C_{1-12}$ alkyl, - NHC(O)(CH$_2$)$_{1-4}$NR$^f$R$^g$, -NHC(O)(CH$_2$)$_{0-4}$ CHR$^f$(NR$^f$R'$^g$), -NHC(O)(CH$_2$)$_{0-4}$ CHR$^f$R$^g$, - NHC(O)(CH$_2$)$_{0-4}$-$C_{3-7}$ cycloalkyl, -NHC(O)(CH$_2$)$_{0-4}$-5- to 10-membered heterocycloalkyl, NHC(O)(CH$_2$)$_{0-4}$$C_{6-10}$ aryl, -NHC(O)(CH$_2$)$_{0-4}$-5- to10-membered heteroaryl, -(CH$_2$)$_{1-4}$-$C_{3-7}$ cy-

cloalkyl, $-(CH_2)_{1-4}$-5- to 10-membered heterocycloalkyl, $-(CH_2)_{1-4}C_{6-10}$ aryl, $-(CH_2)_{1-4}$-5- to10-membered heteroaryl, optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 4- to 10-membered heterocycloalkyl, and wherein each $R^f$and $R^g$ are independently H or $C_{1-6}$ alkyl.

In certain embodiments, the the protein binding moiety is a residue of a compound having a structure of Formula (C-2):

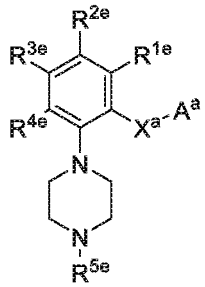
(C-2),

wherein $R^{5e}$ is independently selected from the group consisting of H, $COOC_{1-10}$alkyl, -NHC(O)-optionally substituted $-C_{1-12}$ alkyl, optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkylsubstituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkyl.

In certain embodiments, $A^a$ is selected from an optionally substituted $C_{6-10}$ aryl, optionally substituted $C_{3-7}$ cycloalkyl, optionally substituted 5- to 10 membered heteroaryl, and optionally substituted 5-to 10-membered heterocycloalkyl. In certain embodiments, $A^a$ is an optionally substituted $C_{6-10}$ aryl.

In certain embodiments, the the protein binding moiety is a residue of a compound having a structure of Formula (C-3):

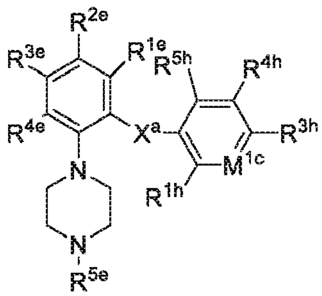
(C-3),

wherein:

$M^{1c}$ is $CR^{2h}$ or N; and

each $R^{1h}$, $R^{2h}$, $R^{3h}$, $R^{4b}$, and $R^{5h}$ are independently selected from the group consisting of H, OH, $-NO_2$, halogen, amine, COOH, $COOC_{1-10}$alkyl, -NHC(O)-optionally substituted $-C_{1-12}$ alkyl, - $NHC(O)(CH_2)_{1-4}NR^fR^g$, $-NHC(O)(CH_2)_{0-4}$ $CHR^f(NR^fR^g)$, $-NHC(O)(CH_2)_{0-4}$ $CHR^fR^g$, - $NHC(O)(CH_2)_{0-4}$-$C_{3-7}$ cycloalkyl, $-NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}$-$C_{3-7}$ cycloalkyl, $-(CH_2)_{1-4}$-5- to 10-membered heterocycloalkyl, $-(CH_2)_1$-$_4C_{6-10}$ aryl, $-(CH_2)_{1-4}$-5- to10-membered heteroaryl, optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkyl, wherein each $R^f$ and $R^g$ are independently H or $C_{1-6}$ alkyl.

In certain embodiments, each $R^{1h}$and $R^{5h}$ are independently hydrogen, halogen, or $C_{1-6}$ alkyl. In certain em-

bodiments, each $R^{2h}$ and $R^{3h}$ are independently H, OH, $-NO_2$, halogen, $C_{1-4}$ haloalkyl, amine, COOH, $COOC_{1-10}$alkyl, -NHC(O)-optionally substituted $-C_{1-12}$ alkyl, $-NHC(O)(CH_2)_{1-4}NR^fR^g$, - $NHC(O)(CH_2)_{0-4}$ CHR'(NR'R"), $-NHC(O)(CH_2)_{0-4}$ $CHR^fR^g$, $-NHC(O)(CH_2)_{0-4}-C_{3-7}$ cycloalkyl, - $NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}-C_{3-7}$ cycloalkyl, $-(CH_2)_{1-4}$-5- to 10-membered heterocycloalkyl, $-(CH_2)_{1-4}C_{6-10}$ aryl, $-(CH_2)_{1-4}$-5- to10-membered heteroaryl, optionally substituted $-C_{2-10}$ alkenyl, optionally substituted $-C_{2-10}$ alkynyl, optionally substituted $-C_{1-12}$ alkoxyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkyl. In certain embodiments, $R^{1e}$, $R^{3e}$, and $R^{4e}$ are hydrogen.

In certain embodiments, $R^{2e}$ is selected from the group consisting of H, OH, $-NO_2$, halogen, amine, COOH, $COOC_{1-10}$alkyl, -NHC(O)-optionally substituted $-C_{1-12}$ alkyl, $-NHC(O)(CH_2)_{1-4}NR^fR^g$, - $NHC(O)(CH_2)_{0-4}$ $CHR^f(NR^fR^g)$, $-NHC(O)(CH_2)_{0-4}$ $CHR^fR^g$, $-NHC(O)(CH_2)_{0-4}-C_{3-7}$ cycloalkyl, - $NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}-C_{3-7}$ cycloalkyl, $-(CH_2)_{1-4}$-5- to 10-membered heterocycloalkyl, $-(CH_2)_{1-4}C_{6-10}$ aryl, $-(CH_2)_{1-4}$-5- to10-membered heteroaryl, optionally substituted $-C_{1-12}$ alkyl, -optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5-to 10-membered heteroaryl, and optionally substituted 5- to 10-membered heterocycloalkyl, wherein each $R^f$ and $R^g$ are independently H or $C_{1-6}$ alkyl.

In certain embodiments, $R^{2e}$ is an phenyl or pyridinyl optionally substituted with 1-3 substituents, wherein the substituent is independently selected from the group consisting of OH, $-NO_2$, halogen, amine, COOH, $COOC_{1-10}$alkyl, NHC(O) $-C_{1-12}$ alkyl, $-NHC(O)(CH_2)_{1-4}NR^fR^g$, $-NHC(O)(CH_2)_{0-4}$ $CHR^f$ $(NR^fR^g)$, - $NHC(O)(CH_2)_{0-4}$ $CHR^fR^g$, $-NHC(O)(CH_2)_{0-4}-C_{3-7}$ cycloalkyl, $-NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}-C_{3-7}$ cycloalkyl, $-(CH_2)_{1-4}$-5- to 10-membered heterocycloalkyl, $-(CH_2)_{1-4}C_{6-10}$ aryl, $-(CH_2)_{1-4}$-5- to10-membered heteroaryl, $-C_{1-12}$ alkoxyl, $C_{1-12}$ haloalkyl, $C_{6-10}$ aryl, $C_{3-7}$ cycloalkyl, 5- to 10-membered heteroaryl, and 5- to 10-membered heterocycloalkyl, wherein each $R^f$ and $R^g$ are independently H or $C_{1-6}$ alkyl

In certain embodiments, $A^a$ is a $C_{6-10}$ aryl substituted with 1-4 substituents, and each substituent is independently selected from halogen, OH, $NO_2$, an optionally substituted $-C_{1-12}$ alkyl, optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10 membered heteroaryl, and optionally substituted 5- to 10-membered heterocycloalkyl.

In certain embodiments, the protein binding moiety is a residue of a compound having the structure of Formula (C-4):

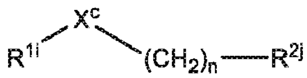
(C-4),

wherein:

- $R^{1c}$ is an optionally substituted $C_{6-10}$ aryl or an optionally substituted 5- to 10-membered heteroaryl,
- $X^c$ is -C(O)NH-, -C(O), $-S(O_2)-$, -NH-, or $-C_{1-4}$alkyl-NH,
- n is 0-10,
- $R^{2j}$ is $-NR^{3j}R^{4j}$, optionally substituted $C_{6-10}$ aryl, optionally substituted $C_{3-7}$ cycloalkyl, optionally substituted 5- to 10-membered heteroaryl, or optionally substituted 4- to 10-membered heterocycloalkyl; and
- each $R^{3j}$ and $R^{4j}$ are independently H or optionally substituted $-C_{1-12}$ alkyl.

In some embodiments, $R^{2j}$ is $-NHC(CH_3)_3$, or a 4- to 10-membered heterocycloalkyl substituted with $C_{1-12}$ alkyl.

In certain embodiments, the protein binding moiety is a residue of a compound having the structure of Formula (C-5):

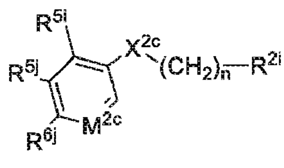
(C-5),

wherein:

$X^{2c}$ is a bond, C(O), $SO_2$, or $CHR^{3c}$;
$M^{2c}$ is CH or N;
n is 0-10,
$R^{2j}$ is $-NR^{3j}R^{4j}$, optionally substituted $C_{6-10}$ aryl, optionally substituted $C_{3-7}$ cycloalkyl, optionally substituted 5- to 10-membered heteroaryl, or optionally substituted 4- to 10-membered heterocycloalkyl;
each $R^{5j}$ is independently $-NR^{3j}R^{4j}$, $-C(O)R^{3j}$, -COOH, $-C(O)NHC_{1-6}$alkyl, an optionally substituted $C_{6-10}$ aryl, or an optionally substituted 5- to 10-membered heteroaryl;
$R^{6j}$ is $-NR^{3j}R^{4j}$, $-C(O)R^{3j}$, an optionally substituted $C_{6-10}$ aryl, or an optionally substituted 5-to 10-membered heteroaryl; and
each $R^{3j}$ and $R^{4j}$ are independently H, an optionally substituted $C_{6-10}$ aryl, optionally substituted 4- to 10-membered heterocycloalkyl, or optionally substituted $-C_{1-12}$ alkyl.

In certain embodiments, $R^{2j}$ is a 4- to 10-membered heterocycloalkyl substituted by a 4- to 10-membered heterocycloalkyl. In certain embodiments, $R^{6j}$ is $-C(O)R^{3j}$, and $R^{3j}$ is a 4- to 10-membered heterocycloalkyl substituted by a 4- to 10-membered heterocycloalkyl. In certain embodiments, each $R^{5j}$ is independently H, $-C(O)R^{3j}$, -COOH, $-C(O)NHC_{1-6}$alkyl, $-NH-C_{6-10}$ aryl, or optionally substituted $C_{6-10}$ aryl

In certain embodiments, the protein binding moiety is a residue of a compound having the structure of Formula (C-6):

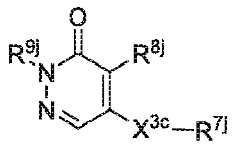 (C-6),

wherein:

$X^{3c}$ is a bond, NH, $C_{1-4}$ alkylene, or $NC_{1-4}$ alkyl;
$R^{7j}$ is an optionally substituted $C_{1-6}$ alkyl, an optionally substituted cyclic amine, an optionally substituted aryl, an optionally substituted 5- to 10-membered heteroaryl, or optionally substituted 4- to 10-membered heterocycloalkyl,
$R^{8j}$ is H, halogen, or $C_{1-6}$ alkyl; and
$R^{9j}$ is H, or $C_{1-6}$ alkyl.

In certain embodiments, $R^{7j}$ is an optionally substituted cyclic secondary or tertiary amine. In certain embodiments, $R^{7j}$ is a tetrahydroisoquinoline optionally substituted with $C_{1-4}$ alkyl.

In certain embodiments, the protein binding moiety is a residue of a compound having the structure of Formula (C-7):

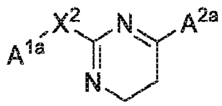 (C-7),

wherein:

$A^{1a}$ is an optionally substituted aryl or heteroaryl;
$X^2$ is a bond, $(CH_2)_{1-4}$, or NH; and
$A^{2a}$ is an optionally substituted aryl, heterocyclic, or heteroaryl, linked to an amide group.

In certain embodiments, $A^{1a}$ is an aryl substituted with one or more halogen, $C_{1-6}$alkyl, hydroxyl, $C_{1-6}$alkoxy, or $C_{1-6}$ haloalkyl. In certain embodiments, $X^2$ is NH. In certain embodiments, $A^{2a}$ is a heterocyclic group. In certain embodiments, $A^{2a}$ is a pyrrolidine. In certain embodiments, $A^{2a}$ is an optionally substituted phenyl. In certain embodiments, $A^{2a}$ is a phenyl optionally substituted with one or more halogen, $C_{1-6}$ alkyl, hydroxyl, $C_{1-6}$ alkoxy, or $C_{1-6}$ haloalkyl.

In certain embodiments, the protein binding moiety is a residue of a compound having the structure of Formula (C-8):

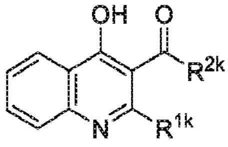

(C-8),

wherein $R^{1k}$ is H or $C_{1-25}$ alkyl and $R^{2k}$ is OH or $-OC_{1-12}$ alkyl.

In certain embodiments, the protein binding moiety is a residue of a compound having the structure of Formula (C-9):

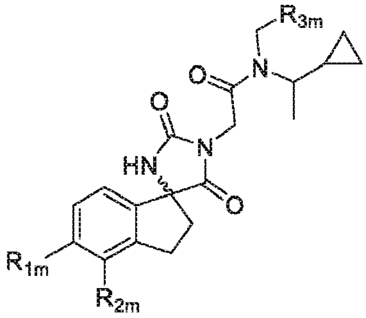

(C-9),

wherein $R_{1m}$ is H, OH, $-CONH_2$, -COOH, -NHC(O)-$C_{1-6}$ alkyl,-NHC(O)O-$C_{1-6}$ alkyl,-NHS$(O)_2$-$C_{1-6}$alkyl, -$C_{1-6}$ alkyl, -$C_{1-6}$ alkoxyl, or -NHC(O)NH-$C_{1-6}$alkyl;

$R_{2m}$ is H, CN, or $CONH_2$; and

$R_{3m}$ is an optionally substituted $C_{6-10}$ aryl.

In certain embodiments, the protein binding moiety is a residue of a compound having the structure of Formula (C-10):

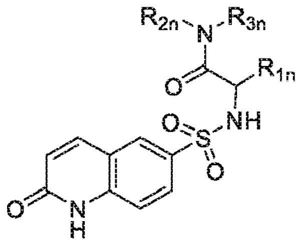

(C-10),

wherein $R_{1n}$ is an optionally substituted $C_{6-10}$ aryl or optionally substituted 5- to 10-membered heteroaryl, and each $R_{2n}$ and $R_{3n}$ are independently H, -$C_{1-4}$ alkyl-$C_{6-10}$ aryl, -$C_{1-4}$alkyl-5-to10-membered heteroaryl, $C_{6-10}$ aryl, or -5-to10-membered heteroaryl, or

$R_{2n}$ and $R_{3n}$ together with N form an optionally substituted 4-10 membered heterocyclic or heteroaryl group.

In certain embodiments, the regulatory molecule is not a bromodomain-containing protein chosen from BRD2, BRD3, BRD4, and BRDT.

In certain embodiments, the regulatory molecule is BRD4. In certain embodiments, the recruiting moiety is a BRD4 activator. In certain embodiments, the BRD4 activator is chosen from JQ-1, OTX015, RVX208 acid, and RVX208 hydroxyl.

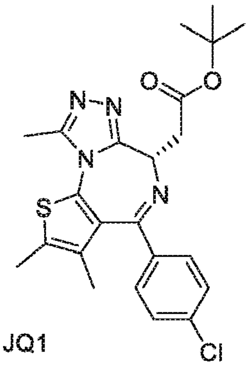
JQ1

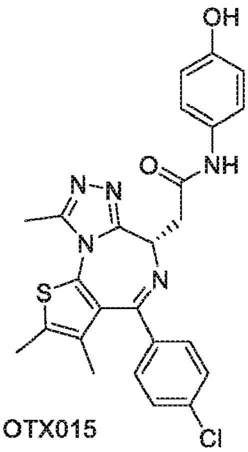
OTX015

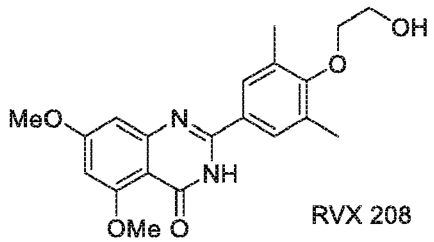
RVX 208

In certain embodiments, the regulatory molecule is BPTF. In certain embodiments, the recruiting moiety is a BPTF activator. In certain embodiments, the BPTF activator is AU1.

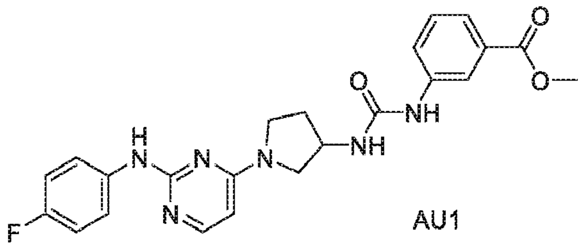
AU1

In certain embodiments, the regulatory molecule is histone acetyltransferase ("HAT"). In certain embodiments, the recruiting moiety is a HAT activator. In certain embodiments, the HAT activator is a oxopiperazine helix mimetic OHM. In certain embodiments, the HAT activator is selected from OHM1, OHM2, OHM3, and OHM4 (BB Lao et al., PNAS USA 2014, 111(21), 7531-7536). In certain embodiments, the HAT activator is OHM4.

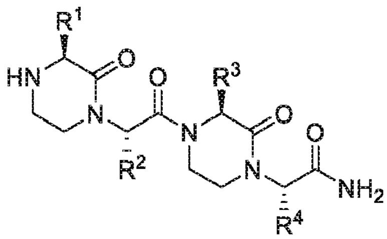

OHM1: $R^1$ = *i*-Bu; $R^2$ = *i*-Bu; $R^3$ = Me; $R^4$ = $CH_2CH_2CONH_2$.

OHM2: $R^1$ = *i*-Bu; $R^2$ = *i*-Bu; $R^3$ = Me; $R^4$ =Me.

OHM3: $R^1$ = *i*-Bu; $R^2$ = Me; $R^3$ = Me; $R^4$ = $CH_2CH_2CONH_2$.

OHM4: $R^1$ = Me; $R^2$ = Me; $R^3$ = Me; $R^4$ = Me.

In certain embodiments, the regulatory molecule is histone deacetylase ("HDAC"). In certain embodiments, the recruiting moiety is an HDAC activator. In certain embodiments, the HDAC activator is chosen from SAHA and 109 (Soragni E Front. Neurol. 2015, 6, 44, and references therein).

SAHA

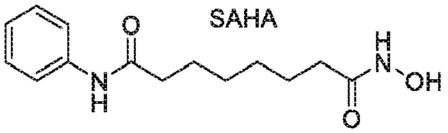

109

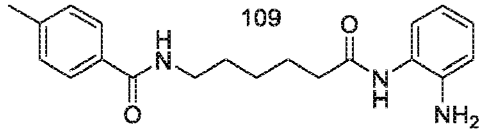

In certain embodiments, the regulatory molecule is histone deacetylase ("HDAC"). In certain embodiments, the recruiting moiety is an HDAC inhibitor. In certain embodiments, the HDAC inhibitor is an inositol phosphate.

In certain embodiments, the regulatory molecules is O-linked β-N-acetylglucosamine transferase ("OGT"). In certain embodiments, the recruiting moiety is an OGT activator. In certain embodiments, the OGT activator is chosen from ST045849, ST078925, and ST060266 (Itkonen HM, "Inhibition of O-GicNAc transferase activity reprograms prostate cancer cell metabolism", Oncotarget 2016, 7(11), 12464-12476).

ST045849

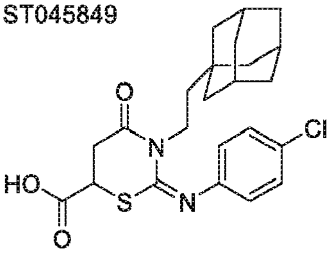

ST078925

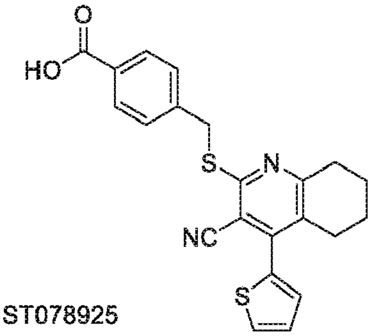

ST060266

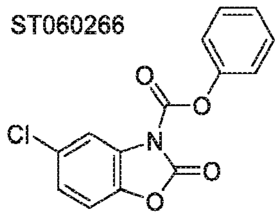

In certain embodiments, the regulatory molecule is chosen from host cell factor 1 ("HCF1") and octamer binding transcription factor ("OCT1"). In certain embodiments, the recruiting moiety is chosen from an HCF1 activator and an OCT1 activator. In certain embodiments, the recruiting moiety is chosen from VP16 and VP64.

In certain embodiments, the regulatory molecule is chosen from CBP and P300. In certain embodiments, the recruiting moiety is chosen from a CBP activator and a P300 activator. In certain embodiments, the recruiting moiety is CTPB.

CTPB

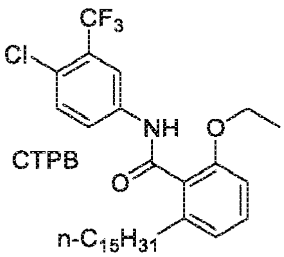

In certain embodiments, the regulatory molecule is P300/CBP-associated factor ("PCAF"). In certain embodiments, the recruiting moiety is a PCAF activator. In certain embodiments, the PCAF activator is embelin.

embelin

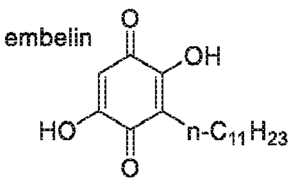

In certain embodiments, the regulatory molecule modulates the rearrangement of histones.

In certain embodiments, the regulatory molecule modulates the glycosylation, phosphorylation, alkylation, or acylation of histones.

In certain embodiments, the regulatory molecule is a transcription factor.

In certain embodiments, the regulatory molecule is an RNA polymerase.

In certain embodiments, the regulatory molecule is a moiety that regulates the activity of RNA polymerase.

In certain embodiments, the regulatory molecule interacts with TATA binding protein.

In certain embodiments, the regulatory molecule interacts with transcription factor II D.

In certain embodiments, the regulatory molecule comprises a CDK9 subunit.

In certain embodiments, the regulatory molecule is P-TEFb.

In certain embodiments, X binds to the regulatory molecule but does not inhibit the activity of the regulatory molecule. In certain embodiments, X binds to the regulatory molecule and inhibits the activity of the regulatory molecule. In certain embodiments, X binds to the regulatory molecule and increases the activity of the regulatory molecule.

In certain embodiments, X binds to the active site of the regulatory molecule. In certain embodiments, X binds to a regulatory site of the regulatory molecule.

In certain embodiments, the recruiting moiety is chosen from a CDK-9 inhibitor, a cyclin T1 inhibitor, and a PRC2 inhibitor.

In certain embodiments, the recruiting moiety is a CDK-9 inhibitor. In certain embodiments, the CDK-9 inhibitor is chosen from flavopiridol, CR8, indirubin-3'-monoxime, a 5-fluoro-N2,N4-diphenylpyrimidine-2,4-diamine, a 4-(thiazol-5-yl)-2-(phenylamino)pyrimidine, TG02, CDKI-73, a 2,4,5-trisubstited pyrimidine derivatives, LCD000067, Wogonin, BAY-1000394 (Roniciclib), AZD5438, and DRB (F Morales et al. "Overview of CDK9 as a target in cancer research", Cell Cycle 2016, 15(4), 519-527, and references therein).

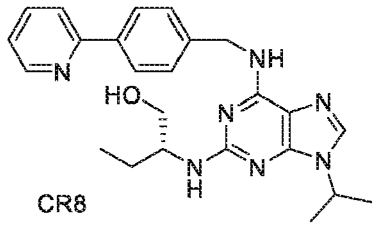

CR8

TG02

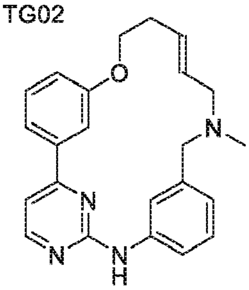

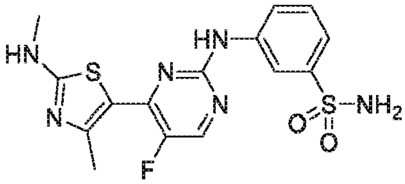

CDKI-73

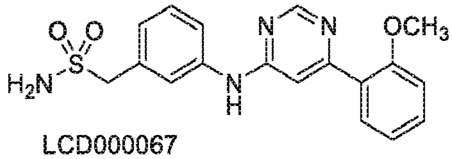

LCD000067

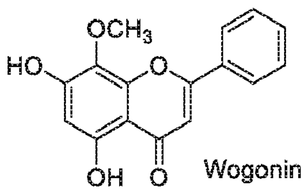

Wogonin

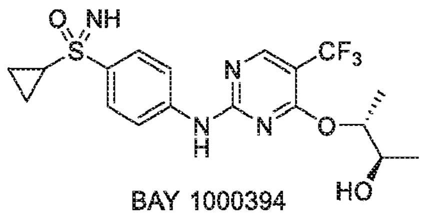

BAY 1000394

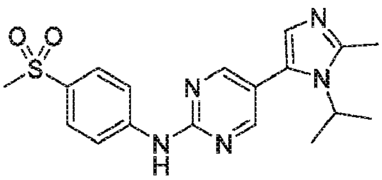

AZD5438

DRB

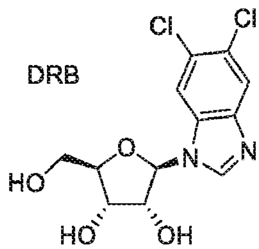

In certain embodiments, the regulatory molecule is a histone demethylase. In certain embodiments, the histone demethylase is a lysine demethylase. In certain embodiments, the lysine demethylase is KDM5B. In certain embodiments, the recruiting moiety is a KDM5B inhibitor. In certain embodiments, the KDM5B inhibitor is AS-8351 (N. Cao, Y. Huang, J. Zheng,et al., "Conversion of human fibroblasts into functional cardiomyocytes by small molecules", Science 2016, 352(6290),1216-1220, and references therein.)

AS-8351

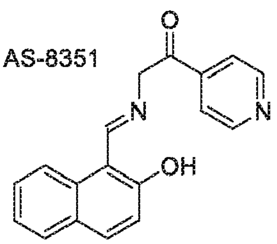

In certain embodiments, the regulatory molecule is the complex between the histone lysine methyltransferases ("HKMT") GLP and G9A ("GLP/G9A"). In certain embodiments, the recruiting moiety is a GLP/G9A inhibitor. In certain embodiments, the GLP/G9A inhibitor is BIX-01294 (Chang Y, "Structural basis for G9a-like protein lysine methyltransferase inhibition by BIX-01294", Nature Struct. Mol. Biol. 2009, 16, 312-317, and references therein).

BIX-01294

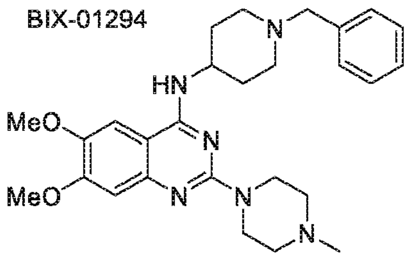

In certain embodiments, the regulatory molecule is a DNA methyltransferase ("DNMT"). In certain embodiments, the regulatory moiety is DNMT1. In certain embodiments, the recruiting moiety is a DNMT1 inhibitor. In certain embodiments, the DNMT1 inhibitor is chosen from RG108 and the RG108 analogues 1149, T1, and G6. (B Zhu et al. BioorgMed Chem 2015, 23(12), 2917-2927 and references therein).

RG108

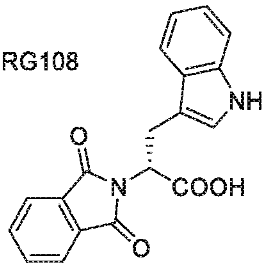

1149

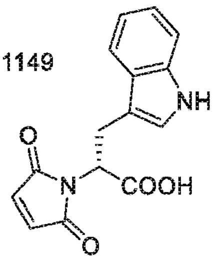

T1

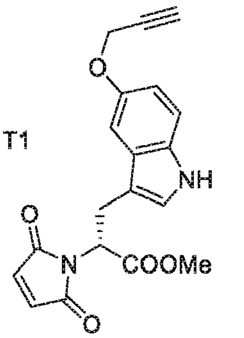

G6

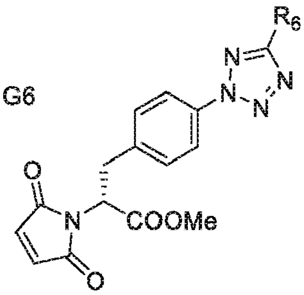

In certain embodiments, the recruiting moiety is a PRC1 inhibitor. In certain embodiments, the PRC1 inhibitor is chosen from UNC4991, UNC3866, and UNC3567 (JI Stuckey et al. Nature Chem Biol 2016, 12(3), 180-187 and references therein; KD Barnash et al. ACS Chem. Biol. 2016, 11(9), 2475-2483, and references therein).

UNC4991

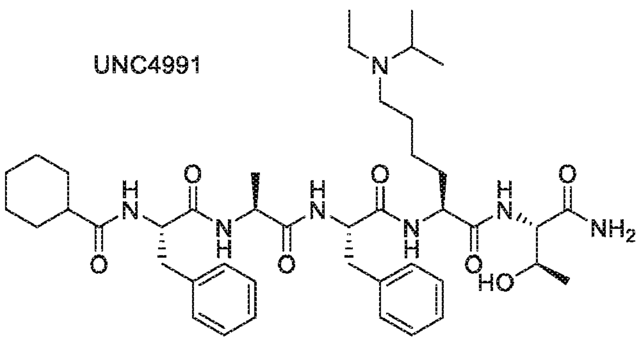

UNC3866

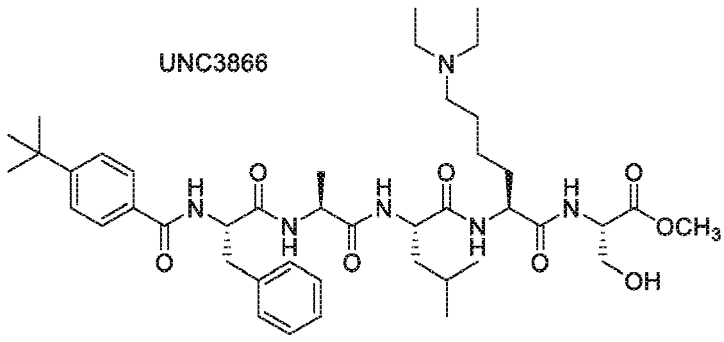

UNC3567

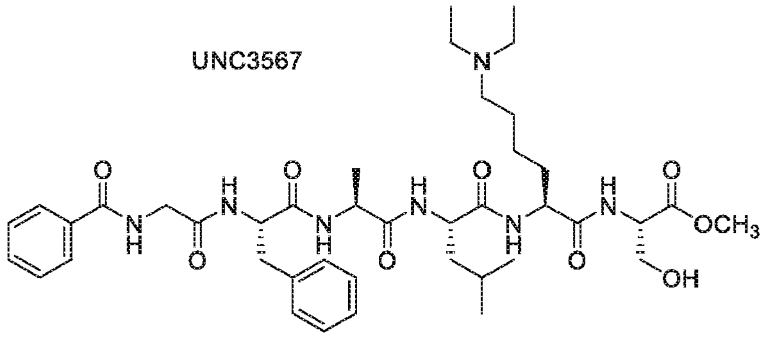

In certain embodiments, the recruiting moiety is a PRC2 inhibitor. In certain embodiments, the PRC2 inhibitor is chosen from A-395, MS37452, MAK683, DZNep, EPZ005687, EI1, GSK126, and UNC1999 (Konze KD ACS Chem Biol 2013, 8(6), 1324-1334, and references therein).

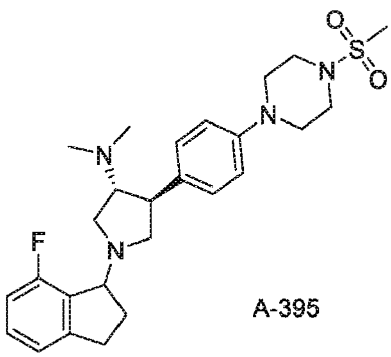

A-395

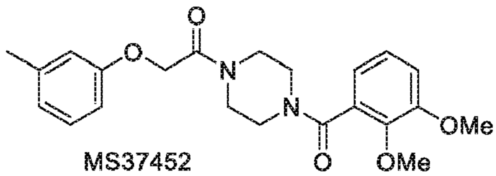

MS37452

MAK683

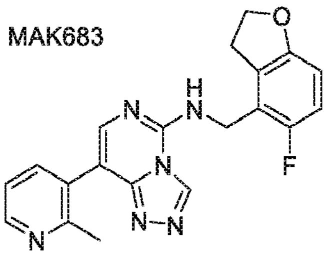

DZNep

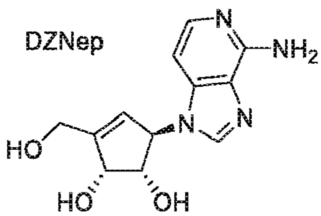

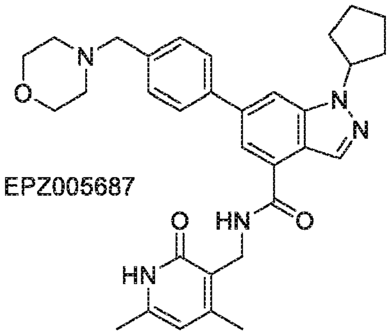

EPZ005687

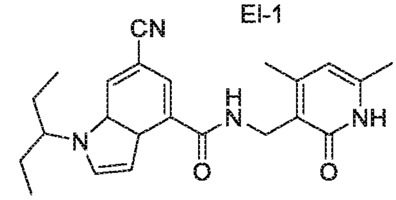

EI-1

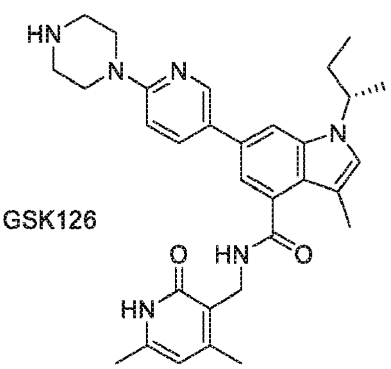

GSK126

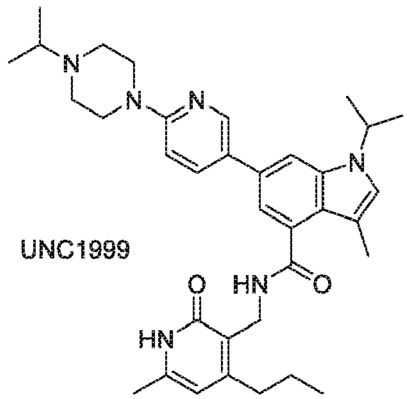

UNC1999

In certain embodiments, the recruiting moiety is rohitukine or a derivative of rohitukine.

In certain embodiments, the recruiting moiety is DB08045 or a derivative of DB08045.

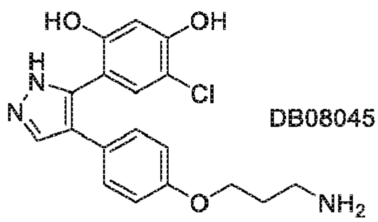

DB08045

In certain embodiments, the recruiting moiety is A-395 or a derivative of A-395.

In certain embodiments, the regulatory molecule is chosen from a bromodomain-containing protein, a nucleosome remodeling factor (NURF), a bromodomain PHD finger transcription factor (BPTF), a ten-eleven translocation enzyme (TET), methylcytosine dioxygenase (TET1), a DNA demethylase, a helicase, an acetyltransferase, and a histone deacetylase ("HDAC").

In certain embodiments, the regulatory molecule is a bromodomain-containing protein chosen from BRD2, BRD3, BRD4, and BRDT.

In certain embodiments, the regulatory molecule is BRD4. In certain embodiments, the recruiting moiety is a BRD4 activator. In certain embodiments, the BRD4 activator is chosen from JQ-1, OTX015, RVX208 acid, and RVX208 hydroxyl.

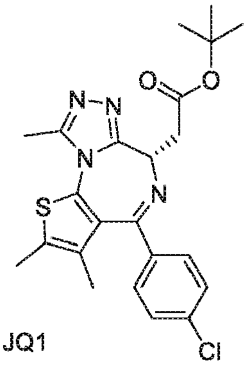

JQ1

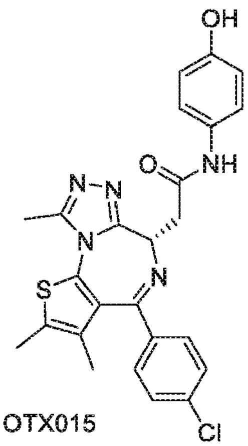

OTX015

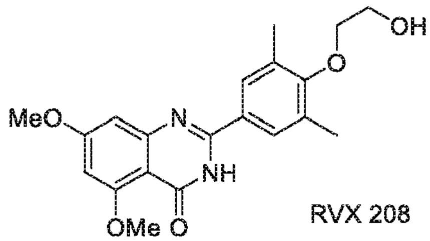

RVX 208

In certain embodiments, the regulatory molecule is BPTF. In certain embodiments, the recruiting moiety is a BPTF activator. In certain embodiments, the BPTF activator is AU1.

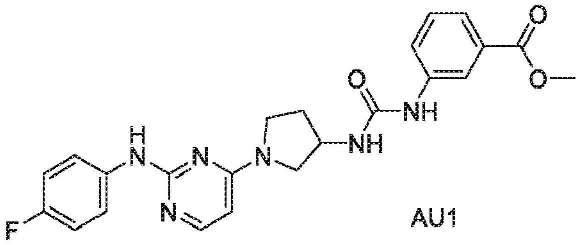

AU1

In certain embodiments, the regulatory molecule is histone acetyltransferase ("HAT"). In certain embodiments, the recruiting moiety is a HAT activator. In certain embodiments, the HAT activator is a oxopiperazine helix mimetic OHM. In certain embodiments, the HAT activator is selected from OHM1, OHM2, OHM3, and OHM4 (BB Lao et al., PNAS USA 2014, 111(21), 7531-7536). In certain embodiments, the HAT activator is OHM4.

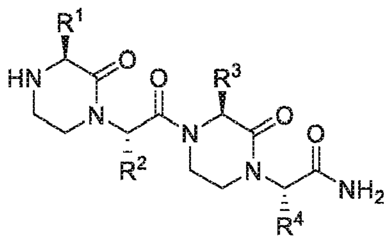

OHM1: $R^1$ = *i*-Bu; $R^2$ = *i*-Bu; $R^3$ = Me; $R^4$ = $CH_2CH_2CONH_2$.
OHM2: $R^1$ = *i*-Bu; $R^2$ = *i*-Bu; $R^3$ = Me; $R^4$ =Me.
OHM3: $R^1$ = *i*-Bu; $R^2$ = Me; $R^3$ = Me; $R^4$ = $CH_2CH_2CONH_2$.
OHM4: $R^1$ = Me; $R^2$ = Me; $R^3$ = Me; $R^4$ = Me.

In certain embodiments, the regulatory molecule is histone deacetylase ("HDAC"). In certain embodiments, the recruiting moiety is an HDAC activator. In certain embodiments, the HDAC activator is chosen from SAHA and 109 (Soragni E Front. Neurol. 2015, 6, 44, and references therein).

SAHA

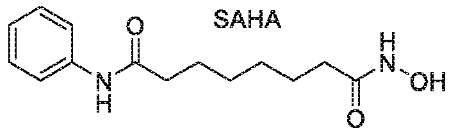

109

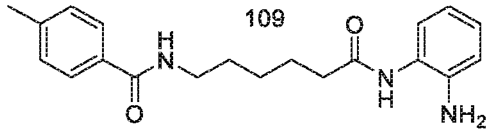

In certain embodiments, the regulatory molecule is histone deacetylase ("HDAC"). In certain embodiments, the recruiting moiety is an HDAC inhibitor. In certain embodiments, the HDAC inhibitor is an inositol phosphate.

In certain embodiments, the regulatory molecules is O-linked β-N-acetylglucosamine transferase ("OGT"). In certain embodiments, the recruiting moiety is an OGT activator. In certain embodiments, the OGT activator is chosen from ST045849, ST078925, and ST060266 (Itkonen HM, "Inhibition of O-GlcNAc transferase activity reprograms prostate cancer cell metabolism", Oncotarget 2016, 7(11), 12464-12476).

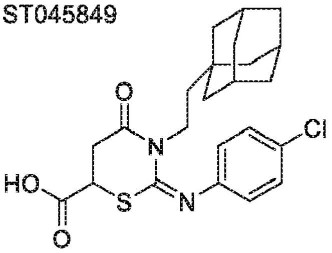
ST045849

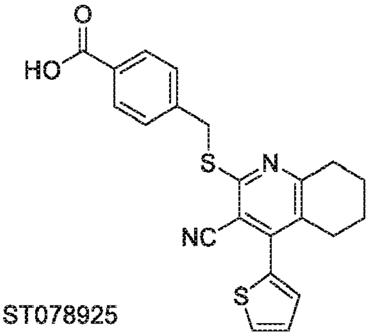
ST078925

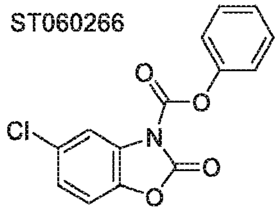
ST060266

In certain embodiments, the regulatory molecule is chosen from host cell factor 1 ("HCF1") and octamer binding transcription factor ("OCT1"). In certain embodiments, the recruiting moiety is chosen from an HCF1 activator and an OCT1 activator. In certain embodiments, the recruiting moiety is chosen from VP16 and VP64.

In certain embodiments, the regulatory molecule is chosen from CBP and P300. In certain embodiments, the recruiting moiety is chosen from a CBP activator and a P300 activator. In certain embodiments, the recruiting moiety is CTPB.

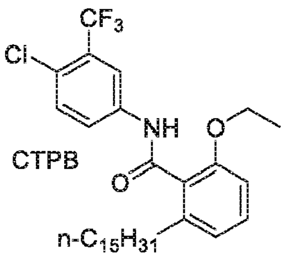
CTPB

In certain embodiments, the regulatory molecule is P300/CBP-associated factor ("PCAF"). In certain embodiments, the recruiting moiety is a PCAF activator. In certain embodiments, the PCAF activator is embelin.

embelin
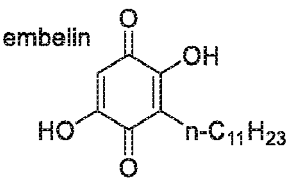

In certain embodiments, the regulatory molecule modulates the rearrangement of histones.

In certain embodiments, the regulatory molecule modulates the glycosylation, phosphorylation, alkylation, or acylation of histones.

In certain embodiments, the regulatory molecule is a transcription factor.

In certain embodiments, the regulatory molecule is an RNA polymerase.

In certain embodiments, the regulatory molecule is a moiety that regulates the activity of RNA polymerase.

In certain embodiments, the regulatory molecule interacts with TATA binding protein.

In certain embodiments, the regulatory molecule interacts with transcription factor II D.

In certain embodiments, the regulatory molecule comprises a CDK9 subunit.

In certain embodiments, the regulatory molecule is P-TEFb.

In certain embodiments, the recruiting moiety binds to the regulatory molecule but does not inhibit the activity of the regulatory molecule. In certain embodiments, the recruiting moiety binds to the regulatory molecule and inhibits the activity of the regulatory molecule. In certain embodiments, the recruiting moiety binds to the regulatory molecule and increases the activity of the regulatory molecule.

In certain embodiments, the recruiting moiety binds to the active site of the regulatory molecule. In certain embodiments, the recruiting moiety binds to a regulatory site of the regulatory molecule.

In certain embodiments, the recruiting moiety is chosen from a CDK-9 inhibitor, a cyclin T1 inhibitor, and a PRC2 inhibitor.

In certain embodiments, the recruiting moiety is a CDK-9 inhibitor. In certain embodiments, the CDK-9 inhibitor

is chosen from flavopiridol, CR8, indirubin-3'-monoxime, a 5-fluoro-N2,N4-diphenylpyrimidine-2,4-diamine, a 4-(thiazol-5-yl)-2-(phenylamino)pyrimidine, TG02, CDKI-73, a 2,4,5-trisubstited pyrimidine derivatives, LCD000067, Wogonin, BAY-1000394 (Roniciclib), AZD5438, and DRB (F Morales et al. "Overview of CDK9 as a target in cancer research", Cell Cycle 2016, 15(4), 519-527, and references therein).

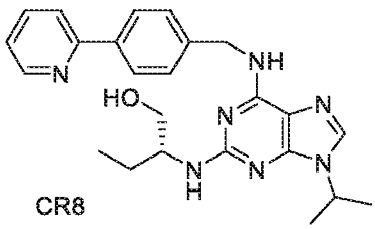

CR8

TG02

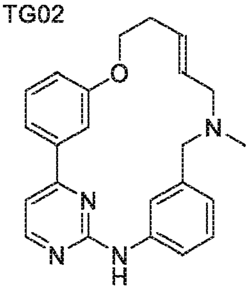

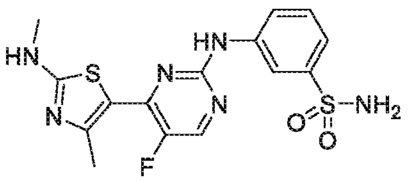

CDKI-73

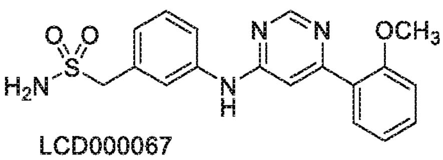

LCD000067

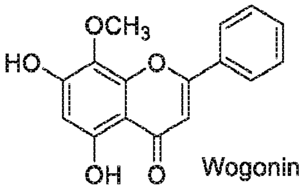

Wogonin

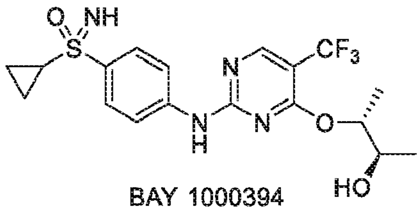

BAY 1000394

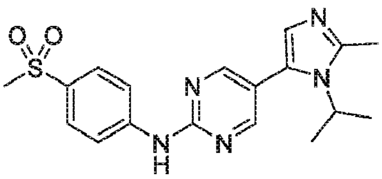

AZD5438

DRB

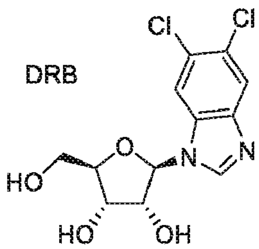

In certain embodiments, the regulatory molecule is a histone demethylase. In certain embodiments, the histone demethylase is a lysine demethylase. In certain embodiments, the lysine demethylase is KDM5B. In certain embodiments, the recruiting moiety is a KDM5B inhibitor. In certain embodiments, the KDM5B inhibitor is AS-8351 (N. Cao, Y. Huang, J. Zheng,et al., "Conversion of human fibroblasts into functional cardiomyocytes by small molecules", Science 2016, 352(6290),1216-1220, and references therein.)

AS-8351

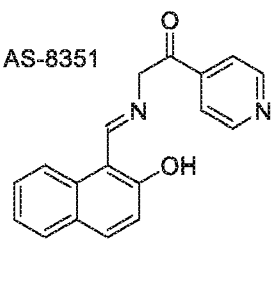

In certain embodiments, the regulatory molecule is the complex between the histone lysine methyltransferases ("HKMT") GLP and G9A ("GLP/G9A"). In certain embodiments, the recruiting moiety is a GLP/G9A inhibitor. In certain

embodiments, the GLP/G9A inhibitor is BIX-01294 (Chang Y, "Structural basis for G9a-like protein lysine methyltransferase inhibition by BIX-01294", Nature Struct. Mol. Biol. 2009, 16, 312-317, and references therein).

BIX-01294

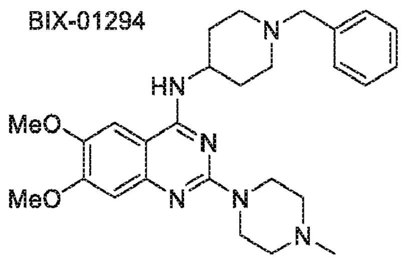

In certain embodiments, the regulatory molecule is a DNA methyltransferase ("DNMT"). In certain embodiments, the regulatory moiety is DNMT1. In certain embodiments, the recruiting moiety is a DNMT1 inhibitor. In certain embodiments, the DNMT1 inhibitor is chosen from RG108 and the RG108 analogues 1149, T1, and G6. (B Zhu et al. BioorgMed Chem 2015, 23(12), 2917-2927 and references therein).

RG108

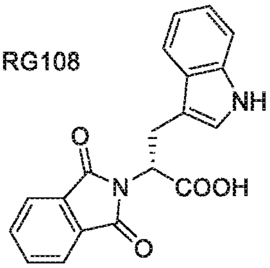

1149

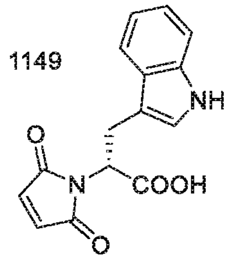

T1

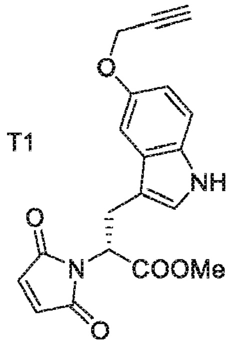

G6

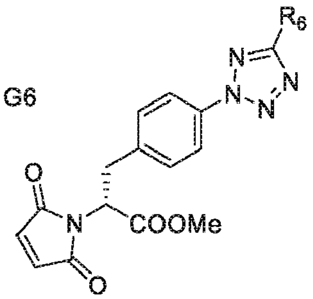

In certain embodiments, the recruiting moiety is a PRC1 inhibitor. In certain embodiments, the PRC1 inhibitor is chosen from UNC4991, UNC3866, and UNC3567 (JI Stuckey et al. Nature Chem Biol 2016, 12(3), 180-187 and references therein; KD Barnash et al. ACS Chem. Biol. 2016, 11(9), 2475-2483, and references therein).

UNC4991

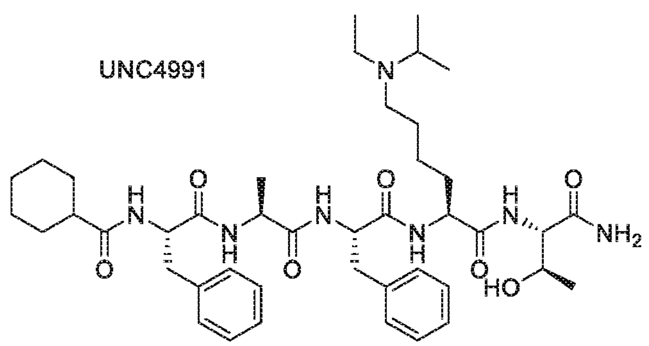

UNC3866

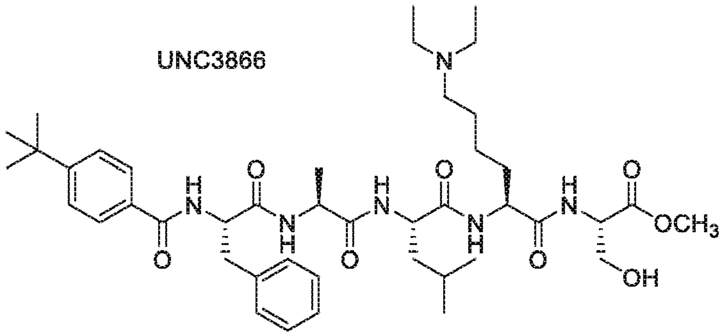

UNC3567

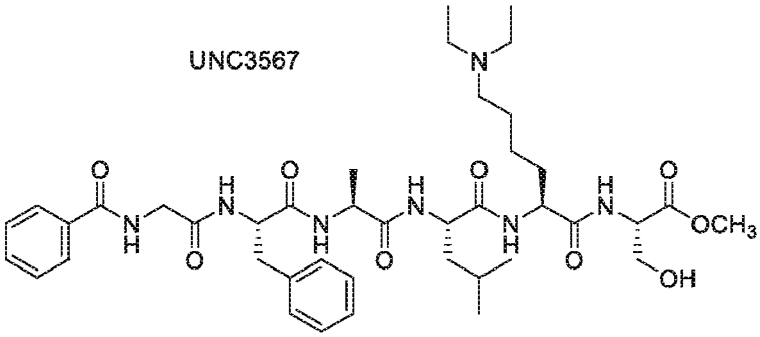

In certain embodiments, the recruiting moiety is a PRC2 inhibitor. In certain embodiments, the PRC2 inhibitor is chosen from A-395, MS37452, MAK683, DZNep, EPZ005687, EI1, GSK126, and UNC1999 (Konze KD ACS Chem Biol 2013, 8(6), 1324-1334, and references therein).

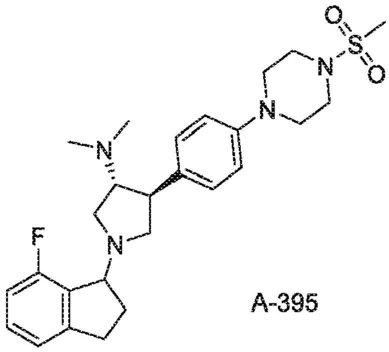

A-395

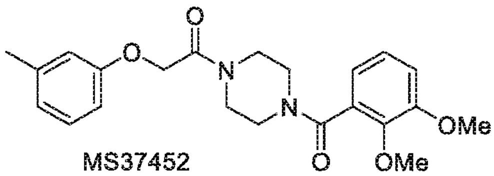

MS37452

MAK683

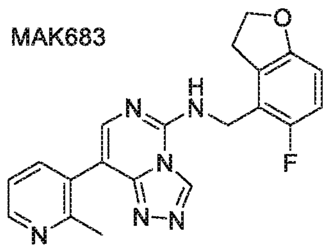

DZNep

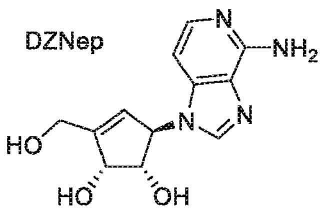

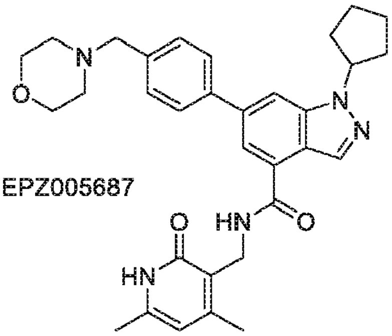

EPZ005687

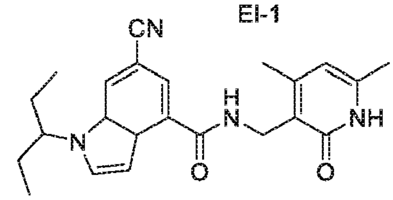

EI-1

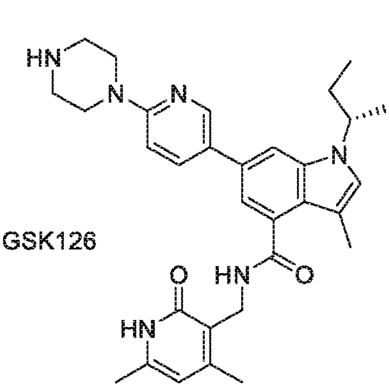

GSK126

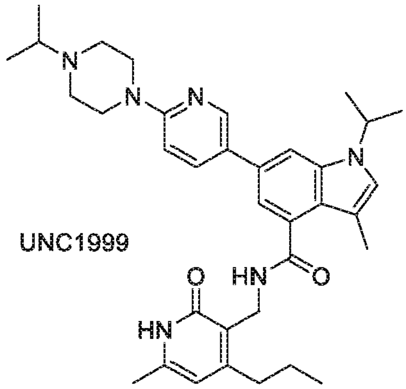

UNC1999

In certain embodiments, the recruiting moiety is rohitukine or a derivative of rohitukine.

In certain embodiments, the recruiting moiety is DB08045 or a derivative of DB08045.

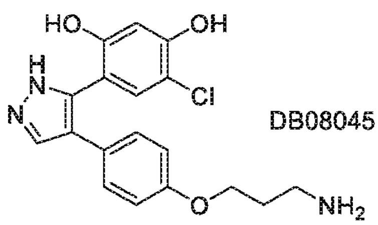

DB08045

In certain embodiments, the recruiting moiety is A-395 or a derivative of A-395.

*Oligomeric Backbone and Linker*

The Oligomeric backbone contains a linker that connects the first terminus and the second terminus and brings the regulatory molecule in proximity to the target gene to modulate gene expression.

The length of the linker depends on the type of regulatory protein and also the target gene. In some embodiments, the linker has a length of less than about 50 Angstroms. In some embodiments, the linker has a length of about 20 to 30 Angstroms.

In some embodiments, the linker comprises between 5 and 50 chain atoms.

In some embodiments, the linker comprises a multimer having 2 to 50 spacing moieties, wherein the spacing moiety is independently selected from the group consisting of $-((CR^{3a}R^{3b})_x-O)_y-$, - $((CR^{3a}R^{3b})_x-NR^{4a})_y-$, $-((CR^{3a}R^{3b})_x-CH=CH-(CR^{3a}R^{3b})_x-O)_y-$, optionally substituted $-C_{1-12}$ alkyl, optionally substituted $C_{2-10}$ alkenyl, optionally substituted $C_{2-10}$ alkynyl, optionally substituted $C_{6-10}$ arylene, optionally substituted $C_{3-7}$ cycloalkylene, optionally substituted 5- to 10-membered heteroarylene, optionally substituted 4- to 10-membered heterocycloalkylene, amino acid residue, -O-, $-C(O)NR^{4a}-$, - $NR^{4a}C(O)-$, -C(O)-, $-NR^1-$,-C(O)O-,-O-, -S-, -S(O)-, $-SO_2-$, $-SO_2NR^{4a}-$, - $NR^{4a}SO_2-$, and -P(O)OH-, and any combinations thereof, wherein

each x is independently 2-4;

each y is independently 1-10;

each $R^{3a}$ and $R^{3b}$ are independently selected from hydrogen, optionally substituted alkyl, optionally substituted

alkenyl, optionally substituted alkynyl, optionally substituted alkoxy, optionally substituted amino, carboxyl, carboxyl ester, acyl, acyloxy, acyl amino, amino acyl, optionally substituted alkylamide, sulfonyl, optionally substituted thioalkoxy, optionally substituted aryl, optionally substituted heteroaryl, optionally substituted cycloalkyl, and optionally substituted heterocyclyl; and

each $R^{4a}$ is independently a hydrogen or an optionally substituted $C_{1-6}$ alkyl.

In some embodiments, the oligomeric backbone comprises $-(T^1-V^1)_a-(T^2-V^2)_b-(T^3-V^3)_c-(T^4-V^4)_d-(T^5-V^5)_e-$,

wherein a, b, c, d and e are each independently 0 or 1, and where the sum of a, b, c, d and e is 1 to 5;

$T^1$, $T^2$, $T^3$, $T^4$ and $T^5$ are each independently selected from an optionally substituted ($C_1$-$C_{12}$)alkylene, optionally substituted alkenylene, optionally substituted alkynylene, $(EA)_w$, $(EDA)_m$, $(PEG)_n$, (modified PEG)$_n$ $(AA)_p$, $-(CR^{2a}OH)_h-$,optionally substituted ($C_6$-$C_{10}$) arylene, optionally substituted $C_{3-7}$ cycloalkylene, optionally substituted 5- to 10 membered heteroarylene, optionally substituted 4- to 10-membered heterocycloalkylene, an acetal group, a disulfide, a hydrazine, a carbohydrate, a beta-lactam, and an ester,

(a) w is an integer from 1 to 20;
(b) m is an integer from 1 to 20;
(c) n is an integer from 1 to 30;
(d) p is an integer from 1 to 20;
(e) h is an integer from 1 to 12;
(f) EA has the following structure

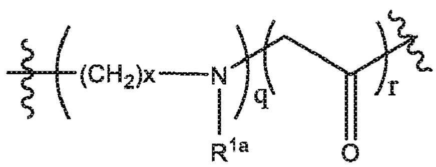

(g) EDA has the following structure:

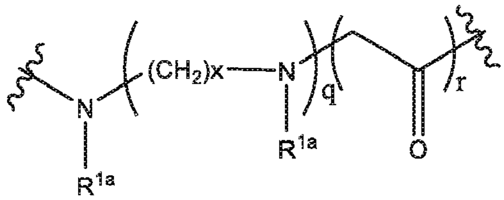

wherein each q is independently an integer from 1 to 6, each x is independently an integer from 1 to 4, and each r is independently 0 or 1;
(h) $(PEG)_n$ has the structure of $-(CR^{2a}R^{2b}-CR^{2a}R^{2b}-O)_n-CR^{2a}R^{2b}-$;-
(i) (modified PEG)$_n$ has the structure of replacing at least one $-(CR^{2a}R^{2b}-CR^{2a}R^{2b}-O)-$ in $(PEG)_n$ with $-(CH_2-CR^{2a}=CR^{2a}-CH_2-O)-$ or $-(CR^{2a}R^{2b}-CR^{2a}R^{2b}-S)-$;
(j) AA is an amino acid residue;
(k) $V^1$, $V^2$, $V^3$, $V^4$ and $V^3$ are each independently selected from the group consisting of a bond, CO-, $-NR^{1a}-$, $-CONR^{1a}-$, $-NR^{1a}CO-$, $-CONR^{1a}C_{1-4}$ alkyl-, $-NR^{1a}CO-C_{1-4}$ alkyl-, -C(O)O-, -OC(O)-, -0-, -S-, - S(O)-, $-SO_2-$, $-SO_2NR^{1a}-$, $-NR^{1a}SO_2-$ and -P(O)OH-;
(l) each $R^{1a}$ is independently hydrogen or and optionally substituted $C_{1-6}$ alkyl; and
(m) each $R^{2a}$ and $R^{2b}$ are independently selected from hydrogen, alkyl, substituted alkyl, alkenyl, substituted alkenyl, alkynyl, substituted alkynyl, halogen, alkoxy, substituted alkoxy, amino, substituted amino, carboxyl, carboxyl ester, acyl, acyloxy, acyl amino, amino acyl, alkylamide, substituted alkylamide, sulfonyl, thioalkoxy, substituted thioalkoxy, aryl, substituted aryl, heteroaryl, substituted heteroaryl, cycloalkyl, substituted cycloalkyl, heterocyclyl, and substituted heterocyclyl.

In some embodiments, the a, b, c, d and e are each independently 0 or 1, where the sum of a, b, c, d and e is 1. In some embodiments, the a, b, c, d and e are each independently 0 or 1, where the sum of a, b, c, d and e is 2. In some embodiments, the a, b, c, d and e are each independently 0 or 1, where the sum of a, b, c, d and e is 3. In some

embodiments, the a, b, c, d and e are each independently 0 or 1, where the sum of a, b, c, d and e is 4. In some embodiments, the a, b, c, d and e are each independently 0 or 1, where the sum of a, b, c, d and e is 5.

In some embodiments, n is 3-9. In some embodiments, n is 4-8. In some embodiments, n is 5 or 6.

In some embodiments, $T^1$, $T^2$, $T^3$, and $T^4$, and $T^5$ are each independently selected from $(C_1\text{-}C_{12})$alkyl, substituted $(C_1\text{-}C_{12})$alkyl, $(EA)_w$, $(EDA)_m$, $(PEG)_n$, (modified PEG)$_n$, $(AA)_p$, $-(CR^{2a}OH)_h-$, phenyl, substituted phenyl, piperidin-4-amino (P4A), para-amino-benzyloxycarbonyl (PABC), meta-amino-benzyloxycarbonyl (MABC), para-amino-benzyloxy (PABO), meta-amino-benzyloxy (MABO), para-aminobenzyl, an acetal group, a disulfide, a hydrazine, a carbohydrate, a beta-lactam, an ester, $(AA)_p$-MABC-$(AA)_p$, $(AA)_p$-MABO-$(AA)_p$, $(AA)_p$-PABO-$(AA)_p$ and $(AA)_p$-PABC-$(AA)_p$, In some embodiments, piperidin-4-amino (P4A) is

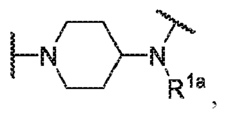

wherein $R^{1a}$ is H or $C_{1-6}$ alkyl.

In some embodiments, $T^1$, $T^2$, $T^3$, $T^4$ and $T^5$ are each independently selected from $(C_1\text{-}C_{12})$alkyl, substituted $(C_1\text{-}C_{12})$alkyl, $(EA)_w$, $(EDA)_m$, $(PEG)_n$, (modified PEG)$_n$, $(AA)_p$,$-(CR^{2a}OH)_h-$, optionally substituted $(C_6\text{-}C_{10})$ arylene, 4-10 membered heterocycloalkene, optionally substituted 5-10 membered heteroarylene. In some embodiments, EA has the following structure:

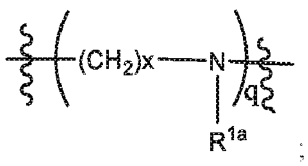

and EDA has the following structure:

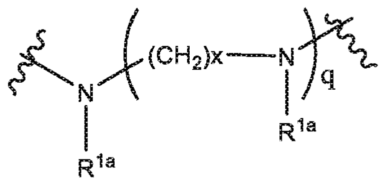

In some embodiments, x is 2-3 and q is 1-3 for EA and EDA. In some embodiments, $R^{1a}$ is H or $C_{1-6}$ alkyl.

In some embodiments, $T^4$ or $T^5$ is an optionally substituted $(C_6\text{-}C_{10})$ arylene.

In some embodiments, $T^4$ or $T^5$ is phenylene or substituted phenylene. In some embodiments, $T^4$ or $T^5$ is phenylene or phenylene substituted with 1-3 substituents selected from $-C_{1-6}$ alkyl, halogen, OH or amine. In some embodiments, $T^4$ or $T^5$ is 5-10 membered heteroarylene or substituted heteroarylene. In some embodiments, $T^4$ or $T^5$ is 4-10 membered heterocylcylene or substituted heterocylcylene. In some embodiments, $T^4$ or $T^5$ is heteroarylene or heterocylcylene optionally substituted with 1-3 substituents selected from $-C_{1-6}$ alkyl, halogen, OH or amine.

In some embodiments, $T^1$, $T^2$, $T^3$, $T^4$ and $T^5$ and $V^1$, $V^2$, $V^3$, $V^4$ and $V^5$ are selected from the following

Table 6:

| $T^1$ | $V^1$ | $T^2$ | $V^2$ | $T^3$ | $V^3$ | $T^4$ | $V^4$ | $T^5$ | $V^5$ |
|---|---|---|---|---|---|---|---|---|---|
| $(C_1-C_{12})$ alkylene | $CONR^{1a}$ | $(EA)_w$ | CO | $(PEG)_n$ | $NR^{11}CO$ | ---- | ---- | ---- | ---- |
| $(C_1-C_{12})$ alkylene | $CONR^{1a}$ | $(EA)_w$ | CO | $(PEG)_n$ | O | arylene | $NR^{11}CO$ | ----- | ----- |
| $(C_1-C_{12})$ alkylene | $CONR^{1a}$ | $(EA)_w$ | CO | $(PEG)_n$ | O | Subst. arylene | $NR^{11}CO$ | ----- | ----- |
| $(C_1-C_{12})$ alkylene | $CONR^{1a}$ | $(EA)_w$ | CO | $(PEG)_n$ | O | $NR^{11}C$ O | $(C_1-C_{12})$ alkyl | Subst. arylene | $NR^{11}CO$ |
| $(C_1-C_{12})$ alkylene | $CONR^{1a}$ | $(EA)_w$ | CO | $(C_1-C_{12})$ alkyl | $NR^{11}CO-C_{1-4}$ alkyl | Subst. arylene | $NR^{11}$ | ---- | ---- |
| $(C_1-C_{12})$ alkylene | $CONR^{1a}$ | $(EA)_w$ | CO | $(PEG)_n$ | O | Subst. arylene | ---- | ---- | ---- |
| $(PEG)_n$ | $CONR^{1a}$- $C_{1-4}$ alkyl | ---- | ---- | ---- | ---- | ---- | ---- | ---- | --- |
| $(EA)_w$ | CO | $(C_1-C_{12})$ alkyl | $CONR^{11}$- $C_{1-4}$ alkyl | ---- | ---- | ---- | ---- | ---- | --- |
| $(C_1-C_{12})$ alkylene | $CONR^{1a}$ | $(EA)_w$ | CO | $(PEG)_n$ | $NR^{11}CO-C_{1-4}$ alkyl | ---- | ---- | ---- | ---- |
| $(EA)_w$ | CO | $(PEG)_n$ | O | phenyl | $NR^{11}CO-C_{1-4}$ alkyl | ---- | ---- | ---- | ---- |
| $(C_1-C_{12})$ alkylene | $CONR^{1a}$ | $(PEG)_n$ | CO | ---- | ---- | ---- | ---- | ---- | ---- |
| $(C_1-C_{12})$ alkylene | $CONR^{1a}$ | $(EA)_w$ | CO | modifd. $(PEG)_n$ | O | arylene | $NR^{11}CO$ | ---- | ---- |

In some embodiments, the linker comprises

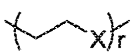 , or 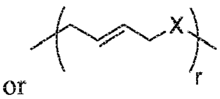 ;

or any combinations thereof, wherein r is an integer between 1 and 10, preferably between 3 and 7; and X is O, S, or $NR^{1a}$. In some embodiments, X is O or $NR^{1a}$. In some embodiments, X is O.

In some embodiments, the linker comprise a

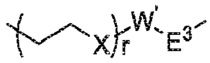 , or 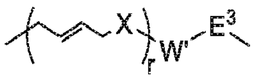 ;

or any combinations thereof, wherein at least one $-(CH_2-CH_2-O)-$ is replaced with $-((CR^{1a}R^{1b})_x-CH=CH-(CR^{1a}R^{1b})_x-O)-$, or any combinations thereof; W' is absent, $(CH_2)_{1-5}$, $-(CH_2)_{1-5}O$, $(CH_2)_{1-5}-C(O)NH-(CH_2)_{1-5}-O$, $(CH_2)_{1-5}-C(O)NH-(CH_2)_{1-5}$, $-(CH_2)_{1-5}NHC(O)-(CH_2)_{1-5}-O$, or $-(CH_2)_{1-5}-NHC(O)-(CH_2)_{1-5}-$; $E^3$ is an optionally substituted $C_{6-10}$ arylene group, optionally substituted 4-10 membered heterocycloalkylene, or optionally substituted 5-10 membered heteroarylene; X is O, S, or NH; each $R^{1a}$ and $R^{1b}$ are independently H or $C_{1-6}$ alkyl; r is an integer between 1 and 10; and x is an integer between 1 and 15. In some embodiments, X is O. In some embodiments, X is NH. In some embodiments, $E^3$ is a $C_{6-10}$ arylene group optionally substituted with 1-3 substituents selected from $-C_{1-6}$ alkyl, halogen, OH or amine.

In some embodiments, $E^3$ is a phenylene or substituted phenylene.

In some embodiments, the linker comprise a

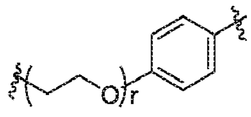 ; 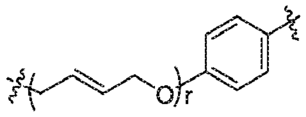 ;

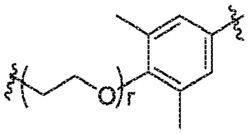 or 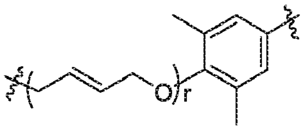 .

In some embodiments, the linker comprises $-X(CH_2)_m(CH_2CH_2O)_n-$, wherein X is -O-, -NH-, or - S-, wherein m is 0 or greater and n is at least 1.

In some embodiments, the linker comprises

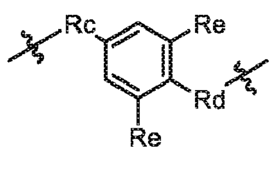

following the second terminus, wherein $R_c$ is selected from a bond, $-N(R^{1a})-$, -O-, and -S-; $R_d$ is selected from $-N(R^{1a})-$, -O-, and -S-; and $R_e$ is independently selected from hydrogen and optionally substituted $C_{1-6}$ alkyl

In some embodiments, the linker comprises one or more structures selected from

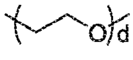 ,

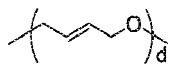
,

$-C_{1-12}$ alkyl, arylene, cycloalkylene, heteroarylene, heterocycloalkylene, -O-, $-C(O)NR^{1a}$-,-C(O)-, $-NR^{1a}$-, $-(CH_2CH_2CH_2O)_y$-, and $-(CH_2CH_2CH_2NR^{1a})_y$- ,wherein each d and y are independently 1-10, andeach $R^{1a}$ is independently hydrogen or $C_{1-6}$ alkyl. In some embodiments, d is 4-8.

In some embodiments, the linker comprises

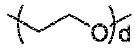

and each d is independently 3-7. In some embodiments, d is 4-6.

In some embodiments, the linker comprises $N(R^{1a})(CH_2)_xN(R^{1b})(CH_2)_xN$-, wherein $R^{1a}$ and$R^{1b}$ are each independently selected from hydrogen or optionally substituted $C_1$-$C_6$ alkyl; and each x is independently an integer in the range of 1-6..

In some embodiments, the linker comprises the linker comprises $-(CH_2$ $-C(O)N(R'')-(CH_2)_q-N(R')-(CH_2)_q-N(R'')C(O)-(CH_2)_x-C(O)N(R'')-A-$, $-(CH_2)_x-C(O)N(R'')-(CH_2$ $CH_2O)_y(CH_2)_x-C(O)N(R'')-A-$, - $C(O)N(R'')-(CH_2)_q-N(R')-(CH_2)_q-N(R'')C(O)-(CH_2)_x-A-$, $-(CH_2)_x-O-(CH_2$ $CH_2O)_y-(CH_2)_x-N(R'')C(O)-(CH_2)_x-A-$, or $-N(R'')C(O)-(CH_2)-C(O)N(R'')-(CH_2)_x-O(CH_2CH_2O)_y(CH_2)_x-A-$; wherein R' is methyl; R" is hydrogen; each x and y are independently an integer from 1 to 10; each q is independently an integer from 2 to 10; and each A is independently selected from a bond, an optionally substituted $C_{1-12}$ alkyl, an optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene.

In some embodiments, the linker is joined with the first terminus with a group selected from-CO-, $-NR^{1a}$-,$-CONR^{1a}$-, $-NR^{1a}CO$-, $-CONR^{1a}C_{1-4}$alkyl-, $-NR^{1a}CO-C_{1-4}$alkyl -, -C(O)O-, -OC(O)-, -O-, —S—, -S(O)-, $-SO_2$-, $-SO_2NR^{1a}$-, $-NR^1SO_2$-, -P(O)OH-,$-((CH_2)_x-O)$-, $-((CH_2)_y-NR^{1a})$-, optionally substituted $-C_{1-12}$ alkylene, optionally substituted $C_{2-10}$ alkenylene, optionally substituted $C_{2-10}$ alkynylene, optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene, wherein each x is independently 1-4, each y is independently 1-4, and each $R^{1a}$ is independently a hydrogen or optionally substituted $C_{1-6}$ alkyl.

In some embodiments, the linker is joined with the first terminus with a group selected from-CO-, $-NR^{1a}$-, $C_{1-12}$ alkyl, $-CONR^{1a}$-, and $-NR^{1a}CO$-.

In some embodiments, the linker is joined with second terminus with a group selected from —CO—, $-NR^{1a}$-,$-CONR^{1a}$-, $-NR^{1a}CO$-, $-CONR^{1a}C_{1-4}$alkyl-, $-NR^{1a}CO-C_{1-4}$alkyl -, -C(O)O-,-OC(O)-, -O-, -S-, -S(O)-, $-SO_2$-, $-SO_2NR^{1a}$-, $-NR^1SO_2$-, -P(O)OH-,$-((CH_2)_x-O)$-, $-((CH_2)_y-NR^{1a})$-, optionally substituted $-C_{1-12}$ alkylene, optionally substituted $C_{2-10}$ alkenylene, optionally substituted $C_{2-10}$ alkynylene, optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene, wherein each x is independently 1-4, each y is independently 1-4, and each $R^{1a}$ is independently a hydrogen or optionally substituted $C_{1-6}$ alkyl.

In some embodiments, the linker is joined with second terminus with a group selected from —CO—, $-NR^{1a}$-, $-CONR^{1a}$-, $-NR^{1a}CO$-,$-((CH_2)_x-O)$-, $-((CH_2)_y-NR^{1a})$-, -O-, optionally substituted $-C_{1-12}$ alkyl, optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene, wherein each x is independently 1-4, each y is independently 1-4, and each R' is independently a hydrogen or optionally substituted $C_{1-6}$ alkyl.

*Cell-penetrating ligand*

In certain embodiments, the compounds comprise a cell-penetrating ligand moiety.

In certain embodiments, the cell-penetrating ligand moiety is a polypeptide.

In certain embodiments, the cell-penetrating ligand moiety is a polypeptide containing fewer than 30 amino acid residues.

In certain embodiments, the polypeptide is chosen from any one of SEQ ID NO. 1 to SEQ ID NO. 37, inclusive.

In some embodiments, the second terminus does not comprise a structure of Formula (C-1 1):

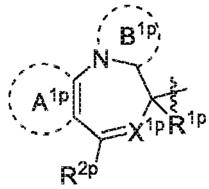

(C-11),

wherein:

each of $A^{1p}$ and $B^{1p}$ is independently an optionally substituted aryl or heteroaryl ring;
$X^{1p}$ is CH or N;
$R^{1p}$ is hydrogen, halogen, or an optionally substituted $C_{1-6}$ alkyl group; and
$R^{2p}$ is an optionally substituted $C_{1-6}$ alkyl, cycloalkyl, $C_{6-10}$ aryl, or heteroaryl.

In some ebmbodiments, the protein binding moiety does not have the structure of Formula (C-12):

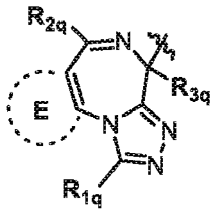

(C-12),

wherein:

$R_{1q}$ is a hydrogen or an optionally substituted alkyl, hydroxyalkyl, aminoalkyl, alkoxyalkyl, halogenated alkyl, hydroxyl, alkoxy, or $-COOR_{4q}$;
$R_{4q}$ is hydrogen, or an optionally substituted aryl, aralkyl, cycloalkyl, heteroaryl, heteroaralkyl, heterocycloalkyl, alkyl, alkenyl, alkynyl, or cycloalkylalkyl group, optionally containing one or more heteroatoms;
$R_{2q}$ is an optionally substituted aryl, alkyl, cycloalkyl, or aralkyl group;
$R_{3q}$ is hydrogen, halogen, or an optionally substituted alkyl group, preferably $(CH_2)_x$-C(O)N($R_{20}$)($R_{21}$), or $(CH_2)_x$-N($R_{20}$)-C(O)$R_{21}$; or halogenated alkyl group;
wherein x is an integer from 1 to 10; and $R_{20}$ and $R_{21}$ are each independently hydrogen or $C_1$-$C_6$ alkyl group, preferably $R_{20}$ is hydrogen and $R_{21}$ ismethyl; and
Ring E is an optionally substituted aryl or heteroaryl group.

Also provided are embodiments wherein any compound disclosed above, including compounds of Formulas I - VIII, are singly, partially, or fully deuterated. Methods for accomplishing deuterium exchange for hydrogen are known in the art.

Also provided are embodiments wherein any embodiment above may be combined with any one or more of these embodiments, provided the combination is not mutually exclusive.

As used herein, two embodiments are "mutually exclusive" when one is defined to be something which is different than the other. For example, an embodiment wherein two groups combine to form a cycloalkyl is mutually exclusive with an embodiment in which one group is ethyl the other group is hydrogen. Similarly, an embodiment wherein one group is $CH_2$ is mutually exclusive with an embodiment wherein the same group is NH.

## Method of Treatment

The present disclosure also relates to a method of modulating the transcription of *c9orf72* comprising the step of contacting *c9orf72* with a compound as described herein. The cell phenotype, cell proliferation, transcription of *c9orf72,* production of mRNA from transcription of *c9orf72,* translation of *c9orf72*, change in biochemical output produced by the protein coded by *c9orf72,* or noncovalent binding of the protein coded by *c9orf72* with a natural binding partner may be monitored. Such methods may be modes of treatment of disease, biological assays, cellular assays, biochemical assays, or the like.

Also provided herein is a method of treatment of a disease mediated by transcription of *c9orf72* comprising the

administration of a therapeutically effective amount of a compound as disclosed herein, or a salt thereof, to a patient in need thereof.

In certain embodiments, the disease is chosen from ALS, FTD, and ALS-FTD.

Also provided herein is a compound as disclosed herein for use as a medicament.

Also provided herein is a compound as disclosed herein for use as a medicament for the treatment of a disease mediated by transcription of *c9orf72.*

Also provided is the use of a compound as disclosed herein as a medicament.

Also provided is the use of a compound as disclosed herein as a medicament for the treatment of a disease mediated by transcription of *c9orf72.*

Also provided is a compound as disclosed herein for use in the manufacture of a medicament for the treatment of a disease mediated by transcription of *c9orf72.*

Also provided is the use of a compound as disclosed herein for the treatment of a disease mediated by transcription of *c9orf72.*

Also provided herein is a method of modulation of transcription of *c9orf72* comprising contacting *c9orf72* with a compound as disclosed herein, or a salt thereof.

Also provided herein is a method for achieving an effect in a patient comprising the administration of a therapeutically effective amount of a compound as disclosed herein, or a salt thereof, to a patient, wherein the effect is chosen from muscular atrophy, ataxia, fasciculations, and dementia.

Certain compounds of the present disclosure may be effective for treatment of subjects whose genotype has 5 or more repeats of GGGGCC. Certain compounds of the present disclosure may be effective for treatment of subjects whose genotype has 10 or more repeats of GGGGCC. Certain compounds of the present disclosure may be effective for treatment of subjects whose genotype has 20 or more repeats of GGGGCC. Certain compounds of the present disclosure may be effective for treatment of subjects whose genotype has 50 or more repeats of GGGGCC. Certain compounds of the present disclosure may be effective for treatment of subjects whose genotype has 100 or more repeats of GGGGCC. Certain compounds of the present disclosure may be effective for treatment of subjects whose genotype has 200 or more repeats of GGGGCC. Certain compounds of the present disclosure may be effective for treatment of subjects whose genotype has 500 or more repeats of GGGGCC.

Also provided is a method of modulation of a *c9orf72*-mediated function in a subject comprising the administration of a therapeutically effective amount of a compound as disclosed herein.

Also provided is a pharmaceutical composition comprising a compound as disclosed herein, together with a pharmaceutically acceptable carrier.

In certain embodiments, the pharmaceutical composition is formulated for oral administration.

In certain embodiments, the pharmaceutical composition is formulated for intravenous injection and/or infusion.

In certain embodiments, the oral pharmaceutical composition is chosen from a tablet and a capsule.

In certain embodiments, *ex vivo* methods of treatment are provided. *Ex vivo* methods typically include cells, organs, and/or tissues removed from the subject. The cells, organs and/or tissues can, for example, be incubated with the agent under appropriate conditions. The contacted cells, organs, and/or tissues are typically returned to the donor, placed in a recipient, or stored for future use. Thus, the compound is generally in a pharmaceutically acceptable carrier.

In certain embodiments, administration of the pharmaceutical composition modulates expression of *c9orf72* within 6 hours of treatment. In certain embodiments, administration of the pharmaceutical composition modulates expression of *c9orf72* within 24 hours of treatment. In certain embodiments, administration of the pharmaceutical composition modulates expression of *c9orf72* within 72 hours of treatment.

In certain embodiments, administration of the pharmaceutical composition causes a 2-fold increase in expression of *c9orf72.* In certain embodiments, administration of the pharmaceutical composition causes a 5-fold increase in expression of *c9orf72.* In certain embodiments, administration of the pharmaceutical composition causes a 10-fold increase in expression of *c9orf72.* In certain embodiments, administration of the pharmaceutical composition causes a 20-fold increase in expression of *c9orf72.*

In certain embodiments, administration of the pharmaceutical composition causes a 20 % decrease in expression of *c9orf72.* In certain embodiments, administration of the pharmaceutical composition causes a 50 % decrease in expression of *c9orf72.* In certain embodiments, administration of the pharmaceutical composition causes a 80 % decrease in expression of *c9orf72.* In certain embodiments, administration of the pharmaceutical composition causes a 90 % decrease in expression of *c9orf72*. In certain embodiments, administration of the pharmaceutical composition causes a 95 % decrease in expression of *c9orf72*. In certain embodiments, administration of the pharmaceutical composition causes a 99 % decrease in expression of *c9orf72.*

In certain embodiments, administration of the pharmaceutical composition causes expression of *c9orf72* to fall within 25 % of the level of expression observed for healthy individuals. In certain embodiments, administration of the pharmaceutical composition causes expression of *c9orf72* to fall within 50 % of the level of expression observed for healthy individuals. In certain embodiments, administration of the pharmaceutical composition causes expression of

*c9orf72* to fall within 75 % of the level of expression observed for healthy individuals. In certain embodiments, administration of the pharmaceutical composition causes expression of *c9orf72* to fall within 90 % of the level of expression observed for healthy individuals.

Pharmaceutical Composition and Administration

Also provided is a method of modulation of a *c9orf72*-mediated function in a subject comprising the administration of a therapeutically effective amount of a compound as disclosed herein.

Also provided is a pharmaceutical composition comprising a compound as disclosed herein, together with a pharmaceutically acceptable carrier.

In certain embodiments, the pharmaceutical composition is formulated for oral administration.

In certain embodiments, the pharmaceutical composition is formulated for intravenous injection or infusion.

In certain embodiments, the oral pharmaceutical composition is chosen from a tablet and a capsule.

In certain embodiments, *ex vivo* methods of treatment are provided. *Ex vivo* methods typically include cells, organs, or tissues removed from the subject. The cells, organs or tissues can, for example, be incubated with the agent under appropriate conditions. The contacted cells, organs, or tissues are typically returned to the donor, placed in a recipient, or stored for future use. Thus, the compound is generally in a pharmaceutically acceptable carrier.

In certain embodiments, the compound is effective at a concentration less than about 5 μM. In certain embodiments, the compound is effective at a concentration less than about 1 μM. In certain embodiments, the compound is effective at a concentration less than about 400 nM. In certain embodiments, the compound is effective at a concentration less than about 200 nM. In certain embodiments, the compound is effective at a concentration less than about 100 nM. In certain embodiments, the compound is effective at a concentration less than about 50 nM. In certain embodiments, the compound is effective at a concentration less than about 20 nM. In certain embodiments, the compound is effective at a concentration less than about 10 nM.

*Abbreviations and Definitions*

As used herein, the terms below have the meanings indicated.

It is to be understood that certain radical naming conventions can include either a mono-radical or a di-radical, depending on the context. For example, where a substituent requires two points of attachment to the rest of the molecule, it is understood that the substituent is a di-radical. For example, a substituent identified as alkyl that requires two points of attachment includes di-radicals such as $-CH_2-$, $-CH_2CH_2-$, $-CH_2CH(CH_3)CH_2-$, and the like. Other radical naming conventions clearly indicate that the radical is a di-radical such as "alkylene," "alkenylene," "arylene", "heteroarylene."

When two R groups are said to form a ring (e.g., a carbocyclyl, heterocyclyl, aryl, or heteroaryl ring) "together with the atom to which they are attached," it is meant that the collective unit of the atom and the two R groups are the recited ring. The ring is not otherwise limited by the definition of each R group when taken individually. For example, when the following substructure is present:

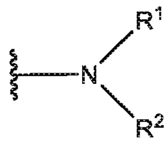

and $R^1$ and $R^2$ are defined as selected from the group consisting of hydrogen and alkyl, or $R^1$ and $R^2$ together with the nitrogen to which they are attached form a heterocyclyl, it is meant that $R^1$ and $R^2$ can be selected from hydrogen or alkyl, or alternatively, the substructure has structure:

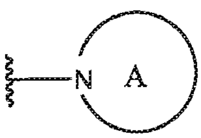

where ring A is a heteroaryl ring containing the depicted nitrogen.

Similarly, when two "adjacent" R groups are said to form a ring "together with the atom to which they are attached," it is meant that the collective unit of the atoms, intervening bonds, and the two R groups are the recited ring. For example, when the following substructure is present:

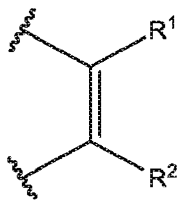

and $R^1$ and $R^2$ are defined as selected from the group consisting of hydrogen and alkyl, or $R^1$ and $R^2$ together with the atoms to which they are attached form an aryl or carbocylyl, it is meant that $R^1$ and $R^2$ can be selected from hydrogen or alkyl, or alternatively, the substructure has structure:

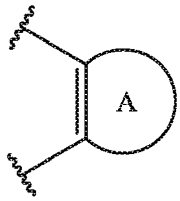

where A is an aryl ring or a carbocylyl containing the depicted double bond.

Wherever a substituent is depicted as a di-radical (*i.e.*, has two points of attachment to the rest of the molecule), it is to be understood that the substituent can be attached in any directional configuration unless otherwise indicated. Thus, for example, a substituent depicted as -AE- or

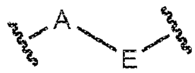

includes the substituent being oriented such that the **A** is attached at the leftmost attachment point of the molecule as well as the case in which **A** is attached at the rightmost attachment point of the molecule.

When ranges of values are disclosed, and the notation "from $n_1$ ... to $n_2$" or "between $n_1$ ... and $n_2$" is used, where $n_1$ and $n_2$ are the numbers, then unless otherwise specified, this notation is intended to include the numbers themselves and the range between them. This range may be integral or continuous between and including the end values. By way of example, the range "from 2 to 6 carbons" is intended to include two, three, four, five, and six carbons, since carbons come in integer units. Compare, by way of example, the range "from 1 to 3 μM (micromolar)," which is intended to include 1 μM, 3 μM, and everything in between to any number of significant figures (e.g., 1.255 μM, 2.1 μM, 2.9999 μM, etc.).

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

The term "polyamide" refers to polymers of linkable units chemically bound by amide (i.e., CONH) linkages; optionally, polyamides include chemical probes conjugated therewith. Polyamides may be synthesized by stepwise condensation of carboxylic acids (COOH) with amines (RR'NH) using methods known in the art. Alternatively, polyamides may be formed using enzymatic reactions in vitro, or by employing fermentation with microorganisms.

The term "linkable unit" refers to methylimidazoles, methylpyrroles, and straight and branched chain aliphatic functionalities (e.g., methylene, ethylene, propylene, butylene, and the like) which optionally contain nitrogen Substituents, and chemical derivatives thereof. The aliphatic functionalities of linkable units can be provided, for example, by condensation of B-alanine or dimethylaminopropylaamine during synthesis of the polyamide by methods well known in the art.

The term "linker" refers to a chain of at least 10 contiguous atoms. In certain embodiments, the linker contains no more than 20 non-hydrogen atoms. In certain embodiments, the linker contains no more than 40 non-hydrogen atoms. In certain embodiments, the linker contains no more than 60 non-hydrogen atoms. In certain embodiments, the linker contains atoms chosen from C, H, N, O, and S. In certain embodiments, every non-hydrogen atom is chemically bonded either to 2 neighboring atoms in the linker, or one neighboring atom in the linker and a terminus of the linker. In certain embodiments, the linker forms an amide bond with at least one of the two other groups to which it is attached. In certain

embodiments, the linker forms an ester or ether bond with at least one of the two other groups to which it is attached. In certain embodiments, the linker forms a thiolester or thioether bond with at least one of the two other groups to which it is attached. In certain embodiments, the linker forms a direct carbon-carbon bond with at least one of the two other groups to which it is attached. In certain embodiments, the linker forms an amine or amide bond with at least one of the two other groups to which it is attached. In certain embodiments, the linker comprises -($CH_2OCH_2$)- units. In certain embodiments, the linker comprises -($CH(CH_3)OCH_2$)- units. In certain embodiments, the linker comprises -($CH_2NR_NCH_2$) units, for $R_N$ = $C_{1-4}$alkyl. In certain embodiments, the linker comprises an arylene, cycloalkylene, or heterocycloalkylene moiety.

The term "spacer" refers to a chain of at least 5 contiguous atoms. In certain embodiments, the spacer contains no more than 10 non-hydrogen atoms. In certain embodiments, the spacer contains atoms chosen from C, H, N, O, and S. In certain embodiments, the spacer forms amide bonds with the two other groups to which it is attached. In certain embodiments, the spacer comprises -($CH_2OCH_2$)- units. In certain embodiments, the spacer comprises -($CH_2NR_NCH_2$)- units, for $R_N$ = $C_{1-4}$alkyl. In certain embodiments, the spacer contains at least one positive charge at physiological pH.

The term "turn component" refers to a chain of about 4 to 10 contiguous atoms. In certain embodiments, the turn component contains atoms chosen from C, H, N, O, and S. In certain embodiments, the turn component forms amide bonds with the two other groups to which it is attached. In certain embodiments, the turn component contains at least one positive charge at physiological pH.

The terms "nucleic acid and "nucleotide" refer to ribonucleotide and deoxyribonucleotide, and analogs thereof, well known in the art.

The term "oligonucleotide sequence" refers to a plurality of nucleic acids having a defined sequence and length (e.g., 2, 3, 4, 5, 6, or even more nucleotides). The term "oligonucleotide repeat sequence" refers to a contiguous expansion of oligonucleotide sequences.

The term "transcription," well known in the art, refers to the synthesis of RNA (i.e., ribonucleic acid) by DNA-directed RNA polymerase. The term "modulate transcription" refers to a change in transcriptional level which can be measured by methods well known in the art, for example, assay of mRNA, the product of transcription. In certain embodiments, modulation is an increase in transcription. In other embodiments, modulation is a decrease in transcription

The term "acyl," as used herein, alone or in combination, refers to a carbonyl attached to an alkenyl, alkyl, aryl, cycloalkyl, heteroaryl, heterocycle, or any other moiety were the atom attached to the carbonyl is carbon. An "acetyl" group refers to a -$C(O)CH_3$ group. An "alkylcarbonyl" or "alkanoyl" group refers to an alkyl group attached to the parent molecular moiety through a carbonyl group. Examples of such groups include methylcarbonyl and ethylcarbonyl. Examples of acyl groups include formyl, alkanoyl and aroyl.

The term "alkenyl," as used herein, alone or in combination, refers to a straight-chain or branched-chain hydrocarbon radical having one or more double bonds and containing from 2 to 20 carbon atoms. In certain embodiments, said alkenyl will comprise from 2 to 6 carbon atoms. The term "alkenylene" refers to a carbon-carbon double bond system attached at two or more positions such as ethenylene [(-CH=CH-),(-C::C-)]. Examples of suitable alkenyl radicals include ethenyl, propenyl, 2-methylpropenyl, 1,4-butadienyl and the like. Unless otherwise specified, the term "alkenyl" may include "alkenylene" groups.

The term "alkoxy," as used herein, alone or in combination, refers to an alkyl ether radical, wherein the term alkyl is as defined below. Examples of suitable alkyl ether radicals include methoxy, ethoxy, n-propoxy, isopropoxy, n-butoxy, iso-butoxy, sec-butoxy, tert-butoxy, and the like.

The term "alkyl," as used herein, alone or in combination, refers to a straight-chain or branched-chain alkyl radical containing from 1 to 20 carbon atoms. In certain embodiments, said alkyl will comprise from 1 to 10 carbon atoms. In further embodiments, said alkyl will comprise from 1 to 8 carbon atoms. Alkyl groups may be optionally substituted as defined herein. Examples of alkyl radicals include methyl, ethyl, n-propyl, isopropyl, n-butyl, isobutyl, sec-butyl, tert-butyl, pentyl, iso-amyl, hexyl, octyl, noyl and the like. The term "alkylene," as used herein, alone or in combination, refers to a saturated aliphatic group derived from a straight or branched chain saturated hydrocarbon attached at two or more positions, such as methylene
(-$CH_2$-). Unless otherwise specified, the term "alkyl" may include "alkylene" groups.

The term "alkylamino," as used herein, alone or in combination, refers to an alkyl group attached to the parent molecular moiety through an amino group. Suitable alkylamino groups may be mono- or dialkylated, forming groups such as, for example, N-methylamino, N-ethylamino, N,N-dimethylamino, N,N-ethylmethylamino and the like.

The term "alkylidene," as used herein, alone or in combination, refers to an alkenyl group in which one carbon atom of the carbon-carbon double bond belongs to the moiety to which the alkenyl group is attached.

The term "alkylthio," as used herein, alone or in combination, refers to an alkyl thioether (R-S-) radical wherein the term alkyl is as defined above and wherein the sulfur may be singly or doubly oxidized. Examples of suitable alkyl thioether radicals include methylthio, ethylthio, n-propylthio, isopropylthio, n-butylthio, iso-butylthio, sec-butylthio, tert-butylthio, methanesulfonyl, ethanesulfinyl, and the like.

The term "alkynyl," as used herein, alone or in combination, refers to a straight-chain or branched chain hydrocarbon radical having one or more triple bonds and containing from 2 to 20 carbon atoms. In certain embodiments, said alkynyl comprises from 2 to 6 carbon atoms. In further embodiments, said alkynyl comprises from 2 to 4 carbon atoms. The term "alkynylene" refers to a carbon-carbon triple bond attached at two positions such as ethynylene (-C:::C-, -C≡C-). Examples of alkynyl radicals include ethynyl, propynyl, hydroxypropynyl, butyn-1-yl, butyn-2-yl, pentyn-1-yl, 3-methylbutyn-1-yl, hexyn-2-yl, and the like. Unless otherwise specified, the term "alkynyl" may include "alkynylene" groups.

The terms "amido" and "carbamoyl,"as used herein, alone or in combination, refer to an amino group as described below attached to the parent molecular moiety through a carbonyl group, or vice versa. The term "C-amido" as used herein, alone or in combination, refers to a -C(O)N(RR') group with R and R' as defined herein or as defined by the specifically enumerated "R" groups designated. The term "N-amido" as used herein, alone or in combination, refers to a RC(O)N(R')- group, with R and R' as defined herein or as defined by the specifically enumerated "R" groups designated. The term "acylamino" as used herein, alone or in combination, embraces an acyl group attached to the parent moiety through an amino group. An example of an "acylamino" group is acetylamino ($CH_3C(O)NH$-).

The term "amide," as used herein, alone in combination, refers to -C(O)NRR', wherein R and R' are independently chosen from hydrogen, alkyl, acyl, heteroalkyl, aryl, cycloalkyl, heteroaryl, and heterocycloalkyl, any of which may themselves be optionally substituted. Additionally, R and R' may combine to form heterocycloalkyl, either of which may be optionally substituted. Amides may be formed by direct condensation of carboxylic acids with amines, or by using acid chlorides. In addition, coupling reagents are known in the art, including carbodiimide-based compounds such as DCC and EDCI.

The term "amino," as used herein, alone or in combination, refers to -NRR', wherein R and R' are independently chosen from hydrogen, alkyl, acyl, heteroalkyl, aryl, cycloalkyl, heteroaryl, and heterocycloalkyl, any of which may themselves be optionally substituted. Additionally, R and R' may combine to form heterocycloalkyl, either of which may be optionally substituted.

The term "aryl," as used herein, alone or in combination, means a carbocyclic aromatic system containing one, two or three rings wherein such polycyclic ring systems are fused together. The term "aryl" embraces aromatic groups such as phenyl, naphthyl, anthracenyl, and phenanthryl. The term "arylene" embraces aromatic groups such as phenylene, naphthylene, anthracenylene, and phenanthrylene.

The term "arylalkenyl" or "aralkenyl," as used herein, alone or in combination, refers to an aryl group attached to the parent molecular moiety through an alkenyl group.

The term "arylalkoxy" or "aralkoxy," as used herein, alone or in combination, refers to an aryl group attached to the parent molecular moiety through an alkoxy group.

The term "arylalkyl" or "aralkyl," as used herein, alone or in combination, refers to an aryl group attached to the parent molecular moiety through an alkyl group.

The term "arylalkynyl" or "aralkynyl," as used herein, alone or in combination, refers to an aryl group attached to the parent molecular moiety through an alkynyl group.

The term "arylalkanoyl" or "aralkanoyl" or "aroyl,"as used herein, alone or in combination, refers to an acyl radical derived from an aryl-substituted alkanecarboxylic acid such as benzoyl, napthoyl, phenylacetyl, 3-phenylpropionyl (hydrocinnamoyl), 4-phenylbutyryl, (2-naphthyl)acetyl, 4-chlorohydrocinnamoyl, and the like.

The term aryloxy as used herein, alone or in combination, refers to an aryl group attached to the parent molecular moiety through an oxy.

The terms "benzo" and "benz," as used herein, alone or in combination, refer to the divalent radical $C_6H_4$= derived from benzene. Examples include benzothiophene and benzimidazole.

The term "carbamate," as used herein, alone or in combination, refers to an ester of carbamic acid (-NHCOO-) which may be attached to the parent molecular moiety from either the nitrogen or acid end, and which may be optionally substituted as defined herein.

The term "O-carbamyl" as used herein, alone or in combination, refers to a -OC(O)NRR', group-with R and R' as defined herein.

The term "N-carbamyl" as used herein, alone or in combination, refers to a ROC(O)NR'- group, with R and R' as defined herein.

The term "carbonyl," as used herein, when alone includes formyl [-C(O)H] and in combination is a - C(O)- group.

The term "carboxyl" or "carboxy," as used herein, refers to -C(O)OH or the corresponding "carboxylate" anion, such as is in a carboxylic acid salt. An "O-carboxy" group refers to a RC(O)O- group, where R is as defined herein. A "C-carboxy" group refers to a -C(O)OR groups where R is as defined herein.

The term "cyano," as used herein, alone or in combination, refers to -CN.

The term "cycloalkyl," or, alternatively, "carbocycle," as used herein, alone or in combination, refers to a saturated or partially saturated monocyclic, bicyclic or tricyclic alkyl group wherein each cyclic moiety contains from 3 to 12 carbon atom ring members and which may optionally be a benzo fused ring system which is optionally substituted as defined herein. In certain embodiments, said cycloalkyl will comprise from 5 to 7 carbon atoms. Examples of such cycloalkyl groups include cyclopropyl, cyclobutyl, cyclopentyl, cyclohexyl, cycloheptyl, tetrahydronapthyl, indanyl, octahydronaphthyl, 2,3-dihydro-1H-indenyl, adamantyl and the like. "Bicyclic" and "tricyclic" as used herein are intended to include both fused ring systems, such as decahydronaphthalene, octahydronaphthalene as well as the multicyclic (multicentered) saturated or partially unsaturated type. The latter type of isomer is exemplified in general by, bicyclo[1,1,1]pentane, camphor, adamantane, and bicyclo[3,2,1]octane.

The term "ester," as used herein, alone or in combination, refers to a carboxy group bridging two moieties linked at carbon atoms.

The term "ether," as used herein, alone or in combination, refers to an oxy group bridging two moieties linked at carbon atoms.

The term "halo," or "halogen," as used herein, alone or in combination, refers to fluorine, chlorine, bromine, or iodine.

The term "haloalkoxy," as used herein, alone or in combination, refers to a haloalkyl group attached to the parent molecular moiety through an oxygen atom.

The term "haloalkyl," as used herein, alone or in combination, refers to an alkyl radical having the meaning as defined above wherein one or more hydrogens are replaced with a halogen. Specifically embraced are monohaloalkyl, dihaloalkyl and polyhaloalkyl radicals. A monohaloalkyl radical, for one example, may have an iodo, bromo, chloro or fluoro atom within the radical. Dihalo and polyhaloalkyl radicals may have two or more of the same halo atoms or a combination of different halo radicals. Examples of haloalkyl radicals include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. "Haloalkylene" refers to a haloalkyl group attached at two or more positions. Examples include fluoromethylene (-CFH-), difluoromethylene ($-CF_2-$), chloromethylene (-CHCl-) and the like.

The term "heteroalkyl," as used herein, alone or in combination, refers to a stable straight or branched chain, or combinations thereof, fully saturated or containing from 1 to 3 degrees of unsaturation, consisting of the stated number of carbon atoms and from one to three heteroatoms chosen from N, O, and S, and wherein the N and S atoms may optionally be oxidized and the N heteroatom may optionally be quaternized. The heteroatom(s) may be placed at any interior position of the heteroalkyl group. Up to two heteroatoms may be consecutive, such as, for example, $-CH_2-NH-OCH_3$.

The term "heteroaryl," as used herein, alone or in combination, refers to a 3 to 15 membered unsaturated heteromonocyclic ring, or a fused monocyclic, bicyclic, or tricyclic ring system in which at least one of the fused rings is aromatic, which contains at least one atom chosen from N, O, and S. In certain embodiments, said heteroaryl will comprise from 1 to 4 heteroatoms as ring members. In further embodiments, said heteroaryl will comprise from 1 to 2 heteroatoms as ring members. In certain embodiments, said heteroaryl will comprise from 5 to 7 atoms. The term also embraces fused polycyclic groups wherein heterocyclic rings are fused with aryl rings, wherein heteroaryl rings are fused with other heteroaryl rings, wherein heteroaryl rings are fused with heterocycloalkyl rings, or wherein heteroaryl rings are fused with cycloalkyl rings. Examples of heteroaryl groups include pyrrolyl, pyrrolinyl, imidazolyl, pyrazolyl, pyridyl, pyrimidinyl, pyrazinyl, pyridazinyl, triazolyl, pyranyl, furyl, thienyl, oxazolyl, isoxazolyl, oxadiazolyl, thiazolyl, thiadiazolyl, isothiazolyl, indolyl, isoindolyl, indolizinyl, benzimidazolyl, quinolyl, isoquinolyl, quinoxalinyl, quinazolinyl, indazolyl, benzotriazolyl, benzodioxolyl, benzopyranyl, benzoxazolyl, benzoxadiazolyl, benzothiazolyl, benzothiadiazolyl, benzofuryl, benzothienyl, chromonyl, coumarinyl, benzopyranyl, tetrahydroquinolinyl, tetrazolopyridazinyl, tetrahydroisoquinolinyl, thienopyridinyl, furopyridinyl, pyrrolopyridinyl and the like. Exemplary tricyclic heterocyclic groups include carbazolyl, benzidolyl, phenanthrolinyl, dibenzofuranyl, acridinyl, phenanthridinyl, xanthenyl and the like.

The terms "heterocycloalkyl" and, interchangeably, "heterocycle," as used herein, alone or in combination, each refer to a saturated, partially unsaturated, or fully unsaturated (but nonaromatic) monocyclic, bicyclic, or tricyclic heterocyclic group containing at least one heteroatom as a ring member, wherein each said heteroatom may be independently chosen from nitrogen, oxygen, and sulfur. In certain embodiments, said hetercycloalkyl will comprise from 1 to 4 heteroatoms as ring members. In further embodiments, said hetercycloalkyl will comprise from 1 to 2 heteroatoms as ring members. In certain embodiments, said hetercycloalkyl will comprise from 3 to 8 ring members in each ring. In further embodiments, said hetercycloalkyl will comprise from 3 to 7 ring members in each ring. In yet further embodiments, said hetercycloalkyl will comprise from 5 to 6 ring members in each ring. "Heterocycloalkyl" and "heterocycle" are intended to include sulfones, sulfoxides, N-oxides of tertiary nitrogen ring members, and carbocyclic fused and benzo fused ring systems; additionally, both terms also include systems where a heterocycle ring is fused to an aryl group, as defined herein, or an additional heterocycle group. Examples of heterocycle groups include tetrhydroisoquinoline, aziridinyl, azetidinyl, 1,3-benzodioxolyl, dihydroisoindolyl, dihydroisoquinolinyl, dihydrocinnolinyl, dihydrobenzodioxinyl, dihydro[1,3]oxazolo[4,5-b]pyridinyl, benzothiazolyl, dihydroindolyl, dihy-dropyridinyl, 1,3-dioxanyl, 1,4-dioxanyl, 1,3-dioxolanyl, isoindolinyl, morpholinyl, piperazinyl, pyrrolidinyl, tetrahydropyridinyl, piperidinyl, thiomorpholinyl, and the like. The heterocycle groups may be optionally substituted unless specifically prohibited.

The term "hydrazinyl" as used herein, alone or in combination, refers to two amino groups joined by a single bond, i.e., -N-N-.

The term "hydroxy," as used herein, alone or in combination, refers to -OH.

The term "hydroxyalkyl," as used herein, alone or in combination, refers to a hydroxy group attached to the parent molecular moiety through an alkyl group.

The term "imino," as used herein, alone or in combination, refers to =N-.

The term "iminohydroxy," as used herein, alone or in combination, refers to =N(OH) and =N-O-.

The phrase "in the main chain" refers to the longest contiguous or adjacent chain of carbon atoms starting at the point of attachment of a group to the compounds of any one of the formulas disclosed herein.

The term "isocyanato" refers to a -NCO group.

The term "isothiocyanato" refers to a -NCS group.

The phrase "linear chain of atoms" refers to the longest straight chain of atoms independently selected from carbon, nitrogen, oxygen and sulfur.

The term "lower," as used herein, alone or in a combination, where not otherwise specifically defined, means containing from 1 to and including 6 carbon atoms (i.e., $C_1$-$C_6$ alkyl).

The term "lower aryl," as used herein, alone or in combination, means phenyl or naphthyl, either of which may be optionally substituted as provided.

The term "lower heteroaryl," as used herein, alone or in combination, means either 1) monocyclic heteroaryl comprising five or six ring members, of which between one and four said members may be heteroatoms chosen from N, O, and S, or 2) bicyclic heteroaryl, wherein each of the fused rings comprises five or six ring members, comprising between them one to four heteroatoms chosen from N, O, and S.

The term "lower cycloalkyl," as used herein, alone or in combination, means a monocyclic cycloalkyl having between three and six ring members (i.e., $C_3$-$C_6$ cycloalkyl). Lower cycloalkyls may be unsaturated. Examples of lower cycloalkyl include cyclopropyl, cyclobutyl, cyclopentyl, and cyclohexyl.

The term "lower heterocycloalkyl," as used herein, alone or in combination, means a monocyclic heterocycloalkyl having between three and six ring members, of which between one and four may be heteroatoms chosen from N, O, and S (i.e., $C_3$-$C_6$ heterocycloalkyl). Examples of lower heterocycloalkyls include pyrrolidinyl, imidazolidinyl, pyrazolidinyl, piperidinyl, piperazinyl, and morpholinyl. Lower heterocycloalkyls may be unsaturated.

The term "lower amino," as used herein, alone or in combination, refers to -NRR’, wherein R and R are independently chosen from hydrogen and lower alkyl, either of which may be optionally substituted.

The term "mercaptyl" as used herein, alone or in combination, refers to an RS- group, where R is as defined herein.

The term "nitro," as used herein, alone or in combination, refers to $-NO_2$.

The terms "oxy" or "oxa," as used herein, alone or in combination, refer to -O-.

The term "oxo," as used herein, alone or in combination, refers to =O.

The term "perhaloalkoxy" refers to an alkoxy group where all of the hydrogen atoms are replaced by halogen atoms.

The term "perhaloalkyl" as used herein, alone or in combination, refers to an alkyl group where all of the hydrogen atoms are replaced by halogen atoms.

The terms "sulfonate," "sulfonic acid," and "sulfonic," as used herein, alone or in combination, refer the $-SO_3H$ group and its anion as the sulfonic acid is used in salt formation.

The term "sulfanyl," as used herein, alone or in combination, refers to -S-.

The term "sulfinyl," as used herein, alone or in combination, refers to -S(O)-.

The term "sulfonyl," as used herein, alone or in combination, refers to $-S(O)_2-$.

The term "N-sulfonamido" refers to a $RS(=O)_2NR'$- group with R and R’ as defined herein.

The term "S-sulfonamido" refers to a $-S(=O)_2NRR'$, group, with R and R’ as defined herein.

The terms "thia" and "thio," as used herein, alone or in combination, refer to a -S- group or an ether wherein the oxygen is replaced with sulfur. The oxidized derivatives of the thio group, namely sulfinyl and sulfonyl, are included in the definition of thia and thio.

The term "thiol," as used herein, alone or in combination, refers to an -SH group.

The term "thiocarbonyl," as used herein, when alone includes thioformyl -e(S)H and in combination is a -e(S)- group.

The term "N-thiocarbamyl" refers to an ROC(S)NR’- group, with R and R’as defined herein.

The term "O-thiocarbamyl" refers to a -OC(S)NRR’, group with R and R’as defined herein.

The term "thiocyanate" refers to a -CNS group.

The term "trihalomethanesulfonamido" refers to a $X_3CS(O)_2NR$- group with X is a halogen and R as defined herein.

The term "trihalomethanesulfonyl" refers to a $X_3CS(O)_2$- group where X is a halogen.

The term "trihalomethoxy" refers to a $X_3CO$- group where X is a halogen.

The term "trisubstituted silyl," as used herein, alone or in combination, refers to a silicone group substituted at

its three free valences with groups as listed herein under the definition of substituted amino. Examples include trimethysilyl, tert-butyldimethylsilyl, triphenylsilyl and the like.

Any definition herein may be used in combination with any other definition to describe a composite structural group. By convention, the trailing element of any such definition is that which attaches to the parent moiety. For example, the composite group alkylamido would represent an alkyl group attached to the parent molecule through an amido group, and the term alkoxyalkyl would represent an alkoxy group attached to the parent molecule through an alkyl group.

When a group is defined to be "null," what is meant is that said group is absent.

The term "optionally substituted" means the anteceding group may be substituted or unsubstituted. When substituted, the substituents of an "optionally substituted" group may include, without limitation, one or more substituents independently selected from the following groups or a particular designated set of groups, alone or in combination: lower alkyl, lower alkenyl, lower alkynyl, lower alkanoyl, lower heteroalkyl, lower heterocycloalkyl, lower haloalkyl, lower haloalkenyl, lower haloalkynyl, lower perhaloalkyl, lower perhaloalkoxy, lower cycloalkyl, phenyl, aryl, aryloxy, lower alkoxy, lower haloalkoxy, oxo, lower acyloxy, carbonyl, carboxyl, lower alkylcarbonyl, lower carboxyester, lower carboxamido, cyano, hydrogen, halogen, hydroxy, amino, lower alkylamino, arylamino, amido, nitro, thiol, lower alkylthio, lower haloalkylthio, lower perhaloalkylthio, arylthio, sulfonate, sulfonic acid, trisubstituted silyl, $N_3$, SH, $SCH_3$, $C(O)CH_3$, $CO_2CH_3$, $CO_2H$, pyridinyl, thiophene, furanyl, lower carbamate, and lower urea. Where structurally feasible, two substituents may be joined together to form a fused five-, six-, or seven-membered carbocyclic or heterocyclic ring consisting of zero to three heteroatoms, for example forming methylenedioxy or ethylenedioxy. An optionally substituted group may be unsubstituted (e.g., $-CH_2CH_3$), fully substituted (e.g., $-CF_2CF_3$), monosubstituted (e.g., $-CH_2CH_2F$) or substituted at a level anywhere in-between fully substituted and monosubstituted (e.g., $-CH_2CF_3$). Where substituents are recited without qualification as to substitution, both substituted and unsubstituted forms are encompassed. Where a substituent is qualified as "substituted," the substituted form is specifically intended. Additionally, different sets of optional substituents to a particular moiety may be defined as needed; in these cases, the optional substitution will be as defined, often immediately following the phrase, "optionally substituted with."

As used herein, a substituted group is derived from the unsubstituted parent group in which there has been an exchange of one or more hydrogen atoms for another atom or group. Unless otherwise indicated, when a group is deemed to be "substituted," it is meant that the group is substituted with one or more substituents independently selected from $C_1$-$C_6$ alkyl, $C_1$-$C_6$ alkenyl, $C_1$-$C_6$ alkynyl, $C_1$-$C_6$ heteroalkyl, $C_3$-$C_7$ carbocyclyl (optionally substituted with halo, $C_1$-$C_6$ alkyl, $C_1$-$C_6$ alkoxy, $C_1$-$C_6$ haloalkyl, and $C_1$-$C_6$ haloalkoxy), $C_3$-$C_7$-carbocyclyl-$C_1$-$C_6$-alkyl (optionally substituted with halo, $C_1$-$C_6$ alkyl, $C_1$-$C_6$ alkoxy, $C_1$-$C_6$ haloalkyl, and $C_1$-$C_6$ haloalkoxy), 3-10 membered heterocyclyl (optionally substituted with halo, $C_1$-$C_6$ alkyl, $C_1$-$C_6$ alkoxy, $C_1$-$C_6$ haloalkyl, and $C_1$-$C_6$ haloalkoxy), 3-10 membered heterocyclyl-$C_1$-$C_6$-alkyl (optionally substituted with halo, $C_1$-$C_6$ alkyl, $C_1$-$C_6$ alkoxy, $C_1$-$C_6$ haloalkyl, and $C_1$-$C_6$ haloalkoxy), aryl (optionally substituted with halo, $C_1$-$C_6$ alkyl, $C_1$-$C_6$ alkoxy, $C_1$-$C_6$ haloalkyl, and $C_1$-$C_6$ haloalkoxy), aryl($C_1$-$C_6$)alkyl (optionally substituted with halo, $C_1$-$C_6$ alkyl, $C_1$-$C_6$ alkoxy, $C_1$-$C_6$ haloalkyl, and $C_1$-$C_6$ haloalkoxy), 5-10 membered heteroaryl (optionally substituted with halo, $C_1$-$C_6$ alkyl, $C_1$-$C_6$ alkoxy, $C_1$-$C_6$ haloalkyl, and $C_1$-$C_6$ haloalkoxy), 5-10 membered heteroaryl($C_1$-$C_6$)alkyl (optionally substituted with halo, $C_1$-$C_6$ alkyl, $C_1$-$C_6$ alkoxy, $C_1$-$C_6$ haloalkyl, and $C_1$-$C_6$ haloalkoxy), halo, cyano, hydroxy, $C_1$-$C_6$ alkoxy, $C_1$-$C_6$ alkoxy($C_1$-$C_6$)alkyl (i.e., ether), aryloxy, sulfhydryl (mercapto), halo($C_1$-$C_6$)alkyl (e.g., $-CF_3$), halo($C_1$-$C_6$)alkoxy (e.g., $-OCF_3$), $C_1$-$C_6$ alkylthio, arylthio, amino, amino($C_1$-$C_6$)alkyl, nitro, O-carbamyl, N-carbamyl, O-thiocarbamyl, N-thiocarbamyl, C-amido, N-amido, S-sulfonamido, N-sulfonamido, C-carboxy, O-carboxy, acyl, cyanato, isocyanato, thiocyanato, isothiocyanato, sulfinyl, sulfonyl, and oxo (=O). Wherever a group is described as "optionally substituted" that group can be substituted with the above substituents.

The term R or the term R', appearing by itself and without a number designation, unless otherwise defined, refers to a moiety chosen from hydrogen, alkyl, cycloalkyl, heteroalkyl, aryl, heteroaryl and heterocycloalkyl, any of which may be optionally substituted. Such R and R' groups should be understood to be optionally substituted as defined herein. Whether an R group has a number designation or not, every R group, including R, R' and $R^n$ where n=(1, 2, 3, ...n), every substituent, and every term should be understood to be independent of every other in terms of selection from a group. Should any variable, substituent, or term (e.g. aryl, heterocycle, R, etc.) occur more than one time in a formula or generic structure, its definition at each occurrence is independent of the definition at every other occurrence. Those of skill in the art will further recognize that certain groups may be attached to a parent molecule or may occupy a position in a chain of elements from either end as written. For example, an unsymmetrical group such as -C(O)N(R)- may be attached to the parent moiety at either the carbon or the nitrogen.

Asymmetric centers exist in the compounds disclosed herein. These centers are designated by the symbols "R" or "S," depending on the configuration of substituents around the chiral carbon atom. It should be understood that the disclosure encompasses all stereochemical isomeric forms, including diastereomeric, enantiomeric, and epimeric forms,as well as d-isomers and 1-isomers, and mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, direct separation of enantiomers on chiral chromatographic columns,

or any other appropriate method known in the art. Starting compounds of particular stereochemistry are either commercially available or can be made and resolved by techniques known in the art. Additionally, the compounds disclosed herein may exist as geometric isomers. The present disclosure includes all cis, trans, syn, anti, entgegen (E), and zusammen (Z) isomers as well as the appropriate mixtures thereof. Additionally, compounds may exist as tautomers; all tautomeric isomers are provided by this disclosure. Additionally, the compounds disclosed herein can exist in unsolvated as well as solvated forms with pharmaceutically acceptable solvents such as water, ethanol, and the like. In general, the solvated forms are considered equivalent to the unsolvated forms.

The term "bond" refers to a covalent linkage between two atoms, or two moieties when the atoms joined by the bond are considered to be part of larger substructure. A bond may be single, double, or triple unless otherwise specified. A dashed line between two atoms in a drawing of a molecule indicates that an additional bond may be present or absent at that position.

The term "disease" as used herein is intended to be generally synonymous, and is used interchangeably with, the terms "disorder," "syndrome," and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The term "combination therapy" means the administration of two or more therapeutic agents to treat a therapeutic condition or disorder described in the present disclosure. Such administration encompasses coadministration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the conditions or disorders described herein.

The phrase "therapeutically effective" is intended to qualify the amount of active ingredients used in the treatment of a disease or disorder or on the effecting of a clinical endpoint.

The term "therapeutically acceptable" refers to those compounds (or salts, prodrugs, tautomers, zwitterionic forms, etc.) which are suitable for use in contact with the tissues of patients without undue toxicity, irritation, and allergic response, are commensurate with a reasonable benefit/risk ratio, and are effective for their intended use.

As used herein, reference to "treatment" of a patient is intended to include prophylaxis. Treatment may also be preemptive in nature, i.e., it may include prevention of disease. Prevention of a disease may involve complete protection from disease, for example as in the case of prevention of infection with a pathogen, or may involve prevention of disease progression. For example, prevention of a disease may not mean complete foreclosure of any effect related to the diseases at any level, but instead may mean prevention of the symptoms of a disease to a clinically significant or detectable level. Prevention of diseases may also mean prevention of progression of a disease to a later stage of the disease.

The term "patient" is generally synonymous with the term "subject" and includes all mammals including humans. Examples of patients include humans, livestock such as cows, goats, sheep, pigs, and rabbits, and companion animals such as dogs, cats, rabbits, and horses. Preferably, the patient is a human.

The term "prodrug" refers to a compound that is made more active in vivo. Certain compounds disclosed herein may also exist as prodrugs, as described in Hydrolysis in Drug and Prodrug Metabolism : Chemistry, Biochemistry, and Enzymology (Testa, Bernard and Mayer, Joachim M. Wiley-VHCA, Zurich, Switzerland 2003). Prodrugs of the compounds described herein are structurally modified forms of the compound that readily undergo chemical changes under physiological conditions to provide the compound. Additionally, prodrugs can be converted to the compound by chemical or biochemical methods in an ex vivo environment. For example, prodrugs can be slowly converted to a compound when placed in a transdermal patch reservoir with a suitable enzyme or chemical reagent. Prodrugs are often useful because, in some situations, they may be easier to administer than the compound, or parent drug. They may, for instance, be bioavailable by oral administration whereas the parent drug is not. The prodrug may also have improved solubility in pharmaceutical compositions over the parent drug. A wide variety of prodrug derivatives are known in the art, such as those that rely on hydrolytic cleavage or oxidative activation of the prodrug. An example, without limitation, of a prodrug would be a compound which is administered as an ester (the "prodrug"), but then is metabolically hydrolyzed to the carboxylic acid, the active entity. Additional examples include peptidyl derivatives of a compound.

The compounds disclosed herein can exist as therapeutically acceptable salts. The present disclosure includes compounds listed above in the form of salts, including acid addition salts. Suitable salts include those formed with both organic and inorganic acids. Such acid addition salts will normally be pharmaceutically acceptable. However, salts of non-pharmaceutically acceptable salts may be of utility in the preparation and purification of the compound in question. Basic addition salts may also be formed and be pharmaceutically acceptable. For a more complete discussion of the preparation and selection of salts, refer to Pharmaceutical Salts: Properties, Selection, and Use (Stahl, P. Heinrich. Wiley-VCHA, Zurich, Switzerland, 2002).

Basic addition salts can be prepared during the final isolation and purification of the compounds by reacting a carboxy group with a suitable base such as the hydroxide, carbonate, or bicarbonate of a metal cation or with ammonia

or an organic primary, secondary, or tertiary amine. The cations of therapeutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, *N,N*-dimethylaniline, *N*-methylpiperidine, *N*-methylmorpholine, dicyclohexylamine, procaine, dibenzylamine, *N,N*-dibenzylphenethylamine, 1-ephenamine, and *N,N*'-dibenzylethylenediamine. Other representative organic amines useful for the formation of base addition salts include ethylenediamine, ethanolamine, diethanolamine, piperidine, and piperazine.

Other carrier materials and modes of administration known in the pharmaceutical art may also be used. Pharmaceutical compositions of the disclosure may be prepared by any of the well-known techniques of pharmacy, such as effective formulation and administration procedures. Preferred unit dosage formulations are those containing an effective dose, as herein below recited, or an appropriate fraction thereof, of the active ingredient.

It should be understood that in addition to the ingredients particularly mentioned above, the formulations described above may include other agents conventional in the art having regard to the type of formulation in question, for example those suitable for oral administration may include flavoring agents.

The amount of active ingredient that may be combined with the carrier materials to produce a single dosage form will vary depending upon the host treated and the particular mode of administration.

The compounds can be administered in various modes, e.g. orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the indication or condition being treated. In addition, the route of administration may vary depending on the condition and its severity. The above considerations concerning effective formulations and administration procedures are well known in the art and are described in standard textbooks.

*Combinations and Combination Therapy*

In certain instances, it may be appropriate to administer at least one of the compounds described herein (or a pharmaceutically acceptable salt thereof) in combination with another therapeutic agent. By way of example only, if one of the side effects experienced by a patient upon receiving one of the compounds herein is hypertension, then it may be appropriate to administer an anti-hypertensive agent in combination with the initial therapeutic agent. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced). Or, by way of example only, the benefit of experienced by a patient may be increased by administering one of the compounds described herein with another therapeutic agent (which also includes a therapeutic regimen) that also has therapeutic benefit. By way of example only, in a treatment for diabetes involving administration of one of the compounds described herein, increased therapeutic benefit may result by also providing the patient with another therapeutic agent for diabetes. In any case, regardless of the disease, disorder or condition being treated, the overall benefit experienced by the patient may simply be additive of the two therapeutic agents or the patient may experience a synergistic benefit.

In any case, the multiple therapeutic agents (at least one of which is a compound disclosed herein) may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may be any duration of time ranging from a few minutes to four weeks.

Thus, in another aspect, certain embodiments provide methods for treating *c9orf72*-mediated disorders in a human or animal subject in need of such treatment comprising administering to said subject an amount of a compound disclosed herein effective to reduce or prevent said disorder in the subject, in combination with at least one additional agent for the treatment of said disorder that is known in the art. In a related aspect, certain embodiments provide therapeutic compositions comprising at least one compound disclosed herein in combination with one or more additional agents for the treatment of *c9orf72*-mediated disorders.

Besides being useful for human treatment, certain compounds and formulations disclosed herein may also be useful for veterinary treatment of companion animals, exotic animals and farm animals, including mammals, rodents, and the like. More preferred animals include horses, dogs, and cats.

Compound Synthesis

Compounds of the present disclosure can be prepared using methods illustrated in general synthetic schemes and experimental procedures detailed below. General synthetic schemes and experimental procedures are presented

for purposes of illustration and are not intended to be limiting. Starting materials used to prepare compounds of the present disclosure are commercially available or can be prepared using routine methods known in the art.

List of Abbreviations

$Ac_2O$ = acetic anhydride; AcCl = acetyl chloride; AcOH = acetic acid; AIBN = azobisisobutyronitrile; aq. = aqueous; $Bu_3SnH$ = tributyltin hydride; $CD_3OD$ = deuterated methanol; $CDCl_3$ = deuterated chloroform; CDI = 1,1'-Carbonyldiimidazole; DBU = 1,8-diazabicyclo[5.4.0]undec-7-ene; DCM = dichloromethane; DEAD = diethyl azodicarboxylate; DIBAL-H = di-iso-butyl aluminium hydride; DIEA = DIPEA = N,N-diisopropylethylamine; DMAP = 4-dimethylaminopyridine; DMF = N,N-dimethylformamide; DMSO-$d_6$ = deuterated dimethyl sulfoxide; DMSO = dimethyl sulfoxide; DPPA = diphenylphosphoryl azide; EDC.HCl = EDCI.HCl = 1-ethyl-3-(3-dimethylaminopropyl)carbodiimide hydrochloride; $Et_2O$ = diethyl ether; EtOAc = ethyl acetate; EtOH = ethanol; h = hour; HATU=2-(1H-7-azabenzotriazol-1-yl)-1,1,3,3-tetramethyl uronium hexafluorophosphate methanaminium; HMDS = hexamethyldisilazane; HOBT = 1-hydroxybenzotriazole; i-PrOH = isopropanol; LAH = lithium aluminium hydride; LiHMDS = Lithium bis(trimethylsilyl)amide; MeCN = acetonitrile; MeOH = methanol; MP-carbonate resin = macroporous triethylammonium methylpolystyrene carbonate resin; MsCl = mesyl chloride; MTBE = methyl tertiary butyl ether; MW = microwave irradiation ; n-BuLi = n-butyllithium; NaHMDS = Sodium bis(trimethylsilyl)amide; NaOMe = sodium methoxide; NaOtBu = sodium t-butoxide; NBS = N-bromosuccinimide; NCS = N-chlorosuccinimide; NMP = N-Methyl-2-pyrrolidone; $Pd(Ph_3)_4$ = tetrakis(triphenylphosphine)palladium(0); $Pd_2(dba)_3$ = tris(dibenzylideneacetone)dipalladium(0); $PdCl_2(PPh_3)_2$ = bis(triphenylphosphine)palladium(II) dichloride; PG = protecting group; prep-HPLC = preparative high-performance liquid chromatography; PyBop = (benzotriazol-1-yloxy)-tripyrrolidinophosphonium hexafluorophosphate; Pyr = pyridine; RT = room temperature; Ru-Phos = 2-dicyclohexylphosphino-2',6'-diisopropoxybiphenyl; sat. = saturated; ss = saturated solution; t-BuOH = tert-butanol; T3P = Propylphosphonic Anhydride; TBS = TBDMS = tert-butyldimethylsilyl; TBSCl = TBDMSCl = *tert*-butyldimethylchlorosilane; TEA = $Et_3N$ = triethylamine; TFA = trifluoroacetic acid; TFAA = trifluoroacetic anhydride; THF = tetrahydrofuran; Tol = toluene; TsCl = tosyl chloride; XPhos = 2-dicyclohexylphosphino-2',4',6'-triisopropylbiphenyl.

General Synthetic Methods for Preparing Compounds

In general, polyamides of the present disclosure may be synthesized by solid supported synthetic methods, using compounds such as Boc-protected straight chain aliphatic and heteroaromatic amino acids, and alkylated derivatives thereof, which are cleaved from the support by aminolysis, deprotected (e.g., with sodium thiophenoxide), and purified by reverse-phase HPLC, as well known in the art. The identity and purity of the polyamides may be verified using any of a variety of analytical techniques available to one skilled in the art such as $^1$H-NMR, analytical HPLC, or mass spectrometry.

The following scheme can be used to practice the present disclosure.

Scheme I: Synthesis of polyamides

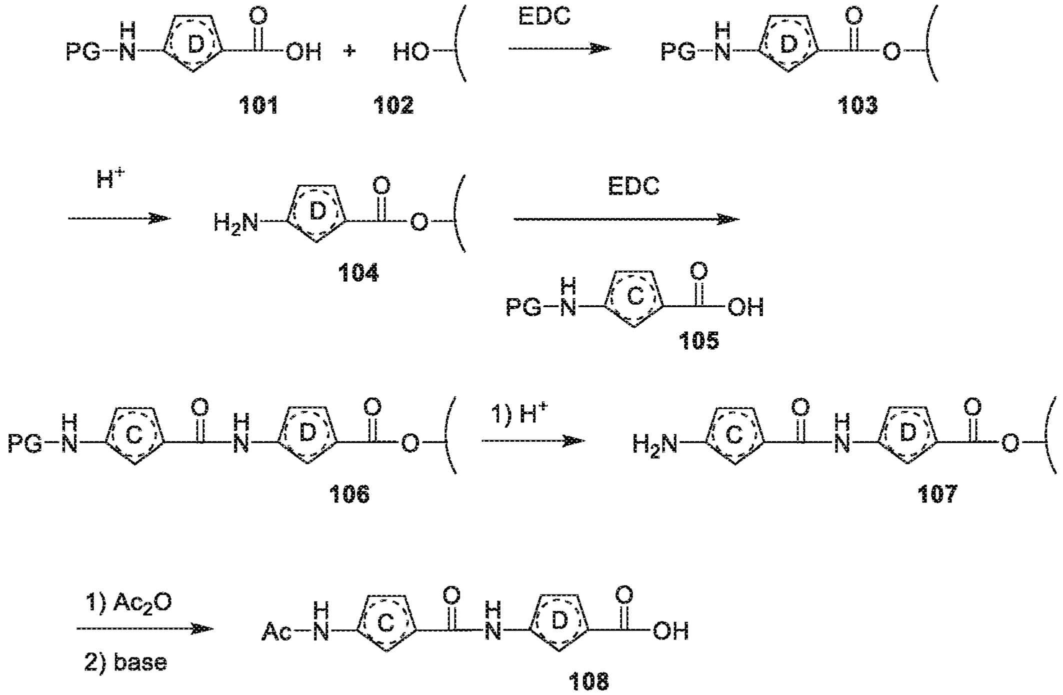


101 102 103 104 105 106 107 108

The compounds disclosed herein can be synthesized using Scheme I. For clarity and compactness, the scheme depicts the synthesis of a diamide comprising subunits "C" and "D", both of which are represented as unspecified five-membered rings having amino and carboxy moieties. The amino group of subunit "D" is protected with a protecting group "PG" such as a Boc or CBz carbamate to give **101**. The free )carboxylic acid is then reacted with a solid support, using a coupling reagent such as EDC, to give the supported compound 103. Removal of PG under acidic conditions gives the free amine **104**, which is coupled with the nitrogen-protected carboxylic acid **105** to give amide **106**. Removal of PG under acidic conditions gives the free amine **107**. In this example, the free amine is reacted with acetic anhydride to form an acetamide (not shown. The molecule is then cleaved from the solid support under basic conditions to give carboxylic acid **108**. Methods for attachment of the linker L and recruiting moiety X are disclosed below.

The person of skill will appreciate that many variations of the above scheme are available to provide a wide range of compounds:

1) The sequence **104** -**106** -**107** can be repeated as often as desired, in order to form longer polyamine sequences.

2) A variety of amino heterocycle carboxylic acids can be used, to form different subunits. Table 3, while not intended to be limiting, provides several heterocycle amino acids that are contemplated for the synthesis of the compounds in this disclosure. Carbamate protecting groups PG can be incorporated using techniques that are well established in the art.

Table 3. Heterocyclic amino acids.

| Structure |
|---|
| 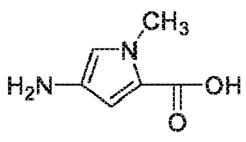 Py |
| 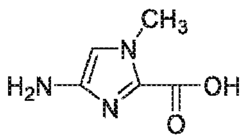 Im |

(continued)

| Structure |
| --- |
| 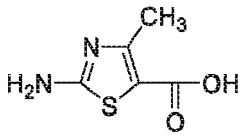 Th |
| 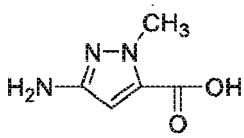 Pz |
| 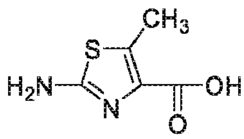 Nt |
| 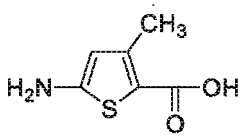 Tn |
| 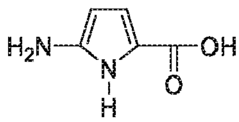 Nh |
| 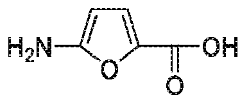 Fr |
| 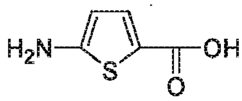 Tp |
| 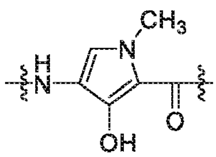 Hp |
| 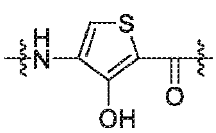 Ht |
| 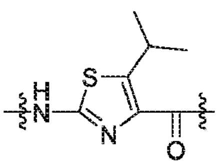 iNt |

(continued)

| Structure |
| --- |
| 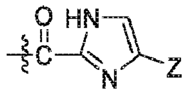 PyT (Z is H, $C_{1-6}$ alkyl, amine, or halogen) |
| 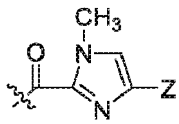 ImT (Z is H, $C_{1-6}$ alkyl, amine, or halogen) |
| 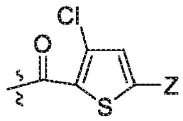 CTh |
| 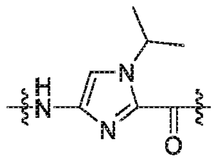 iIm |
| 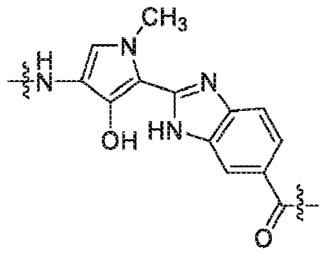 HpBi |
| 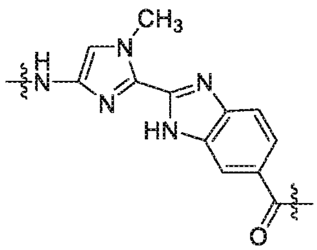 ImBi |
| 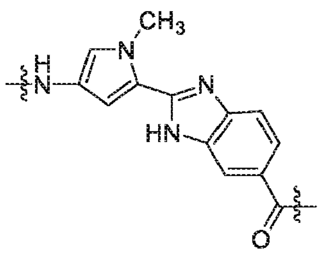 PyBi |

(continued)

| Structure |
|---|
| 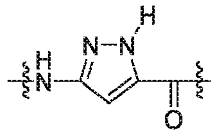 |
| 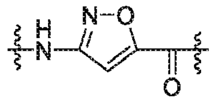 |
| 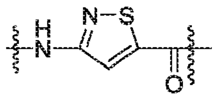 |
| 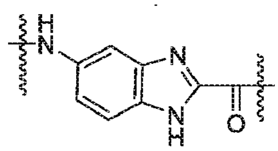 |

3) Hydroxy-containing heterocyclic amino acids can be incorporated into Scheme I as their TBS ethers. While not intended to be limiting, Scheme II provides the synthesis of TBSprotected heterocyclic amino acids contemplated for the synthesis of the compounds in this disclosure.

Scheme II: Synthesis of TBS-protected heterocyclic amino acids

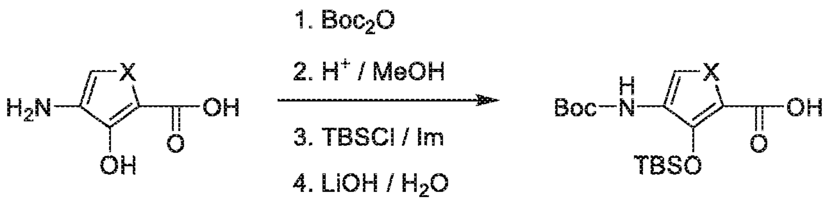


X = $N(CH_3)$; S

4) Aliphatic amino acids can be used in the above synthesis for the formation of spacer units "W" and subunits for recognition of DNA nucleotides. Table 4, while not intended to be limiting, provides several aliphatic amino acids contemplated for the synthesis of the compounds in this disclosure.

Table 4. Aliphatic amino acids.

| Structure |
|---|
| 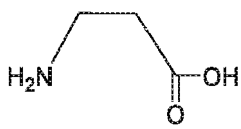 beta-alanine (β) |
| 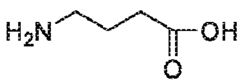 gamma-aminobutyric acid ("gAB" or γ) |

(continued)

| Structure |
|---|
| 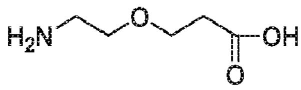 3-(2-aminoethoxy)propanoic acid |
| 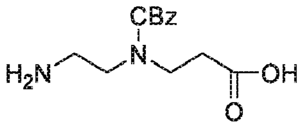 3-((2-aminoethyl)(2-oxo-2-phenyl-1λ$^{2}$-ethyl)amino)propanoic acid |
| 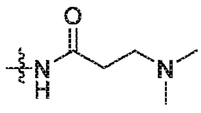 Dp |
| 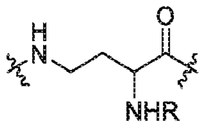 (R is H, $C_{1-6}$ alkyl) |
| 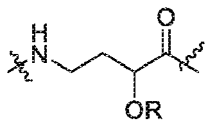 (R is H, $C_{1-6}$ alkyl, aryl, or heteroaryl) |
| 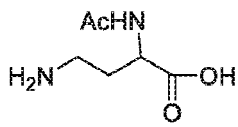 |
| 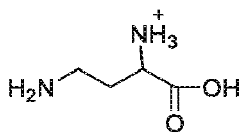 |
| 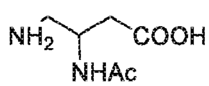 |
| 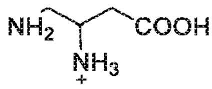 |
| 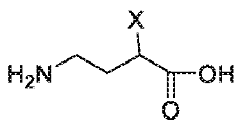 X is F or OH |

Scheme III: Synthesis of polyamide / recruiting agent / linker conjugate.

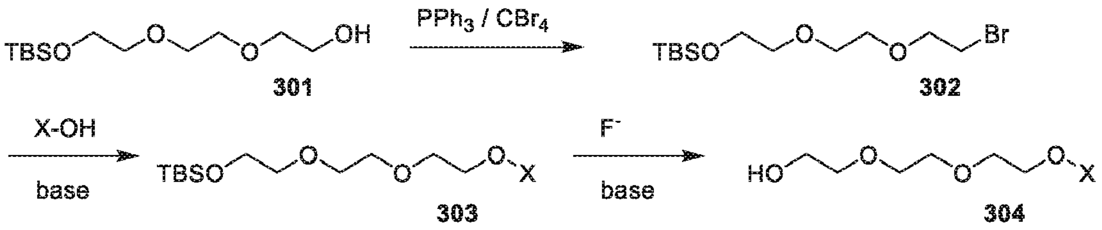


301 302 303 304

Attachment of the linker L and recruiting moiety X can be accomplished with the methods disclosed in Scheme III, which uses a triethylene glycol moiety for the linker L. The mono-TBS ether of triethylene glycol **301** is converted to the bromo compound **302** under Mitsunobu conditions. The recruiting moiety X is attached by displacement of the bromine with a hydroxyl moiety, affording ether **303**. The TBS group is then removed by treatment with fluoride, to provide alcohol **304**, which will be suitable for coupling with the polyamide moiety. Other methods will be apparent to the person of skill in the art for inclusion of alternate linkers L, including but not limited to propylene glycol or polyamine linkers, or alternate points of attachment of the recruiting moiety X, including but not limited to the use of amines and thiols.

Scheme IV: Synthesis of polyamide / recruiting agent / linker conjugate.

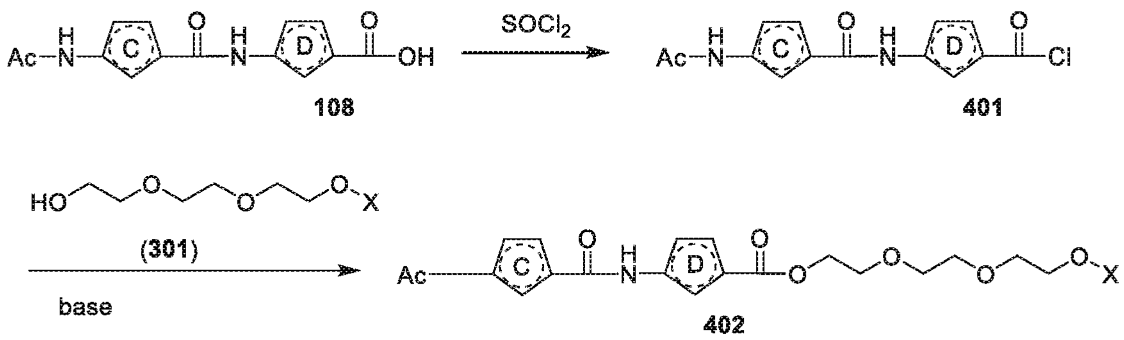


108 401 402

Synthesis of the X-L-Y molecule can be completed with the methods set forth in Scheme IV. Carboxylic acid **108** is converted to the acid chloride **401**. Reaction with the alcohol functionality of **301** under basic conditions provides the coupled product **402**. Other methods will be apparent to the person of skill in the art for performing the coupling procedure, including but not limited to the use of carbodiimide reagents. For instance, the amide coupling reagents can be used, but not limited to, are carbodiimides such as dicyclohexylcarbodiimide (DCC), diisopropylcarbodiimide (DIC), ethyl-(N',N'-dimethylamino)propylcarbodiimide hydrochloride (EDC), in combination with reagents such as 1-hydroxybenzotriazole (HOBt), 4-(N,N-dimethylamino)pyridine (DMAP) and diisopropylethylamine (DIEA). Other reagents are also often used depending the actual coupling reactions are (Benzotriazol-1-yloxy)tris(dimethylamino)phosphonium hexafluorophosphate (BOP), (Benzotriazol-1-yloxy)tripyrrolidinophosphonium hexafluorophosphate (PyBOP), (7-Azabenzotriazol-1-yloxy)tripyrrolidinophosphonium hexafluorophosphate (PyAOP), Bromotripyrrolidinophosphonium hexafluorophosphate (PyBrOP), Bis(2-oxo-3-oxazolidinyl)phosphinic chloride (BOP-Cl), O-(Benzotriazol-1-yl)-N,N,N',N'-tetramethyluronium hexafluorophosphate (HBTU), O-(Benzotriazol-1-yl)- N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)- N,N,N',N'-tetramethyluronium tetrafluoroborate (TATU), O-(6-Chlorobenzotriazol-1-yl)-N,N,N',N'-tetramethyluronium hexafluorophosphate (HCTU), Carbonyldiimidazole (CDI), and N,N,N',N'-Tetramethylchloroformamidinium Hexafluorophosphate (TCFH).

Scheme V: Proposed synthesis of rohitukine-based CDK9 inhibitor

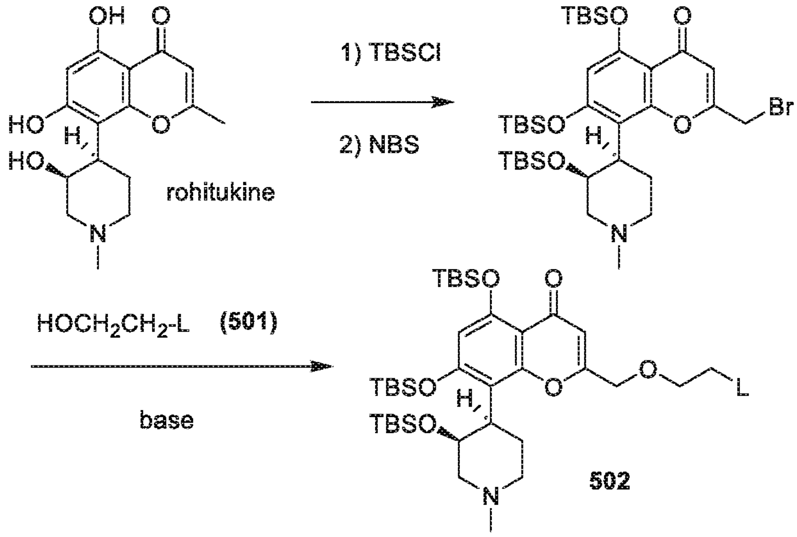


A proposed synthesis of a rohitukine-based CDK9 inhibitor is set forth in Scheme V. Synthesis begins with the natural product rohitukine, which is a naturally available compound that has been used as a precursor for CDK9-active drugs such as Alvocidib. The existing hydroxy groups are protected as TBS ethers, the methyl group is brominated, and the bromo compound is coupled with a suitably functionalized linker reagent such as **501** to afford the linked compound **502**. Variants of this procedure will be apparent to the person of skill.

Scheme VI: Proposed synthesis of DB08045-based cyclin T1 inhibitor

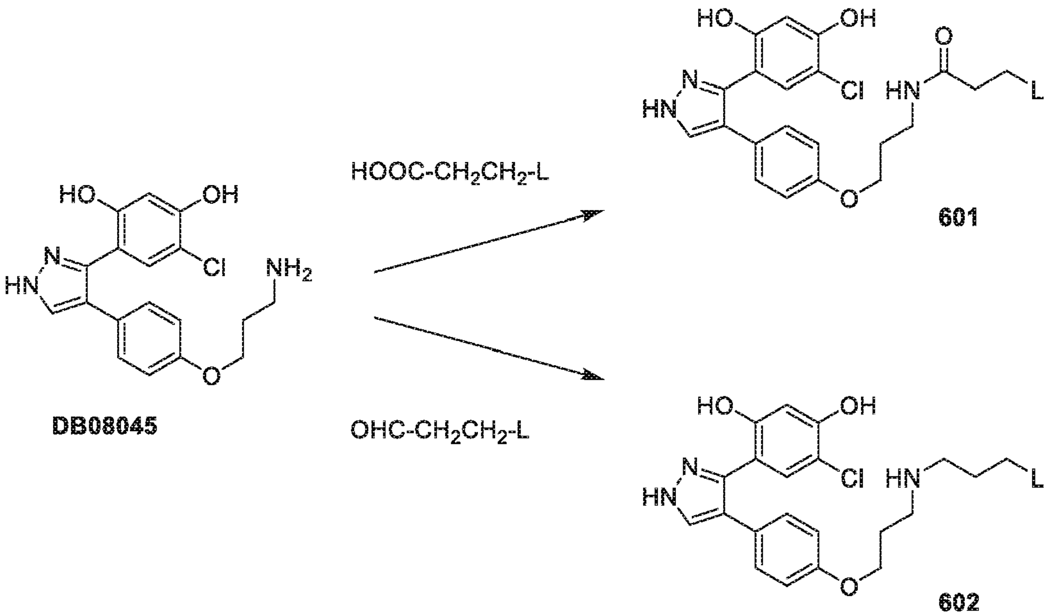


Proposed syntheses of DB08045-based cyclin T1 inhibitors are set forth in Scheme VI. Synthesis begins with DB08045, which contains a primary amino group that is available for functionalization. Coupling of the amino group with a carboxylic acid under conventional conditions gives amide **601**. Alternatively, reductive amination with a carboxaldehyde gives amine **602**. Variants of this procedure will be apparent to the person of skill.

Scheme VII: Proposed synthesis of A-395 based PRC2 inhibitor

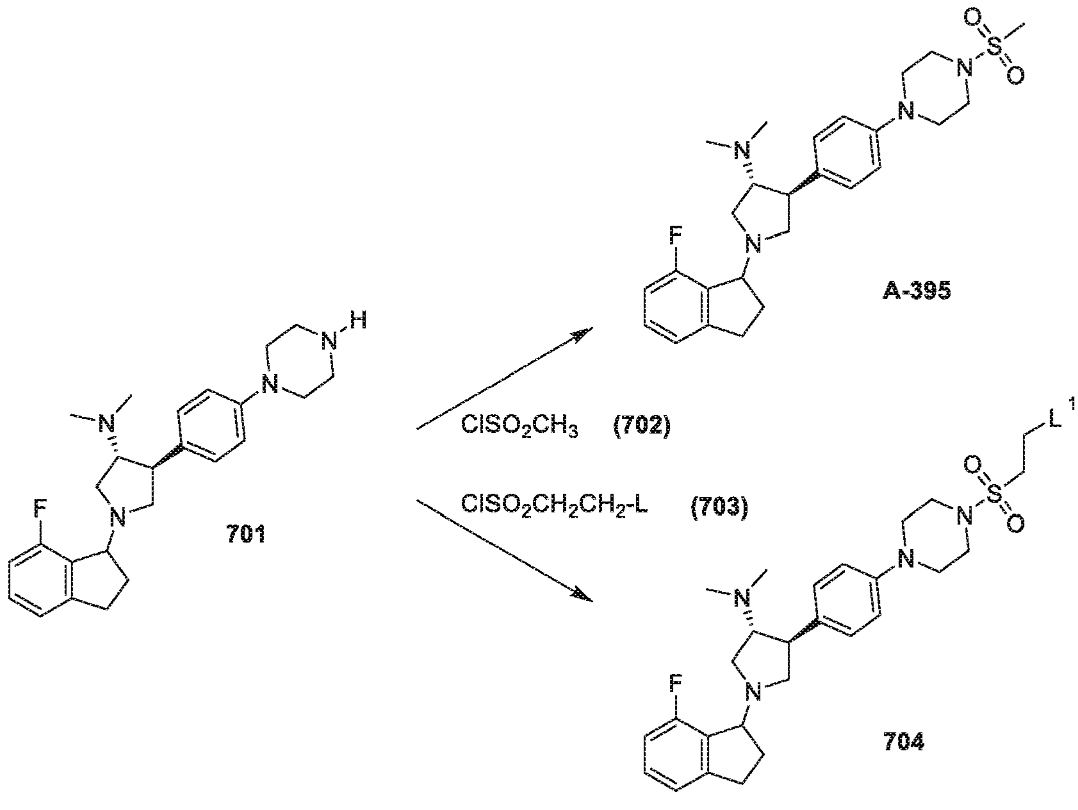


A proposed synthesis of an A-395 based PRC2 inhibitor is set forth in Scheme VII. The piperidine compound **701**, a precursor to A-395, can be reacted with methanesulfonyl chloride **702** to give A-395. In a variation of this synthesis, **701** is reacted with linked sulfonyl chloride **703**, to provide linked A-395 inhibitor **704**

***Attaching protein binding molecules to oligomeric backbone***

Generally the oligomeric backbone is functionalized to adapt to the type of chemical reactions can be performed to link the oligomers to the attaching position in protein binding moieties. The type reactions are suitable but not limited to, are amide coupling reactions, ether formation reactions (O-alkylation reactions), amine formation reactions (*N*-alkylation reactions), and sometimes carbon-carbon coupling reactions. The general reactions used to link oligomers and protein binders are shown in below schemes (VIII through X). The compounds and structures shown in Table 2 can be attached to the oligomeric backbone described herein at any position that is chemically feasible while not interfering with the hydrogen bond between the compound and the regulatory protein.

Scheme VIII. Amide Couplings

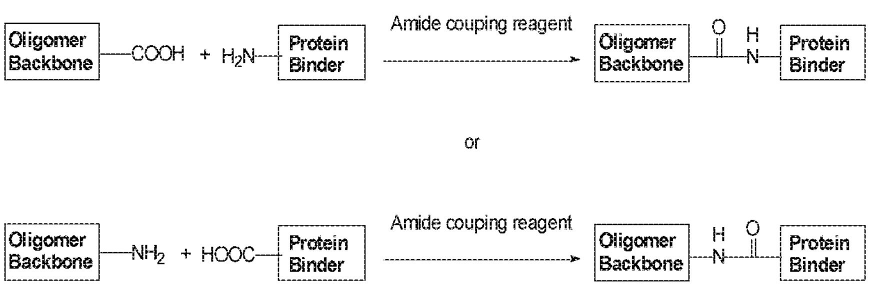


Either the oligomer or the protein binder can be functionalized to have a carboxylic acid and the other coupling counterpart being functionalized with an amino group so the moieties can be conjugated together mediated by amide coupling reagents. The amide coupling reagents can be used, but not limited to, are carbodiimides such as dicyclohexylcarbodiimide (DCC), diisopropylcarbodiimide (DIC), ethyl-(N',N'-dimethylamino)propylcarbodiimide hydrochloride

(EDC), in combination with reagents such as 1-hydroxybenzotriazole (HOBt), 4-(N,N-dimethylamino)pyridine (DMAP) and diisopropylethylamine (DIEA). Other reagents are also often used depending the actual coupling reactions are (Benzotriazol-1-yloxy)tris(dimethylamino)phosphonium hexafluorophosphate (BOP), (Benzotriazol-1-yloxy)tripyrrolidinophosphonium hexafluorophosphate (PyBOP), (7-Azabenzotriazol-1-yloxy)tripyrrolidinophosphonium hexafluorophosphate (PyAOP), Bromotripyrrolidinophosphonium hexafluorophosphate (PyBrOP), Bis(2-oxo-3-oxazolidinyl)phosphinic chloride (BOP-Cl), O-(Benzotriazol-1-yl)-N,N,N',N'-tetramethyluronium hexafluorophosphate (HBTU), O-(Benzotriazol-1-yl)- N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)- N,N,N',N'-tetramethyluronium tetrafluoroborate (TATU), O-(6-Chlorobenzotriazol-1-yl)-N,N,N',N'-tetramethyluronium hexafluorophosphate (HCTU), Carbonyldiimidazole (CDI), and N,N,N',N'-Tetramethylchloroformamidinium Hexafluorophosphate (TCFH).

Scheme IX. Ether Formation Reactions (O-alkylation reactions)

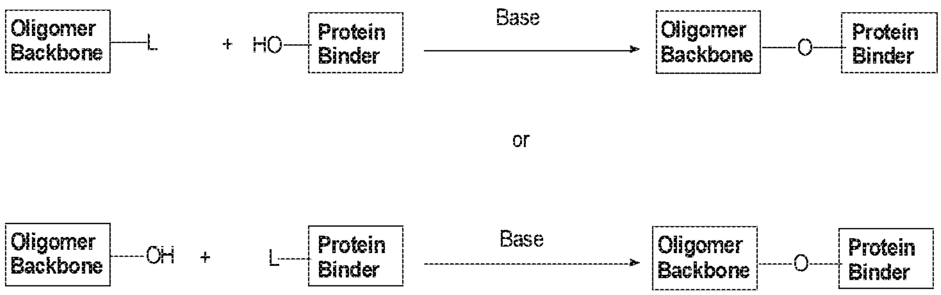


L = leaving group such as iodide, bromide, chloride, mesylate, besylate, tosylate

In an ether formation reaction, either the oligomer or the protein binder can be functionalized to have an hydroxyl group (phenol or alcohol) and the other coupling counterpart being functionalized with a leaving group such as halide, tosylate and mesylate so the moieties can be conjugated together mediated by a base or catalyst. The bases can be selected from, but not limited to, sodium hydride, potassium hydride, sodium hydroxide, potassium hydroxide, sodium carbonate, potassium carbonate. The catalyst can be selected from silver oxide, phase transfer reagents, iodide salts, and crown ethers.

Scheme X. Amine Formation Reactions (N-alkylation reactions)

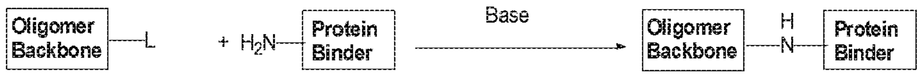


or

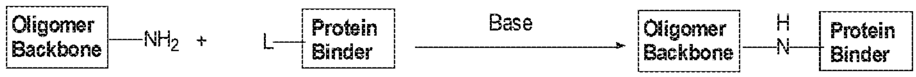


L = leaving group such as iodide, bromide, chloride, mesylate, besylate, tosylate

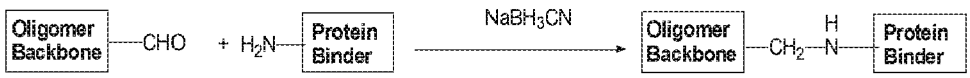


or

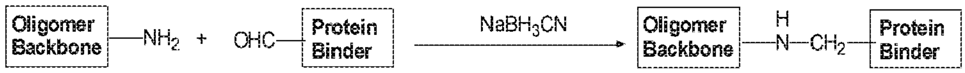


In an N-alkylation reaction, either the oligomer or the protein binder can be functionalized to have an amino group (arylamine or alkylamine) and the other coupling counterpart being functionalized with a leaving group such as halide, tosylate and mesylate so the moieties can be conjugated together directly or with a base or catalyst. The bases can be selected from, but not limited to, sodium hydride, potassium hydride, sodium hydroxide, potassium hydroxide, sodium carbonate, potassium carbonate. The catalyst can be selected from silver oxide, phase transfer reagents, iodide salts, and crown ethers. The alkylation of amines can also be achieved through reductive amination reactions, where in either the oligomer or the protein binder can be functionalized to have an amino group (arylamine or alkylamine) and the other coupling counterpart being functionalized with an aldehyde or ketone group so the moieties can be conjugated together with the treatment of a reducing reagent (hydride source) directly or in combination with a dehydration agent. The reducing reagents can be selected from, but not limited to, $NaBH_4$, $NaHB(OAc)_3$, $NaBH_3CN$, and dehydration agents are normally $Ti(iPrO)_4$, $Ti(OEt)_4$, $Al(iPrO)_3$, orthoformates and activated molecular sieves.

***Cell-penetrating ligand***

In one aspect, the compounds of the present disclosure comprises a cell penetrating ligand moiety. The cell-penetrating ligand moiety serves to facilitate transport of the compound across cell membranes. In certain embodiments, the cell-penetrating ligand moiety is a polypeptide. Several peptide sequences can facilitate passage into the cell, including polycationic sequences such as poly-R; arginine-rich sequences interspersed with spacers such as $(RXR)_n$ (X = 6-aminohexanoic acid) and $(RXRRBR)_n$ (B = beta-alanine); sequences derived from the Penetratin peptide; and sequences derived from the PNA/PMO internalisation peptide (Pip). The Pip5 series is characterized by the sequence ILFQY.

In certain embodiments, the cell penetrating polypeptide comprises an Nterminal cationic sequence $H_2N-(R)_n-CO-$, with n = 5-10, inclusive. In certain embodiments, the N-terminal cationic sequence contains 1, 2, or 3 substitutions of R for amino acid resides independently chosen from beta-alanine and 6-aminohexanoic acid.

In certain embodiments, the cell-penetrating polypeptide comprises the ILFQY sequence. In certain embodiments, the cell-penetrating polypeptide comprises the QFLY sequence. In certain embodiments, the cellpenetrating polypeptide comprises the QFL sequence.

In certain embodiments, the cell penetrating polypeptide comprises a C-terminal cationic sequence $-HN-(R)_n-COOH$, with n = 5-10, inclusive. In certain embodiments, the C-terminal cationic sequence contains 1, 2, or 3 substitutions of R for amino acid resides independently chosen from beta-alanine and 6-aminohexanoic acid. In certain

embodiments, the C-terminal cationic sequence is substituted at every other position with an amino acid residue independently chosen from beta-alanine and 6-aminohexanoic acid. In certain embodiments, the C-terminal cationic sequence is -HN-RXRBRXRB-COOH.

Table 5. Cell-penetrating peptides

| SEQ ID NO. | Sequence |
|---|---|
| SEQ ID NO. 1 | GRKKRRQRRRPPQ |
| SEQ ID NO. 2 | RQIKIWFQNRRMICWKK |
| SEQ ID NO. 3 | KLALKLALKALKAALKLA |
| SEQ ID NO. 4 | GWTLNS/AGYLLGKINLKALAALAKKIL |
| SEQ ID NO. 5 | NAKTRRHERRRKLAIER |
| SEQ ID NO. 6 | RRRRRRRR |
| SEQ ID NO. 7 | RRRRRRRRR |
| SEQ ID NO. 8 | GALFLGFLGAAGSTMGA |
| SEQ ID NO. 9 | KETWWETWWTEWSQPKKKRKV |
| SEQ ID NO. 10 | LLIILRRRIRKQAHAHSK |
| SEQ ID NO. 11 | YTAIAWVKAFIRKLRK |
| SEQ ID NO. 12 | IAWVKAFIRKLRKGPLG |
| SEQ ID NO. 13 | MVTVLFRRLRIRRACGPPRVRV |
| SEQ ID NO. 14 | GLWRALWRLLRSLWRLLWRA |
| SEQ ID NO. 15 | RRRRRRR QIKIWFQNRRMKWKKGG |
| SEQ ID NO. 16 | RXRRXRRXRIKILFQNRRMKWKK |
| SEQ ID NO. 17 | RXRRXRRXRIdKILFQNdRRMKWHKB |
| SEQ ID NO. 18 | RXRRXRRXRIHILFQNdRRMKWHKB |
| SEQ ID NO. 19 | RXRRBRRXRILFQYRXRBRXRB |
| SEQ ID NO. 20 | RXRRBRRXRILFQYRXRXRXRB |
| SEQ ID NO. 21 | RXRRXRELFQYRXRRXR |
| SEQ ID NO. 22 | RBRRXRRBRELFQYRBRXRBRB |
| SEQ ID NO. 23 | RBRRXRRBRILFQYRXRBRXRB |
| SEQ ID NO. 24 | RBRRXRRBRELFQYRXRRXRB |
| SEQ ID NO. 25 | RBRRXRRBRILFQYRXRBRXB |
| SEQ ID NO. 26 | RXRRBRRXRELFQYRXRRXRB |
| SEQ ID NO. 27 | RXRRBRRXRILFQYRXRBRXB |
| SEQ ID NO. 28 | RXRRBRRXRYQFLIRXRBRXRB |
| SEQ ID NO. 29 | RXRRBRRXRIQFLIRXRBRXRB |
| SEQ ID NO. 30 | RXRRBRRXRQFLIRXRBRXRB |
| SEQ ID NO. 31 | RXRRBRRXRQFLRXRBRXRB |
| SEQ ID NO. 32 | RXRRBRRXYRFLIRXRBRXRB |
| SEQ ID NO. 33 | RXRRBRRXRFQILYRXRBRXRB |
| SEQ ID NO. 34 | RXRRBRRXYRFRLIXRBRXRB |
| SEQ ID NO. 35 | RXRRBRRXILFRYRXRBRXRB |

(continued)

| SEQ ID NO. | Sequence |
|---|---|
| SEQ ID NO. 36 | Ac-RRLSYSRRRFXBpgG |
| SEQ ID NO. 37 | Ac-RRLSYSRRRFPFVYLIXBpgG |
| Ac = acetyl; Bpg = L-bis-homopropargylglycine = 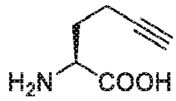 ; B = beta-alanine; X = 6-aminohexanoic acid; dK/dR = corresponding D-amino acid. | |

## EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments described herein may be employed. It is intended that the following claims define the scope of the invention and that methods and structures within the scope of these claims and their equivalents be covered thereby.

Example 1.

Scheme A describes the steps involved for preparing the polyamide, attaching the polyamide to the oligomeric backbone, and then attaching the ligand to the other end of the oligomeric backbone. The second terminus can include any structure in Table 2. The oligomeric backbone can be selected from the various combinations of linkers shown in Table 6. The transcription modulator molecule such as those listed in Table 7 below can be prepared using the synthesis scheme shown below.

Table 6. Examples of oligomeric backbone as represented by $-(T^1-V^1)_a-(T^2-V^2)_b-(T^3-V^3)_c-(T^4-V^4)_d-(T^5-V^5)_e-$

| $T^1$ | $V^1$ | $T^2$ | $V^2$ | $T^3$ | $V^3$ | $T^4$ | $V^4$ | $T^5$ | $V^5$ |
|---|---|---|---|---|---|---|---|---|---|
| $(C_1-C_{12})$ alkylene | $CONR^{1a}$ | $(EA)_w$ | CO | $(PEG)_n$ | $NR^{11}CO$ | ---- | ---- | ---- | ---- |
| $(C_1-C_{12})$ alkylene | $CONR^{1a}$ | $(EA)_w$ | CO | $(PEG)_n$ | O | arylene | $NR^{11}CO$ | ---- | ---- |
| $(C_1-C_{12})$ alkylene | $CONR^{1a}$ | $(EA)_w$ | CO | $(PEG)_n$ | O | Subst. arylene | $NR^{11}CO$ | ---- | ---- |
| $(C_1-C_{12})$ alkylene | $CONR^{1a}$ | $(EA)_w$ | CO | $(PEG)_n$ | O | $NR^{11}C$ O | $(C_1-C_{12})$ alkyl | Subst. arylene | $NR^{11}CO$ |
| $(C_1-C_{12})$ alkylene | $CONR^{1a}$ | $(EA)_w$ | CO | $(C_1-C_{12})$ alkyl | $NR^{11}CO-C_{1-4}$ alkyl | Subst. arylene | $NR^{11}$ | ---- | ---- |
| $(C_1-C_{12})$ alkylene | $CONR^{1a}$ | $(EA)_w$ | CO | $(PEG)_n$ | O | Subst. arylene | ---- | ---- | ---- |
| $(PEG)_n$ | $CONR^{1a}$- $C_{1-4}$ alkyl | ---- | ---- | ---- | ---- | ---- | ---- | ---- | ---- |
| $(EA)_w$ | CO | $(C_1-C_{12}$ alkyl | $CONR^{11}$- $C_{1-4}$ alkyl | ---- | ---- | ---- | ---- | ---- | ---- |
| $(C_1-C_{12})$ alkylene | $CONR^{1a}$ | $(EA)_w$ | CO | $(PEG)_n$ | $NR^{11}CO-C_{1-4}$ alkyl | ---- | ---- | ---- | ---- |
| $(EA)_w$ | CO | $(PEG)_n$ | O | phenyl | $NR^{11}CO-C_{1-4}$ alkyl | ---- | ---- | ---- | ---- |
| $(C_1-C_{12})$ alkylene | $CONR^{1a}$ | $(PEG)_n$ | CO | ---- | ---- | ---- | ---- | ---- | ---- |
| $(C_1-C_{12})$ alkylene | $CONR^{1a}$ | $(EA)_w$ | CO | modifd. $(PEG)_n$ | O | arylene | $NR^{11}CO$ | ---- | ---- |

Table 7. Examples of transcription modulator molecules

| First terminus | Oligomeric backbone | Second terminus |
|---|---|---|
| Im-Im-Nt-Im-Py | 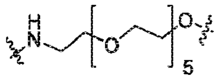 | 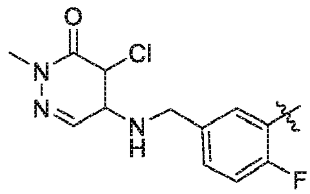 (CBP binder) |
| Im-Im-Nt-Im-Py | 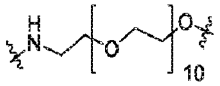 | 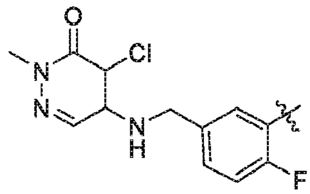 (CBP binder) |
| Im-Im-Nt-Im-Py | 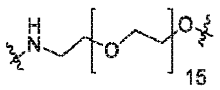 | 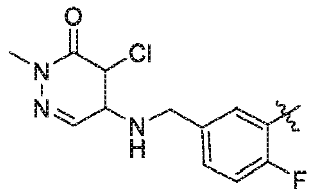 (CBP binder) |
| Im-Im-β-β-gAB-Im-Im-β-Py | 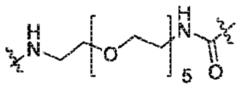 | 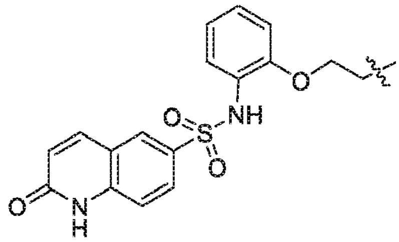 (OGT binder) |
| Im-Im-β-β-gAB-Im-Im -β-Py | 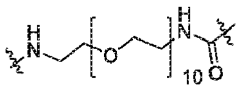 | 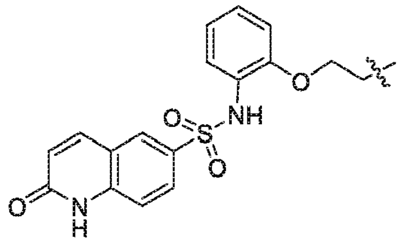 (OGT binder) |
| Im-Im-β-β-gAB-Im-Im-β-Py | 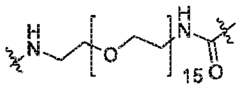 | 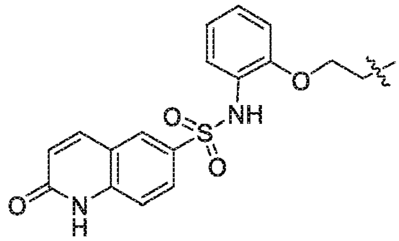 (OGT binder) |

(continued)

| First terminus | Oligomeric backbone | Second terminus |
|---|---|---|
| Im-Im-Im-Py-Py (Linked in the middle - position 3) to Im-Im-Py-Py-Py | 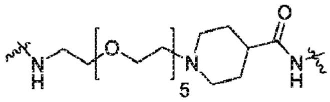 | 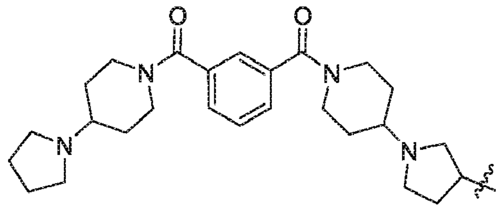 (methyl lysine binder) |
| Im-Im-Im-Py-Py (Linked in the middle - position 3) to Im-Im-Py-Py-Py | 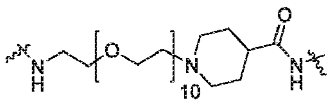 | 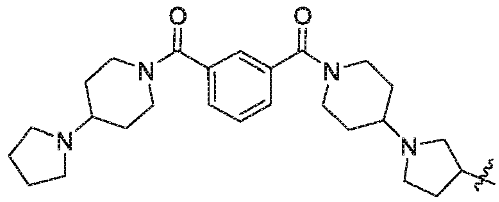 (methyl lysine binder) |
| Im-Im-Im-Py-Py (Linked in the middle - position 3) to Im-Im-Py-Py-Py | 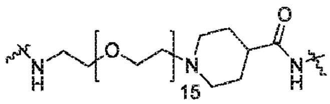 | 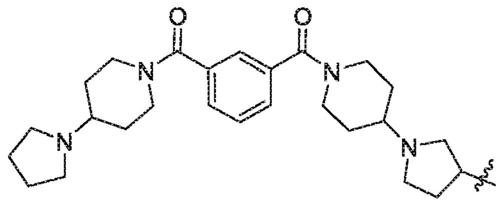 (methyl lysine binder) |
| Im-Im-Im-Py-Py (Linked in the middle - position 3) to Im-Im-Py-Py-Py | 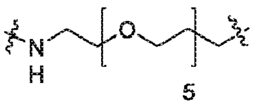 | 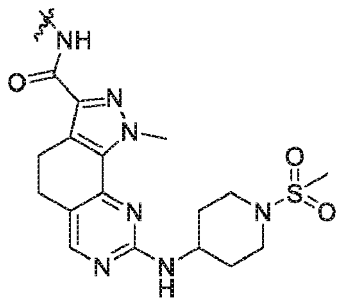 (CDK 1, 2, 5) |
| Im-Im-Im-Py-Py (Linked in the middle - position 3) to Im-Im-Py-Py-Py | 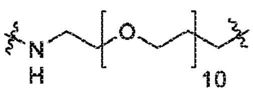 | 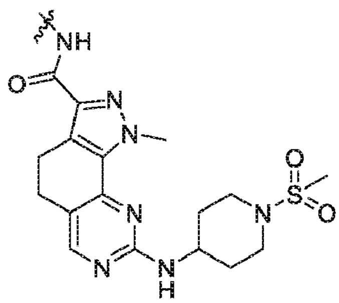 (CDK 1, 2, 5) |

(continued)

| First terminus | Oligomeric backbone | Second terminus |
|---|---|---|
| Im-Im-Im-Py-Py (Linked in the middle - position 3) to Im-Im-Py-Py-Py | 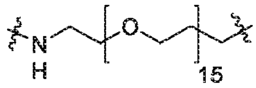 | 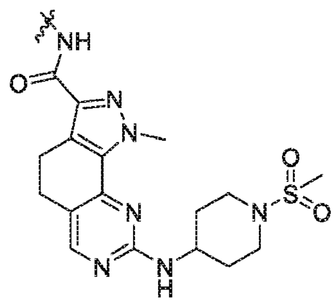 (CDK 1, 2, 5) |
| Im-Im-Im-Py-Py (Linked in the middle - position 3) to Im-Im-Py-Py-Py | 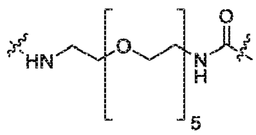 | 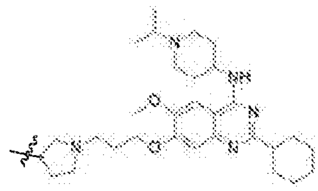 (methyl transferase) |
| Im-Im-Im-Py-Py (Linked in the middle - position 3) to Im-Im-Py-Py-Py | 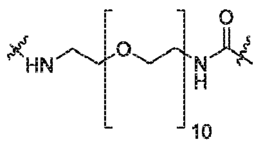 | 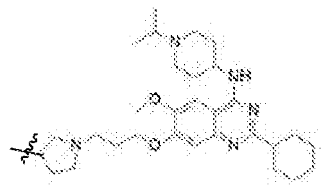 (methyl transferase) |
| Im-Im-Im-Py-Py (Linked in the middle - position 3) to Im-Im-Py-Py-Py | 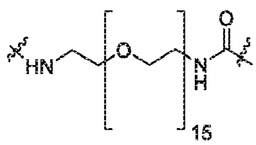 | 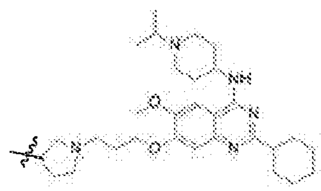 (methyl transferase) |
| Im-Im-Im-Py-Py (Linked in the middle - position 3) to Im-Im-Py-Py-Py | 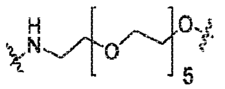 | 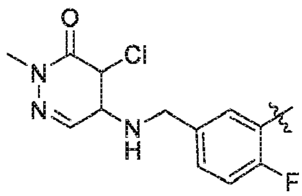 (CBP binder) |
| Im-Im-Im-Py-Py (Linked in the middle - position 3) to Im-Im-Py-Py-Py | 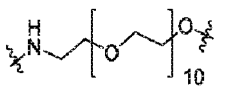 | 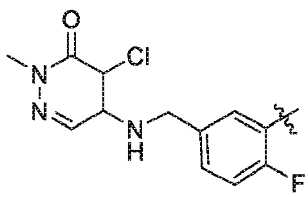 (CBP binder) |
| Im-Im-Im-Py-Py (Linked in the middle - position 3) to Im-Im-Py-Py-Py | 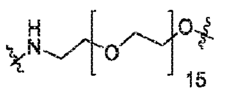 | 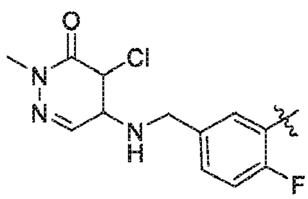 (CBP binder) |

(continued)

| First terminus | Oligomeric backbone | Second terminus |
|---|---|---|
| Im-Im-Im-Py-Py (Linked in the end Py to the end Im) to Im-Im-Py-Py-Py | 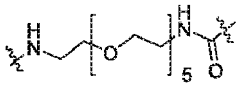 | 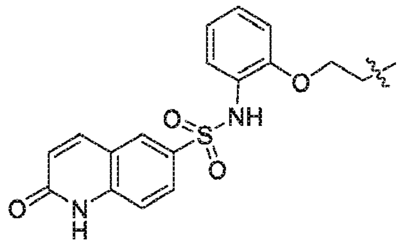 (OGT binder) |
| Im-Im-Im-Py-Py (Linked in the end Py to the end Im) to Im-Im-Py-Py-Py | 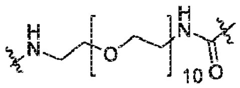 | 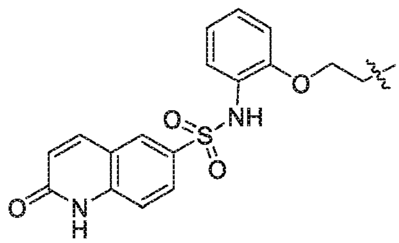 (OGT binder) |
| Im-Im-Im-Py-Py (Linked in the end Py to the end Im) to Im-Im-Py-Py-Py | 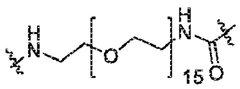 | 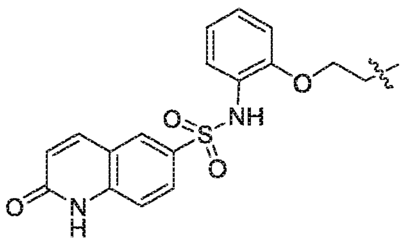 (OGT binder) |
| Im-Im-Im-Py-Py (Linked in the end Py to the end Im) to Im-Im-Py-Py-Py | 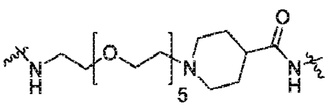 | 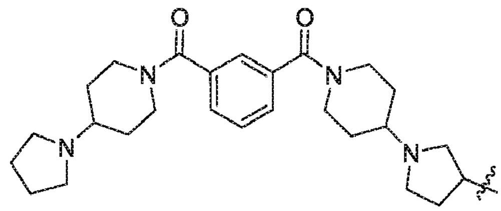 (methyl lysine binder) |
| Im-Im-Im-Py-Py (Linked in the end Py to the end Im) to Im-Im-Py-Py-Py | 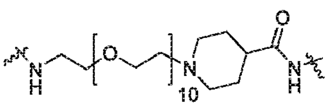 | 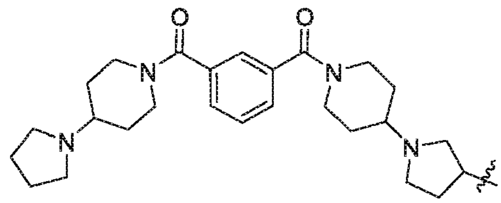 (methyl lysine binder) |
| Im-Im-Im-Py-Py (Linked in the end Py to the end Im) to Im-Im-Py-Py-Py | 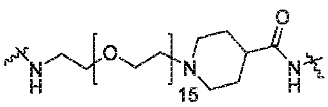 | 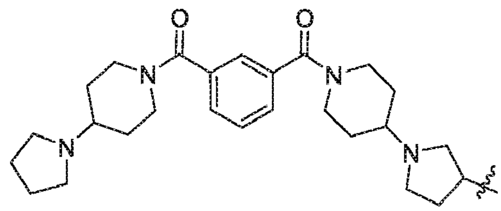 (methyl lysine binder) |

(continued)

| First terminus | Oligomeric backbone | Second terminus |
|---|---|---|
| Im-Im-Im-Py-Py (Linked in the end Py to the end Im) to Im-Im-Py-Py-Py | 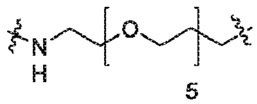 | 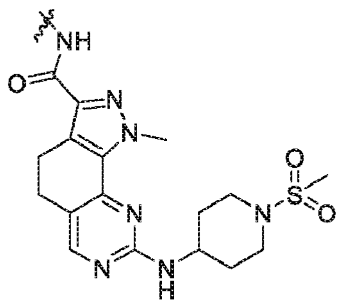 (CDK 1, 2, 5) |
| Im-Im-Im-Py-Py (Linked in the end Py to the end Im) to Im-Im-Py-Py-Py | 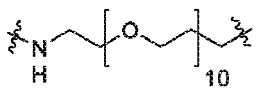 | 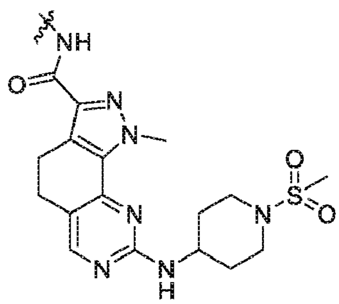 (CDK 1, 2, 5) |
| Im-Im-Im-Py-Py (Linked in the end Py to the end Im) to Im-Im-Py-Py-Py | 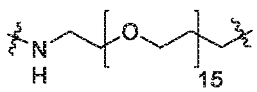 | 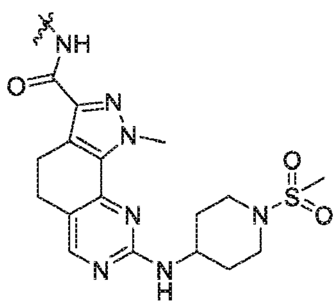 (CDK 1, 2, 5) |
| Im-Im-Im-Py-Py (Linked in the end Py to the end Im) to Im-Im-Py-Py-Py | 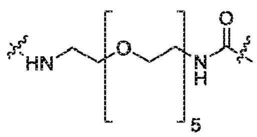 | 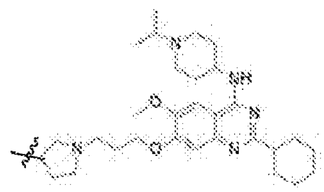 |
| Im-Im-Im-Py-Py (Linked in the end Py to the end Im) to Im-Im-Py-Py-Py | 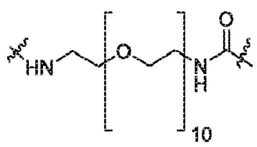 | 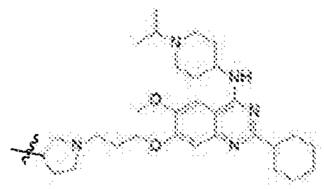 |
| Im-Im-Im-Py-Py (Linked in the end Py to the end Im) to Im-Im-Py-Py-Py | 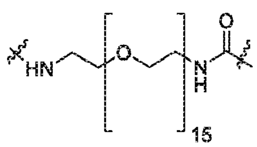 | 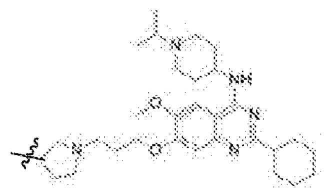 |

Scheme A: Synthesis of first terminus / second terminus / linker conjugate.

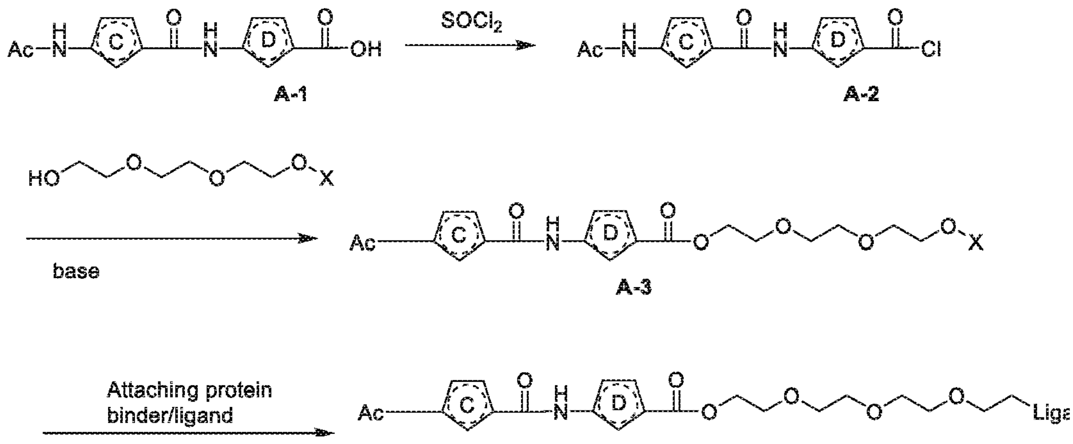


The ligand or protein binder can be attached to the oligomeric backbone using the schemes described below. The oligomeric backbone can be linked to the protein binder at any position on the protein binder that is chemically feasible while not interfering with the binding between the protein binder and the regulatory protein. The protein binder binds to the regulatory protein often through hydrogen bonds, and linking the oligomeric backbone and the regulatory protein should not interfere the hydrogen bond formation. The protein binder is attached to the oligomeric backbone through an amide or ether bond. Scheme B through Scheme D demonstrate several examples of linking the oligomeric backbone and protein binder.

Scheme B. Example for Amide Coupling

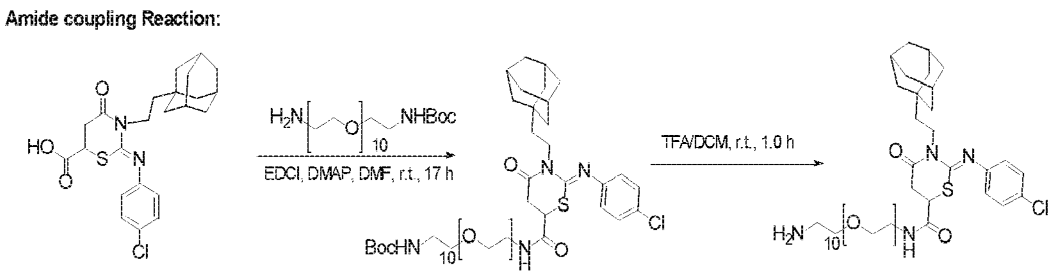


Scheme C. Example for Ether Formation Reaction (O-alkylation reaction)

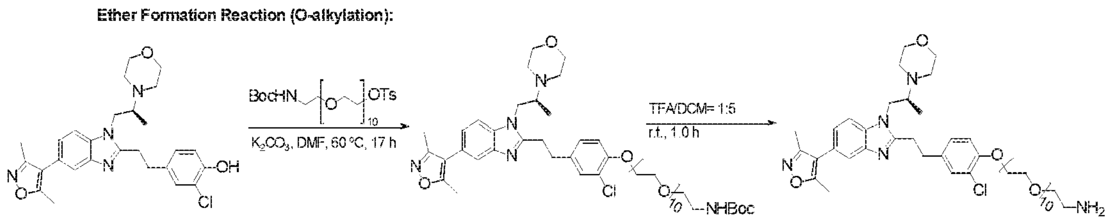

Scheme D. Example for Amine Formation Reaction (N-alkylation reaction)

AmineFormation Reaction (*N*-alkylation):

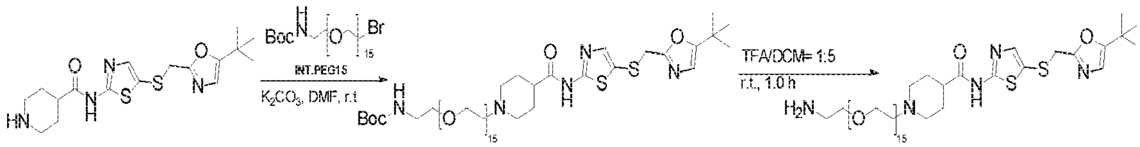


Example 2. Biological Activity Assays

The methods as set forth below will be used to demonstrate the binding of the disclosed compounds and the efficacy in treatment. In general, the assays are directed at evaluating the effect of the disclosed compounds on the level of expression of *c9orf72.*

*Gene expression*

Expression of *c9orf72* will be assayed by techniques known in the field. These assays include, but are not limited to quantitative reverse transcription polymerase chain reaction (RT-PCR), microarray, or multiplexed RNA sequencing (RNA-seq), with the chosen assay measuring either total expression, or the allele specific expression of the *fmr* gene. Exemplary assays are found at: Freeman WM et al., "Quantitative RT-PCR: pitfalls and potential", BioTechniques 1999, 26, 112-125; Dudley AM et al, "Measuring absolute expression with microarrays with a calibrated reference sample and an extended signal intensity range", PNAS USA 2002, 99(11), 7554-7559; Wang Z et al., "RNA-Seq: a revolutionary tool for transcriptomics" Nature Rev. Genetics 2009, 10, 57-63.

Production of the FMRP protein will be assayed by techniques known in the field. These assays include, but are not limited to Western blot assay, with the chosen assay measuring either total protein expression, or allele specific expression of the *fmr* gene.

For use in assay, two tissue models and two animal models are contemplated.

*Disease Model I: Human cell culture*

This model will constitute patient-derived cells, including fibroblasts, induced pluripotent stem cells and cells differentiated from stem cells. Attention will be made in particular to cell types that show impacts of the disease, e.g., neuronal cell types.

*Disease Model II: Murine cell culture*

This model will constitute cell cultures from mice from tissues that are particularly responsible for disease symptoms, which will include fibroblasts, induced pluripotent stem cells and cells differentiated from stem cells and primary cells that show impacts of the disease, e.g., neuronal cell types.

*Disease Model III: Murine*

This model wil constitute mice whose genotypes contain the relevant number of repeats for the disease phenotype - these models should show the expected altered gene expression (e.g., a variation in *c9orf72* expression).

*Disease Model IV: Murine*

This model will constitute mice whose genotypes contain a knock in of the human genetic locus from a diseased patient - these models should show the expected altered gene expression (e.g., increase or decrease in *c9orf72* expression).

All references, patents or applications, U.S. or foreign, cited in the application are hereby incorporated by reference as if written herein in their entireties. Where any inconsistencies arise, material literally disclosed herein controls.

From the foregoing description, one skilled in the art can easily ascertain the essential characteristics of this disclosure, and without departing from the spirit and scope thereof, can make various changes and modifications of the disclosure to adapt it to various usages and conditions.

The following numbered paragraphs set out preferred features and preferred combinations of features of the

present invention:

1. A transcription modulator molecule having a first terminus, a second terminus, and an oligomeric backbone, wherein:

a) the first terminus comprises a DNA-binding moiety capable of noncovalently binding to a nucleotide repeat sequence GGGGCC;
b) the second terminus comprises a protein-binding moiety binding to a regulatory molecule that modulates an expression of a gene comprising the nucleotide repeat sequence GGGGCC; and
c) the oligomeric backbone comprising a linker between the first terminus and the second terminus, with the proviso that the second terminus is not a Brd4 binding moiety.

2. The transcription modulator molecule of para 1, wherein the first terminus comprises a polyamide selected from the group consisting of a linear polyamide, a hairpin polyamide, a H-pin polyamide, an overlapped polyamide, a slipped polyamide, a cyclic polyamide, a tandem polyamide, and an extended polyamide.
3. The transcription modulator molecule of para 1 or 2, wherein the first terminus comprises a linear polyamide.
4. The transcription modulator molecule of para 1 or 2, wherein the first terminus comprises a hairpin polyamide.
5. The transcription modulator molecule of any one of paras 2-4, wherein the polyamide is capable of binding the DNA with an affinity of less than 500 nM.
6. The transcription modulator molecule of any one of paras 1-5, wherein the first terminus comprises -NH-Q-C(O)-, wherein Q is an optionally substituted $C_{6-10}$ arylene, optionally substituted 4-10 membered heterocyclene, optionally substituted 5-10 membered heteroarylene group, or an optionally substituted alkylene group.
7. The transcription modulator molecule of any one of paras 1-6, wherein the first terminus comprises at least three heteroaromatic carboxamide moieties comprising at least one heteroatom selected from O, N, and S, and at least one aliphatic amino acid residue chosen from the group consisting of glycine, β-alanine, γ-aminobutyric acid, 2,4-diaminobutyric acid, and 5-aminovaleric acid.
8. The transcription modulator molecule of para 7, wherein the heteroaromatic carboxamide moiety is a monocyclic or bicyclic moiety.
9. The transcription modulator molecule of para 7, wherein the first terminus comprises one or more carboxamide moieties selected from the group consisting of optionally substituted pyrrole carboxamide monomer, optionally substituted imidazole carboxamide monomer, and β-alanine monomer.
10. The transcription modulator molecule of any one of paras 7-9, wherein the carboxamide moieties are selected based on the pairing principle shown in Table 1A, Table 1B, Table 1C, or Table 1D.
11. The transcription modulator molecule of any one of paras 1-10, wherein the first terminus comprises Im corresponding to the nucleotide G, Py or β corresponding to the nucleotide pair C, Py or β corresponding to the nucleotide pair A, Py, β, or Hp corresponding to the nucleotide T, and wherein Im is N-$C_{1-6}$alkyl imidazole, Py is N-$C_{1-6}$alkyl pyrrole, Hp is 3 -hydroxy N-$C_{1-6}$alkyl pyrrole, and β-alanine.
12. The transcription modulator molecule of any one of paras 1-10, wherein the first terminus comprises Im/Py to correspond to the nucleotide pair G/C, Py/Im to correspond to the nucleotide pair C/O, Py/Py to correspond to the nucleotide pair A/T, Py/Py to correspond to the nucleotide pair T/A, Hp/Py to correspond to the nucleotide pair T/A, and wherein Im is N-$C_{1-6}$alkyl imidazole, Py is N-$C_{1-6}$alkyl pyrrole, and Hp is 3 -hydroxy N-$C_{1-6}$alkyl pyrrole.
13. The transcription modulator molecule of any one of paras 1-12, wherein the first terminus comprises a structure of Formula (A-1):

$$-L_{1a}-[A-M]_p-E_1 \qquad (A-1)$$

wherein:

each [A-M] appears p times and p is an integer in the range of 1 to 10;
$L_{1a}$ is a bond, a $C_{1-6}$ alkylene, -$NR^a$-$C_{1-6}$ alkylene-C(O)-, -NRC(O)-, -$NR^a$-$C_{1-6}$ alkylene, -O-, or -O-$C_{1-6}$ alkylene;
each A is selected from the group consisting of a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, -$C_{1-10}$ alkylene-$NR^a$-, —CO—, -$NR^a$-,-$CONR^a$-,-$CONR^aC_{1-4}$alkylene-, -$NR^aCO$-$C_{1-4}$alkylene-, -C(O)O-, -O-, -S-, - C(=S)-NH-, -C(O)-NH-NH-, -C(O)-N=N-, -C(O)-CH=CH-, $(CH_2)_{0-4}$-CH=CH-$(CH_2)_{0-4}$, -N($CH_3$)-$C_{1-6}$ alkylene,

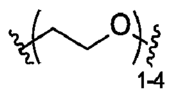
,

-NH-$C_{1-6}$ alkylene-NH-, -O-$C_{1-6}$ alkylene-O-, -NH-N=N-, - NH-C(O)-NH-, and any combinations thereof, and at least one A is -CONH-;

each M is an optionally substituted $C_{6-10}$ arylene group, optionally substituted 4-10 membered heterocyclene, optionally substituted 5-10 membered heteroarylene group, or an optionally substituted alkylene;

$E_1$ is H or -$A^E$-G;

$A^E$ is absent or -NHCO-;

G is selected from the group consisting of optionally substituted H, $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)$C_{1-5}$alkylene-$NR^aR^b$, $C_{0-4}$ alkylene-NHC(=NH)$R^a$, -CO-halogen, and optionally substituted amine; and

each $R^a$ and $R^b$ are independently selected from the group consisting of H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, and optionally substituted 5-10 membered heteroaryl.

14. The transcription modulator molecule of any one of paras 1-12, wherein the first terminus comprises a structure of Formula (A-2):

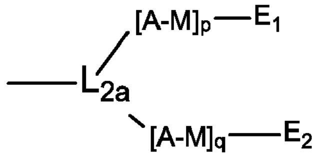

Formula (A-2),

wherein:

$L_{2a}$ is a linker selected from -$C_{1-12}$ alkylene-$CR^a$, -CH, N, -$C_{1-6}$ alkylene-N, -C(O)N, -$NR^a$-$C_{1-6}$ alkylene-CH, -O-$C_{0-6}$ alkylene-CH, ,

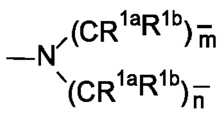

, or,

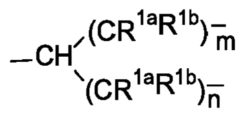

,

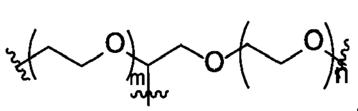

,

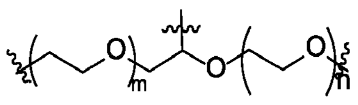

;

each p and q are independently an integer in the range of 1 to 10;

each m and n are independently an integer in the range of 0 to 10;

each A is independently selected from a bond, $C_{1-10}$ alkylene, -$C_{1-10}$ alkylene-C(O)-, -$C_{1-10}$ alkylene-$NR^a$-, -CO-, -$NR^a$-, -$CONR^a$-,-$CONR^aC_{1-4}$alkylene-, -$NR^aCO$-$C_{1-4}$ alkylene-, -C(O)O-, -O-, -S-, -C(=S)-NH-, -C(O)-NH-NH-, -C(O)-N=N-, or -C(O)-CH=CH-, and at least one A is -CONH-;

each M is independently an optionally substituted $C_{6-10}$ arylene group, optionally substituted 4-10 membered heterocyclene, optionally substituted 5-10 membered heteroarylene group, or an optionally substituted alkylene;

each $E_1$ and $E_2$ are independently H or -$A^E$-G,

each $A^E$ is independently absent or NHCO,

G is selected from the group consisting of H, $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)$C_{1-5}$alkylene-$NR^aR^b$, $C_{0-4}$ alkylene-NHC(=NH) $R^a$, -CO-halogen, and optionally substituted amine; and

each $R^a$ and $R^b$ are independently selected from the group consisting of H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, and an optionally substituted 5-10 membered heteroaryl; and

each $R^{1a}$ and $R^{1b}$ is independently H, or $C_{1-6}$ alkyl.

15. The transcription modulator molecule of para 14, wherein integers p and q are $2 \leqq p+q \leqq 20$.

16. The transcription modulator molecule of para 14 or 15, wherein $L_{2a}$ is-$C_{2-8}$ alkylene-CH,

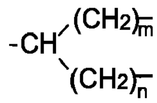

or

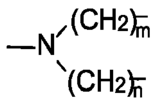

and wherein each m and n is independently an integer in the range of 0 to 10.

17. The transcription modulator molecule of any one of paras 1-12, wherein the first terminus comprises a structure of Formula (A-3):

$$-L_{1a}-[A-M]_{p1}-L_{3a}-[M-A]_{q1}-E_1 \qquad \text{(A-3)}$$

wherein:

$L_{1a}$ is a bond, a $C_{1-6}$ alkylene, -NH-$C_{0-6}$ alkylene-C(O)-, -N($CH_3$)-$C_{0-6}$ alkylene, or -O-$C_{0-6}$ alkylene;
$L_{3a}$ is a bond, $C_{1-6}$ alkylene, -NH-$C_{0-6}$ alkylene-C(O)-, -N($CH_3$)-$C_{0-6}$ alkylene, -O-$C_{0-6}$ alkylene, -$(CH_2)_a$-$NR^a$-$(CH_2)_b$-, -$(CH_2)_a$-, -$(CH_2)_a$-O-$(CH_2)_b$-, -$(CH_2)_a$-CH($NHR^a$)-, -$(CH_2)_a$-CH($NHR^a$)-, -$(CR^{1a}R^{1b})_a$-, or -$(CH_2)_a$-CH($NR^aR^b$)-$(CH_2)_b$-;
each a and b are independently an integer between 2 and 4;
each $R^a$ and $R^b$ are independently selected from H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, and an optionally substituted 5-10 membered heteroaryl;
each $R^{1a}$ and $R^{1b}$ is independently H, halogen, OH, NHAc, or $C_{1-4}$ alkyl;
each [A-M] appears $p^1$ times and $p^1$ is an integer in the range of 1 to 10;
each [M-A] appears $q^1$ times and $q^1$ is an integer in the range of 1 to 10;
each A is selected from a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, -$C_{1-10}$ alkylene-$NR^a$-, -CO-, -$NR^a$-, -$CONR^a$-,-$CONR^aC_{1-4}$alkylene-, -$NR^aCO$-$C_{1-4}$alkylene-, -C(O)O-, -O-, -8--, -C(=S)-NH-, -C(O)-NH-NH-, —C(O)-N=N—, —C(O)-CH=CH—, $(CH_2)_{0-4}$-CH=CH-$(CH_2)_{0-4}$, -N($CH_3$)-$C_{1-6}$ alkylene,

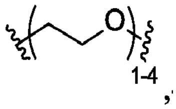

-NH-$C_{1-6}$ alkylene-NH-, -O- $C_{1-6}$ alkylene-O-, -NH-N=N-, -NH-C(O)-NH-, and any combinations thereof, and at least one A is -CONH-;
each M in each [A-M] and [M-A] unit is independently an optionally substituted $C_{6-10}$ arylene group, optionally substituted 4-10 membered heterocyclene, optionally substituted 5-10 membered heteroarylene group, or an optionally substituted alkylene; and
$E_1$ is selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR_2$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine.

18. The transcription modulator molecule of any one of paras 13 to 17, when M is a 10 membered bicyclic aryle or heteroaryl ring, at least one A adjacent to M is a bond.

19. The transcription modulator molecule of 18, wherein M is anthracene or benzimidazole.

20. The transcription modulator molecule of any one of paras 13 to 17, wherein one A is a 4-10 membered heterocyclyl or 5-10 membered heteraryl having at least one nitrogen, optioanlly substituted by one or more groups selected from oxo and $C_{1-6}$ alkyl.

21. The transcription modulator molecule of any one of paras 13 to 17, wherein at least one A is a triazole or a 4-10 membered heterocyclyl having a cyclic amide or cyclic amine.

22. The transcription modulator molecule of any one of paras 13 to 17, wherein integers $p^1$ and $q^1$ are $2 \leqq p^1+q^1 \leqq 20$.

23. The transcription modulator molecule of any one of paras 1-12, wherein the first terminus comprises a structure of Formula (A-4a) or (A-4b):

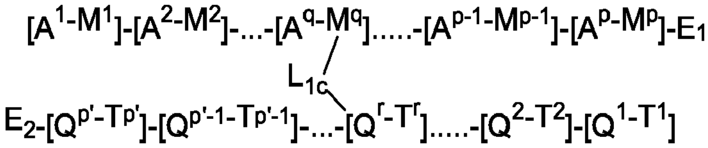

Formula (A-4a)

Or

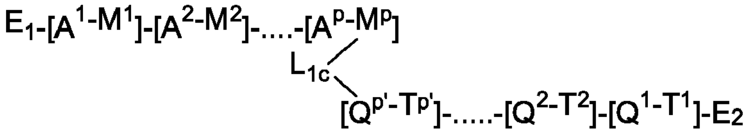

Formula (A-4b)

wherein:

$L_{1c}$ is a bivalent or trivalent group selected from

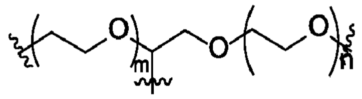

,

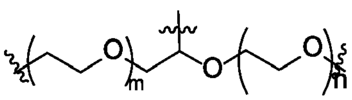

, 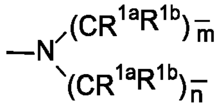

, 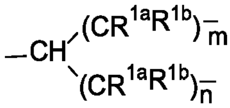

,

a $C_{1-10}$ alkylene, -NH-$C_{0-6}$ alkylene-C(O)-, -N($CH_3$)-$C_{0-6}$ alkylene, and

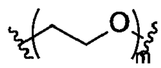

;

p is an integer in the range of 2 to 10;
p' is an integer in the range of 2 to 10;

$$2 \leqq q \leqq (p-1);$$

$$2 \leqq r \leqq (p-1);$$

m and n are each independently an integer in the range of 0 to 10;
each $A^2$ through $A^p$ is independently selected from the group consisting of a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, - $C_{1-10}$alkylene-$NR^a$-, -CO-, -$NR^a$-, -CON$R^a$-,-CON$R^a$$C_{1-4}$alkylene-, -$NR^a$CO-$C_{1-4}$alkylene-, -C(O)O-, -O-, -S-, -C(=S)-NH-, -C(O)-NH-NH-, -C(O)-N=N-,-C(O)-CH=CH-, $(CH_2)_{0-4}$-CH=CH-$(CH_2)_{0-4}$, -N($CH_3$)-$C_{1-6}$ alkylene, and

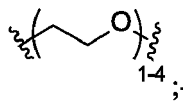

;

-NH-$C_{1-6}$ alkylene-NH-, -O- $C_{1-6}$ alkylene-O-, -NH-N=N-, -NH-C(O)-NH-, and any combinations thereof, and at least one of $A^2$ through $A^p$ is -CONH-;

each $M^1$ through $M^p$ is an optionally substituted $C_{6-10}$ arylene group, optionally substituted 4-10 membered heterocyclene, optionally substituted 5-10 membered heteroarylene group, or an optionally substituted alkylene;

each $T^2$ through $T^{p'}$ in formula (A-4a) is independently selected from the group consisting of a bond, $C_{1-10}$ alkylene, optionally substituted $C_{6-10}$ arylene group, optionally substituted 4-10 membered heterocyclene, optionally substituted 5-10 membered heteroarylene group, -$C_{1-10}$ alkylene-C(O)-, -$C_{1-10}$ alkylene-$NR^a$-, -CO-, -$NR^a$-, -CON$R^a$-,-CON$R^a$$C_{1-4}$alkylene-, - $NR^a$CO-$C_{1-4}$alkylene-, -C(O)O-, -O-, -S-, —C(=S)-NH—, -C(O)-NH-NH-, -C(O)-N=N—, —C(O)-CH=CH—, $(CH2)_{0-4}$-CH=CH-$(CH_2)_{0-4}$, -N($CH_3$)-$C_{1-6}$ alkylene, and

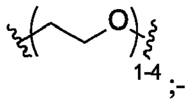

;-

NH- $C_{1-6}$ alkylene-NH-, -O- $C_{1-6}$ alkylene-O-, -NH-N=N-, -NH-C(O)-NH-, and any combinations thereof, and at least one of $T^2$ through $T^p$ is -CONH-;

each $Q^1$ to $Q^{p'}$ is an optionally substituted $C_{6-10}$ arylene group, optionally substituted 4-10 membered heterocyclene, optionally substituted 5-10 membered heteroarylene group, or an optionally substituted alkylene;

each $A^1$, $T^1$, $E_1$, and $E_2$ are independently H or -$A^E$-G,

each $A^E$ is independently absent or NHCO,

each G is independently selected from the group consisting of optionally substituted H, $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;

when $L_{1c}$ is a trivalent group, the oligomeric backbone is attached to the first terminus through $L_{1c}$, when $L_{1c}$ is a bivalent group, the oligomeric backbone is attached to the first terminus through one of $A^1$, $T^1$, $E_1$, and $E_2$, or the oligomeric backbone is attached to the first terminus through a nitrogen or carbon atom on one of $M^1$, $M^2$, ...$M^{p-1}$, $M^p$, $T^1$, $T^2$, ... and $T^{p'}$, and

each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl;

each $R^{1a}$ and $R^{1b}$ are independently H or an optionally substituted $C_{1-6}$ alkyl.

24. The transcription modulator molecule of para 23, wherein when one of $M^1$ through $M^p$ or $M^1$ through $M^p$ is a 10 membered bicyclic aryl or heteroaryl ring, the adjacent A or T is a bond.

25. The transcription modulator molecule of para 23 or 24, wherein $L_{1c}$ is

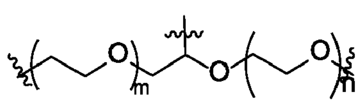

or

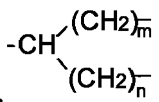

,

$C_{1-10}$ alkylene, or

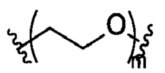

.

26. The transcription modulator molecule of any one of paras 23 to 25, wherein $L_{1c}$ is

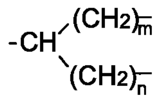

,

and wherein 2≦m+n≦10.

27. The transcription modulator molecule of 26, wherein 3≦m+n≦7.

28. The transcription modulator molecule of 23, wherein $L_{1c}$ is $C_{3-8}$ alkylene.

29. The transcription modulator molecule of any one of paras 23 to 28, wherein $M^q$ is a five membered heteroaryl ring comprising at least one nitrogen; $Q^q$ is a five membered heteroaryl ring comprising at least one nitrogen; $L_{1c}$ is linked to the nitrogen atom on $M^q$ and $L_{1c}$ is linked to the nitrogen atom on $Q^q$.

30. The transcription modulator molecule of any one of paras 23 to 29, wherein each $M^1$ through $M^p$ is independently selected from an optionally substituted pyrrolylene, an optionally substituted imidazolylene, an optionally substituted pyrazolylene, an optionally substituted thioazolylene, an optionally substituted diazolylene, an optionally substituted benzopyridazinylene, an optionally substituted benzopyrazinylene, an optionally substituted phenylene, an optionally substituted pyridinylene, an optionally substituted thiophenylene, an optionally substituted furanylene, an optionally substituted piperidinylene, an optionally substituted pyrimidinylene, an optionally substituted anthracenylene, an optionally substituted quinolinylene, and an optionally substituted $C_{1-6}$ alkylene.

31. The transcription modulator molecule of any one of paras 23 to 30, wherein each $Q^1$ to $Q^{p'}$ is independently selected from an optionally substituted pyrrolylene, an optionally substituted imidazolylene, an optionally substituted pyrazolylene, an optionally substituted thioazolylene, an optionally substituted diazolylene, an optionally substituted benzopyridazinylene, an optionally substituted benzopyrazinylene, an optionally substituted phenylene, an optionally substituted pyridinylene, an optionally substituted thiophenylene, an optionally substituted furanylene, an optionally substituted piperidinylene, an optionally substituted pyrimidinylene, an optionally substituted anthracenylene, an optionally substituted quinolinylene, and an optionally substituted $C_{1-6}$ alkylene.

32. The transcription modulator molecule of any one of paras 23 to 31, wherein each $A^2$ through $A^p$ is independently selected from a bond, $C_{1-10}$ alkylene, optionally substituted phenylene, optionally substituted thiophenylene, optionally substituted furanylene, optionally substituted triazole, a 4-10 membered heterocyclyl having a cyclic amide, -$C_{1-10}$ alkylene-C(O)-, -$C_{1-10}$ alkylene-NH-, -CO-, - $NR^a$-, -$CONR^a$-,-$CONR^aC_{1-4}$alkylene-, -$NR^aCO$-$C_{1-4}$alkylene-, -C(O)O-, -O-, -S-, - C(=S)-NH-, -C(O)-NH-NH-, -C(O)-N=N-, -C(O)-CH=CH-, -CH=CH-, -NH-N=N-, -NH-C(O)-NH-, -$N(CH_3)$-$C_{1-6}$ alkylene, and

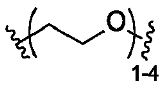

;

-NH- $C_{1-6}$ alkylene-NH-, -O-$C_{1-6}$ alkylene-O-, and any combinations thereof.

33. The transcription modulator molecule of any one of paras 23 to 32, wherein each $T^2$ through $T^{p'}$ is independently selected from a bond, $C_{1-10}$ alkylene, optionally substituted phenylene, optionally substituted thiophenylene, optionally substituted furanylene, optionally substituted triazole, a 4-10 membered heterocyclyl having a cyclic amide, -$C_{1-10}$ alkylene-C(O)-, -$C_{1-10}$ alkylene-NH-, -CO-, - $NR^a$-, -$CONR^a$-,-$CONR^aC_{1-4}$alkylene-, -$NR^aCO$-$C_{1-4}$alkylene-, -C(O)O-, -O-, -8--, - C(=S)-NH-, -C(O)-NH-NH-, -C(O)-N=N-, -C(O)-CH=CH-, -CH=CH-, -NH-N=N-, -NH-C(O)-NH-, -N(CH3)-$C_{1-6}$ alkylene, and

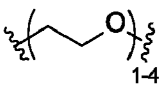

;

-NH- $C_{1-6}$ alkylene-NH-, -O-$C_{1-6}$ alkylene-O-, and any combinations thereof.

34. The transcription modulator molecule of any one of paras 23 to 33, wherein each G is an end group independently selected from the group consisting of optionally substituted $C_{6-10}$ aryl, optionally substituted 4-10 membered heterocyclyl, a 5-10 membered heteroaryl optionally substituted with 1-3 substituents selected from $C_{1-6}$ alkyl, -NHCOH, halogen, -$NR^aR^b$, , an optionally substituted $C_{1-6}$ alkyl, $C_{0-4}$ alkylene-NHC(=NH)NH, $C_{0-4}$ alkylene-NHC(=NH)-$R_E$, -$C_{1-4}$ alkylene-, -CN, -$C_{0-4}$alkylene-CH(=NH)($NR^aR^b$), -$C_{0-4}$alkylene-CH(=$N^+H_2$)($NR^aR^b$)$C_{1-5}$ alkylene-$NR^aR^b$, $C_{0-4}$ alkylene-NHC(=NH) $R^a$, - CO-halogen, and optionally substituted amine.

35. The transcription modulator molecule of any one of paras 23 to 34, wherein each G is independently selected from

$C_{1-4}alkylNHC(NH)NH_2$,

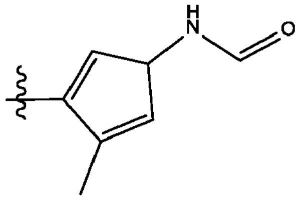,

$-CH(=NH)(NH_2)$,

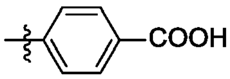, 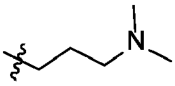,-CN, 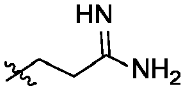, 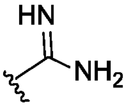, 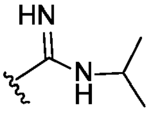, 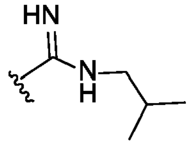,

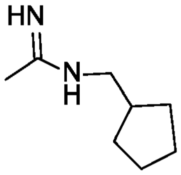, 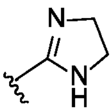, 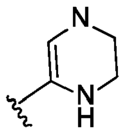, 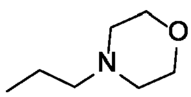, 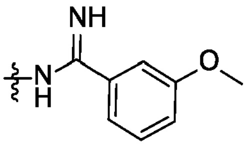, 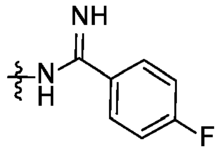,

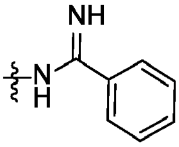, 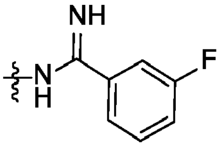, 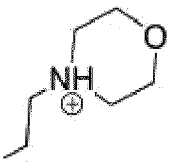, 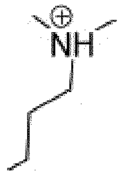, 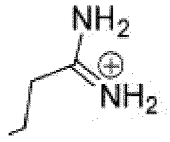, , 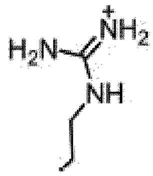,

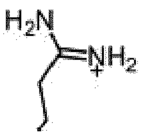, 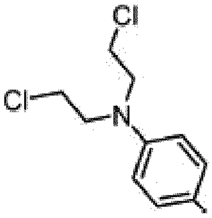, 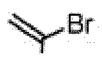, 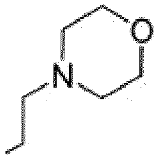, 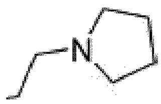, 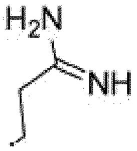,

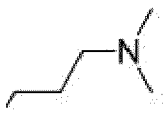, 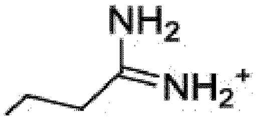, 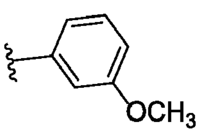, 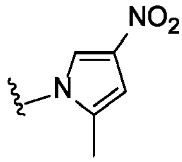, 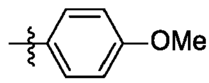,

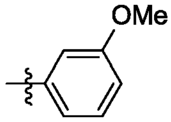, 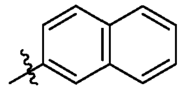, 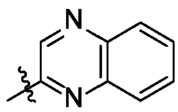, 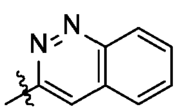, 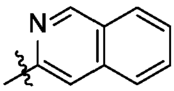, 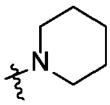, 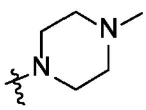,

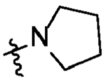, 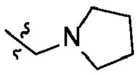, and 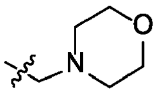.

36. The transcription modulator molecule of any one of paras 13-15, wherein each $E_1$ independently comprises an optionally substituted thiophene-containing moiety, optionally substituted pyrrole containing moiety, optionally substituted imidazole containing moiety, or optionally substituted amine.
37. The transcription modulator molecule of para 14, wherein each $E_2$ independently comprises an optionally substituted thiophene-containing moiety, optionally substituted pyrrole containing moiety, optionally substituted imidazole containing moiety, or optionally substituted amine.
38. The transcription modulator molecule of para 18 or 37, wherein each $E_1$ and $E_2$ are independently selected from the group consisting of optionally substituted N-methylpyrrole, optionally substituted N-methylimidazole, optionally substituted benzimidazole moiety, and optionally substituted 3-(dimethylamino)propanamidyl.
39. The transcription modulator molecule of para 38, wherein each $E_1$ and $E_2$ independently comprises thiophene, benzothiophene, C-C linked benzimidazole/thiophene-containing moiety, or C-C linked hydroxybenzimidazole/thiophene-containing moiety.
40. The transcription modulator of para 38 or 39, wherein each $E_1$ or $E_2$ are independently selected from the group consisting of isophthalic acid; phthalic acid; terephthalic acid; morpholine; N,N-dimethylbenzamide; N,N-bis(trifluoromethyl)benzamide; fluorobenzene; (trifluoromethyl)benzene; nitrobenzene; phenyl acetate; phenyl 2,2,2-trifluoroacetate; phenyl dihydrogen phosphate; 2H-pyran; 2H-thiopyran; benzoic acid; isonicotinic acid; and nicotinic acid; wherein one, two, or three ring members in any of the end-group candidates can be independently substituted with C, N, S or O; and where any one, two, three, four or five of the hydrogens bound to the ring can be substituted with $R^{3a}$ , wherein $R_5$ may be independently selected from H, OH, halogen, $C_{1-10}$ alkyl, $NO_2$, $NH_2$, $C_{1-10}$ haloalkyl, -$OC_{1-10}$ haloalkyl, COOH, and $CONR^{1c}R^{1d}$; wherein each $R^{1c}$ and $R^{1d}$ are independently H, $C_{1-10}$ alkyl, $C_{1-10}$ haloalkyl, or -$C_{1-10}$ alkoxyl.
41. The transcription modulator molecule of para any one of paras 1-12, wherein the first terminus comprises the structure of Formula (A-5a) or Formula (A-Sb);

$A^{1a}$-NH-$Q^1$-C(O)-NH-$Q^2$-C(O)-NH-$Q^3$-C(O)... -NH-$Q^{p-1}$C(O)-NH-C(O)NH-G (Formula A-5a)

or

$T^{1a}$-C(O)-$Q^1$-NH-C(O)-$Q^2$NH-C(O)-$Q^3$-NH-... -C(O)-$Q^{p-1}$NH-C(O)-$Q^p$-NHC(O)-G (Formula A-5b)

wherein:

each $Q^1$, $Q^2$, $Q^3$... through $Q^p$ are independently an optionally substituted $C_{6-10}$ arylene group, optionally substituted 4-10 membered heterocyclene, optionally substituted 5-10 membered heteroarylene group, or an optionally substituted alkylene;
each $A^{1a}$ and $T^{1a}$ are independently a H, bond, a -$C_{1-6}$ alkylene-, -NH-$C_{0-6}$ alkylene-C(O)-, - N($CH_3$)-$C_{0-6}$ alkylene, -C(O)-, -C(O)-$C_{1-10}$alkylene, and -O-$C_{0-6}$ alkylene, optionally substituted $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene-$NR^aR^b$, $C_{0-4}$ alkylene-NHC(=NH) $R^a$, -CO-halogen, and optionally substituted amine;
p is an integer between 2 and 10; and
G is selected from the group consisting of an optionally substituted $C_{6-10}$ aryl, optionally substituted 4-10 membered heterocyclyl, optionally substituted 5-10 membered heteroaryl, or an optionally substituted alkyl, $C_{0-4}$ alkylene-NHC(=NH)NH, -CN, -$C_{0-4}$alkylene-CH(=NH)($NR^aR^b$), - $C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;
each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl; and
wherein the first terminus is connected to the oligomeric backbone through either $A^1$ or $T^1$, or through a nitrogen or carbon atom on one of $Q^1$ through $Q^p$.

42. The transcription modulator molecule of para any one of paras 1-12, wherein the first terminus comprises the structure of Formula (A-5c) or (A-5d):

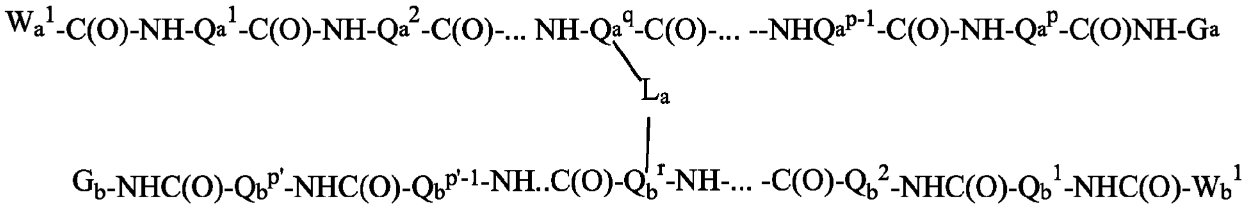

(A-5c)

or

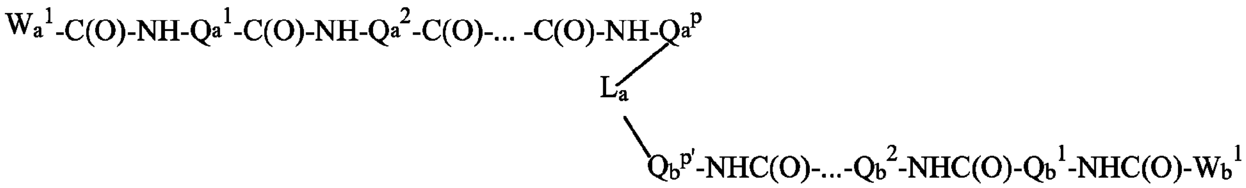

(A-5d)

wherein:

each $Q_a^1$, $Q_a^2$...$Q_a^q$...through $Q_a^p$ are independently an optionally substituted $C_{6-10}$ arylene group, optionally substituted 4-10 membered heterocyclene, optionally substituted 5-10 membered heteroarylene group, or an optionally substituted alkylene;

each $Q_b^1$,$Q_b^2$...$Q_b^r$....through $Q_b^{p'}$ are independently an optionally substituted $C_{6-10}$ arylene group, optionally substituted 4-10 membered heterocyclene, optionally substituted 5-10 membered heteroarylene group, or an optionally substituted alkylene;

p and p' are independently an integer between 3 and 10;

$$2 \leqq q \leqq (p-1);$$

$$2 \leqq r \leqq (p-1);$$

L, is selected from a divalent or trivalent group selected from the group consisting of

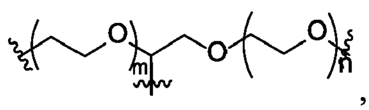,
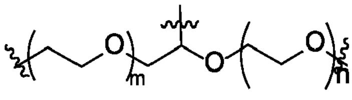,
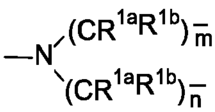,

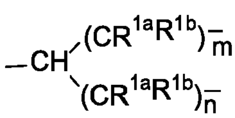,

a $C_{1-10}$ alkylene, -NH-$C_{0-6}$ alkylene-C(O)-, -N($CH_3$)-$C_{0-6}$ alkylene, and

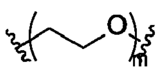;

each m and n are independently an integer in the range of 1 to 10;

n is an integer in the range of 1 to 10;

each $R^{1a}$ and $R^{1b}$ are independently H, or $C_{1-6}$ alkyl;

each $W_a{}^1$, $G_a$, $G_b$, and $W_b{}^1$ are end groups independently selected from the group consisting of optionally substituted H, $C_{6-10}$ aryl, optionally substituted 4-10 membered heterocyclyl, optionally substituted 5-10 membered heteroaryl, an optionally substituted $C_{1-6}$ alkyl, $C_{0-4}$ alkylene-NHC(=NH)NH, -CN, -$C_{0-4}$alkylene-CH(=NH)($NR^aR^b$), -$C_{0-4}$alkylene-CH(=$N^+H_2$)($NR^aR^b$)C1-5alkylene- $NR^aR^b$, $C_{0-4}$ alkylene-NHC(=NH) $R^a$, -CO-halogen, and optionally substituted amine;

when $L_a$ is a trivalent group, the oligomeric backbone is attached to the first terminus through L,; and when L, is a divalent group, the oligomeric backbone is attached to the first terminus through one of $W_a{}^1$, F., $E_b$, and $W_b{}^1$, or the oligomeric backbone is attached to the first terminus through a nitrogen or carbon atom on one of $Q_a{}^1$, $Q_a{}^2$, ... $Q_a{}^{p-1}$, $Q_a{}^p$, $Q_b{}^1$, $Q_a{}^2$, ... $Q_b{}^{p'-1}$, and $Q_b{}^{p'}$; and

each $R^a$ and $R^b$ are independently H, an optionally substituted $C_{1-6}$ alkyl, an optionally substituted $C_{3-10}$ cycloalkyl, optionally substituted $C_{6-10}$ aryl, optionally substituted 4-10 membered heterocyclyl, or an optionally substituted 5-10 membered heteroaryl.

43. The transcription modulator molecule of para 42, wherein L, is

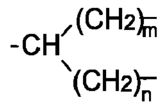

or a $C_{2-8}$ alkylene.

44. The transcription modulator molecule of para any one of paras 1-41, wherein the first terminus comprises at least one $C_{3-5}$ achiral aliphatic or heteroaliphatic amino acid.

45. The transcription modulator molecule of para 44, wherein the first terminus comprises one or more subunits selected from the group consisting of optionally substituted pyrrole, optionally substituted imidazole, optionally substituted thiophene, optionally substituted furan, optionally substituted beta-alanine, γ-aminobutyric acid, (2-aminoethoxy)-propanoic acid, 3((2-aminoethyl)(2-oxo-2-phenyl-1λ²-ethyl)amino)-propanoic acid, and dimethylaminopropylamide monomer.

46. The transcription modulator molecule of any one of paras 1-12, wherein the first terminus comprises a polyamide having the structure of Formula (A-6):

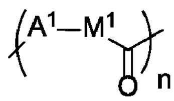

(A-6)

wherein:

each $A^1$ is -NH- or -NH-$(CH_2)_m$-$CH_2$-C(O)-NH-;

each $M^1$ is an optionally substituted $C_{6-10}$ arylene group, optionally substituted 4-10 membered heterocyclene, optionally substituted 5-10 membered heteroarylene group, or optionally substituted alkylene;

m is an integer between 1 to 10; and

n is an integer between 1 and 6.

47. The transcription modulator molecule as recited in any one of paras 1-12 and 46, wherein the first terminus has the structure of Formula (A-7):

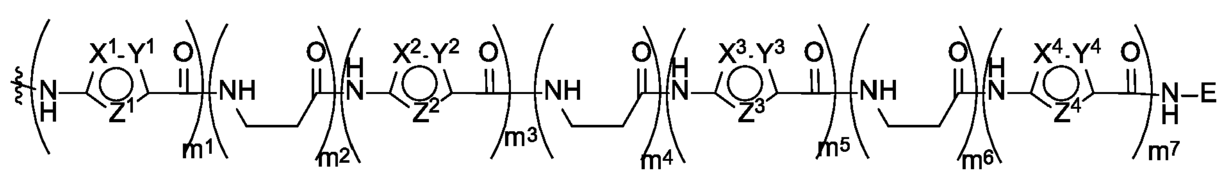

(A-7),

or a salt thereof, wherein:

E is an end subunit which comprises a moiety chosen from a heterocyclic group or a straight chain aliphatic group, which is chemically linked to its single neighbor;
$X^1$, $Y^1$, and $Z^1$ in each $m^1$ unit are independently selected from $CR^4$, N, $NR^5$, O, or S;
$X^2$, $Y^2$, and $Z^2$ in each $m^3$ unit are independently selected from $CR^4$, N, $NR^5$, O, or S;
$X^3$, $Y^3$, and $Z^3$ in each $m^5$ unit are independently selected from $CR^4$, N, $NR^5$, O, or S;
$X^4$, $Y^4$, and $Z^4$ in each $m^7$ unit are independently selected from $CR^4$, N, $NR^5$, O, or S;
each $R^4$ is independently H, -OH, halogen, $C_{1-6}$ alkyl, or $C_{1-6}$ alkoxyl;
each $R^5$ is independently H, $C_{1-6}$ alkyl, or $C_{1-6}$ alkylamine;
each $m^1$, $m^3$, $m^5$ and $m^7$ are independently an integer between 0 and 5;
each $m^2$, $m^4$ and $m^6$ are independently an integer between 0 and 3; and $m^1 + m^2 + m^3 + m^4 + m^5 + m^6 + m^7$ is between 3 and 15.

48. The transcription modulator molecule as recited in any one of paras 1-12 and 46, wherein the first terminus has the structure of Formula (A-8):

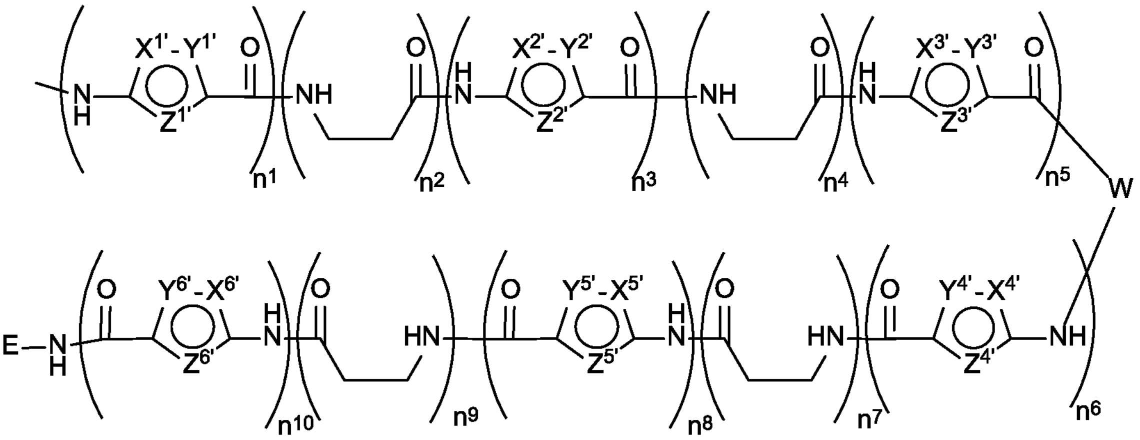

(A-8),

or a salt thereof, wherein:

E is an end subunit which comprises a moiety chosen from a heterocyclic group or a straight chain aliphatic group, which is chemically linked to its single neighbor;
W is $C_{1-6}$ alkylene,

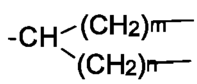 , or 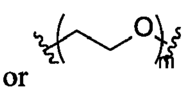 ;

$X^{1'}$, $Y^{1'}$, and $Z^{1'}$ in each $n^1$ unit are independently selected from $CR^4$, N, $NR^5$, O, or S;
$X^{2'}$, $Y^{2'}$, and $Z^{2'}$ in each $n^3$ unit are independently selected from $CR^4$, N, $NR^5$, O, or S;
$X^{3'}$, $Y^{3'}$, and $Z^{3'}$ in each $n^5$ unit are independently selected from $CR^4$, N, $NR^5$, O, or S;
$X^{4'}$, $Y^{4'}$, and $Z^{4'}$ in each $n^6$ unit are independently selected from $CR^4$, N, $NR^5$, O, or S;
$X^{5'}$, $Y^{5'}$, and $Z^{5'}$ in each $n^8$ unit are independently selected from $CR^4$, N, $NR^5$, O, or S;
$X^{6'}$, $Y^{6'}$, and $Z^{6'}$ in each $n^{10}$ unit are independently selected from $CR^4$, N, $NR^5$, O, or S;
each $R^4$ is independently H, -OH, halogen, $C_{1-6}$ alkyl, $C_{1-6}$ alkoxyl;
each $R^5$ is independently H, $C_{1-6}$ alkyl or $C_{1-6}$alkylamine;
n is an integer between 1 and 5;
each $n^1$, $n^3$, $n^5$, $n^6$, $n^8$ and $n^{10}$ are independently an integer between 0 and 5;
each $n^2$, $n^4$, $n^7$ and $n^9$ are independently an integer between 0 and 3, and
$n^1 + n^2 + n^3 + n^4 + n^5 + n^6 + n^7 + n^8 + n^9 + n^{10}$ is between 3 and 15.

49. The transcription modulator molecule as recited in any one of paras 1-12 and 46, wherein the first terminus has the structure of Formula (A-9):

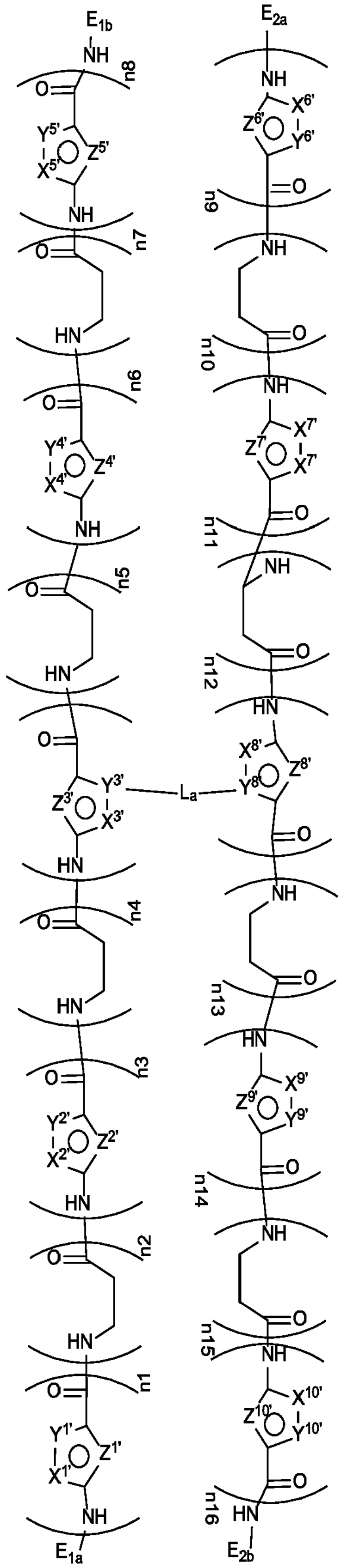

(A-9),

or a salt thereof, wherein:

$X^{1'}$, $Y^{1'}$, and $Z^{1'}$ in each $n^1$ unit are independently selected from $CR^4$, N, $NR^5$, O, or S;
$X^{2'}$, $Y^2$, and $Z^{2'}$ in each $n^3$ unit are independently selected from $CR^4$, N, $NR^5$, O, or S;
$X^{3'}$, $Y^{3'}$, and $Z^{3'}$ are independently selected from $CR^4$, N, $NR^5$, O, or S;
$X^{4'}$, $Y^{4'}$, and $Z^{4'}$ in each $n^6$ unit are independently selected from $CR^4$, N, $NR^5$, O, or S;
$X^{5'}$, $Y^{5'}$, and $Z^{5'}$ in each $n^8$ unit are independently selected from $CR^4$, N, $NR^5$, O, or S;
$X^{6'}$, $Y^{6'}$, and $Z^{6'}$ in each $n^9$ unit are independently selected from $CR^4$, N, $NR^5$, O, or S;
$X^{7'}$, $Y^{7'}$, and $Z^{7'}$ in each $n^{11}$ unit are independently selected from $CR^4$, N, $NR^5$, O, or S;
$X^{8'}$, $Y^{8'}$, and $Z^{8'}$ are independently selected from $CR^4$, N, $NR^5$, O, or S;
$X^{9'}$, $Y^{9'}$, and $Z^{9'}$ in each $n^{14}$ unit are independently selected from $CR^4$, N, $NR^5$, O, or S;
$X^{10'}$, $Y^{10'}$, and $Z^{10'}$ in each $n^{16}$ unit are independently selected from $CR^4$, N, $NR^5$, O, or S;
each $R^4$ is independently H, -OH, halogen, $C_{1-6}$ alkyl, $C_{1-6}$ alkoxyl;
each $R^5$ is independently H, $C_{1-6}$ alkyl or $C_{1-6}$alkylamine;
each $n^1$, $n^3$, $n^6$, $n^8$, $n^9$, $n^{11}$, $n^{14}$, and $n^{16}$ are independently an integer between 0 and 5;
each $n^2$, $n^4$, $n^5$, $n^7$, $n^{10}$, $n^{13}$, and $n^{13}$ are independently an integer between 0 and 3,
$n^1 + n^2 + n^3 + n^4 + n^5 + n^6 + n^7 + n^8 + n' + n^{10} + n^{11} + n^{12} + n^{13} + n^{14} + n^{15} + n^{16}$ is between 3 and 18
or a salt thereof, wherein:

$L_a$ is selected from a divalent or trivalent group selected from the group consisting of

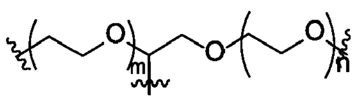,
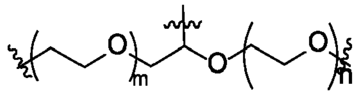,
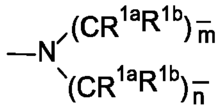,

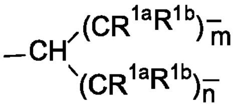,

a $C_{1-10}$ alkylene, -NH-$C_{0-6}$ alkylene-C(O)-, -N($CH_3$)-$C_{0-6}$ alkylene, and

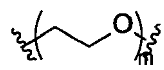;

each $R^{1a}$ and $R^{1b}$ are independently H, or an $C_{1-6}$ alkyl;
each m and n are independently an integer between 1 and 10;
each $E_{1a}$, $E_{2a}$, $E_{1b}$, and $E_{2b}$ are end groups independently selected from the group consisting of optionally substituted $C_{6-10}$ aryl, optionally substituted 4-10 membered heterocyclyl, optionally substituted 5-10 membered heteroaryl, an optionally substituted $C_{1-6}$ alkyl, and optionally substituted amine;

when L, is a trivalent group, the oligomeric backbone is attached to the first terminus through L,;
when L, is a divalent group, the oligomeric backbone is attached to the first terminus through one of $E_{1a}$, $E_{2a}$, $E_{1b}$, and $E_{2b,}$ or the oligomeric backbone is attached to the first terminus through a nitrogen or carbon atom on one of five-membered heteroaryl rings.

50. The transcription modulator molecule of any one of paras 1-12 and 46, wherein the first terminus comprises a polyamide having the structure of Formula (A-10):

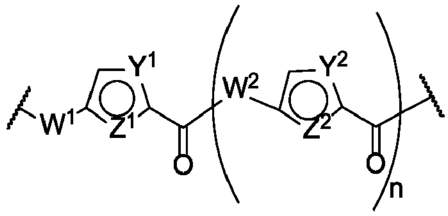
(A-10),

wherein:

each $Y^1$, $Y^2$, $Z^1$, and $Z^2$ are independently $CR^4$, N, $NR^5$, O, or S;
each $R^4$ is independently H, -OH, halogen, $C_{1-6}$ alkyl, or $C_{1-6}$ alkoxyl;
each $R^5$ is independently H, $C_{1-6}$ alkyl, or $C_{1-6}$ alkylamine;
each $W^1$ and $W^2$ are independently a bond, NH, $C_{1-6}$ alkylene, -NH-$C_{1-6}$ alkylene, -NH-5-10 membered heteroarylene, -NH-5-10 membered heterocyclene, -N($CH_3$)-$C_{0-6}$ alkylene, -C(O)-$C_{1-10}$ alkylene, or -O-$C_{0-6}$ alkylene; and
n is an integer between 2 and 11.

51. The transcription modulator molecule of any one of paras 47-50, wherein $R^4$ is selected from the group consisting of H, COH, Cl, NO, N-acetyl, benzyl, $C_{1-6}$ alkyl, $C_{1-6}$ alkoxyl, $C_{1-6}$ alkenyl, $C_{1-6}$ alkynyl , $C_{1-6}$ alkylamine, -C(O)NH-$(CH_2)_{1-4}$-C(O)NH -$(CH_2)_{1-4}$-$NR^aR^b$; and each $R^a$ and $R^b$ are independently hydrogen or $C_{1-6}$ alkyl.
52. The transcription modulator molecule of any one of paras 47-50, wherein $R^5$ is independently selected from the group consisting of H, $C_{1-6}$ alkyl, and $C_{1-6}$ alkyl$NH_2$, preferably H, methyl, or isopropyl.
53. The transcription modulator molecule of any one of paras 1-52, wherein the first terminus comprises a polyamide having one or more subunits independently selected from

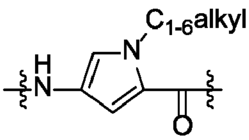
(Py),
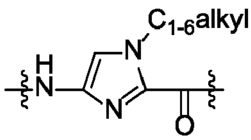
(Im),
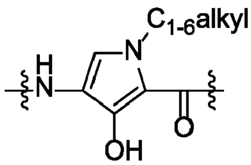
(Hp),
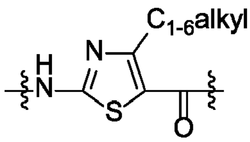
(Th),
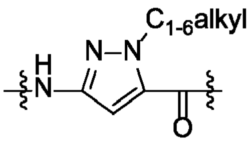
(Pz),
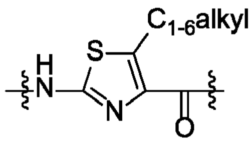
(Nt),
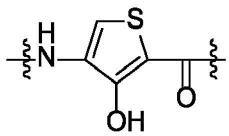
(Ht),
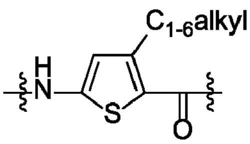
(Tn),
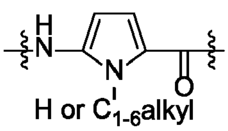
(Nh),
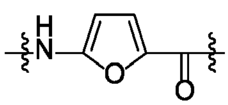
(Fr),
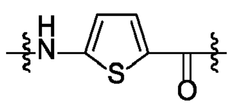
(Tp),
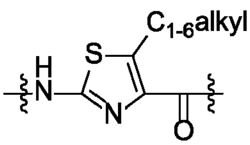
(iNt),
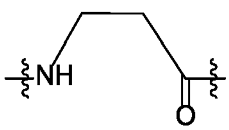
(β),
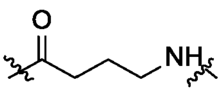
(gAB),
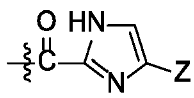

(PyT), 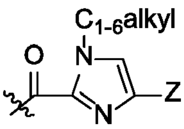 (ImT), 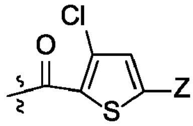 (CTh), 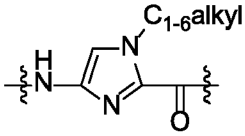 (iIm), 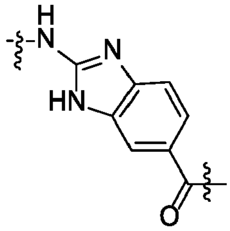

(Bi), 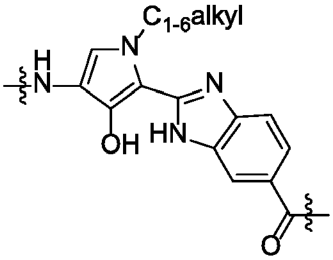 (HpBi), 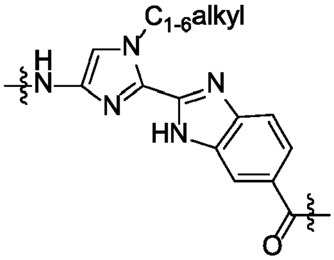 (ImBi),

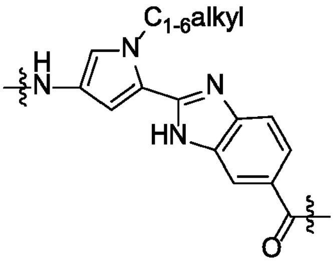 (PyBi), 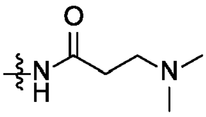 ("Dp"), 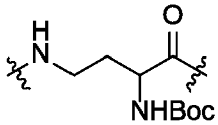, 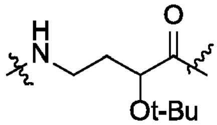,

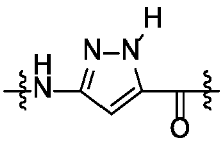, 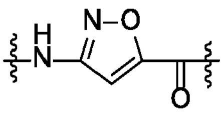, 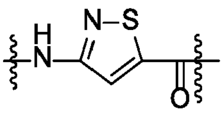,

-NH-benzopyrazinylene-CO-, -NH-phenylene-CO-, -NH-pyridinylene-CO-, -NH-piperidinylene-CO-, -NH-pyrimidinylene-CO-, -NH-anthracenylene-CO-, -NH-quinolinylene-CO-, and

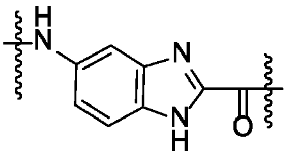,

wherein Z is H, $NH_2$, $C_{1-6}$ alkyl, $C_{1-6}$ haloalkyl or $C_{1-6}$ alkyl-$NH_2$.

54. The transcription modulator molecule of para 53, wherein Py is

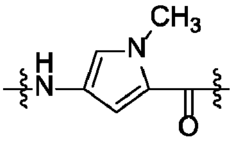,

Im is

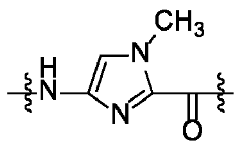

,

Hp is

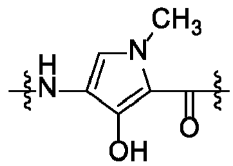

,

Th is

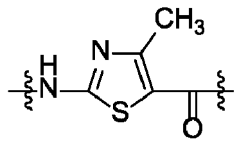

,

Pz is

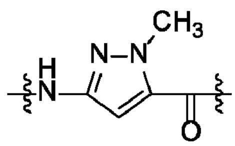

,

Nt is

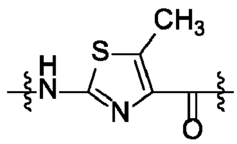

,

Tn is

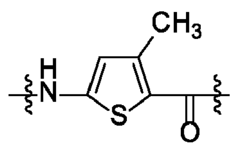

,

Nh is

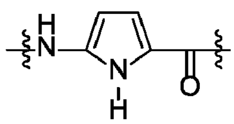

,

iNt is

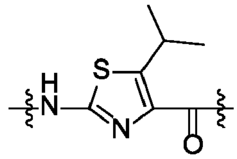

,

iIm is

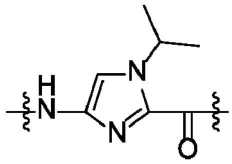

,

HpBi is

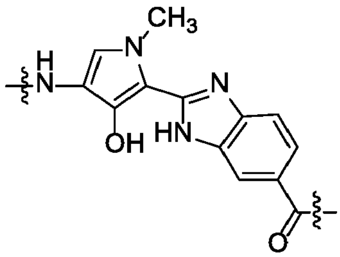

,

ImBi is

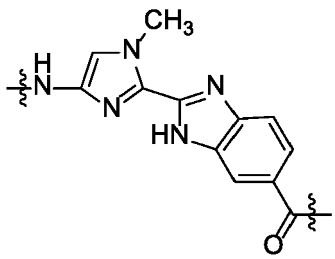

,

PyBi is

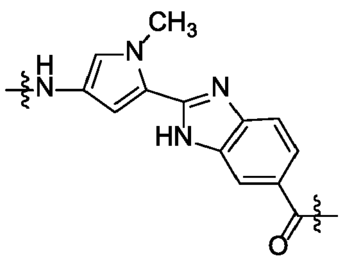

,

Dp is

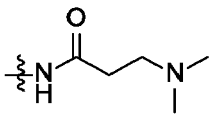,

-NH-benzopyrazinylene-CO- is

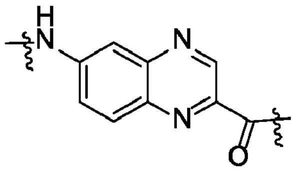,

-NH-phenylene-CO- is

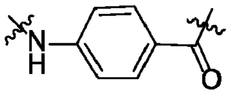,

-NH-pyridinylene-CO- is

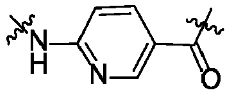,

-NH-piperidinylene-CO- is

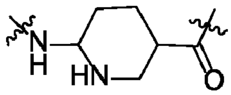,

-NH-pyrazinylene-CO- is

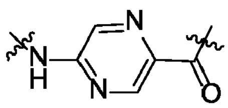,

-NH-anthracenylene-CO- is

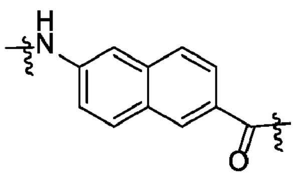,

and -NH-quinolinylene-CO- is

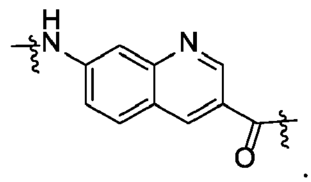

55. The transcription modulator molecule of para 53, wherein the first terminus comprises one or more subunits selected from the group consisting of optionally substituted N-methylpyrrole, optionally substituted N-methylimidazole, and β-alanine (β).

56. The transcription modulator molecule of any one of paras 1-55, wherein the first terminus does not have thestructure of

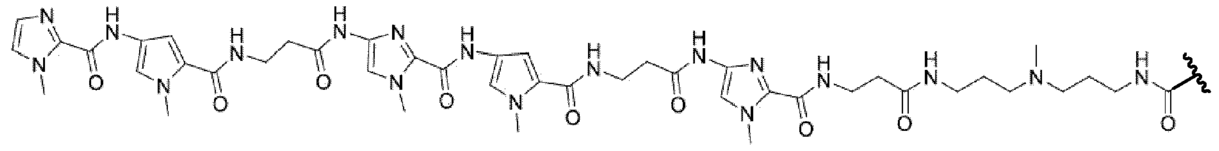

57. The transcription modulator molecule of any one of paras 1-56, wherein the linker has a length of less than about 50 Angstroms.

58. The transcription modulator molecule of any one of paras 1-57, wherein the linker has a length of about 20 to 30 Angstroms.

59. The transcription modulator molecule of any one of paras 1-58, wherein the linker comprises between 5 and 50 chain atoms.

60. The transcription modulator molecule of any one of paras 1-59, wherein the linker comprises a multimer having from 2 to 50 spacing moieties, and wherein the spacing moiety is independently selected from the group consisting of $-((CR^{3a}R^{3b})_x-O)_y-$, $-((CR^{3a}R^{3b})_x-NR^{4a})_y-$, $-((CR^{3a}R^{3b})_x-CH=CH-(CR^{3a}R3^{b})_x-O)_y-$, optionally substituted $-C_{1-12}$ alkyl, optionally substituted $C_{2-10}$ alkenyl, optionally substituted $C_{2-10}$ alkynyl, optionally substituted $C_{6-10}$ arylene, optionally substituted $C_{3-7}$ cycloalkylene, optionally substituted 5- to 10-membered heteroarylene, optionally substituted 4- to 10-membered heterocycloalkylene, an amino acid residue, -O-, $-C(O)NR^{4a}-$, $-NR^{4a}C(O)-$, -C(O)-, $-NR^{4a}-$,-C(O)O-,-O-, -S-, -S(O)-, $-SO_2-$, $-SO_2NR^{4a}-$, $-NR^{4a}SO_2-$, and -P(O)OH-, and any combinations thereof, wherein

each x is independently 2-4;
each y is independently 1-10;
each $R^{3a}$ and $R^{3b}$ are independently selected from hydrogen, optionally substituted alkyl, optionally substituted alkenyl, optionally substituted alkynyl, optionally substituted alkoxy, optionally substituted amino, carboxyl, carboxyl ester, acyl, acyloxy, acyl amino, amino acyl, optionally substituted alkylamide, sulfonyl, optionally substituted thioalkoxy, optionally substituted aryl, optionally substituted heteroaryl, optionally substituted cycloalkyl, and optionally substituted heterocyclyl; and
each $R^{4a}$ is independently a hydrogen or an optionally substituted $C_{1-6}$ alkyl.

61. The transcription modulator molecule of any one of paras 1-60, wherein the oligomeric backbone comprises $-(T^1-V^1)_a-(T^2-V^2)_b-(T^3-V^3)_c-(T^4-V^4)_d-(T^5-V^5)_e-$,

wherein a, b, c, d and e are each independently 0 or 1, and where the sum of a, b, c, d and e is 1 to 5;
$T^1$, $T^2$, $T^3$, $T^4$ and $T^5$ are each independently selected from an optionally substituted $(C_1-C_{12})$ alkylene, optionally substituted alkenylene, optionally substituted alkynylene, $(EA)_w$, $(EDA)_m$, $(PEG)_n$, (modified PEG)$_n$, $(AA)_p$, $-(CR^{2a}OH)_h-$, optionally substituted $(C_6-C_{10})$ arylene, optionally substituted $C_{3-7}$ cycloalkylene, optionally substituted 5- to 10 membered heteroarylene, optionally substituted 4- to 10-membered heterocycloalkylene, a disulfide, a hydrazine, a carbohydrate, a beta-lactam, and an ester;
each m, p, and w are independently an integer from 1 to 20;
n is an integer from 1 to 30;
h is an integer from 1 to 12;
EA has the following structure:

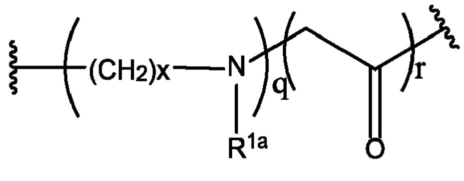

;

EDA has the following structure:

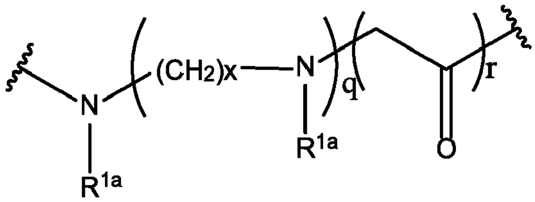

;

wherein each q is independently an integer from 1 to 6;
each x is independently an integer from 2 to 4 and
each r is independently 0 or 1;

$(PEG)_n$ has the structure of $-(CR^{2a}R^{2b}-CR^{2a}R^{2b}-O)_n-CR^{2a}R^{2b}-$; (modified PEG)$_n$ has the structure of replacing at least one $-(CR^{2a}R^{2b}-CR^{2a}R^{2b}-O)-$ in $(PEG)_n$ with $-(CH_2-CR^{2a}=CR^{2a}-CH_2-O)-$ or $-(CR^{2a}R^{2b}-CR^{2a}R^{2b}-S)-$;
AA is an amino acid residue;
$V^1$, $V^2$, $V^3$, $V^4$ and $V^5$ are each independently selected from the group consisting of a bond, - CO-, $-NR^{1a}-$, $-CONR^{1a}-$, $-NR^{1a}-CO-$, $-CONR^{1a}C_{1-4}$ alkyl-, $-NR^{1a}CO-C_{1-4}$ alkyl-, -C(O)O-, -OC(O)-, - O-, -S-, -S(O)-, $-SO_2-$, $-SO_2NR^{1a}-$, $-NR^{1a}SO_2-$ and -P(O)OH-;
each $R^{1a}$ is independently hydrogen or and optionally substituted $C_{1-6}$ alkyl; and each $R^{2a}$ and $R^{2b}$ are independently selected from hydrogen, alkyl, substituted alkyl, alkenyl, substituted alkenyl, alkynyl, substituted alkynyl, halogen, alkoxy, substituted alkoxy, amino, substituted amino, carboxyl, carboxyl ester, acyl, acyloxy, acyl amino, amino acyl, alkylamide, substituted alkylamide, sulfonyl, thioalkoxy, substituted thioalkoxy, aryl, substituted aryl, heteroaryl, substituted heteroaryl, cycloalkyl, substituted cycloalkyl, heterocyclyl, and substituted heterocyclyl.

62. The transcription modulator molecule of para 61, wherein $T^1$, $T^2$, $T^3$, $T^4$, and $T^5$ are each independently selected from $(C_1-C_{12})$alkyl, substituted $(C_1-C_{12})$alkyl, $(EA)_w$, $(EDA)_m$, $(PEG)_n$, (modified PEG)$_n$, $(AA)_p$, $-(CR^{2a}OH)_h-$, an optionally substituted phenyl, piperidin-4-amino (P4A), piperidine-3-amino, piperazine, pyrrolidin-3-amino, azetidine-3-amino, para-amino-benzyloxycarbonyl (PABC), meta, amino-benzyloxycarbonyl (MABC), para-amino-benzyloxy (PABO), meta-amino-benzyloxy (MABO), para-aminobenzyl, an acetal group, a disulfide, a hydrazine, a carbohydrate, a beta-lactam, an ester, $(AA)_p$-MABC-$(AA)_p$, $(AA)_p$-MABO-$(AA)_p$, $(AA)_p$-PABO-$(AA)_p$ and $(AA)_p$-PABC-$(AA)_p$.
63. The transcription modulator molecule of para 62, wherein piperidin-4-amino (P4A) is

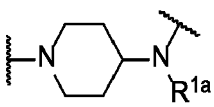

,

wherein $R^{1a}$ is H or $C_{1-6}$ alkyl.
64. The transcription modulator molecule of para 61, wherein $T^1$, $T^2$, $T^3$, $T^4$ and $T^5$ are each independently selected from $(C_1-C_{12})$alkyl, substituted $(C_1-C_{12})$alkyl, $(EA)_w$, $(EDA)_m$, $(PEG)_n$, (modified PEG)$_n$, $(AA)_p$,$-(CR^{2a}OH)_h-$, optionally substituted $(C_6-C_{10})$ arylene, 4-10 membered heterocycloalkene, and optionally substituted 5-10 membered heteroarylene.
65. The transcription modulator molecule of para 61, wherein $T^4$ or $T^5$ is an optionally substituted $(C_6-C_{10})$ arylene.
66. The transcription modulator molecule of para 61, wherein $T^4$ or $T^5$ is an optionally substituted phenylene.
67. The transcription modulator molecule of para 1, wherein $T^1$, $T^2$, $T^3$, $T^4$ and $T^5$; and $V^1$, $V^2$, $V^3$, $V^4$ and $V^5$ are selected from the following Table:

| $T^1$ | $V^1$ | $T^2$ | $V^2$ | $T^3$ | $V^3$ | $T^4$ | $V^4$ | $T^5$ | $V^5$ |
|---|---|---|---|---|---|---|---|---|---|
| $(C_1-C_{12})$ alkylene | $CONR^{1a}$ | $(EA)_w$ | CO | $(PEG)_n$ | $NR^{11}CO$ | ---- | ---- | ----- | ---- |
| $(C_1-C_{12})$ alkylene | $CONR^{1a}$ | $(EA)_w$ | CO | $(PEG)_n$ | O | arylene | $NR^{11}CO$ | ---- | ---- |
| $(C_1-C_{12})$ alkylene | $CONR^{1a}$ | $(EA)_w$ | CO | $(PEG)_n$ | O | Subst arylene | $NR^{11}CO$ | ---- | ---- |
| $(C_1-C_{12})$ alkylene | $CONR^{1a}$ | $(EA)_w$ | CO | $(PEG)_n$ | O | $NR^{11}C$ O | $(C_1-C_{12})$ alkyl | Subst. arylene | $NR^{11}CO$ |
| $(C_1-C_{12})$ alkylene | $CONR^{1a}$ | $(EA)_w$ | CO | $(C_1-C_{12})$ alkyl | $NR^{11}CO-C_{1-4}$ alkyl | Subst arylene | $NR^{11}$ | ---- | ---- |
| $(C_1-C_{12})$ alkylene | $CONR^{1a}$ | $(EA)_w$ | CO | $(PEG)_n$ | O | Subst arylene | --- | ---- | ---- |
| $(PEG)_n$ | $CONR^{1a}$- $C_{1-4}$ alkyl | ---- | ---- | ---- | ---- | ---- | ---- | ---- | --- |
| $(EA)_w$ | CO | $(C_1-C_{12})$ alkyl | $CONR^{11}$- $C_{1-4}$ alkyl | ---- | ---- | ---- | ---- | ---- | --- |
| $(C_1-C_{12})$ alkylene | $CONR^{1a}$ | $(EA)_w$ | CO | $(PEG)_n$ | $NR^{11}CO-C_{1-4}$ alkyl | ---- | ---- | ---- | ---- |
| $(EA)_w$ | CO | $(PEG)_n$ | O | phenyl | $NR^{11}CO-C_{1-4}$ alkyl | ---- | ---- | ---- | ---- |
| $(C_1-C_{12})$ alkylene | $CONR^{1a}$ | $(PEG)_n$ | CO | ---- | ---- | ---- | ---- | ---- | ---- |
| $(C_1-C_{12})$ alkylene | $CONR^{1a}$ | $(EA)_w$ | CO | modifd. $(PEG)_n$ | O | arylene | $NR^{11}CO$ | ---- | ---- |

wherein $R^{1a}$ is H or $C_{1-6}$ alkyl, and n is an integer between 1 and 15.

68. The transcription modulator molecule of any one of paras 1-67, wherein the linker comprises

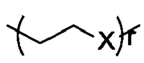 , or 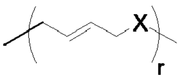 ;

or any combination thereof, wherein r is an integer between 1 and 10, preferably between 3 and 7; X is O, S, or $NR^{1a}$; and $R^{1a}$ is H or $C_{1-6}$ alkyl.

69. The transcription modulator molecule of any one of paras 1-68, wherein the linker comprises

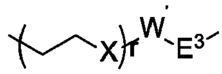 ,

wherein at least one $-(CH_2-CH_2-O)-$ is replaced with $-((CR^{1a}R^{1b})_x-CH=CH-(CR^{1a}R^{1b})_x-O)-$, or any combinations thereof, wherein W' is absent, $(CH_2)_{1-5}$, $-(CH_2)_{1-5}O$, $(CH_2)_{1-5}-C(O)NH-(CH_2)_{1-5}-O$, $(CH_2)_{1-5}-C(O)NH-(CH_2)_{1-5}$, $-(CH_2)_{1-5}NHC(O)-(CH_2)_{1-5}-O$, or $-(CH_2)_{1-5}-NHC(O)-(CH_2)_{1-5}-$; $E^3$ is an optionally substituted $C_{6-10}$ arylene group, optionally substituted 4-10 membered heterocycloalkylene, or optionally substituted 5-10 membered heteroarylene; X is O, S, or N; each $R^{1a}$ and $R^{1b}$ are independently H or $C_{1-6}$ alkyl; r is an integer between 1 and 10; and x is an integer between 1 and 15.

70. The transcription modulator molecule of para 69, wherein $E^3$ is a phenylene or substituted phenylene.

71. The transcription modulator molecule of para 69, wherein the linker comprises

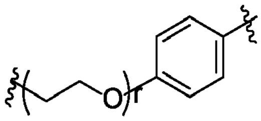 ; 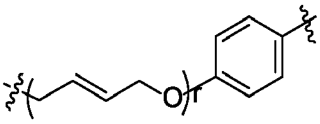 ; 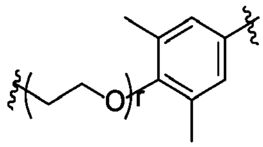 ; or 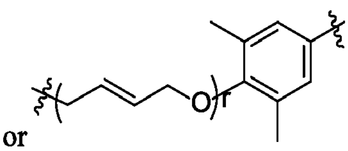 .

72. The transcription modulator molecule of any one of paras 1-69, wherein the linker comprises $-X(CH_2)_m(CH_2CH_2O)_n-$, wherein X is -O-, $N_H$-, or -S-;m is 0 or greater; and n is at least 1.

73. The transcription modulator molecule of any one of paras 1-69, wherein the linker comprises

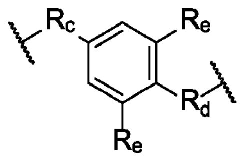

following the second terminus, wherein $R_c$ is selected from a bond, $-N(R^{1a})-$, -O-, and -S-; $R_d$ is selected from $-N(R^{1a})-$, -O-, and -S-; $R_e$ is independently selected from hydrogen and optionally substituted $C_{1-6}$ alkyl; and $R^{1a}$ is H or $C_{1-6}$ alkyl.

74. The transcription modulator molecule of any one of paras 1-69, wherein the linker comprises one or more structures selected from

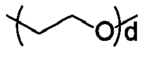 , 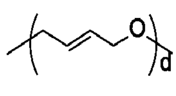 ,

$-C_{1-12}$ alkyl, arylene, cycloalkylene, heteroarylene, heterocycloalkylene, -O-, $-C(O)NR^{1a}$-,-C(O)-, $-NR^{1a}$-, $-(CH_2CH_2CH_2O)_y-$, and $-(CH_2CH_2CH_2NR^{1a})_y-$ ,wherein each d and y are independently 1-10, andeach $R^{1a}$ is independently hydrogen or $C_{1-6}$ alkyl.

75. The transcription modulator molecule of para 74, wherein the linker comprises

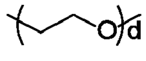 ,

wherein d is 3-7.

76. The transcription modulator molecule of any one of paras 1-75, wherein the linker comprises $-N(R^{1a})(CH_2)_xN(R^{1b})(CH_2)_xN-$, wherein $R^{1a}$ and$R^{1b}$ are each independently selected from hydrogen or optionally substituted $C_1$-$C_6$ alkyl; and each x is independently an integer in the range of 1-6.

77. The transcription modulator molecule of any one of paras 1-76, wherein the linker comprises $-(CH_2-C(O)N(R'')-(CH_2)q-N(R'XCH_2)_q-N(R'')C(O)-(CH_2)_x-C(O)N(R'')-A-$, $-(CH_2)_x-C(O)N(R'')-(CH_2CH_2O)_y(CH_2)_x-C(O)N(R'')-A-$, $-C(O)N(R'')-(CH_2)_q-N(R')-(CH_2)_q-N(R'')C(O)-(CH_2)_x-A-$, $-(CH_2)_x-O-(CH_2CH_2O)_y-(CH_2)_x-N(R'')C(O)-(CH_2)_x-A-$, or $-N(R'')C(O)-(CH_2)-C(O)N(R'')-(CH_2)_x-O(CH_2CH_2O)_y(CH_2)_x-A-$; wherein R' is methyl; R" is hydrogen; each x and y are independently an integer from 1 to 10; each q is independently an integer from 2 to 10; and each A is independently selected from a bond, an optionally substituted $C_{1-12}$ alkyl, an optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene.

78. The transcription modulator molecule of any one of paras 1-77, wherein the linker is joined with the first terminus with a group selected from -CO-, $-NR^{1a}$-,$-CONR^{1a}$-, $-NR^{1a}CO$-, $-CONR^{1a}C_{1-4}$alkyl-, - $NR^{1a}CO-C_{1-4}$alkyl-, -C(O)O-, -OC(O)-, -O-, -S-, -S(O)-, $-SO_2$-, $-SO_2NR^{1a}$-, $-NR^{1a}SO_2$-, -P(O)OH-,$-((CH_2)_x-O)$-, $-((CH_2)_y-NR^{1a})$-, optionally substituted $-C_{1-12}$ alkylene, optionally substituted $C_{2-10}$ alkenylene, optionally substituted $C_{2-10}$ alkynylene, optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene; wherein each x and y are independently 1-4, and each $R^{1a}$ is independently a hydrogen or optionally substituted $C_{1-6}$ alkyl.

79. The transcription modulator molecule of any one of paras 1-78, wherein the linker is joined with the first terminus with a group selected from -CO-, $-NR^{1a}$-, $C_{1-12}$ alkyl, $-CONR^{1a}$-, and $-NR^{1a}CO$-; wherein each $R^{1a}$ is independently a hydrogen or optionally substituted $C_{1-6}$ alkyl.

80. The transcription modulator molecule of any one of paras 1-79, wherein the linker is joined with second terminus with a group selected from -CO-, $-NR^{1a}$-,$-CONR^{1a}$-, $-NR^{1a}CO$-, $-CONR^{1a}C_{1-4}$alkyl-, - $NR^1-CO-C_{1-4}$alkyl-, -C(O)O-, -OC(O)-, -O-, -S-, -S(O)-, $-SO_2$-, $-SO_2NR^{1a}$-, $-NR^{1a}SO_2$-, -P(O)OH-,$-((CH_2)_x-O)$-, $-((CH_2)_y-NR^{1a})$-, optionally substituted $-C_{1-12}$ alkylene, optionally substituted $C_{2-10}$ alkenylene, optionally substituted $C_{2-10}$ alkynylene, optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene, wherein each x and y are independently 1-4, and each $R^{1a}$ is independently a hydrogen or optionally substituted $C_{1-6}$ alkyl.

81. The transcription modulator molecule of para 80, wherein the linker is joined with second terminus with a group selected from -CO-, $-NR^{1a}$-, $-CONR^{1a}$-, $-NR^{1a}CO$-,$-((CH_2)_x-O)$-, $-((CH_2)_y-NR^{1a})$-, -O-, optionally substituted $-C_{1-12}$ alkyl, optionally substituted $C_{6-10}$ arylene, optionally substituted $C_{3-7}$ cycloalkylene, optionally substituted 5- to 10-membered heteroarylene, and optionally substituted 4- to 10-membered heterocycloalkylene, wherein each x and y are independently 1-4, and each $R^{1a}$ is independently a hydrogen or optionally substituted $C_{1-6}$ alkyl.

82. The transcription modulator molecule of any one of paras 1-80, wherein the second terminus comprises one or more optionally substituted $C_{6-10}$ aryl, optionally substituted $C_{4-10}$ carbocyclic, optionally substituted 4 to 10 membered heterocyclic, or optionally substituted 5 to 10 membered heteroaryl.

83. The transcription modulator molecule of any one of paras 1-82, wherein the protein binding moiety that binds to the regulatory molecule is selected from the group consisting of a CREB binding protein (CBP), a P300, an O-linked β-N-acetylglucosamine-transferase- (OGT-), a P300-CBP-associated-factor- (PCAF-), histone methyltransferase, histone demethylase, chromodomain, a cyclin-dependent-kinase-9- (CDK9-), a nucleosome-remocleling-factor-(NURF-), a bromodomain-PHD-finger-transcription-factor- (BPTF-), a ten-eleven-translocation-enzyme- (TET-), a methylcytosine-dioxygenase- (TET1-), histone acetyltransferase (HAT), a histone deacetalyse (HDAC), a host-cell-factor-1(HCF1-), an octamer-binding-transcription-factor- (OCT1-), a P-TEFb-, a cyclin-T1-, a PRC2-, a DNA-demethylase, a helicase, an acetyltransferase, a histone-deacetylase, and methylated histone lysine protein.

84. The transcription modulator molecule of para 83, wherein the second terminus comprises a moiety that binds to an O-linked β-N-acetylglucosamine-transferase(OGT), or CREB binding protein (CBP).

85. The transcription modulator molecule of para 83, wherein the protein binding moiety is a residue of a compound that binds to an O-linked β-N-acetylglucosamine-transferase(OGT), or CREB binding protein (CBP).

86. The transcription modulator molecule of para 1, wherein the protein binding moiety is a residue of a compound selected from Table 2.

87. The transcription modulator molecule of any one of paras 1-85, wherein the second terminus binds the regulatory molecule with an affinity of less than 200 nM.

88. The transcription modulator molecule of any one of paras 1-86, wherein the protein binding moiety is a residue of a compound having a structure of Formula (C-1):

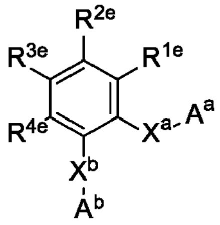

(C-1)

wherein:

$X^a$ is -NHC(O)-, -C(O)-NH-, -$NHSO_2$-, or -$SO_2NH$-;
$A^a$ is selected from an optionally substituted -$C_{1-12}$ alkyl, optionally substituted -$C_{2-10}$ alkenyl, optionally substituted -$C_{2-10}$ alkynyl, optionally substituted -$C_{1-12}$ alkoxyl, optionally substituted -$C_{1-12}$ haloalkyl, optionally substituted $C_{6-10}$ aryl, optionally substituted $C_{3-7}$ cycloalkyl, optionally substituted 5- to 10 membered heteroaryl, and optionally substituted 5- to 10-membered heterocycloalkyl;
$X^b$ is a bond, NH, NH-$C_{1-10}$alkylene, -$C_{1-12}$ alkyl, NHC(O)-, or -C(O)-NH-;
$A^b$ is selected from an optionally substituted -$C_{1-12}$ alkyl, optionally substituted -$C_{2-10}$ alkenyl, optionally substituted -$C_{2-10}$ alkynyl, optionally substituted -$C_{1-12}$ alkoxyl, optionally substituted -$C_{1-12}$ haloalkyl, optionally substituted $C_{6-10}$ aryl, optionally substituted $C_{3-7}$ cycloalkyl, optionally substituted 5- to 10 membered heteroaryl, and optionally substituted 4- to 10-membered heterocycloalkyl; and
each $R^{1e}$, $R^{2e}$, $R^{3e}$, $R^{4e}$ are independently selected from the group consisting of H, OH, - $NO_2$, halogen, amine, COOH, $COOC_{1-10}$alkyl, -NHC(O)-optionally substituted -$C_{1-12}$ alkyl, - $NHC(O)(CH_2)_{1-4}NR^fR^g$, -$NHC(OXCH_2)_{0-4}$ $CHR^f(NR^fR^g)$, -$NHC(O)(CH_2)_{0-4}$ $CHR^fR^g$, - $NHC(O)(CH_2)_{0-4}$-$C_{3-7}$ cycloalkyl, -$NHC(O)(CH_2)_{0-4}$-5- to 10-membered heterocycloalkyl, $NHC(O)(CH_2)_{0-4}C_{6-10}$ aryl, -$NHC(OXCH_2)_{0-4}$-5- to10-membered heteroaryl, -$(CH_2)_{1-4}$-$C_{3-7}$ cycloalkyl, -$(CH_2)_{1-4}$-5- to 10-membered heterocycloalkyl, -$(CH_2)_{1-4}C_{6-10}$ aryl, -$(CH_2)_{1-4}$-5- to10-membered heteroaryl, optionally substituted -$C_{2-10}$ alkenyl, optionally substituted -$C_{2-10}$ alkynyl, optionally substituted -$C_{1-12}$ alkoxyl, optionally substituted -$C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 4- to 10-membered heterocycloalkyl; and
wherein each $R^f$and $R^g$ are independently H or $C_{1-6}$ alkyl.

89. The transcription modulator molecule of para 88, wherein the protein binding moiety is a residue of a compound having a structure of Formula (C-2):

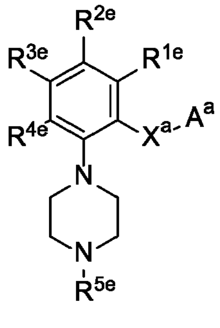

(C-2)

wherein $R^{5e}$ is independently selected from the group consisting of H, $COOC_{1-10}$alkyl,-NHC(O)-optionally substituted -$C_{1-12}$ alkyl, optionally substituted -$C_{2-10}$ alkenyl, optionally substituted -$C_{2-10}$ alkynyl, optionally substituted -$C_{1-12}$ alkoxyl, optionally substituted -$C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkylsubstituted -$C_{2-10}$ alkenyl, optionally substituted -$C_{2-10}$ alkynyl, optionally substituted -$C_{1-12}$ alkoxyl, optionally substituted -$C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkyl.

90. The transcription modulator molecule of para 88, wherein $A^a$ is selected from an optionally substituted $C_{6-10}$ aryl, optionally substituted $C_{3-7}$ cycloalkyl, optionally substituted 5- to 10 membered heteroaryl, and optionally substituted 5- to 10-membered heterocycloalkyl.

91. The transcription modulator molecule of para 88, wherein $A^a$ is an optionally substituted $C_{6-10}$ aryl.

92. The transcription modulator molecule of para 88, wherein the protein binding moiety is a residue of a compound

having a structure of Formula (C-3):

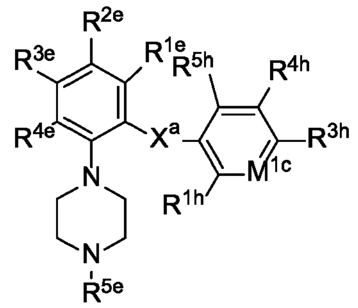

(C-3)

wherein:

$M^{1c}$ is $CR^{2h}$ or N; and
each $R^{1h}$, $R^{2h}$, $R^{3h}$, $R^{4h}$, and $R^{5h}$ are independently selected from the group consisting of H, OH, $-NO_2$, halogen, amine, COOH, COOCi-ioalkyl, -NHC(O)-optionally substituted $-C_{1-12}$ alkyl, - $NHC(O)(CH_2)_{1-4}NR^fR^g$, $-NHC(O)(CH_2)_{0-4}$ $CHR^f(NR^fR^g)$, $-NHC(O)(CH_2)_{0-4}$ $CHR^fR^g$, - $NHC(O)(CH_2)_{0-4}$-$C_{3-7}$cycloalkyl, $-NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}$-$C_{3-7}$ cycloalkyl, $-(CH_2)_{1-4}$-5- to 10-membered heterocycloalkyl, $-(CH_2)_{1-4}C_{6-10}$ aryl, $-(CH_2)_{1-4}$-5- to10-membered heteroaryl, optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkyl, wherein each $R^f$ and $R^g$ are independently H or $C_{1-6}$ alkyl.

93. The transcription modulator molecule of para 92, wherein each $R^{1h}$and $R^{5h}$ are independently hydrogen, halogen, or $C_{1-6}$ alkyl.
94. The transcription modulator molecule of para 92, wherein each $R^{2h}$ and $R^{3h}$ are independently H, OH, $-NO_2$, halogen, $C_{1-4}$ haloalkyl, amine, COOH, $COOC_{1-10}$alkyl, -NHC(O)-optionally substituted $-C_{1-12}$ alkyl, $-NHC(O)(CH_2)_{1-4}NR'R''$, $-NHC(O)(CH_2)_{0-4}$ CHR'(NR'R"), $-NHC(O)(CH_2)_{0-4}$ $CHR^fR^g$, $-NHC(O)(CH_2)_{0-4}$-$C_{3-7}$ cycloalkyl, $-NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}$-$C_{3-7}$ cycloalkyl, - $(CH_2)_{1-4}$-5- to 10-membered heterocycloalkyl, $-(CH_2)_{1-4}C_{6-10}$ aryl, $-(CH_2)_{1-4}$-5- to10-membered heteroaryl, optionally substituted $-C_{2-10}$ alkenyl, optionally substituted $-C_{2-10}$ alkynyl, optionally substituted $-C_{1-12}$ alkoxyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkyl.
95. The transcription modulator molecule of para 87, wherein $A^a$ is a $C_{6-10}$ aryl substituted with 1-4 substituents, and each substituent is independently selected from halogen, OH, $NO_2$, an optionally substituted $-C_{1-12}$ alkyl, optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10 membered heteroaryl, and optionally substituted 5-to 10-membered heterocycloalkyl.
96. The transcription modulator molecule of para 87, wherein $R^{1e}$, $R^{3e}$, and $R^{4e}$ are hydrogen.
97. The transcription modulator molecule of para 87, wherein $R^{2e}$ is selected from the group consisting of H, OH, $-NO_2$, halogen, amine, COOH, $COOC_{1-10}$alkyl, -NHC(O)-optionally substituted $-C_{1-12}$ alkyl, $-NHC(O)(CH_2)_{1-4}NR^fR^g$, $-NHC(O)(CH_2)_{0-4}$ CHR'(NR'R"), $-NHC(O)(CH_2)_{0-4}$ $CHR^fR^g$, - $NHC(O)(CH_2)_{0-4}$-$C_{3-7}$ cycloalkyl, $-NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}$-$C_{3-7}$ cycloalkyl, $-(CH_2)_{1-4}$-5- to 10-membered heterocycloalkyl, $-(CH_2)_{1-4}C_{6-10}$ aryl, $-(CH_2)_{1-4}$-5- to10-membered heteroaryl, optionally substituted $-C_{1-12}$ alkyl, -optionally substituted $-C_{2-10}$ alkenyl, optionally substituted $-C_{2-10}$ alkynyl, optionally substituted $-C_{1-12}$ alkoxyl, optionally substituted $-C_{1-12}$ haloalkyl, optionally substituted $C_{6-10}$ aryl, optionally substituted $C_{3-7}$ cycloalkyl, optionally substituted 5- to 10-membered heteroaryl, and optionally substituted 5- to 10-membered heterocycloalkyl, wherein each $R^f$ and $R^g$ are independently H or $C_{1-6}$ alkyl.
98. The transcription modulator molecule of para 87, wherein $R^{2e}$ is an phenyl or pyridinyl optionally substituted with 1-3 substituents, wherein the substituent is independently selected from the group consisting of OH, $-NO_2$, halogen, amine, COOH, $COOC_{1-10}$alkyl, NHC(O) $-C_{1-12}$ alkyl, $-NHC(O)(CH_2)_{1-4}NR^fR^g$, $-NHC(O)(CH_2)_{0-4}$ $CHR^f(NR^fR^g)$, $-NHC(O)(CH_2)_{0-4}$ $CHR^fR^g$, $-NHC(O)(CH_2)_{0-4}$-$C_{3-7}$ cycloalkyl,$-NHC(O)(CH_2)_{0-4}$-5- to 10-membered heterocycloalkyl, $NHC(O)(CH_2)_{0-4}C_{6-10}$ aryl, $-NHC(O)(CH_2)_{0-4}$-5- to10-membered heteroaryl, $-(CH_2)_{1-4}$-$C_{3-7}$ cycloalkyl, $-(CH_2)_{1-4}$-5- to 10-membered heterocycloalkyl, $-(CH_2)_{1-4}C_{6-10}$ aryl, $-(CH_2)_{1-4}$-5- to10-membered heteroaryl, $-C_{1-12}$alkoxyl, $C_{1-12}$haloalkyl,

$C_{6-10}$aryl, $C_{3-7}$ cycloalkyl, 5- to 10-membered heteroaryl, and 5- to 10-membered heterocycloalkyl, wherein each $R^f$ and $R^g$ are independently H or $C_{1-6}$ alkyl.

99. The transcription modulator of any one of paras 1-87, wherein the protein binding moiety is a residue of a compound having the structure of Formula (C-4):

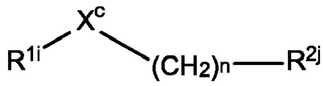
(C-4)

wherein:

$R^{1c}$ is an optionally substituted $C_{6-10}$ aryl or an optionally substituted 5- to 10-membered heteroaryl,
$X^c$ is -C(O)NH-, -C(O), -S($O_2$)-, -NH-, or -$C_{1-4}$alkyl-NH,
n is 0-10,
$R^{2j}$ is -$NR^{3j}R^{4j}$, optionally substituted $C_{6-10}$ aryl, optionally substituted $C_{3-7}$ cycloalkyl, optionally substituted 5- to 10-membered heteroaryl, or optionally substituted 4- to 10-membered heterocycloalkyl; and
each $R^{3j}$ and $R^{4j}$ are independently H or optionally substituted -$C_{1-12}$ alkyl.

100. The transcription modulator molecule of para 99, wherein R^ is -NHC($CH_3$)$_3$, or a 4- to 10-membered heterocycloalkyl substituted with $C_{1-12}$ alkyl.

101. The transcription modulator of any one of paras 1-87, wherein the protein binding moiety is a residue of a compound having the structure of Formula (C-5):

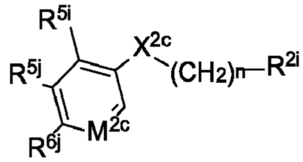
(C-5)

wherein:

$X^{2c}$ is a bond, C(O), $SO_2$, or $CHR^{3c}$;
$M^{2c}$ is CH or N;
n is 0-10,
R^ is -$NR^{3j}R^{4j}$, optionally substituted $C_{6-10}$ aryl, optionally substituted $C_{3-7}$ cycloalkyl, optionally substituted 5- to 10-membered heteroaryl, or optionally substituted 4- to 10-membered heterocycloalkyl;
each $R^{5j}$ is independently -$NR^{3j}R^{4j}$, -C(O)$R^{3j}$, -COOH, -C(O)NH$C_{1-6}$alkyl, an optionally substituted $C_{6-10}$ aryl, or an optionally substituted 5- to 10-membered heteroaryl;
$R^{6j}$ is -$NR^{3j}R^{4j}$, -C(O)$R^{3j}$, an optionally substituted $C_{6-10}$ aryl, or an optionally substituted 5-to 10-membered heteroaryl; and
each $R^{3j}$ and $R^{4j}$ are independently H, an optionally substituted $C_{6-10}$ aryl, optionally substituted 4- to 10-membered heterocycloalkyl, or optionally substituted -$C_{1-12}$ alkyl.

102. The transcription modulator molecule of para 101, wherein $R^{2j}$ is a 4- to 10-membered heterocycloalkyl substituted by a 4- to 10-membered heterocycloalkyl.

103. The transcription modulator molecule of para 101, wherein $R^{6j}$ is -C(O)$R^{3j}$, and $R^{3j}$ is a 4-to 10-membered heterocycloalkyl substituted by a 4- to 10-membered heterocycloalkyl.

104. The transcription modulator molecule of para 101, wherein each $R^{5j}$ is independently H, - C(O)$R^{3j}$, -COOH, -C(O)NH$C_{1-6}$alkyl, -NH-$C_{6-10}$ aryl, or optionally substituted $C_{6-10}$ aryl.

105. The transcription modulator molecule of any one of paras 1-75, wherein the protein binding moiety is a residue of a compound having the structure of Formula (C-6):

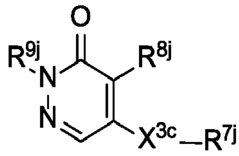
(C-6)

wherein:

$X^{3c}$ is a bond, NH, $C_{1-4}$ alkylene, or $NC_{1-4}$ alkyl;
$R^{7j}$ is an optionally substituted $C_{1-6}$ alkyl, an optionally substituted cyclic amine, an optionally substituted aryl, an optionally substituted 5- to 10-membered heteroaryl, or optionally substituted 4- to 10-membered heterocycloalkyl,
$R^{8j}$ is H, halogen, or $C_{1-6}$ alkyl; and
$R^{9j}$ is H, or $C_{1-6}$ alkyl.

106. The transcription modulator molecule of para 100, wherein $R^{7j}$ is an optionally substituted cyclic secondary or tertiary amine.
107. The transcription modulator molecule of para 100, wherein $R^{7j}$ is a tetrahydroisoquinoline optionally substituted with $C_{1-4}$ alkyl.
108. The transcription modulator molecule of any one of paras 1-75, wherein the protein binding moiety is a residue of a compound having the structure of Formula (C-7):

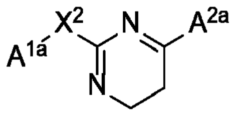
(C-7),

wherein:

$A^{1a}$ is an optionally substituted aryl or heteroaryl;
$X^2$ is a bond, $(CH_2)_{1-4}$, or NH; and
$A^{2a}$ is an optionally substituted aryl, heterocyclic, or heteroaryl, linked to an amide group.

109. The transcription modulator molecule of para 108, wherein $A^{1a}$ is an aryl substituted with one or more halogen, $C_{1-6}$alkyl, hydroxyl, $C_{1-6}$alkoxy, or $C_{1-6}$ haloalkyl.
110. The transcription modulator molecule of para 108, wherein $X^2$ is NH.
111. The transcription modulator molecule of para 108, wherein $A^{2a}$ is a heterocyclic group.
112. The transcription modulator molecule of para 108, wherein $A^{2a}$ is a pyrrolidine.
113. The transcription modulator molecule of para 108, wherein $A^{2a}$ is an optionally substituted phenyl.
114. The transcription modulator molecule of para 108, wherein $A^{2a}$ is a phenyl optionally substituted with one or more halogen, $C_{1-6}$ alkyl, hydroxyl, $C_{1-6}$ alkoxy, or $C_{1-6}$ haloalkyl.
115. The transcription modulator molecule of any one of paras 1-87, wherein the protein binding moiety is a residue of a compound having the structure of Formula (C-8):

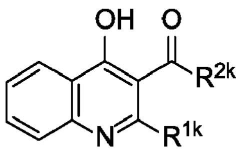
(C-8),

wherein $R^{1k}$ is H or $C_{1-25}$ alkyl and $R^{2k}$ is OH or $-OC_{1-12}$ alkyl.
116. The transcription modulator molecule of any one of paras 1-87, wherein the protein binding moiety is a residue of a compound having the structure of Formula (C-9):

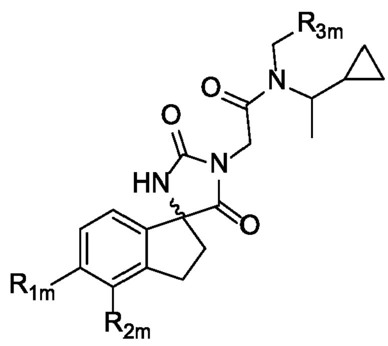

(C-9),

wherein $R_{1m}$ is H, OH, $-CONH_2$, -COOH, -NHC(O)-$C_{1-6}$ alkyl,-NHC(O)O-$C_{1-6}$ alkyl,-NHS$(O)_2$-$C_{1-6}$alkyl, -$C_{1-6}$ alkyl, -$C_{1-6}$ alkoxyl, or -NHC(O)NH-$C_{1-4}$alkyl;
$R_{2m}$ is H, CN, or $CONH_2$; and
$R_{3m}$ is an optionally substituted $C_{6-10}$ aryl.

117. The transcription modulator molecule of any one of paras 1-87, wherein the protein binding moiety is a residue of a compound having the structure of Formula (C-10):

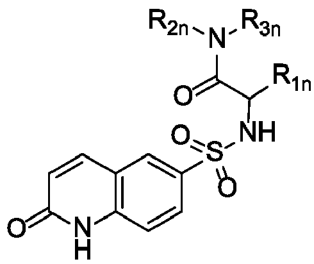

(C-10),

wherein $R_{1n}$ is an optionally substituted $C_{6-10}$ aryl or optionally substituted 5- to 10-membered heteroaryl, and each $R_{2n}$ and $R_{3n}$ are independently H, -$C_{1-4}$ alkyl-$C_{6-10}$ aryl, -$C_{1-4}$alkyl-5-to10-membered heteroaryl, $C_{6-10}$ aryl, or -5-tolO-membered heteroaryl, or
$R_{2n}$ and $R_{3n}$ together with N form an optionally substituted 4-10 membered heterocyclic or heteroaryl group.

118. The transcription modulator molecule of any one of paras 1-87, wherein the methylated histone lysine protein is selected from Ankyrin repeats, WD-40 repeat domains, MBT, Tudor, PWWP, chromodomain plant homeodomain (PHD) fingers, and ADD.
119. The transcription modulator molecule of any one of paras 1-87, wherein the second terminus comprises at least one 5-10 membered heteroaryl group having at least two nitrogen atoms.
120. The transcription modulator molecule of any one of paras 1-119, wherein the second terminus comprises a moiety capable of binding to the regulatory protein, and the moiety is from a compound capable of binding to the regulatory protein.
121. The transcription modulator molecule of any one of paras 1-87, wherein the second terminus comprises at least one group selected from an optionally substituted diazine, an optionally substituted diazepine, and an optionally substituted phenyl.
122. The transcription modulator molecule of any one of paras 1-121, wherein the second terminus does not comprises JQ1, iBET762, OTX015, RVX208, or AU1.
123. The transcription modulator molecule of any one of paras 1-122, wherein the second terminus does not comprises JQ1.
124. The transcription modulator molecule of any one of paras 1-123, wherein the second terminus does not comprises a moiety that binds to a bromodomain protein.
125. The transcription modulator molecule of any one of paras 1-87, wherein the second terminus comprises a diazine or diazepine ring, wherein the diazine or diazepine ring is fused with a $C_{6-10}$ aryl or a 5-10 membered heteroaryl ring comprising one or more heteroatom selected from S, N and O.
126. The transcription modulator molecule of any one of paras 1-87, wherein the second terminus comprises an optionally substituted bicyclic or tricyclic structure.
127. The transcription modulator molecule of para 126, wherein the optionally substituted bicyclic or tricyclic structure

comprises a diazepine ring fused with a thiophene ring.

128. The transcription modulator molecule of para 126, wherein the second terminus comprises an optionally substituted bicyclic structure, wherein the bicyclic structure comprises a diazepine ring fused with a thiophene ring.

129. The transcription modulator molecule of para 126, wherein the second terminus comprises an optionally substituted tricyclic structure, wherein the tricyclic structure is a diazaphine ring that is fused with a thiophene and a triazole.

130. The transcription modulator molecule of any one of paras 1-87, wherein the second terminus comprises an optionally substituted diazine ring.

131. The transcription modulator molecule of any one of paras 1-130, wherein the second terminus does not comprise a structure of Formula (C-11):

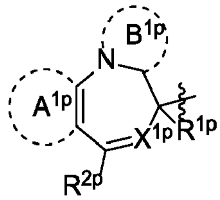

(C-11),

wherein:

each of $A^{1p}$ and $B^{1p}$ is independently an optionally substituted aryl or heteroaryl ring;
$X^{1p}$ is CH or N;
$R^{1p}$ is hydrogen, halogen, or an optionally substituted $C_{1-6}$ alkyl group; and
$R^{2p}$ is an optionally substituted $C_{1-6}$ alkyl, cycloalkyl, $C_{6-10}$ aryl, or heteroaryl.

132. The transcription modulator molecule of para 131, wherein $X^{1p}$ is N.

133. The transcription modulator molecule of para 131, wherein $A^{1p}$ is an aryl or heteroaryl substituted with one or more substituents.

134. The transcription modulator molecule of para 131, wherein $A^{1p}$ is an aryl or heteroaryl substituted with one or more substituents selected from halogen, $C_{1-6}$alkyl, hydroxyl, $C_{1-6}$alkoxy, and $C_{1-6}$haloalkyl.

135. The transcription modulator molecule of para 131, wherein $B^{1p}$ is an optionally substituted aryl or heteroaryl substituted with one or more substituents selected from halogen, $C_{1-6}$alkyl, hydroxyl, $C_{1-6}$alkoxy, and $C_{1-6}$haloalkyl.

136. The transcription modulator molecule of para 131, wherein $A^{1p}$ is an optionally substituted thiophene or phenyl.

137. The transcription modulator molecule of para 131, wherein $A^{1p}$ is a thiophene or phenyl, each substituted with one or more substituents selected from halogen, $C_{1-6}$ alkyl, hydroxyl, $C_{1-6}$ alkoxy, and $C_{1-6}$ haloalkyl.

138. The transcription modulator molecule of para 131, wherein $B^{1p}$ is an optionally substituted triazole.

139. The transcription modulator molecule of para 131, wherein $B^{1p}$ is a triazole substituted with one or more substituents selected from halogen, $C_{1-6}$alkyl, hydroxyl, $C_{1-6}$alkoxy, and $C_{1-6}$haloalkyl.

140. The transcription modulator molecule of any one of paras 1-139, wherein the protein binding moiety is not

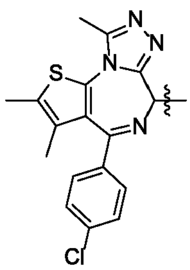

,

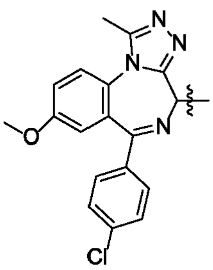

,

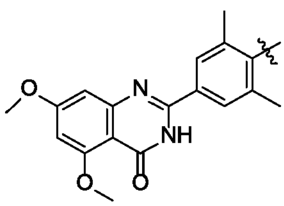

,

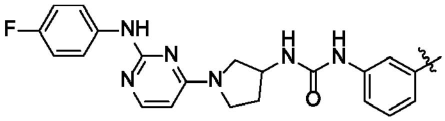, or 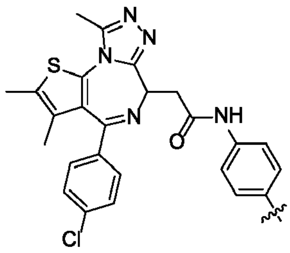.

141. The transcription modulator molecule of any one of paras 1-140, wherein the protein binding moiety is not

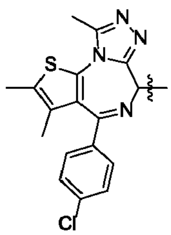.

142. The transcription modulator molecule of any one of paras 1-139, wherein the protein binding moiety does not have the structure ofFormula (C-12):

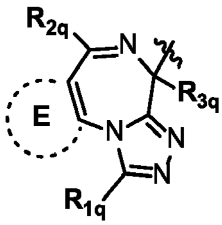

(C-12),

wherein:

$R_{1q}$ is a hydrogen or an optionally substituted alkyl, hydroxyalkyl, aminoalkyl, alkoxyalkyl, halogenated alkyl, hydroxyl, alkoxy, or -$COOR_{4q}$;
$R_{4q}$ is hydrogen, or an optionally substituted aryl, aralkyl, cycloalkyl, heteroaryl, heteroaralkyl, heterocycloalkyl, alkyl, alkenyl, alkynyl, or cycloalkylalkyl group, optionally containing one or more heteroatoms;
$R_{2q}$ is an optionally substituted aryl, alkyl, cycloalkyl, or aralkyl group;
$R_{3q}$ is hydrogen, halogen, or an optionally substituted alkyl group, preferably $(CH_2)_x$ - C(O)N(Rn)($R_{21}$), or $(CH_2)_x$-N($R_{20}$)-C(O)$R_{21}$; or halogenated alkyl group;
wherein x is an integer from 1 to 10; and $R_{20}$ and $R_{21}$ are each independently hydrogen or $C_1$-$C_6$ alkyl group, preferably $R_{20}$ is hydrogen and $R_{21}$ ismethyl; and
Ring E is an optionally substituted aryl or heteroaryl group.

143. A transcription modulator molecule as recited in any one of the proceeding paras for use as a medicament.
144. A transcription modulator molecule as recited in any one of the proceeding paras for use in the manufacture of a medicament for the prevention or treatment of a disease or condition ameliorated by the overexpression of *c9orj72.*
145. A transcription modulator molecule as recited in any one of the proceeding paras for use in the treatment of ALS.
146. A pharmaceutical composition comprising a transcription modulator molecule as recited in any one of the proceeding paras and a pharmaceutically acceptable carrier.
147. A method of modulation of the expression of *c9orf72* comprising contacting *c9orj72* with a transcription modulator molecule as recited in any one of paras 1-134.
148. A method of treatment of a disease caused by expression of a defective *c9orj72* comprising the administration of a therapeutically effective amount of a transcription modulator molecule as recited in any one of paras 1-134 to a patient in need thereof.
149. The method as recited in para 148 wherein said disease is ALS.

150. A method of treatment of a disease caused by expression of a defective *c9orj72* comprising the administration of:

a therapeutically effective amount of a transcription modulator molecule as recited in any one of paras 1-130; and another therapeutic agent.

151. A method for achieving an effect in a patient comprising the administration of a therapeutically effective amount of a transcription modulator molecule as disclosed herein, or a salt thereof, to a patient, wherein the effect is chosen from muscular atrophy, ataxia, fasciculation, and dementia.

152. A compound of structural Formula I:

X-L-Y (I)

or a salt thereof, wherein:

X comprises a recruiting moiety that is capable of noncovalent binding to a regulatory molecule within the nucleus;
Y comprises a DNA recognition moiety that is capable of noncovalent binding to one or more copies of the pentanucleotide repeat sequence GGGGCC; and
L is a linker.

153. The compound as recited in para 152, wherein

L comprises $-(CH(CH_3)OCH2)_m-$; and
m is an integer between 1 to 10, inclusive.

154. The compound as recited in para 152, wherein the DNA recognition moiety Y comprises a polyamide sequence.

155. The compound as recited in para 153, having structural Formula II:

$X\text{-}L\text{-}(Y_1\text{-}Y_2\text{-}Y_3\text{-}Y_4\text{-}Y_5\text{-}Y_6)_n\text{-}Y_0$ (II)

or a salt thereof, wherein:

X comprises a recruiting moiety that is capable of noncovalent binding to a regulatory molecule within the nucleus;
Lis a linker;
$Y_1$, $Y_2$, $Y_3$, $Y_4$, $Y_5$, and $Y_6$ are internal subunits, each of which comprises a moiety chosen from a heterocyclic ring or a $C_{1-6}$ straight chain aliphatic segment, and each of which is chemically linked to its two neighbors;
$Y_0$ is an end subunit which comprises a moiety chosen from a heterocyclic ring or a straight chain aliphatic segment, which is chemically linked to its single neighbor;
each subunit can noncovalently bind to an individual nucleotide in the GGGGCC repeat sequence;
n is an integer between 1 and 5, inclusive; and
$(Y_1\text{-}Y_{2\text{-}}Y_3\text{-}Y_4\text{-}Y_5\text{-}Y6)_n\text{-}Y_0$ combine to form a DNA recognition moiety that is capable of noncovalent binding to one or more copies of the hexanucleotide repeat sequence GGGGCC.

156. The compound as recited in para 155, wherein $Y_1$, $Y_2$, $Y_3$, $Y_4$, $Y_5$, and $Y_6$ each comprise a chemical moiety independently chosen from

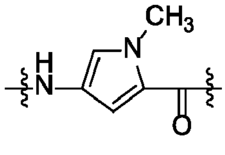 (Py), 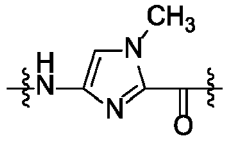 (Im),

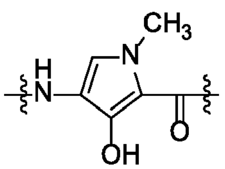 (Hp), 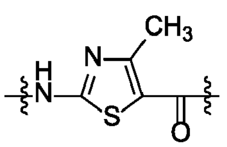 (Th), 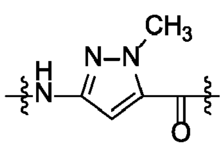 (Pz), 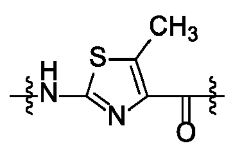 (Nt),

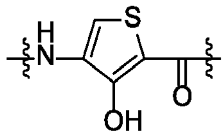 (Ht), 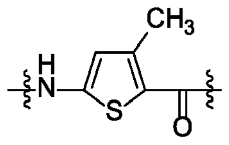 (Tn), 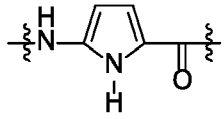 (Nh), 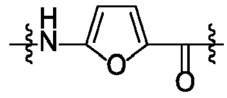 (Fr),

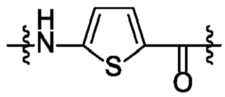 (Tp), 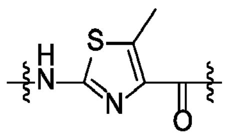 , 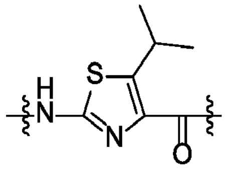 , and 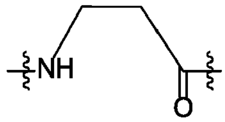 (β).

157. The compound as recited in para 152, having structural Formula III:

$$X\text{-}L\text{-}(Y_1\text{-}Y_{2}\text{-}Y_3\text{-}Y_4\text{-}Y_5\text{-}Y_6)\text{-}(W\text{-}Y_1\text{-}Y_{2}\text{-}Y_{3}\text{-}Y_4\text{-}Y_5\text{-}Y_6)_n\text{-}Y_0 \qquad \text{(III)}$$

or a salt thereof, wherein:

X comprises a recruiting moiety that is capable of noncovalent binding to a regulatory molecule within the nucleus;
L is a linker;
$Y_1$, $Y_2$, $Y_3$, $Y_4$, $Y_5$, and $Y_6$ are internal subunits, each of which comprises a moiety chosen from a heterocyclic ring or a $C_{1\text{-}6}$ straight chain aliphatic segment, and each of which is chemically linked to its two neighbors;
$Y_0$ is an end subunit which comprises a moiety chosen from a heterocyclic ring or a straight chain aliphatic segment, which is chemically linked to its single neighbor;
each subunit can noncovalently bind to an individual nucleotide in the GGGGCC repeat sequence;
W is a spacer;
n is an integer between 1 and 5, inclusive; and
$(Y_1\text{-}Y_2\text{-}Y_3\text{-}Y_4\text{-}Y_5\text{-}Y_6)\text{-}(W\text{-}Y_1\text{-}Y_2\text{-}Y_3\text{-}Y_4\text{-}Y_5\text{-}Y_6)_n\text{-}Y_0$ combine to form a DNA recognition moiety that is capable of noncovalent binding to one or more copies of the hexanucleotide repeat sequence GGGGCC.

158. The compound as recited in para 152, structural Formula IV:

$$X\text{-}L\text{-}(Y_1\text{-}Y_2\text{-}Y_3\text{-}Y_4\text{-}Y_5\text{-}Y_6)\text{-}V\text{-}(Y_7\text{-}Y_8\text{-}Y_9\text{-}Y_{10}\text{-}Y_{11}\text{-}Y_{12})\text{-}Y_0 \qquad \text{(IV)}$$

or a salt thereof, wherein:

X comprises a recruiting moiety that is capable of noncovalent binding to a regulatory molecule within the nucleus;
L is a linker chosen from a $C_{1\text{-}6}$straight chain aliphatic segment and $(CH_2OCH_2)_m$;
$Y_1$, $Y_2$, $Y_3$, $Y_4$, $Y_5$, $Y_6$, $Y_7$, $Y_8$, $Y_9$, $Y_{10}$, $Y_{11}$, and $Y_{12}$ are internal subunits, each of which comprises a moiety chosen from a heterocyclic ring or a $C_{1\text{-}6}$staight chain aliphatic segment, and each of which is chemically linked to its two neighbors;
$Y_0$ is an end subunit which comprises a moiety chosen from a heterocyclic ring or a straight chain aliphatic segment, which is chemically linked to its single neighbor;
each subunit can noncovalently bind to an individual nucleotide in the GGGGCC repeat sequence;
V is a turn component for forming a hairpin turn; and
$(Y_1\text{-}Y_{2}\text{-}Y_3\text{-}Y_4\text{-}Y_5\text{-}Y_6)\text{-}V\text{-}(Y_7\text{-}Y_8\text{-}Y_9\text{-}Y_{10}\text{-}Y_{11}\text{-}Y_{12})\text{-}Y_0$ combine to form a DNA recognition moiety that is capable of noncovalent binding to one or more copies of the hexanucleotide repeat sequence GGGGCC.

159. The compound as recited in para 152, having structural Formula Va:

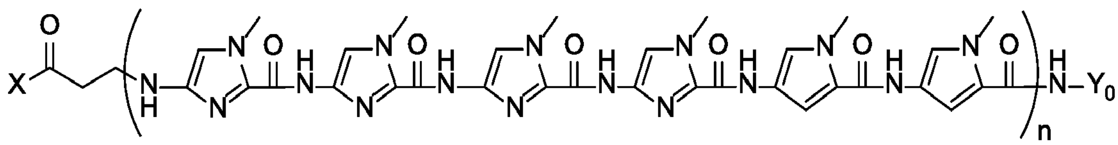

(Va)

or a salt thereof, wherein:

X comprises a recruiting moiety that is capable of noncovalent binding to a regulatory molecule within the nucleus; $Y_0$ is an end subunit which comprises a moiety chosen from a heterocyclic ring or a straight chain aliphatic segment, which is chemically linked to its single neighbor; and n is an integer between 1 and 5, inclusive.

160. The compound as recited in para 1, having structural Formula Vb:

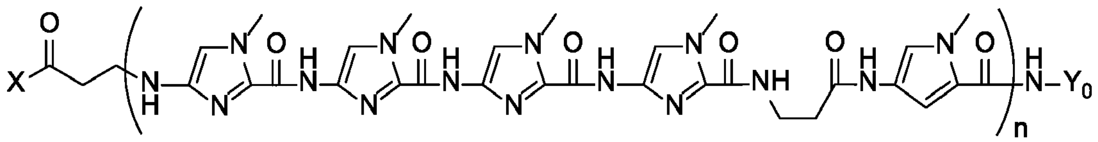

(Vb)

or a salt thereof, wherein:

X comprises a recruiting moiety that is capable of noncovalent binding to a regulatory molecule within the nucleus; $Y_0$ is an end subunit which comprises a moiety chosen from a heterocyclic ring or a straight chain aliphatic segment, which is chemically linked to its single neighbor; and n is an integer between 1 and 5, inclusive.

161. The compound as recited in para 152, having structural VIa:

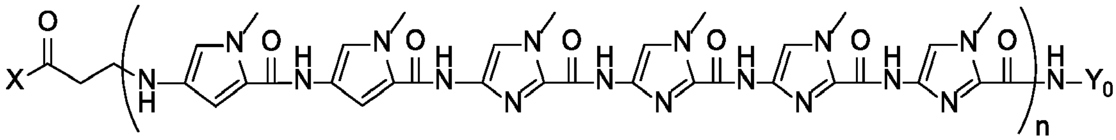

(VIa)

or a salt thereof, wherein:

X comprises a recruiting moiety that is capable of noncovalent binding to a regulatory molecule within the nucleus; and $Y_0$ is an end subunit which comprises a moiety chosen from a heterocyclic ring or a straight chain aliphatic segment, which is chemically linked to its single neighbor; and n is an integer between 1 and 5, inclusive.

162. The compound as recited in para 152, having Formula VIb:

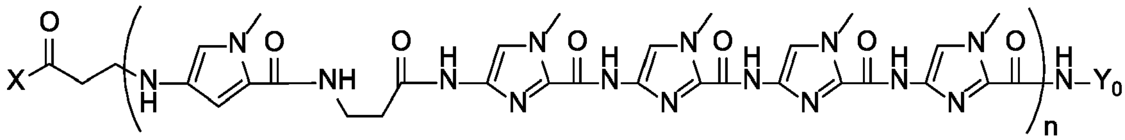

or a salt thereof, wherein:

X comprises a recruiting moiety that is capable of noncovalent binding to a regulatory molecule within the nucleus; $Y_0$ is an end subunit which comprises a moiety chosen from a heterocyclic ring or a straight chain aliphatic segment, which is chemically linked to its single neighbor; and n is an integer between 1 and 5, inclusive.

163. The compound as recited in para 152, having structural Formula VII:

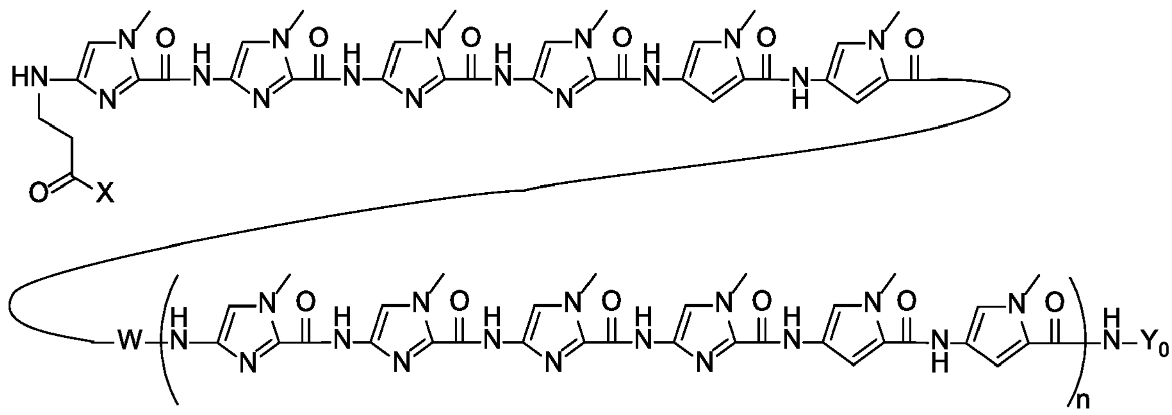

(VII)

or a salt thereof, wherein:

X comprises a recruiting moiety that is capable of noncovalent binding to a regulatory molecule within the nucleus; and
W is a spacer; and
$Y_0$ is an end subunit which comprises a moiety chosen from a heterocyclic ring or a straight chain aliphatic segment, which is chemically linked to its single neighbor; and
n is an integer between 1 and 5, inclusive.

164. The compound as recited in para 152 for use in the treatment of ALS.
165. The compound as recited in para 152, wherein A is selected from a bromodomain inhibitor, a BPTF inhibitor, a methylcytosine dioxygenase inhibitor, a DNA demethylase inhibitor, a helicase inhibitor, an acetyltransferase inhibitor, a histone deacetylase inhibitor, a CDK-9 inhibitor, a positive transcription elongation factor inhibitor, and a polycomb repressive complex inhibitor.
166. The compound as recited in para 165, wherein A is selected from a bromodomain inhibitor and a CDK9 inhibitor.
167. A compound as recited in para 152 for use as a medicament.
168. A compound as recited in para 152 for use in the manufacture of a medicament for the prevention or treatment of a disease or condition ameliorated by the modulation of the expression of the c9orf72 gene.
169. A compound as recited in para 152 for use in the treatment of ALS.
170. A pharmaceutical composition comprising a compound as recited in para 1 together with a pharmaceutically acceptable carrier.
171. A method of modulation of the expression of the c9orf72 gene comprising contacting c9orf72 with a compound as recited in para 152.
172. A method of treatment of a disease associated with the expression of defective *c9orj72* comprising the administration of a therapeutically effective amount of a compound as recited in para 152 to a patient in need thereof.
173. The method as recited in para 172 wherein said disease is spinocerebellar ataxia.
174. The method as recited in para 173 wherein said spinocerebellar ataxia is ALS.
175. A method of treatment of a disease associated with the expression of c9orf72 comprising the administration of

a therapeutically effective amount of a compound as recited in para 152; and
another therapeutic agent.

176. The method as recited in para 175, wherein said other agent is chosen from riluzole (RILUTEK®) and edaravone (RADICAVA®).
177. A method for achieving an effect in a patient comprising the administration of a therapeutically effective amount of a compound as disclosed herein, or a salt thereof, to a patient, wherein the effect is chosen from muscular atrophy, ataxia, fasciculations, and dementia.

**Claims**

**1.** An *ex-vivo* method for modulating transcription of a gene comprising a hexanucleotide repeat sequence GGGGCC

the method comprising contacting a cell comprising the gene with an agent having a first terminus, a second terminus, and an oligomeric backbone, wherein:

(a) the first terminus comprises a DNA-binding moiety capable of noncovalently binding to the hexanucleotide repeat sequence GGGGCC;
(b) the second terminus comprises a protein-binding moiety capable of binding to a regulatory molecule that modulates an expression of the gene comprising the hexanucleotide repeat sequence GGGGCC; and
(c) the oligomeric backbone comprises a linker between the first terminus and the second terminus.

**2.** An agent for use in a method of treatment of a disease, wherein the method comprises contacting a cell comprising a hexanucleotide repeat sequence GGGGCC with the agent, wherein the agent modulates transcription of the gene, and wherein the agent has a first terminus, a second terminus, and an oligomeric backbone, wherein:

(a) the first terminus comprises a DNA-binding moiety capable of noncovalently binding to the hexanucleotide repeat sequence GGGGCC;
(b) the second terminus comprises a protein-binding moiety capable of binding to a regulatory molecule that modulates an expression of the gene comprising the hexanucleotide repeat sequence GGGGCC; and
(c) the oligomeric backbone comprises a linker between the first terminus and the second terminus.

**3.** The method of claim 1 or agent for use of claim 2, wherein the DNA-binding moiety is a polyamide selected from of a linear polyamide, a hairpin polyamide, a H-pin polyamide, an overlapped polyamide, a slipped polyamide, a cyclic polyamide, a tandem polyamide, and an extended polyamide.

**4.** The method of claim 1 or agent for use of claim 2, wherein the hexanucleotide repeat comprises at least 20 repeats, at least 50 repeats, at least 100 repeats, at least 200 repeats, at least 500 repeats, or at least 1000 repeats.

**5.** The method of claim 1 or agent for use of claim 2, wherein the protein-binding moiety is capable of binding to a regulatory molecule that is selected from the group consisting of a CREB binding protein (CBP), a P300, an O-linked β-N-acetylglucosamine-transferase (OGT), a P300-CBP-associated-factor (PCAF), a histone methyltransferase, a histone demethylase, a chromodomain, a cyclin-dependent-kinase-9 (CDK9), a nucleosome-remodeling-factor (NURF), a bromodomain-PHD-finger-transcription-factor (BPTF), a ten-eleven-translocation-enzyme (TET), a methylcytosine-dioxygenase (TET1), a histone acetyltransferase (HAT), a histone deacetylase (HDAC), a host-cell-factor-1 (HCF1), an octamer-binding-transcription-factor (OCTI), a P-TEFb, a cyclin-TI, a PRC2, a DNA-demethylase, a helicase, an acetyltransferase, a histone-deacetylase, a bromodomain-containing protein and a methylated histone lysine protein.

**6.** The method of claim 1 or agent for use of claim 2, wherein the protein-binding moiety is selected from the group consisting of a bromodomain inhibitor, a BPTF inhibitor, a methylcytosine dioxygenase inhibitor, a DNA demethylase inhibitor, a helicase inhibitor, an acetyltransferase inhibitor, a histone deacetylase inhibitor, a CDK-9 inhibitor, a positive transcription elongation factor inhibitor, and a polycomb repressive complex inhibitor.

**7.** The method of claim 1 or agent for use of claim 2, wherein the second terminus does not comprise a moiety that binds to a bromodomain protein.

**8.** The method of claim 1 or agent for use of claim 2, wherein the protein-binding moiety does not comprise JQ1, iBET762, OTX015, RVX208, or AU1.

**9.** The method of claim 1 or agent for use of claim 2, wherein the protein-binding moiety binds the regulatory molecule with an affinity of less than 200 nM.

**10.** The method of claim 1 or agent for use of claim 2, wherein the linker has a length of less than about 50 Angstroms.

**11.** The method of any one of claims 1 and 3-10 or agent for use of any one of claims 2-10, wherein the gene is *C9ORF72.*

**12.** The method or agent for use of claim 11, wherein the DNA-binding moiety is capable of selectively binding to a GGGGCC hexanucleotide repeat sequence of *C9ORF72.*

**13.** The method or agent for use of claim 11, wherein the method comprises decreasing *C9ORF72* expression.

**14.** The method or agent for use of claim 11, wherein the method comprises a 20%, 50%, 80%, 90%, 95%, or 99% decrease in expression of *C9ORF72.*

**15.** The agent for use of any one of claims 2-14, wherein the method further comprises treating a disease mediated by transcription of an allele of *C9ORF72* comprising the GGGGCC hexanucleotide repeat sequence in a patient in need thereof.

**16.** The agent for use of claim 15, wherein the disease is ALS.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62669155

### Non-patent literature cited in the description


- **BB LAO et al.** *PNAS USA,* 2014, vol. 111 (21), 7531-7536
- **ITKONEN HM.** Inhibition of O-GicNAc transferase activity reprograms prostate cancer cell metabolism. *Oncotarget,* 2016, vol. 7 (11), 12464-12476
- **F MORALES et al.** Overview of CDK9 as a target in cancer research. *Cell Cycle,* 2016, vol. 15 (4), 519-527
- **N. CAO ; Y. HUANG ; J. ZHENG.** Conversion of human fibroblasts into functional cardiomyocytes by small molecules. *Science,* 2016, vol. 352 (6290), 1216-1220
- **CHANG Y.** Structural basis for G9a-like protein lysine methyltransferase inhibition by BIX-01294. *Nature Struct. Mol. Biol.,* 2009, vol. 16, 312-317
- **B ZHU et al.** *BioorgMed Chem,* 2015, vol. 23 (12), 2917-2927
- **JI STUCKEY et al.** *Nature Chem Biol,* 2016, vol. 12 (3), 180-187
- **KD BARNASH et al.** *ACS Chem. Biol.,* 2016, vol. 11 (9), 2475-2483
- **KONZE KD.** *ACS Chem Biol,* 2013, vol. 8 (6), 1324-1334
- **SORAGNI E.** *Front. Neurol.,* 2015, vol. 6, 44
- **ITKONEN HM.** Inhibition of O-GlcNAc transferase activity reprograms prostate cancer cell metabolism. *Oncotarget,* 2016, vol. 7 (11), 12464-12476
- **TESTA ; BERNARD ; MAYER.** Hydrolysis in Drug and Prodrug Metabolism : Chemistry, Biochemistry, and Enzymology. Joachim M. Wiley-VHCA, 2003
- **STAHL, P. HEINRICH.** Pharmaceutical Salts: Properties, Selection, and Use. Wiley-VCHA, 2002
- **FREEMAN WM et al.** Quantitative RT-PCR: pitfalls and potential. *BioTechniques,* 1999, vol. 26, 112-125
- **DUDLEY AM et al.** Measuring absolute expression with microarrays with a calibrated reference sample and an extended signal intensity range. *PNAS USA,* 2002, vol. 99 (11), 7554-7559
- **WANG Z et al.** RNA-Seq: a revolutionary tool for transcriptomics. *Nature Rev. Genetics,* 2009, vol. 10, 57-63